# EUROPEAN PATENT SPECIFICATION

(11) **EP 4 396 191 B1**
(45) Date of publication and mention of the grant of the patent: **18.02.2026**
(21) Application number: 22813354.2
(22) Date of filing: 31.08.2022
(51) Int. Cl.: C07D 498/04, A61K 31/553, A61P 35/00

(54) **COMPOUNDS USEFUL IN MODULATION OF AHR SIGNALLING**
VERBINDUNGEN ZUR MODULATION DER AHR-SIGNALISIERUNG
COMPOSÉS UTILES DANS LA MODULATION DE LA SIGNALISATION AHR

(30) Priority: 02.09.2021 SG 10202109626X; 07.09.2021 SG 10202109806R; 21.06.2022 SG 10202250248T; 30.06.2022 SG 10202250348V; 18.08.2022 SG 10202250775C
(43) Date of publication of application: 10.07.2024
(73) Proprietor: JAGUAHR THERAPEUTICS PTE LTD, Singapore 068808 (SG)
(72) Inventor: STOCKS, Michael, Leicestershire LE14 2EY (GB); GRAHAM, Mark, Leicestershire LE12 8QQ (GB); HITCHIN, James, Nottingham Nottinghamshire NG1 1GF (GB); MCINALLY, Tom, Nottingham Nottinghamshire NG1 1GF (GB); CILIBRASI, Vincenzo, 90020 Palermo (IT); COMPAIN, Sandy, Nottingham Nottinghamshire NG15 5BG (GB); SBRAVATI, Davide, 43018 Parma (IT)
(74) Representative: Hambleton, Bernadette Angelina
(86) International application number: PCT/SG2022/050633
(87) International publication number: WO 2023/033740

(56) References cited:
- WO-A1-2020/050409
- WO-A2-2018/195397

## Description

The present invention relates to compounds of the general formula (**I**) as described and defined herein, methods for preparing said compounds, pharmaceutical compositions and combinations comprising said compounds and the use of said compounds and pharmaceutical compositions for the treatment or prevention of diseases, in particular cancer or conditions with dysregulated immune functions, or other conditions associated with aberrant AhR signalling, as a sole agent or in combination with other active Ingredients. Such compounds may also be of utility in the expansion of hematopoietic stem cells (HSCs) and the use of HSCs in autologous or allogenic transplantation for the treatment of patients with inherited Immunological and autoimmune diseases and diverse hematopoietic disorders.

### BACKGROUND

The aryl hydrocarbon receptor (AhR) is a ligand-activated factor that belongs to the family of the basic helix-loop-helix-Per/ARNT/Sim family. Following ligand binding in the cytoplasm, AhR dissociates from its complex with Hsp90 and the AhR-interacting protein, XAP2, allowing ligated AhR to translocate to the nucleus. There, AhR dimerizes with the AhR nuclear translocator (ARNT), that then binds to xenobiotic response elements (XREs) promoting the up- or down-regulation of a multitude of target genes in many different tissues. The AhR is best known for binding to environmental toxins and inducing various members of the cytochrome P450 family including CYP1A1, CYP1A2 and CYP1B1 required for their elimination. Activation of AhR by xenobiotics has demonstrated that this receptor plays a role in a range of physiological processes including embryogenesis, tumourigenesis and inflammation (Esser & Rannug, Pharmacol Rev, 2015, 67:259; Roman et al., Pharmacol Ther, 2018, 185:50).

AhR is expressed in many immune cell types including dendritic cells, macrophages, T cells, NK cells and B cells and plays an important role in immunoregulation (Quintana & Sherr, Pharmacol Rev, 2013, 65:1148; Nguyen et al, Front Immunol, 2014, 5:551). The toxic/adverse effects of classical exogenous AhR agonists, such as 2,3,7,8-Tetrachlorodibenzo-p-dioxin (TCDD) are well known and include profound immunosuppression and initiation of malignancy (Esser et al, Trends Immunol, 2009, 30:447; Feng et al., Biochimica et Biophysica Acta, 2013, 1836:197). Physiological effects of AhR agonists on immune cells include promotion of regulatory T cell (Treg) generation (Pot, Swiss Med Wkly, 2012, 142:w13592), modulation of Th17 cell differentiation and activation (Baricza et al, Cell Mol Life Sci, 2016, 73:95) and stimulation of interleukin-22 (IL-22) expression and/or release from human activated peripheral blood mononuclear cells and T cells (Ramirez et al., Eur J Immunol, 2010, 40:2450; Effner et al., Sci Rep, 2017, 7:44005). AhR also modulates the function of antigen presenting cells, such as dendritic cells and macrophages. AhR activation decreases the expression of class II major histocompatibility complex and co-stimulatory molecules and also the production of Th1 and Th17 polarizing cytokines by dendritic cells (Mezrich et al., J Immunol, 2010, 185:3190; Nguyen et al., Proc Natl Acad Sci USA, 2010, 107:19961; Quintana et al., 2010 Proc Natl Acad Sci USA, 107:20768). Indeed, AhR activation boosts the ability of DCs to promote the differentiation of Tregs (Jurado-Manzano et al., 2017, Immunol Lett, 190:84).

In addition to xenobiotics, the AhR can also bind metabolic products of tryptophan degradation including kynurenine (KYN) and kynurenic acid (KYNA). Indoleamine 2,3 dioxygenase 1 and 2 (IDO1/IDO2) and tryptophan 2,3-dioxygenase 2 (TDO2) catalyse the commitment step of the KYN metabolic pathway and are expressed in immune cells (IDO1) and a range of cancer cells (IDO1 and TDO2)(Pilotte et al., Proc Nat Acad Sci, 2012, 109:2497). Inhibitors of IDO1 have attracted much interest as potential new treatments to stimulate the immune system to recognize and eliminate cancer cells (Cheong & Sun, Trends Pharmacol Sci, 2018, 39:307). Traditionally the immunosuppressive effect of IDO1 has been attributed mainly to reduced levels of tryptophan, which activates the kinase GCN2 (general control non-derepressible 2) and inhibits T cell proliferation/activation both in tumour draining lymph nodes lymph nodes and in the tumour micro-environment. More recently it has become apparent that some of the efficacy of IDO inhibitors may be the result of decreased production of AhR agonists. These endogenously generated AhR agonists have been shown to elicit a range of effects on immune cells including upregulation of IDO1 in dendritic cells (Julliard et al., Front Immunol, 2014, 5:458), inhibition of human T cell proliferation (Frumento et al., J Exp Med, 2002; 196:459; Terness et al, J Exp Med, 2002; 196: 447; Opitz et al., Nature, 2011, 478:197) and up-regulation of PD-1 expression in cytotoxic T lymphocytes (Liu et al., Cancer Cell, 2018; 33:480). As highlighted above, IDO1 is not the only source of endogenous AhR agonists. TDO2 is predominately expressed in the liver but it is also constitutively expressed in some cancers, notably malignant glioma, hepatocellular carcinoma, melanoma, bladder, breast, lung and colorectal cancer (Opitz et al., Nature, 2011, 478:197; Pilotte et al., Proc Nat Acad Sci, 2012, 109:2497; D'Amato et al., Cancer Res, 2015, 75(21):4651; Hsu et al., Oncotarget, 201 6, 7(19): 27584; Chen et al., Dis Markers, 2016, 2016:8169724). Such data suggests that AhR antagonists may have broader efficacy than selective IDO-1 inhibitors, as they will attenuate endogenous AhR agonist signalling regardless of its source. This assertion was given more weight by the recent discovery of another enzyme, Interleukin-4 induced 1 (IL411), capable of generating endogenous AhR agonists (Sadik et al., Cell, 2020, 182:10).

In addition to their effects on immune cells, such endogenous agonists have also been implicated in cancer progression via direct effects on the tumour. For example, KYN increases human glioblastoma cell survival and migration (Opitz et al, Nature, 2011, 478:197). Several other studies also implicate the AhR in cancer progression in the absence of environmental ligands. The AhR-repressor (AHRR) protein acts as a tumour suppressor gene in several human cancers (Zudaire et al., J Clin Invest, 2008, 118:640). AhR expression and "constitutive" (endogenous ligand-driven) activity in breast cancer cells correlate with tumour aggressiveness (Schlezinger et al., Biol Chem, 2006, 387:1175; Yang et al., J Cell Biochem, 2008, 104:402) and control expression of genes associated with tumour invasion (Yang et al., Oncogene, 2005, 24:7869). Ectopic AhR expression in non-malignant human mammary epithelial cells induces an epithelial-to-mesenchymal transition and a > 50% increase in cell growth rates (Brooks & Eltom, Curr Cancer Drug Targets, 2011, 11:654) and AhR knockdown induced gene changes in human breast cancer cell lines consistent with a mesenchymal to epithelial cell reversion to a less aggressive phenotype (Narasimhan et al., Int J Mol Sci, 2018, 19:1388). AhR antagonists or AhR knockdown has been shown to reduce proliferation, survival, invasiveness and migration of human breast cancer cells in culture (Parks et al., Mol Pharmacol, 2014, 86:593; D'Amato et al., Cancer Res, 2015, 75(21):4651; Narasimhan et al., Int J Mol Sci, 2018, 19:1388) and to reduce survival of glioblastoma cells (Gramatzki et al., Oncogene, 2009, 28:2593; Opitz et al., Nature, 2011, 478:197; Guastella et al., J Neuro-oncol, 2018, in press). Finally, AhR antagonists block the formation of tumourspheres (Stanford et al., Mol Cancer Res, 2016, 14:696) which are formed by cancer stem cells (CSCs), a subset of tumour cells that drive the initiation, progression and metastasis of tumours.

Thus, AhR agonists released from immune cells and from tumour cells act in an autocrine and paracrine fashion to promote tumour growth. Agents that reduce or block these effects may therefore find utility in the treatment of cancer and/or conditions with dysregulated immune functions. Thus such agents may also have utility in a range of other diseases/conditions Including but not limited to, obesity (Rojas et al., Int J Obesity, 2020, 44:948) and various viral Infections (Giovannoni et al., Nat Neurosci. 2020, 23:939; Giovannoni et al., Res Sq. 2020, rs.3.rs-25639).

WO2017/202816 relates to compounds and compositions for the treatment or prophylaxis of cancer or conditions with dysregulated immune responses or other disorders associated with aberrant AhR signalling. In particular, WO2017/202816 WO2018/146010 and WO2019/101642 relate *inter alia* to heterocyclic compounds capable of inhibiting AhR function. WO2020/081840 relates to aryl hydrocarbon receptor antagonists, such as substituted imidazopyridines and imidazopyrazines, as well as methods of expanding hematopoietic stem cells by culturing hematopoietic stem or progenitor cells in the presence of these agents. WO2020/039093 relates to compositions and methods for using tetrahydropyridopyrimidine derivatives as AhR modulators.

WO2018/153893 relates to 6-amido-1H-indol-2-yl compounds which can act as aryl hydrocarbon receptor (AhR) modulators and, in particular, as AhR antagonists. The invention further relates to the use of the compounds for the treatment and/or prophylaxis of diseases and/or conditions through binding of said aryl hydrocarbon receptor by said compounds. WO2020/021024 relates to bicyclic compounds which can act as aryl hydrocarbon receptor (AhR) modulators and, in particular, as AhR antagonists. The invention further relates to the use of the compounds for the treatment and/or prophylaxis of diseases and/or conditions through binding of said aryl hydrocarbon receptor by said compounds. WO2020/043880 relates to heterocyclic compounds which are ARH inhibitors, for prevention of diseases, in particular cancer or conditions with dysregulated immune functions, or other conditions associated with aberrant AHR signalling, as a sole agent of in combination with other active ingredients. WO 2020/018848 relates to methods for expanding stem cells and/or lineage committed progenitor cells, such as hematopoietic stems cells and/or lineage committed progenitor cells, at least in part, by using compounds that antagonize AhR. WO2020/050409 relates to novel heterocyclic compound having an aryl hydrocarbon receptor antagonist activity and useful for the promotion of platelet production. WO 2019/236766 relates to methods for expanding stem cells and/or lineage committed progenitor cells, at least in part, by using lactam compounds that antagonize AhR. WO2019/018562 relates to compositions and methods of using heteroaryl amides as AhR modulator compounds, for the treatment of diseases modulated, as least in part, by AhR. WO 2018/195397 relates to compositions and methods for indole AhR Inhibitors. WO 2018/146010 relates to the preparation of 2-heteroaryl-3-oxo-2,3-dihydropyridazine-4-carboxamides for the treatment or prophylaxis of diseases, in particular cancer or conditions with dysregulated immune responses, as a sole agent or in combination with other active ingredients. WO2010/059401 relates to compounds and compositions for expanding the number of CD34+ cells for transplantation. In particular, WO 2010/059401 relates *Inter alia* to heterocyclic compounds capable of down regulating the activity and/or expression of AhR.

WO2012/015914 relates to compositions and methods for modulating AhR activity. In particular, WO2012/015914 relates *Inter alia* to heterocyclic compounds that modulate AhR activity for use in therapeutic compositions to inhibit cancer cell proliferation and tumour cell invasion and metastasis. WO2020/051207 relates to AhR antagonists as well as methods of modulating AhR activity and expanding hematopoietic stem cells by culturing hematopoietic stem or progenitor cells in the presence of these agents. Additionally, this disclosure provides methods of treating various pathologies, such as cancer, by administration of these AhR antagonists US2018/327411 A1 relates to compounds and compositions useful as inhibitors of AhR to treat a variety of diseases, disorders and conditions associated with AhR. US2019/389857 A1 relates to compounds which can act as AhR modulators, and in particular, as AhR antagonists. WO2020/039093 discloses certain AhR modulators.

The presently disclosed compounds have one or more beneficial properties that render them particularly suitable for use as pharmaceuticals, for example high potency (for example a U937 and/or a IL-22 assay), adequate bioavailability, low cardiotoxicity (for example in a hERG assay), adequate cell permeability (for example in a Caco-2 assay), good solubility (for example kinetic solubility), a chromLogD value less than 5, and/or improved metabolic stability (such as Improved CYP3A4 metabolism). A potency assay is, for example described below. The present inventors have generated lots of different templates and structure activity relationship data and it is not easy to design compounds with the level of activity and properties of those described herein.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### SUMMARY OF THE PRESENT DISCLOSURE

1. A compound of formula (**I**) 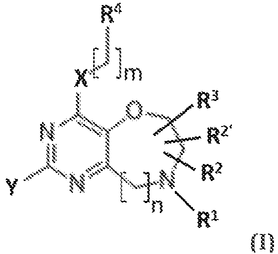 wherein:
   - Y: is phenyl or a 3 to 6 membered ring (such as a 5 or 6 membered ring) optionally comprising 1, 2, or 3 heteroatoms selected from N, O and S, said phenyl or ring substituted with R⁵ and R⁶;
   - R¹: is H, C₁₋₃ alkyl, (-CH₂)pCN, -COC₁₋₃ alkyl, -CO(CH₂)qNR⁷R⁸, -SO₂C₁₋₃ alkyl, -SO₂NR⁷R⁸, -(CH₂)qPh, C₃₋₅ cycloalkyl, C₁₋₃ alkyl bearing 1 to 6 halogen groups, C₁₋₃ alkyl bearing one or more independently selected from OR^{Y} and -NR⁷R⁸ (such as one OR^{Y} and optionally 1 -NR⁷R⁸), -(CH₂)p' OC₁₋₃alkyl substituted with 1 to 6 halogen groups (such as -(CH₂)p' OCF₃), -C₀₋₃alkyleneC(O)C₀₋₃ alkyl wherein the alkyl bears at least one group selected from OR^{Y} and -NR⁷R⁸⁽such as one OR^{Y} and optionally 1 -NR⁷R⁸), Oxetane, Z, -C(O)(CH₂)qZ, -C(O)O(CH₂)qZ -C(O)O(CH₂)pPh, -C(O)(CH₂)qZ', -C(O)O(CH₂)qZ';
   - R²: is H or C₁₋₃ alkyl, C₃₋₅ cycloalkyl halogen and C₁₋₃ alkyl optionally bearing one or more groups independently selected from OR^{Y}, halogen -NR⁷R⁸, such as OR^{Y} and halogen (e.g. F) in particular only one group;
   - R^{2'}: is H, C₁₋₃ alkyl or halogen;
   - R³: is H or C₁₋₃ alkyl;
   - R⁴: is a 9 to 13 membered heterocycle with at least one heteroatom selected from N, O and S (for example an aromatic or partially saturated), with substituents R⁹, R^{9'} and R¹⁰;
   - R⁵: is H, oxo, hydroxy, halogen (such as F, Cl), CN, C₁₋₃ alkyl, C₃₋₅ cycloalkyl, -OC₁₋₃ alkyl (such as -OCH₃), -(O)₀₋₁C₁₋₃ alkyl bearing 1 to 6 halogen groups (such as CF₃ or OCHF₂), C₁₋₃ alkyl bearing one or more OR^{Y} groups, -C(O)C₁₋₃alkylNR⁷R⁸, -SO₂C₁₋₃alkyl, -SO₂ NR⁷R⁸, or NR⁷R⁸ (such as NH₂)
   - R⁶: is H, oxo, hydroxy, halogen (such as F, Cl), CN, C₁₋₃ alkyl, -C(O)C₁₋₃alkylNR⁷R⁸, -SO₂C₁₋₃ alkyl, or -SO₂ NR⁷R⁸,
   - R⁷: is H or C₁₋₃ alkyl, or -C(O)OR^{Y}, for example H or C₁₋₃ alkyl, such as -CH₃;
   - R⁸: is H or C₁₋₃ alkyl, such as -CH₃;
   - R⁹: is H, hydroxy, halogen (such as F, Cl), CN, C₁₋₃ alkyl, C₁₋₃ alkoxy (such as OMe), C₁₋₃ alkyl bearing 1 to 6 halogen groups (such as CF₃), C₁₋₃ alkyl bearing one or more OR^{Y} groups, -C₃₋₅cycloalkyl, -(CH₂)qOC₁₋₃alkyl substituted with 1 to 6 halogen groups (such as - -C₁₋₃ alkyl OCF₃), -CO C₁₋₃ alkylNR⁷R⁸, -SO₂C₁₋₃alkyl, or -SO₂ NR⁷R⁸;
   - R^{9'}: is H, OH, halogen (such as F, Cl), CN, C₁₋₃ alkyl, C₁₋₃ alkoxy (such as OMe), C₁₋₃ alkyl bearing 1 to 6 halo groups (such as CF₃), C₁₋₃ alkyl bearing one or more OH groups,-CO(CH₂)q NR⁷R⁸, -SO₂C₁₋₃ alkyl, or -SO₂ NR⁷R⁸;
   - R¹⁰: is H, hydroxy, halogen (such as F, Cl), CN, C₁₋₃ alkyl, -C(O)C₁₋₃ alkyl NR⁷R⁸; -SO₂C₁₋₃ alkyl, or -SO₂ NR⁷R⁸;
   - R¹¹: is H, C₁₋₃alkyl (such as -CH₃) or C₂₋₆hydroxyalkyl (such as -CH₂CH₂OH);
   - R^{Y}: is H or C₁₋₄ alkyl for example H or C₁₋₃alkyl (such as H, -CH₃ or -CH₂CH₃);
   - X: is CH₂, S, -SO₂, NR¹¹ or O;
   - Z': is a 5 or 6 membered heteroaryl with at least one heteroatom selected from N, O and S, for example 1 or 2 nitrogens, wherein said heteroaryl optionally bears one, two or three substituents selected from hydroxy, halogen (such as F, Cl), CN, C₁₋₃ alkyl;
   - Z: is a 3 to 6 membered ring optionally comprising 1, 2, or 3 heteroatoms selected from N, O and S (for example 1 or 2 nitrogens) bearing R⁵ and R⁶ (for example is a 5 or 6 membered cycloalkyl or heterocycle comprising at least heteroatom selected from N, O and S optionally bears one, two or three substituents selected from hydroxy, halogen (such as F, Cl), CN, C₁₋₃ alkyl (such as bearing no substituents)),
   - n: is 0 or 1 (such as 0);
   - n': is 0 or 1;
   - m: is 0, 1, 2 or 3
   - p: is an integer 1, 2 or 3 (such as 1);
   - p': is an integer 2 or 3 (such as 2);
   - q: is 0, 1, 2 or 3 (such as 0 or 1),
   or a pharmaceutically acceptable salt thereof.
2. A compound of formula (**I**) according to paragraph 1, wherein Y is a 3 to 6 membered ring optionally comprising 1, 2, or 3 heteroatoms selected from N, O and S, each substituted with R⁵ and R⁶, for example a 5 to 6 membered ring comprising 1, 2, or 3 heteroatoms selected from N, O and S, each substituted with R⁵ and R⁶.
3. A compound of formula (**I**) according to paragraph 1 or 2, wherein Y is a 5 or 6 membered nitrogen containing ring.
4. A compound of formula (**I**) according to paragraph 3, wherein the ring is aromatic.
5. A compound of formula (**I**) according to paragraph 3 or 4, wherein the ring is independently selected from pyrimidine, pyridine, triazole and thiazole, in particular pyrimidine and or pyridine.
6. A compound of formula (**I**) according to paragraph 5, wherein R⁵ is located at position 5 on the Y group.
7. A compound according to any one of paragraphs 1 to 6, wherein n is 0.
8. A compound according to paragraph 1 of formula (**II**): 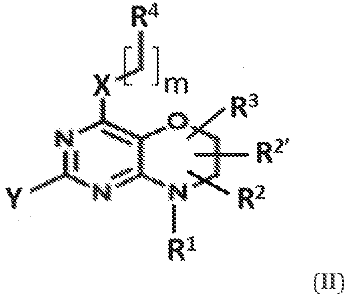 wherein X, R¹, R², R³, R⁴, and m are defined above for compounds of formula (**I**) or a pharmaceutically acceptable salt thereof.
9. A compound according to any one of paragraphs 1 to 8, wherein R¹ is independently selected from hydrogen, -C₁₋₃ alkyl, oxetane, -CH₂CH₂OH, -CH₂CH₂N(CH₃)₂, -SO₂CH₃ for example hydrogen or -C₁₋₃ alkyl, in particular H.
10. A compound according to paragraph 9, wherein in R¹ is selected from methyl, ethyl, propyl and isopropyl, in particular isopropyl.
11. A compound according to any one of paragraphs 1 to 10, wherein R² is independently selected from H, methyl, -CH₂OCH₃, -CH₂OH, CF₃, -CH₂N(CH₃)₂, such as H or methyl.
12. A compound according to any one of paragraphs 1 to 11, wherein R^{2'} is methyl or H, such as H.
13. A compound according to any one of paragraphs 1 to 12, wherein R³ is H.
14. A compound according to any one of paragraphs 1 to 13, wherein R⁴ is a 9 or 13 membered partially saturated or aromatic heterocycle (for example a 9 to 13 membered heteroaryl) for example selected from indole, tetrahydrobenzoindole, tetrahydrocarbazole and tetrahydropyridoindole.
15. A compound according to paragraphs 14, wherein R⁴ is a 9 membered heterocycle, for example a heteroaromatic, such as tryptophan.
16. A compound according to paragraph 15, wherein R⁴ is a 13 membered heterocycle, for example a partially saturated heterocycle, such as tetrahydrocarbazole.
17. A compound according to any one of paragraphs 1 to 16, wherein R⁵ is independently selected from the group comprising oxo, methyl, ethyl, -CF₃, -OCF₃, -OCH₃ and halogen, such as halogen, for example fluoro.
18. A compound according to any one of paragraphs 1 to 17, wherein R⁶ is independently selected from methyl, ethyl, -OCH₃, and H, such as H.
19. A compound according to any one of paragraphs 1 to 18, wherein R⁷ is H.
20. A compound according to any one of paragraphs 1 to 19, wherein R⁸ is H.
21. A compound according to any one of the paragraphs 1 to 20, wherein R⁹ is independently selected from R⁹ is H, methyl, OCF₃ and - C₃₋₅cycloalkyl, such as H, methyl, or - C₃₋₅cycloalkyl, alternatively H, methyl or OCF₃.
22. A compound according to any one of paragraphs 1 to 21, wherein R^{9'} is independently selected from H, F and methyl, such as H or F.
23. A compound according to any one of paragraphs 1 to 22, wherein R¹⁰ is Independently selected from H, F and methyl such as H or F, in particular H.
24. A compound according to paragraph one of paragraphs 1 to 23, wherein X is NR¹¹.
25. A compound according to any one of paragraphs 1 to 24, wherein R¹¹ is H.
26. A compound according to any one of paragraphs 1 to 25, wherein m is 2.
27. A compound according to any one of claims 1 to 25, wherein m is 1.
28. A compound according to any one of claims 1 to 27, wherein n is 1.
29. A compound according to any one of paragraphs 1 to 28, independently selected from:
   2-(5-fluoro-3-pyridyl)-N-[2-(1H-indol-3-yl)ethyl]-7,8-dihydro-6H-pyrimido[5,4-b][1,4]oxazin-4-amine; 2-(4-fluorophenyl)-N-[2-(1H-indol-3-yl)ethyl]-7,8-dihydro-6H-pyrimido[5,4-b][1,4] oxazin-4-amine; 2-(3,5-difluorophenyl)-N-[2-(1H-indol-3-yl)ethyl]-7,8-dihydro-6H-pyrimido[5,4-b][1,4]oxazin-4-amine; 2-(5-fluoro-3-pyridyl)-N-[(3R)-2,3,4,9-tetrahydro-1H-carbazol-3-yl]-7,8-di hydro-6H-pyrimido[5,4-b][1,4]oxazin-4-amine; 2-(5-fluoro-3-pyridyl)-N-[2-(1H-indol-3-yl) ethyl]-7-methyl-7,8-dihydro-6H-pyrimido[5,4-b][1,4]oxazin-4-amine; 2-(5-fluoro-3-pyridyl)-N-[2-(1H-indol-3-yl)ethyl]-8-isopropyl-6,7-dihydropyrimido[5,4-b][1,4]oxazin-4-amine; N-[2-(1H-indol-3-yl) ethyl]-2-(2-methylthiazol-5-yl)-7,8-dihydro-6H-pyrimido[5,4-b][1,4]oxazin-4-amine; (7S)-2-(5-fluoro-3-pyridyl)-N-[2-(1H-indol-3-yl)ethyl]-7-methyl-7,8-dihydro-6H-pyrimido[5,4-b)[1,4]oxazin-4-amine; (7R)-2-(5-fluoro-3-pyridyl)-N-[2-(1H-indol-3-yl)ethyl]-7-methyl-7,8-di hydro-6H-pyrimido[5,4-b][1,4]oxazin-4-amine; N-[2-(1H-indol-3-yl)ethyl]-2-(4-methylthiazol-5-yl)-7,8-dihydro-6H-pyrimido[5,4-b][1,4]oxazin-4-amine, 2-[2-(1H-indol-3-yl)ethyl-[2-(2-methyl thiazol-5-yl)-7,8-dihydro-6H-pyrimido[5,4-b][1,4]oxazin-4-yl]amino]ethanol; 2-(2-ethyl-4-methyl -thiazol-5-yl)-N-[2-(1H-indol-3-yl)ethyl]-7,8-dihydro-6H-pyrimido[5,4-b][1,4]oxazin-4-amine; N-[(3R)-2,3,4,9-tetrahydro- 1H-carbazol-3-yl]-2-thiazol-2-yl-7,8-dihydro-6H-pyrimido[5,4-b] [1,4] oxazin-4-amine; 2-(2-methylthiazol-5-yl)-N-(1,3,4,5-tetrahydrobenzo[cd]indol-4-yl)-7,8-dihydro-6H-pyrimido[5,4-b][1,4]oxazin-4-amine; N-[2-(1H-indol-3-yl)ethyl]-2-(6-methyl-3-pyridyl)-7,8-dihydro-6H-pyrimido[5,4-b][1,4]oxazin-4-amine; 2-(5-fluoro-6-methyl-3-pyridyl)-N-[2-(1H-indol-3-yl)ethyl]-7,8-dihydro-6H-pyrimido[5,4-b][1,4]oxazin-4-amine; 3-[4-[2-(1H-indol-3-yl)ethyl amino]-7,8-dihydro-6H-pyrimido[5,4-b][1,4]oxazin-2-yl]-1H-pyridin-2-one; 3-(4-((2-(1H-indol-3-yl)ethyl)amino)-7,8-dihydro-6H-pyrimido [5,4-b][1,4]oxazin-2-yl)pyridin-2-ol; 5-[4-[2-(1H-indol-3-yl)ethylamino]-7,8-dihydro-6H-pyrimido[5,4-b][1,4]oxazin-2-yl]-1H-pyridin-2-one; 5-(4-((2-(1H-indol-3-yl)ethyl)amino)-7,8-dihydro-6H-pyrimido[5,4-b][1,4]oxazin-2-yl)pyridin-2-ol; 3-[4-[2-(1RH-indol-3-yl)ethylamino]-7,8-dihydro-6H-pyrimido[5,4-b][1,4]oxazin-2-yl]-1-methyl-pyridin-2-one; N-[2-(6-fluoro-1H-indol-3-yl)ethyl]-2-(6-methyl-3-pyridyl)-7,8-dihydro-6H-pyrimido[5,4-b][1,4]oxazin-4-amine; 3-[4-[2-(6-fluoro-1H-indol-3-yl)ethylamino]-7,8-dihydro-6H-pyrimido [5,4-b][1,4]oxazin-2-yl]-1H-pyridin-2-one; 3-(4-((2-(6-fluoro-1H-indol-3-yl)ethyl)amino)-7,8-di hydro-6H-pyrimido[5,4-b][1,4]oxazin-2-yl)pyridin-2-ol; 2-[2-(difluoromethoxy)-3-pyridyl]-N-[2-(1H-indol-3-yl)ethyl]-7,8-dihydro-6H-pyrimido[5,4-b][1,4]oxazin-4amine; N-[2-(1H-indol-3-yl) ethyll-2-(2. methoxy- 5-methyl-3-pyridyl)- 7,8-dihydro-6H-pyrimido[5,4-b][1,4]oxazin-4-amine; 4-[2-(6-fluoro-1H-indol-3-yl)ethoxy]-2-(2-methylthiazol-5-yl)-7,8-dihydro-6H-pyrimido [5,4-b][1,4] oxazine; 3-(4-(2-(1H-indol-3-yl)ethoxy)-7,8-dihydro-6H-pyrimido[5,4-b][1,4]oxazin-2-yl)pyridin-2(1H)-one; 3-(4-(2-(1H-indol-3-yl)ethoxy)-7,8-dihydro-6H-pyrimido[5,4-b][1,4]oxazin-2-yl) pyridin-2-ol; (R)-3-(4-((2-(1H-indol-3-yl)ethyl)amino)-7-(methoxymethyl)-7,8-dihydro-6H-pyrimido [5,4b][1,4]oxazin-2-yl)pyridin-2(1H)-one; (R)-3-(4-((2-(1H-indol-3-yl)ethyl)amino)-7-(methoxymethyl)-7,8-dihydro-6H-pyrimido[5,4-b][1,4]oxazin-2-yl)pyridin-2-ol; (R)-3-(4-((2-(1H-indol-3-yl)ethyl)amino)-7-(hydroxymethyl)-7,8-dihydro-6H-pyrimido[5,4-b][1,4]oxazin-2-yl) pyridin-2(1H)-one; (R)-3-(4-((2-(1H-indol-3-yl)ethyl)amino)-7-(hydroxymethyl)-7,8-dihydro-6H-pyrimido[5,4-b][1,4]oxazin-2-yl)pyridin-2-ol; (7S)-2-(5-fluoro-3-pyridyl)-N-[2-(1H-indol-3-yl) ethyl]-7-(methoxymethyl)-7,8-dihydro-6H-pyrimido[5,4-b][1,4]oxazin-4-amine; (6S)-2-(S-fluoro-3-pyridyl)-N-[2-(1H-indol-3-yl)ethyl]-6-methyl-7,8-dihydro-6H-pyrimldo[5,4-b][1.4]oxazin-4-amine; (6R)-2-(5-fluoro-3-pyridyl)-N-[2-(1H-indol-3-yl)ethyl]-6-methyl-7,8-dihydro-6H-pyrimido[5,4-b][1,4]oxazin-4-amine; 2-(5-fluoro-3-pyridyl)-N-[2-(1H-indol-3-yl)ethyl]-7,7-dimethyl-6,8-dihydropyrimido[5,4-b][1,4]oxazin-4-amine; (7R)-2-(5-fluoro-3-pyridyl)-N-[2-(1H-indol-3-yl)ethyl]-7-(trifluoro methyl)-7,8-dihydro-6H-pyrimido[5,4b][1,4]oxazin-4-amine; N-[2-(1H-indol-3-yl)ethyl]-2-(2-methylthiazol-5-yl)-8-(oxetan-3-yl)-6,7-dihydropyrimido[5,4-b][1,4]oxazin-4-amine; 2-(2-methyl thiazol-5-yl)-N-(6,7,8,9-tetrahydro-5H-pyrido[3,2-b]indol-8-yl)-7,8-dihydro-6H-pyrimido[S.4-b] [1,4]oxazin-4-amine; (7R)-8-[2-(dimethylamino)ethyl]-7-(methoxymethyl)-2-(2-methylthiazol-5-yl)-N-[(3R)-2,3,4,9-tetrahydro-1H-carbazol-3-yl]-6,7-dihydropyrimido[5,4-b][1,4]oxazin-4-amine; [(7R)-8-[2-(dimethylamino)ethyl]-2-(2-methylthiazol-5-yl)-4-[[(3R)-2,3,4,9-tetrahydro-1H-carbazol-3-yl]amino]-6,7-dihydropyrimido[5,4-b][1,4]oxazin-7-yl]methanol; 2-[2-(2-methyl thiazol-5-yl)-4-[[(3R)-2,3,4,9-tetrahydro-1H-carbazol-3-yl]amino]-6,7-dihydropyrimido[5,4-b][1,4]oxazin-8-yl]ethanol; 7-[(dimethylamino)methyl]-2-(2-methylthlazol-5-yl)-N-[(3R)-2,3,4,9-tetrahydro-1H-carbazol-3-yl]-7,8-dihydro-6H-pyrimido[5,4-b][1,4]oxazin-4-amine; 8-methyl sulfonyl-2-(2-methylthiazol-5-yl)-N-[(3R)-2,3,4,9-tetrahydro-1H-carbazol-3-yl]-6,7-dihydro pyrimido[5,4-b][1,4]oxazin-4-amine; 3-(4-((2-(2-chloro-1H-indol-3-yl)ethyl)amino)-7,8-dihydro-6H-pyrimido[5,4-b][1,4]oxazin-2-yl)pyridin-2(1H)-one; 3-(4-((2-(2-chloro-1H-indol-3-yl)ethyl) amino)-7,8-dihydro-6H-pyrimido[5,4-b][1,4]oxazin-2-yl)pyridin-2-ol; 3-[4-[2-(7-fluoro-2-methyl-1H-indol-3-yl)ethylamino]-7,8-dihydro-6H-pyrimido[5,4-b][1,4]oxazin-2-yl]-1H-pyridin-2-one; 3-(4-((2-(7-fluoro-2-methyl- 1H-indol-3-yl)ethyl)amino)-7,8-dihydro-6H-pyrimido[5,4-b][1.4] oxazin-2-yl)pyridin-2-ol; 3-[4-[2-(5,7-difluoro-2-methyl-1H-indol-3-yl)ethylamino]-7,8-dihydro-6H-pyrimido[5,4-b][1,4] oxazin-2-yl]-1H-pyridin-2-one; 3-(4-((2-(5,7-difluoro-2-methyl-1H-indol-3-yl)ethyl) amino)-7,8-dihydro-6H-pyrimido[5,4-b][1,4]oxazin-2-yl)pyridin-2-ol; 3-(4-((2-(6-(trifluoromethoxy)-1H-indol-3-yl)ethyl)amino)-7,8-dihydro-6H-pyrimido[5,4-b][1,4] oxazin-2-yl)pyridin-2(1H)-one; 3-(4-((2-(6-(trifluoromethoxy)-1H-indol-3-yl)ethyl) amino)-7,8-dihydro-6H-pyrimido[5,4-b][1,4]oxazin-2-yl)pyridin-2-ol; 3-(4-((2-(1H-indol-3-yl)ethyl)amino)-8-methyl-7,8-dihydro-6H-pyrimido[5,4-b][1,4]oxazin-2-yl)pyridin-2 (1H)-one; 3-(4-((2-(1H-indol-3-yl)ethyl)amino)-8-methyl-7,8-dihydro-6H-pyrimido[5,4-b][1,4]oxazin-2-yl)pyridin-2-ol; (S)-3-(4-((2-(1H-indol-3-yl)ethyl)amino)-7-(methoxy methyl)-7,8-dihydro-6H-pyrimido[5,4-b][1,4]oxazin-2-yl)pyridin-2(1H)-one; (S)-3-(4-((2-(1H-indol-3-yl)ethyl)amino)-7-(methoxymethyl)-7,8-di hydro-6H-pyrimido[5,4-b][1,4] oxazin-2-yl)pyridin-2-ol; (7R)-2-(5-fluoro-3-pyridyl)-N-[2-(1H-indol-3-yl)ethyl]-7-(methoxymethyl)-7,8-dihydro-6H-pyrimido[5,4-b][1,4]oxazin-4-amine; (R)-3-(4-((2-(6-fluoro-1H-indol-3-yl)ethyl)amino)-7-(methoxymethyl)-7,8-dihydro-6H-pyrimido[5,4-b] [1,4]oxazin-2-yl)pyridin-2(1H)-one; (R)-3-(4-((2-(6-fluoro-1H-indol-3-yl)ethyl)amino)-7-(methoxymethyl)-7,8-dihydro-6H-pyrimido[5,4-b][1,4]oxazin-2-yl)pyridin-2-ol; 3-[(7R)-4-[2-(1H-indol-3-yl)ethoxy]-7-(methoxymethyl)-7,8-dihydro-6H-pyrimido[5,4-b][1,4] oxazin-2-yl]-1H-pyridin-2-one; (R)-3-(4-(2-(1H-indol-3-yl)ethoxy)-7-(methoxymethyl)-7,8-dihydro-6H-pyrimido [5,4-b][1,4]oxazin-2-yl)pyridin-2-ol; (7R)-2-(3,5-difluorophenyl)-N-[2-(1H-indol-3-yl)ethyl]-7-(methoxymethyl)-7,8-dihydro-6H-pyrimido[5,4-b][1,4] oxazin-4-amine; 2-(3-fluorophenyl)-N-[2-(1H-indol-3-yl)ethyl]-7,8-dihydro-6H-pyrimido[5,4-b][1,4]oxazin-4-amine; 2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amine; 2-(2,4-dimethylthiazol-5-yl)-N-[2-(1H-indol-3-yl)ethyl]-7,8-dihydro-6H-pyrimido[5,4-b][1,4] oxazin-4-amine; (7R)-7-(methoxymethyl)-2-(2-methylthiazol-5-yl)-N-[(3R)-2,3,4,9-tetra hydro-1H-carbazol-3-yl]-7,8-dihydro-6H-pyrimido[5,4-b][1,4]oxazin-4amine; [(7R)-2-(2-methylthiazol-5-yl)-4-[[(3R)-2,3,4,9-tetrahydro-1H-carbazol-3-yl]amino]-7,8-dihydro-6H-pyrimido[5,4-b][1,4]oxazin-7-yl]methanol; N-[2-(1H-indol-3-yl)ethyl]-2-(1-methyltriazol-4-yl)-7,8-dihydro-6H-pyrimido[5,4-b][1,4]oxazin-4-amine; (7R)-2-(4-fluorophenyl)-N-[2-(1H-indol-3-yl)ethyl]-7-(methoxymethyl)-7,8-dihydro-6H-pyrimido[5,4-b][1,4]oxazin-4-amine; (7R)-2-(3-fluorophenyl)-N-[2-(1H-indol-3-yl)ethyl]-7-(methoxymethyl)-7,8-dihydro-6H-pyrimido[5,4-b][1,4]oxazin-4-amine; 5-[4-[2-(1H-indol-3-yl)ethylamino]-7,8-dihydro-6H-pyrimido[5,4-b][1.4] oxazin-2-yl]-1-methyl-pyridin-2-one; (7R)-N-[2-(1H-indol-3-yl)ethyl]-7-(methoxymethyl)-2-(2-methylthiazol-5-yl)-7,8-dihydro-6H-pyrimido[5,4-b][1,4]oxazin-4-amine; [(7R)-4-[2-(1H-indol-3-yl)ethylamino]-2-(2-methylthiazol-5-yl)-7,8-dihydro-6H-pyrimido[5,4-b][1,4]oxazin-7-yl] methanol; 2-(2-methylthiazol-5-yl)-N-[(4R)-1,3,4,5-tetrahydrobenzo[cd]indol-4-yl]-7,8-dihydro-6H-pyrimido[5,4-b][1,4]oxazin-4-amine; 2-(2-methylthiazol-5-yl)-N-[(4S)-1,3,4,5-tetra hydrobenzo[cd]indol-4-yl]-7,8-dihydro-6H-pyrimido[5,4-b][1,4]oxazin-4-amine; 3-[4-[[(3R)-2,3,4,9-tetrahydro-1H-carbazol-3-yl]amino]-7,8-dihydro-6H-pyrimido[5,4-b][1,4] oxazin-2-yl]-1H-pyridin-2-one; (R)-3-(4-((2.3,4,9-tetrahydro-1H-carbazol-3-yl)amino)-7,8-dihydro-6H-pyrimido[5,4-b][1,4]oxazin-2-yl)pyridin-2-ol; N-[2-(1H-indol-3-yl)ethyl]-2-(2-methoxy-3-pyridyl)-7,8-dihydro-6H-pyrimido[5,4-b][1,4]oxazin-4-amine; 2-(5-fluoro-2-methoxy-3-pyridyl)-N-[(3R)-2,3,4,9-tetrahydro-1H-carbazol-3-yl]-7,8-dihydro-6H-pyrimido[5,4-b][1,4]oxazin-4-amine; 3-(4-((2-(1H-indol-3-yl)ethyl)amino)-7,3-dihydro-6H-pyrimido[5,4-b][1,4]oxazin-2-yl)-5-fluoropyridin-2(1H)-one; 3-(4-((2-(1H-indol-3-yl)ethyl)amino)-7,8-dihydro-6H-pyrimido(5,4-b][1,4]oxazin-2-yl)-5-fluoropyridin-2-ol; 2-(5-fluoro-2-methoxy-3-pyridyl)-N-[2-(1H-indol-3-yl)ethyl]-7,8-dihydro-6H-pyrimido[5,4-b][1,4]oxazin-4-amine; 3-(4-((2-(1H-indol-3-yl)ethyl)amino)-7,8-dihydro-6H-pyrimido[5,4-b][1,4]oxazin-2-yl)-5-methylpyridin-2(1H)-one; 3-(4-((2-(1H-indol-3-yl)ethyl)amino)-7,8-dihydro-6H-pyrlmido[5,4-b][1,4]oxazin-2-yl)-5-methylpyridin-2-ol; 3-(4-((2-(1H-indol-3-yl)ethyl)amino)-7,8-dihydro-6H-pyrimido[5,4-b][1,4]oxazin-2-yl)-5-(trifluoromethyl)pyridin-2(1H)-one; 3-(4-((2-(1H-indol-3-yl)ethyl)amino)-7,8-dihydro-6H-pyrimido[5,4b][1,4]oxazin-2-yl)-5-(trifluoromethyl)pyridin-2-ol; 5-fluoro-3-(4-((2-(6-fluoro-1H-indol-3-yl)ethyl)amino)-7,8-dihydro-6H-pyrimido[5,4b][1,4]oxazin-2-yl) pyridin-2(1H)-one; 5-fluoro-3-(4-((2-(6-fluoro-1H-indol-3-yl)ethyl)amino)-7,8-dihydro-6H-pyrimido[5,4-b][1,4]oxazin-2-yl)pyridin-2-ol; (R)-5-fluoro-3-(4-((2,3,4,9-tetrahydro-1H-carbazol-3-yl)amino)-7,8-dihydro-6H-pyrimido[5,4-b][1,4]oxazin-2-yl)pyridin-2(1H)-one; (R)-5-fluoro-3-(4-((2,3,4,9-tetrahydro-1H-carbazol-3-yl)amino)-7,8-dihydro-6H-pyrimido[5,4-b][1,4]oxazin-2-yl)pyridin-2-ol; 3-(4-((2-(1H-indol-3-yl)ethyl)amino)-7,8-dihydro-6H-pyrimido[5,4-b][1,4]oxazin-2-yl)-5-chloropyridin-2(1H)-one; 3-(4((2-(1H-indol-3-yl)ethyl)amino)-7,8-dihydro-6H-pyrimido [5,4-b][1,4]oxazin-2-yl)-5-chloropyridin-2-ol; 3-[4-[2-(1H-indol-3-yl)ethylamino]-7,8-dihydro-6H-pyrimldo[5,4-b][1,4]oxazin-2-yl]-6-methyl-1H-pyridin-2-one; 3-(4-((2-(1H-indol-3-yl)ethyl) amino)-7,8-dihydro-6H-pyrimido[5,4-b][1,4]oxazin-2-yl)-6-methylpyridin-2-ol; and 2-(2-amino-3-pyridyl)-N-[2-(1H-indol-3-yl)ethyl]-7,8-dihydro-6H-pyrimido[5.4-b][1,4]oxazin-4-amine, or a pharmaceutically acceptable salt thereof.
28. A compound according to any one of paragraphs 1 to 27, wherein the compound is 3-(4-(2-(1H-indol-3-yl)ethoxy)-7,8-dihydro-6H-pyrimido[5,4-b][1,4]oxazin-2-yl)pyridin-2(1H)-one, or a pharmaceutically acceptable salt thereof.
29. A compound according to any one of paragraphs 1 to 26, where the compound is selected from a novel structure disclosed herein or a pharmaceutically acceptable salt thereof.
30. A compound according to any one of paragraphs 1 to 29, wherein the compound is selected from: 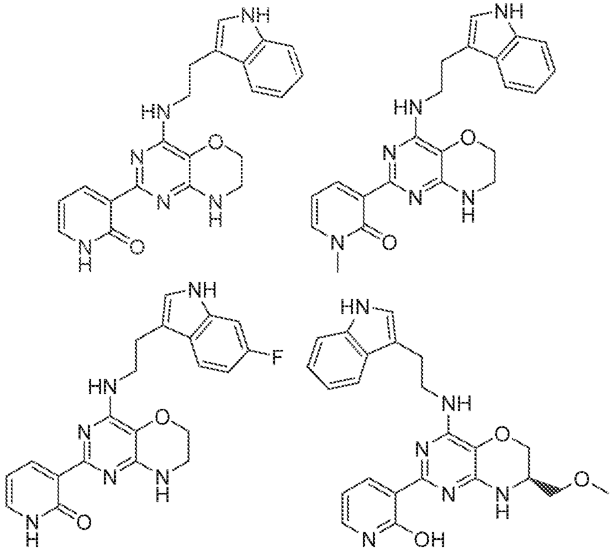 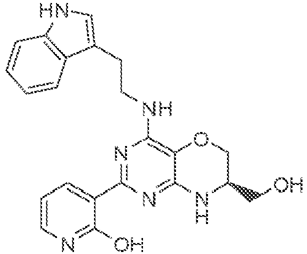 or a pharmaceutically acceptable salt thereof.
31. A compound according to any one of paragraph 1 to 29, where the compound is: 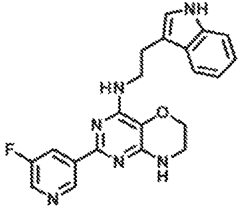 or a pharmaceutically acceptable salt thereof.
32. A compound according to any one of paragraphs 1 to 29, wherein the compound is: 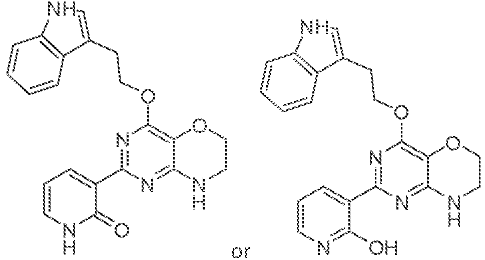 or a pharmaceutically acceptable salt thereof.
33. A compound according to any one of paragraphs 1 to 26, wherein m is 1.
34. A compound according to any one of paragraphs 1 to 26, wherein n is 1.
35. A compound according to paragraph 34, wherein the compound is: 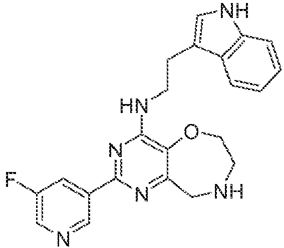 or a pharmaceutically acceptable salt thereof.
36. A pharmaceutical composition comprising a compound according to any one of paragraphs 1 to 35, and a pharmaceutically acceptable excipient diluent or carrier.
37. A compound according to any one of paragraphs 1 to 35 or a composition according to paragraph 36, for use in treatment, in particular the treatment of cancer.
38. Use of a compound according to any one of paragraphs 1 to 35 or a composition according to paragraph 36, in the manufacture of a medicament for the treatment of cancer.
39. A method of treatment comprising administering a therapeutically effective amount of a compound according to any one of paragraphs 1 to 35 or a composition according to paragraph 36 to a patient in need thereof, for example for the treatment of cancer.
40. A compound or composition for use according to paragraph 37, or a use according to paragraph 38, further comprising one or more checkpoint inhibitors, for example selected from the group comprising: PD-1 inhibitor, PD-L1 inhibitor, PD-L2 inhibitor, CTLA-4 inhibitor, checkpoint kinase inhibitor 1 (CHEK1/CHK1), checkpoint kinase inhibitor 2 (CHEK2/ CHK2), Ataxia telangiectasia and Rad3 related (ATR) inhibitor, ataxia-telangiectasia mutated (ATM) inhibitor, Wee1 dual specificity protein kinase (Wee1) inhibitor, Poly ADP Ribose polymerase (PARP) inhibitor and Myt1 inhibitor.
41. A method according to paragraph 39, further comprising administering one or more checkpoint inhibitors, for example selected from the group comprising: PD-1 inhibitor, PD-L1 inhibitor, PD-L2 inhibitor, CTLA-4 inhibitor, checkpoint kinase inhibitor 1 (CHEK1/CHK1), checkpoint kinase inhibitor 2 (CHEK2/ CHK2). Ataxia telangiectasia and Rad3 related (ATR) Inhibitor, ataxia-telanglectasia mutated (ATM) inhibitor, Wee1 dual specificity protein kinase (Wee1) inhibitor, Poly ADP Ribose polymerase (PARP) inhibitor and Myt1 inhibitor.
42. A combination therapy comprising a compound according to any one of paragraphs 1 to 35 or a composition according to paragraph 36, and one or more checkpoint inhibitors, for example selected from the group comprising: PD-1 inhibitor, PD-L1 inhibitor, PD-L2 inhibitor, CTLA-4 inhibitor, checkpoint kinase inhibitor 1 (CHEK1/CHK1), checkpoint kinase inhibitor 2 (CHEK2/ CHK2), Ataxia telangiectasia and Rad3 related (ATR) inhibitor, ataxia-telangiectasia mutated (ATM) inhibitor, Wee1 dual specificity protein kinase (Wee1) inhibitor, Poly ADP Ribose polymerase (PARP) inhibitor and Myt1 inhibitor.
43. A method of preparing a compound according to any one of paragraphs 1 to 35, comprising the following steps: 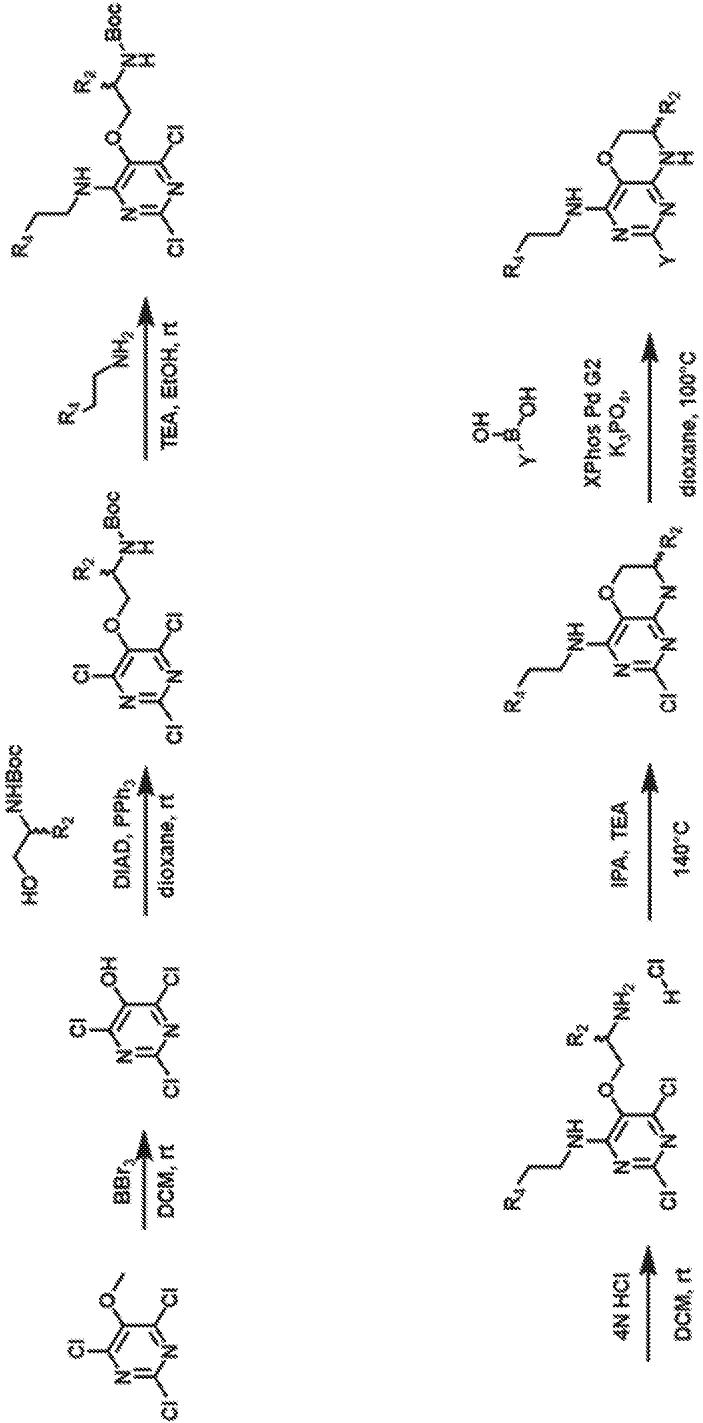 wherein R₂, R₄, and Y are as defined in paragraph 1, and B is boron.
44. A method of preparing a compound according to any one of paragraphs 1 to 35, comprising the following steps: 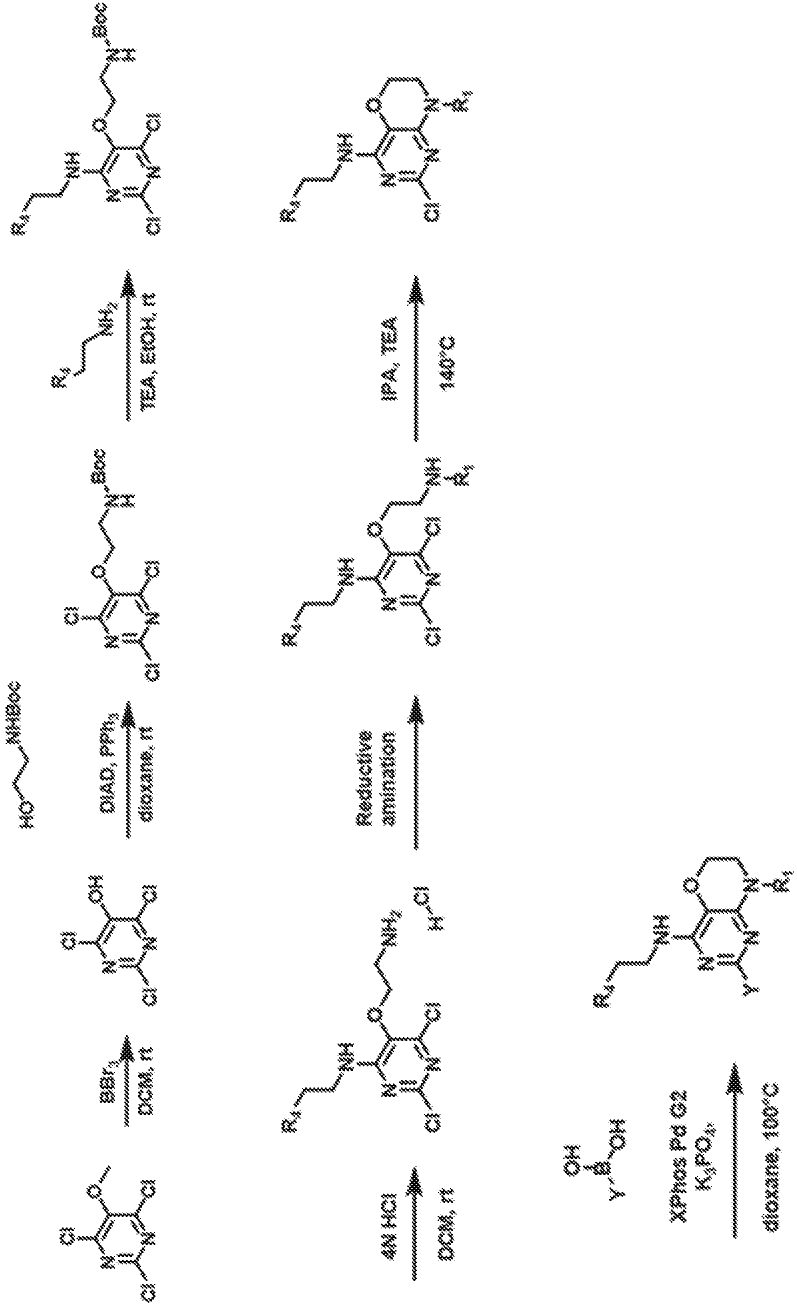 wherein R₄, R₁ and Y are as defined in paragraph 1, and B is boron.
45. A method of preparing a compound according to any one of paragraphs 1 to 35, comprising the following steps: 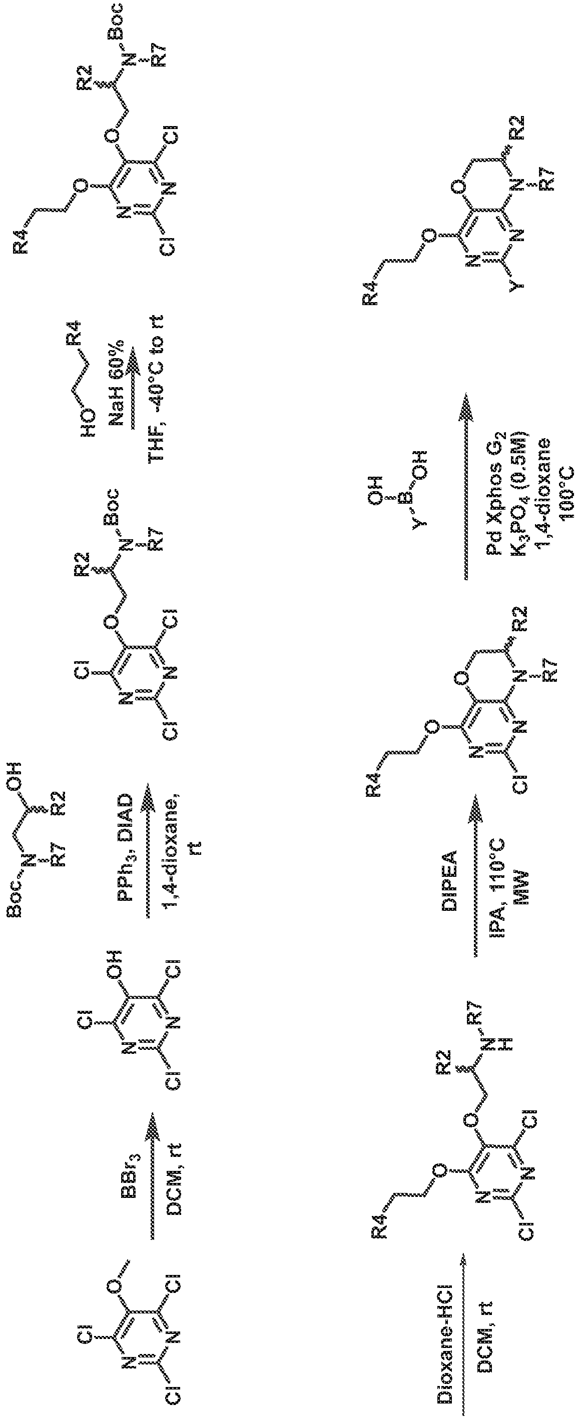 wherein R2, R4, R7 and Y are as defined in paragraph 1, and B is boron.
46. A method of preparing a compound according to any one of paragraphs 1 to 35, comprising the following steps: 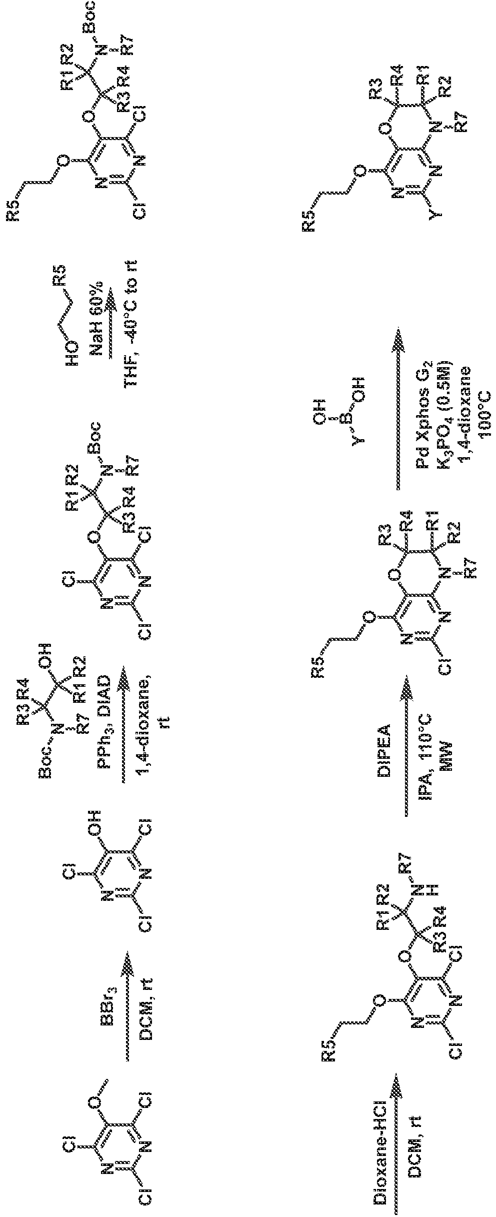 wherein R1, R2, R3, R4, R5, R7 and Y are as defined in paragraph 1, and B is boron.
47. A method of preparing a compound according to any one of paragraphs 1 to 35, comprising the following steps: 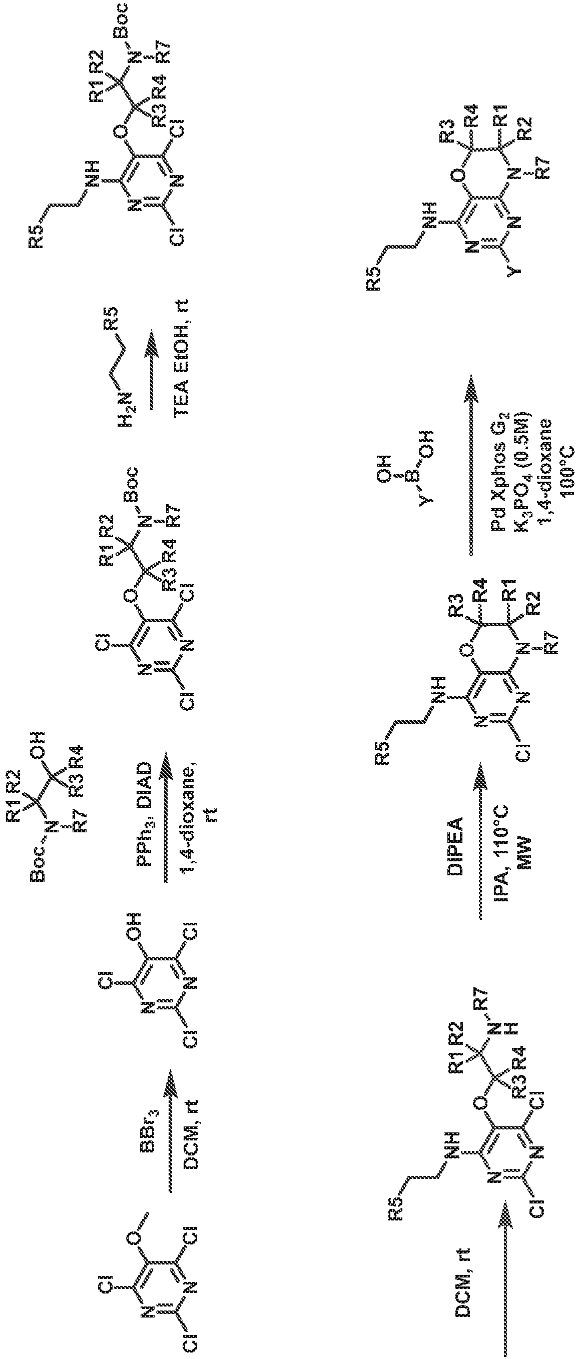 wherein R1, R2, R3, R4, R5, R7and Y are as defined in paragraph 1, and B is boron.
48. A method of preparing a compound according to any one of paragraphs 1 to 35, comprising the following steps: 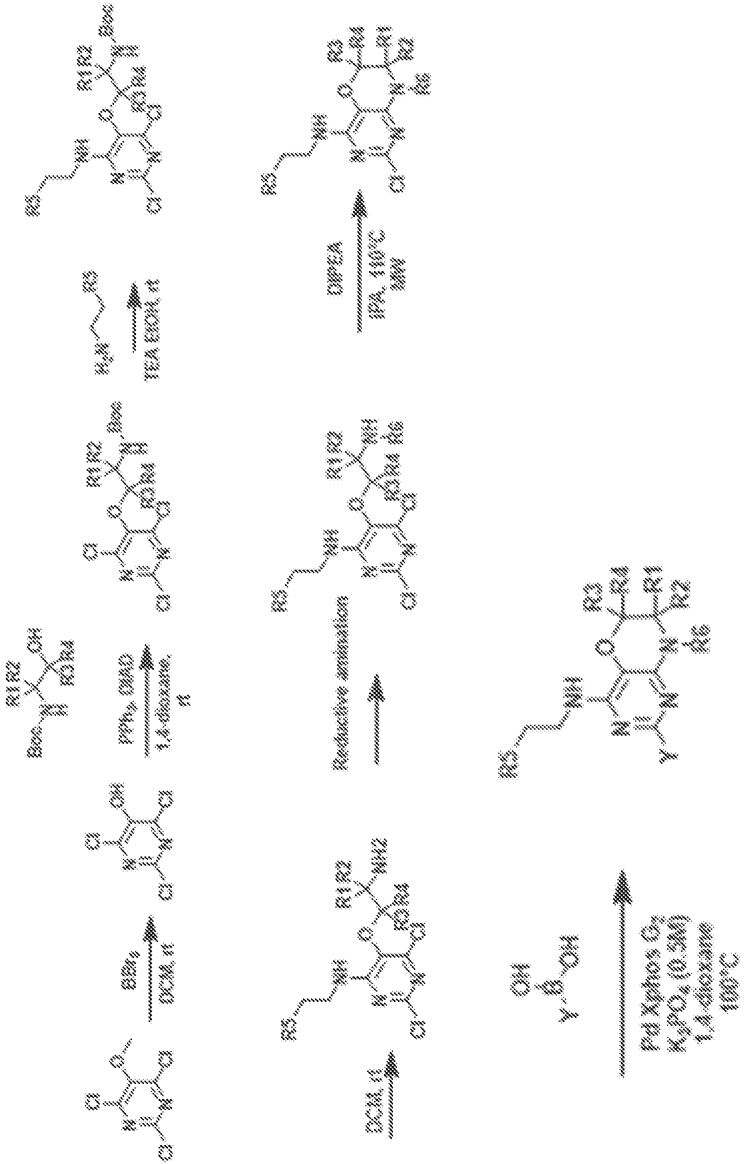 wherein R1, R2, R3, R4, R5, R6 and Y are as defined in paragraph 1, and B is boron.
49. A method of preparing a compound according to any one of paragraphs 1 to 35, comprising the following steps: 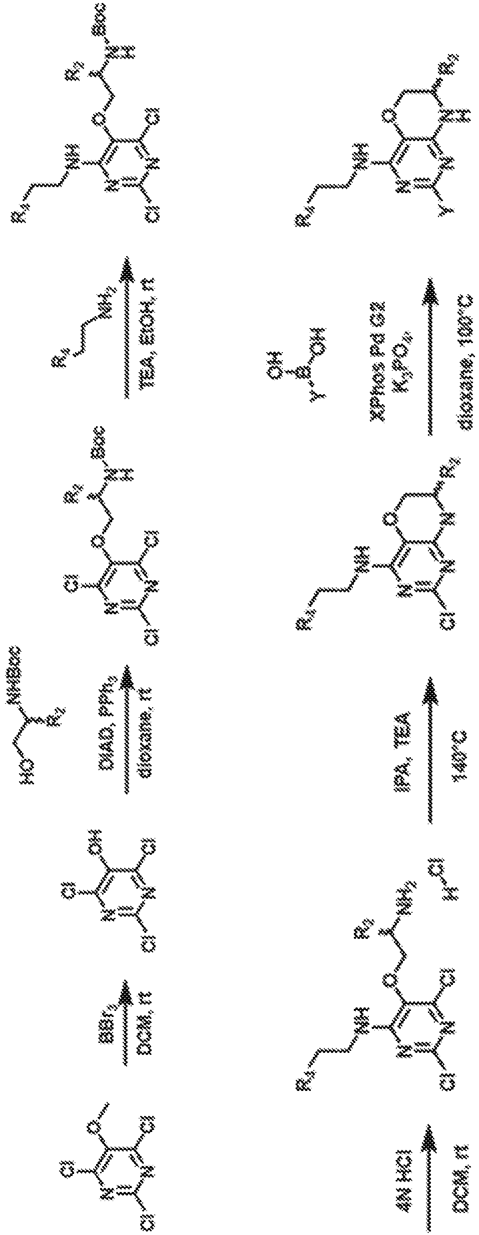 wherein R₂, R₄ and Y are as defined in paragraph 1, and B is boron.
50. A method of preparing a compound according to any one of paragraphs 1 to 35, comprising the following steps: 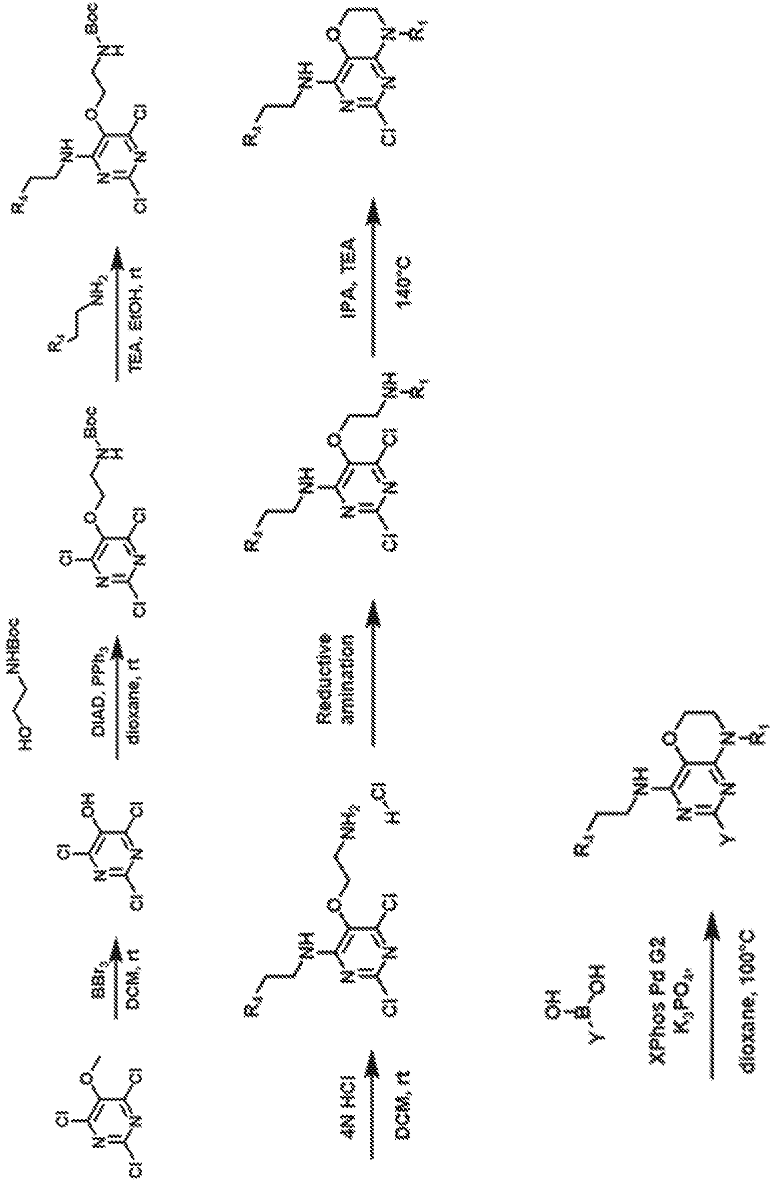 wherein R₁, R₄, and Y are as defined in paragraph 1, and B is boron.
51. A method of preparing a compound according to any one of paragraphs 1 to 35, comprising the following steps: 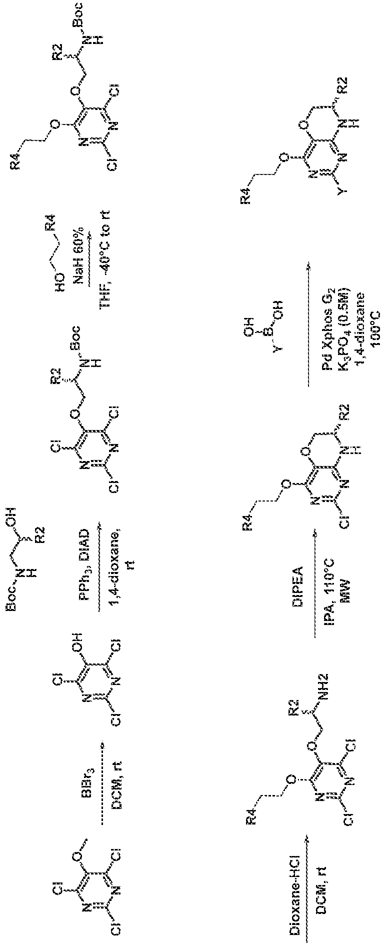 wherein R₂, R₄, and Y are as defined in paragraph 1, and B is boron.
52. A method of preparing a compound according to any one of paragraphs 1 to 35, comprising the following steps: 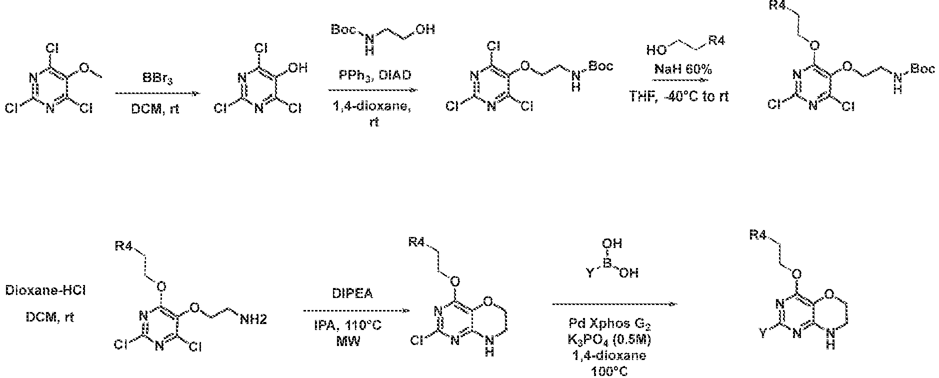 wherein R4 and Y are as defined in paragraph 1, and B is boron.
53. A method of preparing a compound according to any one of paragraphs 1 to 35, comprising the following steps: 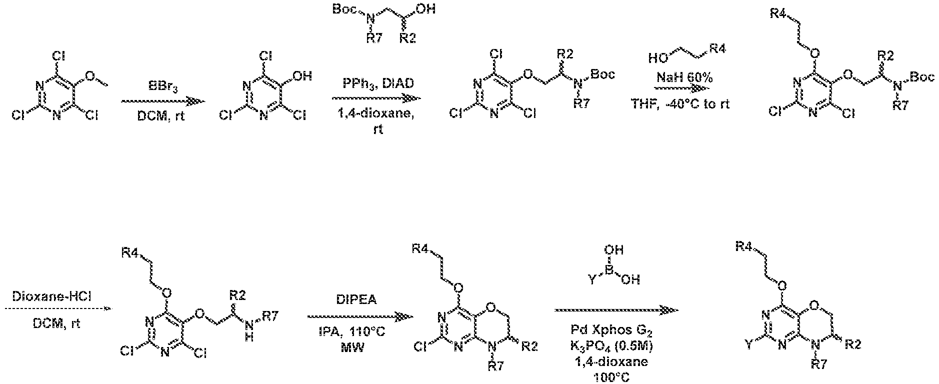 wherein R2, R4, R7 and Y are as defined in paragraph 1, and B is boron.
54. A method of preparing a compound according to any one of paragraphs 1 to 35, comprising the following steps: 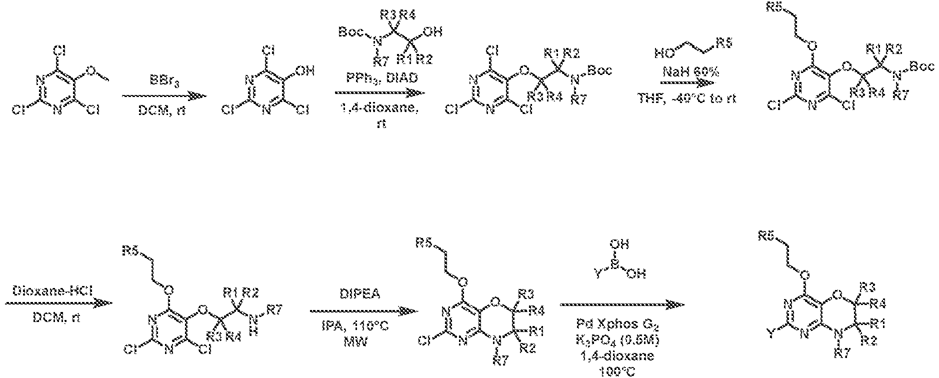 wherein R1, R2. R3, R4, R5, R7 and Y are as defined in paragraph 1, and B is boron.
55. A method of preparing a compound according to any one of paragraphs 1 to 35, comprising the following steps: 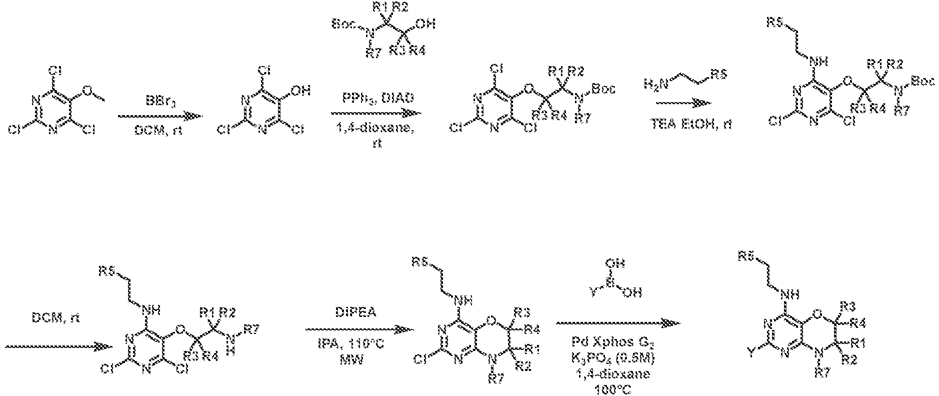 wherein R1, R2, R3, R4, R5, R7 and Y are as defined in paragraph 1, and B is boron.
56. A method of preparing a compound according to any one of paragraphs 1 to 35, comprising the following steps: 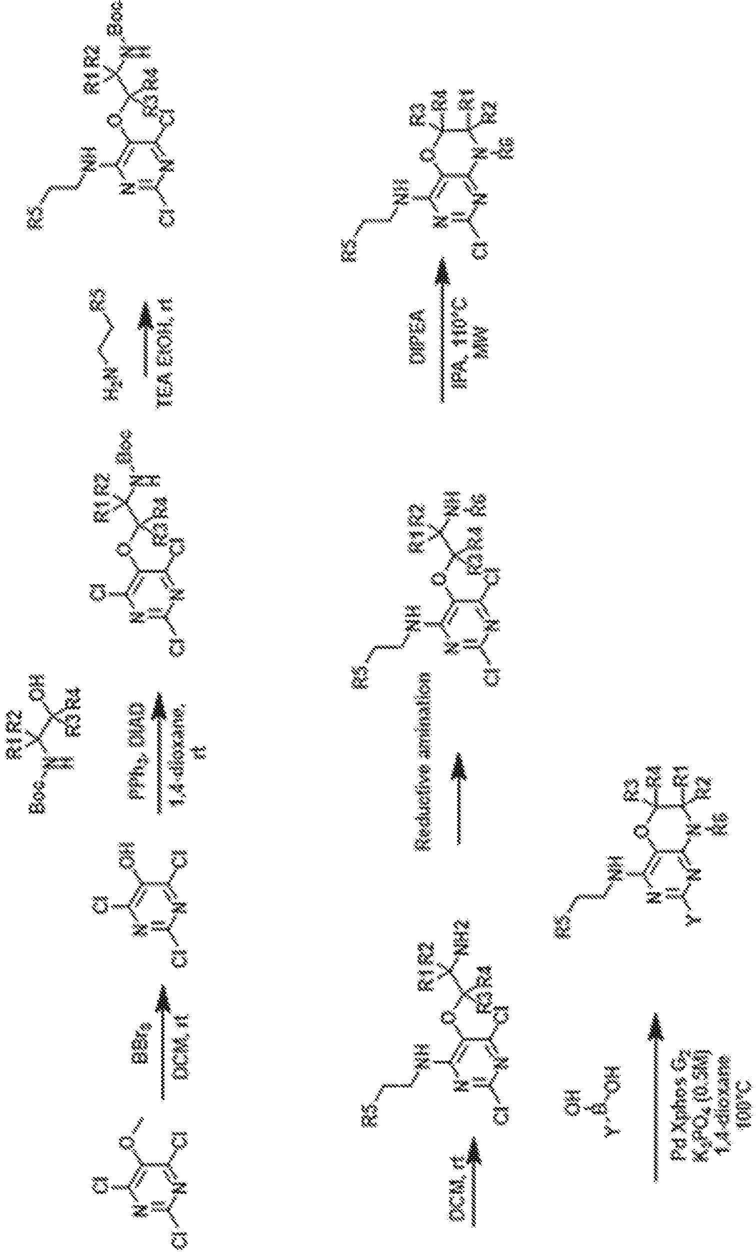 wherein R1, R2, R3, R4, R5, R6 and Y are as defined in paragraph 1, and B is boron.

In one embodiment R¹ is C(O)OR^{Y}. In one embodiment R^{Y} is H.

In one embodiment R^{Y} is C₁₋₃ **alkyl, such as** CH₃. In one embodiment R² is not H. In one embodiment R^{2'} is not H. In one embodiment R³ is not H. In one embodiment R² and R^{2'} are not H. In one embodiment R² and R³ are not H. In one embodiment R^{2'} and R³ are not H. In one embodiment R², R^{2'} and R³ are not H. In one embodiment when m is 1 and n is 1 or 2, and Y is pyridine one or both of R⁵ and R⁶ are not H. In one embodiment when Y Is pyridine one or both of R⁵ and R⁶ are not H. In one embodiment R⁴ is a 9 membered heterocycle, for example a 9 membered heterocycle with a N. In one embodiment R⁴ is indole. In one embodiment R⁵ is not H. In one embodiment R⁵ is hydroxy. In one embodiment R⁵ Is oxo. In one embodiment R⁵ is OCHF₂. In one embodiment R⁵ is - OC₁₋₃ alkyl (such as -OCH₃). In one embodiment R⁶ is not H. In one embodiment R⁹ is H. In one embodiment R⁹ is not H. In one embodiment R^{9'} is not H.

In one embodiment R¹⁰ is not H. In one embodiment R¹¹ is C₂₋₆hydroxyalkyl (such as - CH₂CH₂OH). In one embodiment X is O. In one embodiment n is 0. In one embodiment m is 1.

In one embodiment compounds of the disclosure have an activity of 10nM or less (e.g. 5nM or less) in a U937 assay, such as 9, 8, 7, 6, 5, 4, 3, 2 or 1nM, in particular 1nM.

In one embodiment compounds of the disclosure have an activity of 20nM or less (e.g, 10nM or less) in an IL-22 assay, such as 9, 8, 7, 6, 5, 4, 3, 2nM, in particular 2nM.

In one embodiment the compounds of the disclosure have a ratio of above 6.6/1.7 in Caco-2/efflux assay.

Advantageously, this high potency based on the core, allows the molecule to be optimised for other properties to fall within the candidate drug target profile, thereby balancing all of the properties to ensure the molecule is "drug like".

The compounds of the present invention effectively inhibit AhR. Said compounds are useful for the treatment or prophylaxis of conditions where exogenous and endogenous AhR ligands induce dysregulated immune responses, for example: uncontrolled cell growth, proliferation and/or survival of tumour cells, immunosuppression. This dysregulation may be observed in the context of cancer, Inappropriate cellular immune responses, and inappropriate cellular inflammatory responses.

In one embodiment the compounds of the present disclosure are useful in the treatment of cancer for example, liquid and/or solid tumours, and/or metastases thereof. Examples of cancers include head and neck cancer (such as brain tumours and brain metastases), cancer of the thorax including non-small cell and small cell lung cancer, gastrointestinal cancer (including stomach, oesophageal, colon, and colorectal), biliary tract cancer, pancreatic cancer, liver cancer, endocrine cancer, breast cancer, ovarian cancer, bladder cancer, kidney cancer, prostate cancer, bone cancer and skin cancer.

In one embodiment the cancer is an epithelial cancer. In one embodiment the cancer is a sarcoma. In one embodiment the cancer is metastatic.

### DETAILED DISCLOSURE

In one embodiment Y is Independently selected from pyridine, pyrimidine, thiazole, triazole and pyridone.

In one embodiment R⁵ is independently oxo.

Generally, substituents employed in molecules of the present disclosure will be suitable for use in therapeutic molecules. Reactive molecules, such as epoxides etc will usually one be employed in intermediates.

C₁₋₃ alkyl as employed herein refers to straight or branched chain alkyl, for example methyl, ethyl, propyl or isopropyl. Where the alkyl is optionally substituted as defined herein will generally provide a straight or branched chain alkylene.

C₁₋ₓalkylene as employed herein refers to strait or branched chain alkyl of 1 to X carbons in length bearing terminal substituent, such as an alcohol, for example **-CH₂CH₂CH₂-substituent** is a C₃ straight chain alkylene. When the alkylene is branched then a branch may terminate in an alkyl group to the satisfy the valency of the atoms, for example **-CH₂CH(CH₃)-substituent** is a C₃ branched chain alkylene.

C₁₋₃ alkoxy as employed here refers to a branched or straight chain alkyl chain with an oxygen atom located in the chain, for example so the oxygen connects the alkoxy group to the remainder of the molecule (such as -OCH₃) or a carbon links the alkoxy group to the rest of the molecule and the oxygen Is located internally within the alkoxy chain (such as -CH₂OCH₃).

Halogen as employed herein includes fluoro, chloro, bromo or iodo.

Examples of alkyl bearing up to 6 halogen groups include -CH₂F, -CH₂CL, -CHF₂, -CHCL₂, -CF₃, -CCL₂, -CH₂CF₃, -CF₂CF₃, -CH₂CHCL₂, -CHCCL₃.

C(O) represents carbonyl.

C₃₋₅ cycloalkyl includes cyclopropyl, cyclobutyl and cyclopentyl.

A 3 to 6 membered ring optionally comprising 1, 2 or 3 heteroatoms selected from nitrogen, oxygen and sulfur, refers to a saturated, partially saturated or aromatic ring containing 3 or 6 atoms, for example as defined below.

In one embodiment the 3 to 6 membered ring contains no heteroatoms. In one embodiment the 3 to 6 membered ring comprises 1, 2 or 3 heteroatoms selected from nitrogen, oxygen and sulfur.

In one embodiment the ring is saturated. Examples of saturated rings include cyclopropane, cyclobutene, cyclopentane, cyclohexane, azetidine, oxetane, thietane, tetrahydrofuran, tetrahydrothiophene, oxathiolane, 1,3-dioxolane, pyrazolidine, pyrrolidine, thiolane, imidazoline, piperidine, tetrahydropyran, dioxane, morpholine, thiane, dithiane, piperazine and thiomorpholine.

In one embodiment there is provided a 5 or 6 membered ring as optionally comprising 1, 2 or 3 heteroatoms selected from nitrogen, oxygen and sulfur, refers to a saturated, partially saturated or aromatic ring containing 5 or 6 atoms, including wherein all the atoms are carbon or where there are 1, 2 or 3 heteroatoms independently selected from nitrogen, oxygen and sulfur, for example including: cyclopentadiene, phenyl, thiophene, furan, pyrroline, pyrrole, pyrazoline, pyrazole, imidazoline, imidazole, oxazole, isoxazole, thiazole, isothiazole, oxadiazole, thiadiazole, triazole, tetrazole, pyridine, pyrimidine, pyrazine, triazine, thiazine, oxazine, thiopyran, 2H pyran, 4H pyran, dioxine, 2H thiopyran, 4H thiopyran, 4H-1,2-oxazine, 2H-1,2-oxazine, 6H-1,2-oxazine, 4H-1,3-oxazine, 6H-1,3-oxazine, 4H-1,4-oxazine, 4H-1,4-thiazine, 2H-1,2-thiazine, 6H-1,2-thiazine.

In one embodiment there is provided a 5 or 6 membered ring comprising 1, 2 or 3 heteroatoms selected from nitrogen, oxygen and sulfur, refers to a saturated, partially saturated or aromatic ring containing 5 or 6 atoms, where there are 1, 2 or 3 heteroatoms independently selected from nitrogen, oxygen and sulfur, for example as defined above, such as thiophene, furan, pyrroline, pyrrole, pyrazole, imidazole, oxazole, isoxazole, thiazole, isothiazole, triazole, pyridine, pyrimidine, pyrazine, triazine, thiazine, oxazine, pyrroline, 4-H pyran, thiopyran,.

In one embodiment the ring is saturated.

In one embodiment the ring is a saturated carbocyclic ring. In one embodiment the ring in a saturated heterocyclic ring. In one embodiment the ring is partially saturated or aromatic. In one embodiment the ring is partially saturated or aromatic carbocycle. In one embodiment the ring is partially saturated or aromatic heterocycle. In one embodiment the ring is 5 membered. In one embodiment the ring is 6 membered. In one embodiment the 5 or 6 membered ring is unsaturated or aromatic. In one embodiment the 5 or 6 membered ring is selected from cyclopentadiene, phenyl, pyridine and pyrazine, such as phenyl and pyridine. In one embodiment Z' is a 5 or 6 membered heteroaryl with at least one heteroatom selected from N, O and S, for example 1 or 2 nitrogens, wherein said heteroaryl optionally bears one or two substituents selected from hydroxy, halogen (such as F, Cl), CN, C₁₋₃ alkyl.

5 or 6 membered heteroaryl as employed herein is a ring containing 5 or 6 atoms wherein at least one atom is a heteroatom, for example selected from nitrogen, oxygen or sulphur, such as pyrrole, pyrazole, imidazole, thiophene, oxazole, isothiazole, thiazole, pyridine, pyridazine, pyrimidine, pyrazine, triazine, thiopyran, oxazine and thiazine, such as pyrrole, pyrazole and pyridine and pyrimidine.

Unless the context indicates otherwise 5 to 6 membered heterocycle as employed herein generally refers to a non-aromatic ring containing 5 or 6 atoms wherein at least one atom is a heteroatom (for example 1, 2, 3 or 4 heteroatoms independently selected from O, N and S), for example pyrrolidine, imidazolidine, pyrazolidine, oxathiolane, tetrahydrofuran, morpholine, piperidine, piperazine, tetrahydropyran, thiane, dithiane, thiomorpholine and the like.

9 to 13 membered heterocycle as employed herein refers to a bicyclic or tricyclic system containing 9 to 13 atoms, for example containing 1, 2, 3, or 4 heteroatoms independently selected from nitrogen, oxygen and sulfur, which is saturated, partially unsaturated or aromatic.

9 to 13 membered heteroaryl as employed herein refers to a bicyclic or tricyclic system containing 9 to 13 atoms, wherein at least one ring is aromatic and at least one ring contains a heteroatom, for example containing 1, 2, 3, or 4 heteroatoms independently selected from nitrogen, oxygen and sulfur such as indoline, indole, isoindole, indolizine, indazole, benzimidazole, azaindole, pyrazolopyrimidine, purine, benzofuran, isobenzofuran, benzothiophene, benzoisooxazole, benzoisothiazole, benzoxazole, benzothiadiazole, adenine, guanine, tetrahydroquinoline, dihydroisoquinoline, quinoline, isoquinoline, quinolizine, quinoxaline, phthalazine, cinnoline, napthrhyridine, pyridopyrimidine, pyridopyrazine, pyridopyrazine, pteridine, chromene, isochromene, chromenone, benzoxazine, quinolinone, isoquinolinone, dibenzofuran, carbazole, acridine, phenothiazine, 2,3,4,9-tetrahydro-1H-carbazole.

In one embodiment R⁴ is a 9 or 10 membered heteraryl.

9 to 10 membered heteroaryl as employed herein refers to a bicyclic ring system containing 9 or 10 atoms, wherein at least one ring is aromatic and at least one ring contains a heteroatom, for example containing 1, 2, 3, or 4 heteroatoms independently selected from nitrogen, oxygen and sulfur, such as indoline, indole, isoindole, indolizine, indazole, benzimidazole, azaindole, pyrazolopyrimidine, purine, benzofuran, isobenzofuran, benzothiophene, benzoisooxazole, benzoisothiazole, benzoxazole, benzothiadiazole, adenine, guanine, tetrahydroquinoline, dihydroisoquinoline, quinoline, isoquinoline, quinolizine, quinoxaline, phthalazine, cinnoline, napthrhyridine, pyridopyrimidine, pyridopyrazine, pyridopyrazine, pteridine, chromene, isochromene, chromenone, benzoxazine, quinolinone, and isoquinolinone. In one embodiment the 9 or 10 membered heteroaryl is selected from indolylyl and benzimidazolyl, such as indol-3-yl or benzimidazole-2-yl.

Ph as employed herein refers to phenyl.

In one embodiment R¹ is oxetanyl. In one embodiment n' is 0. In one embodiment n' is 1.

The compounds of the present disclosure can be prepared by methods described herein.

In one embodiment there is provided a process of preparing a compound of formula (I) by reaction a compound of formula (**III**): 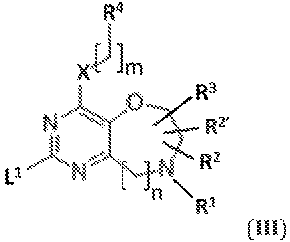 and Y'-R^{x}
wherein R¹, R², R^{2'}, R³, R⁴, X, m and n are defined for compounds of formula (**I**) and Y' is an activated derivative of Y also defined in formula (I) and R^{X} is the activating group. In one embodiment the reaction is a condensation reaction.

**GENERIC ROUTES 1 to 8** can be employed to make certain compounds of the present disclosure:

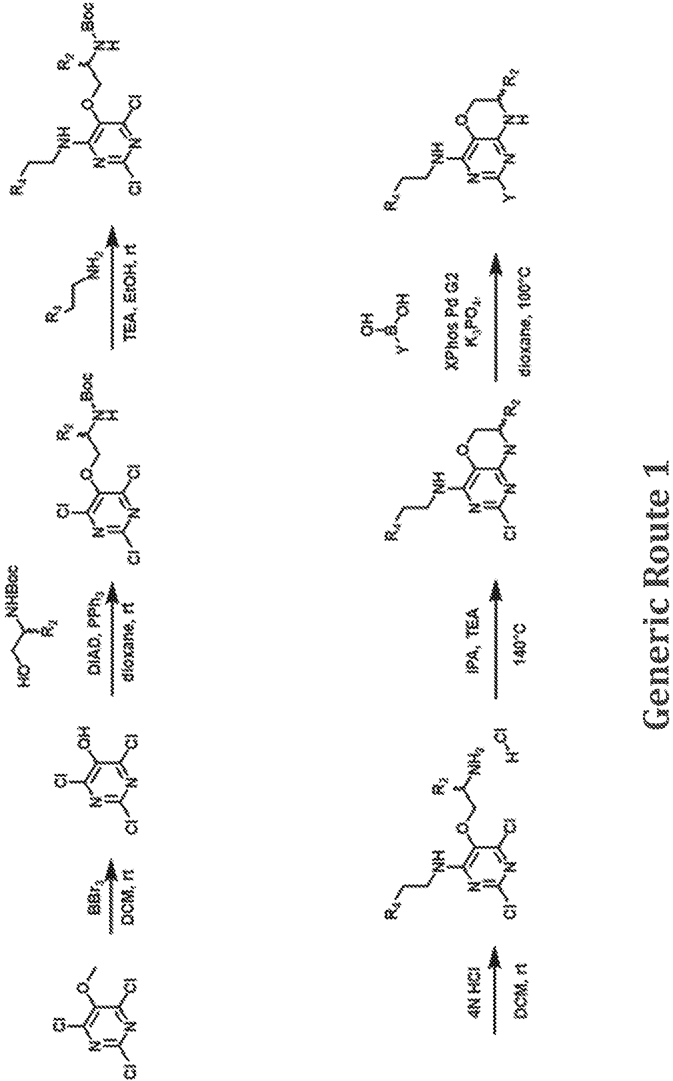

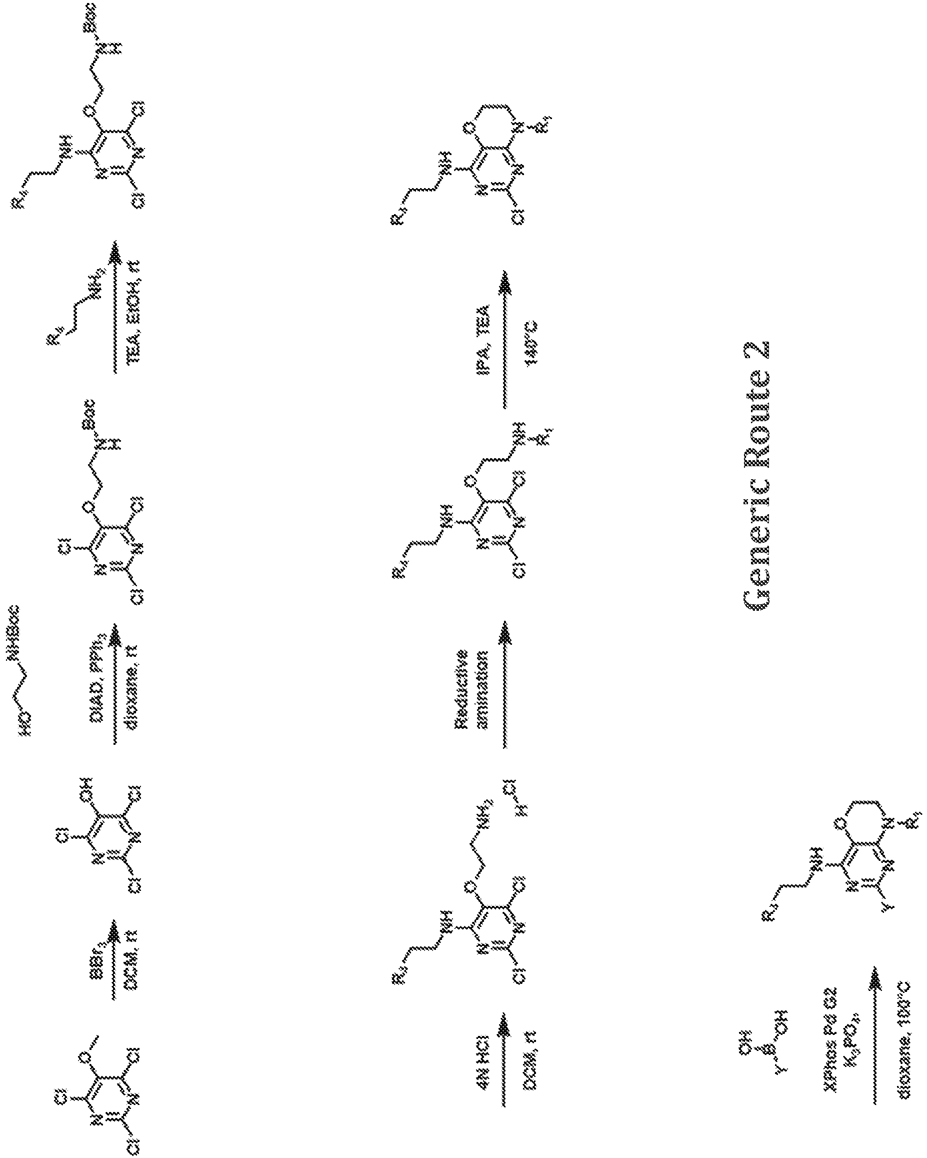

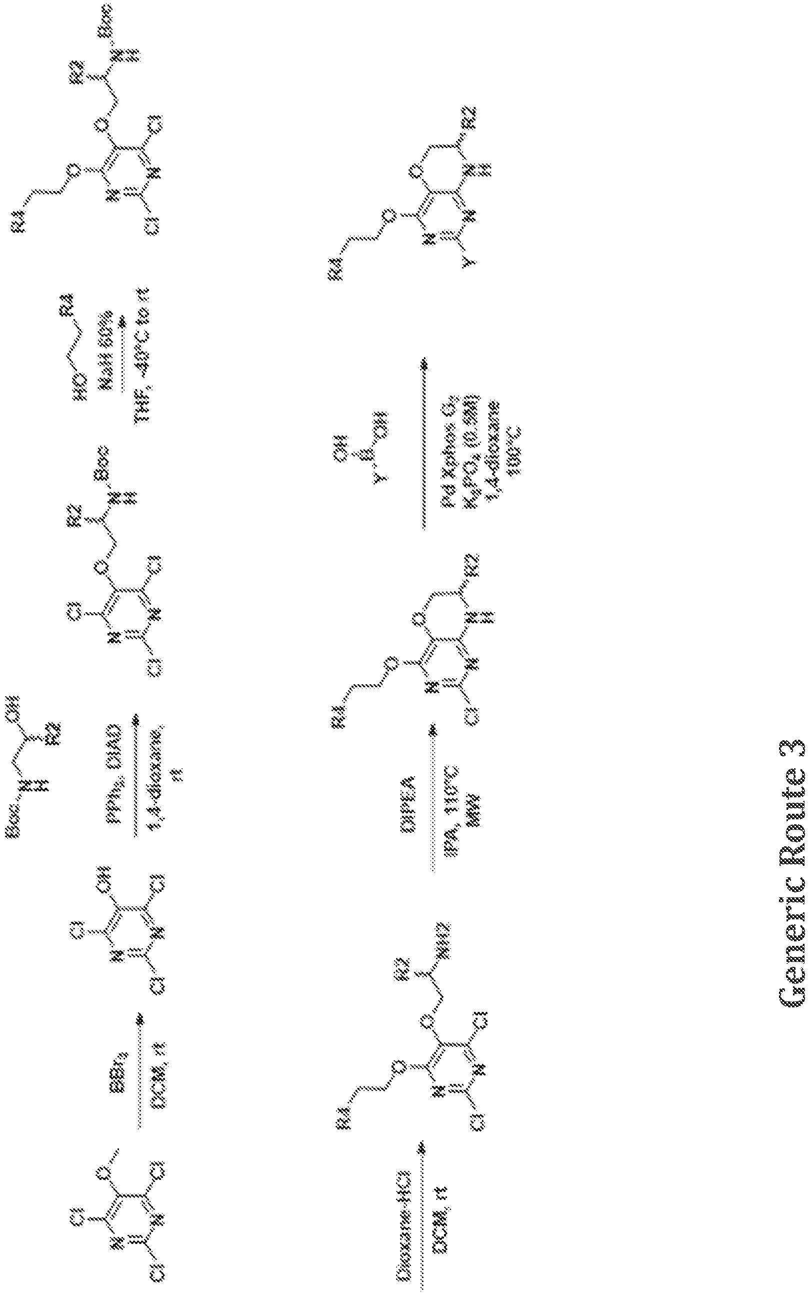

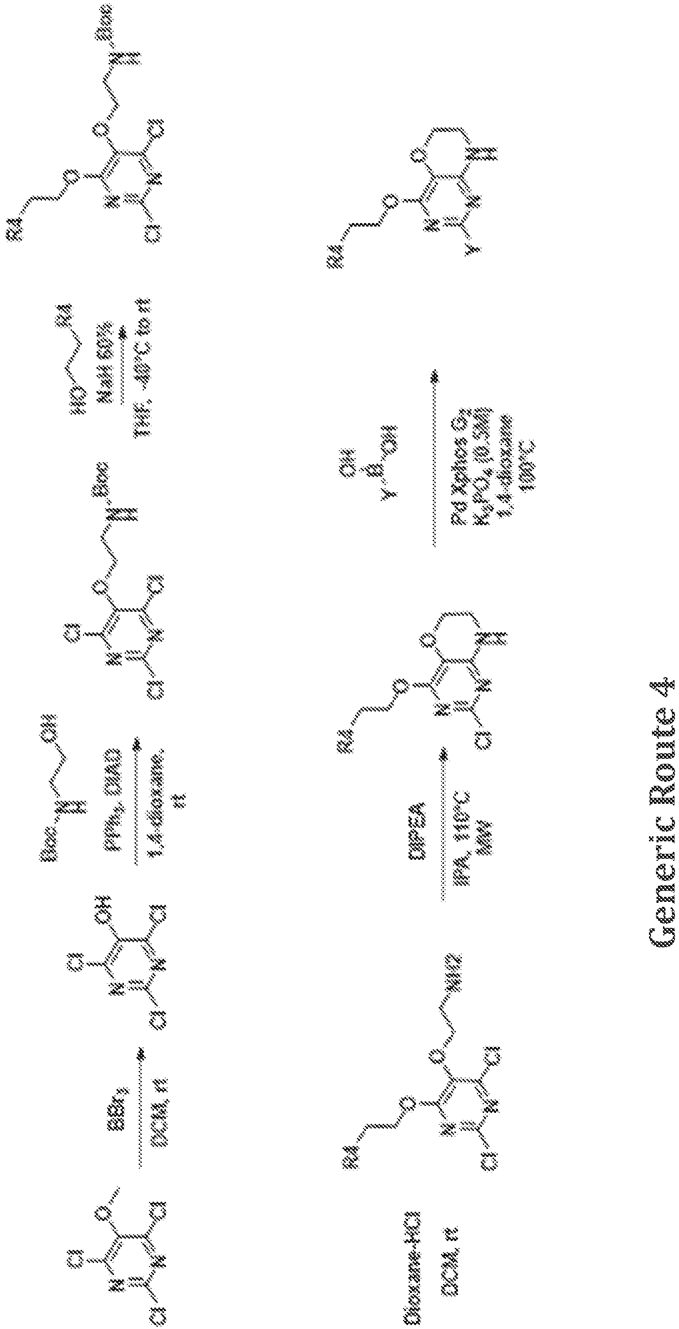

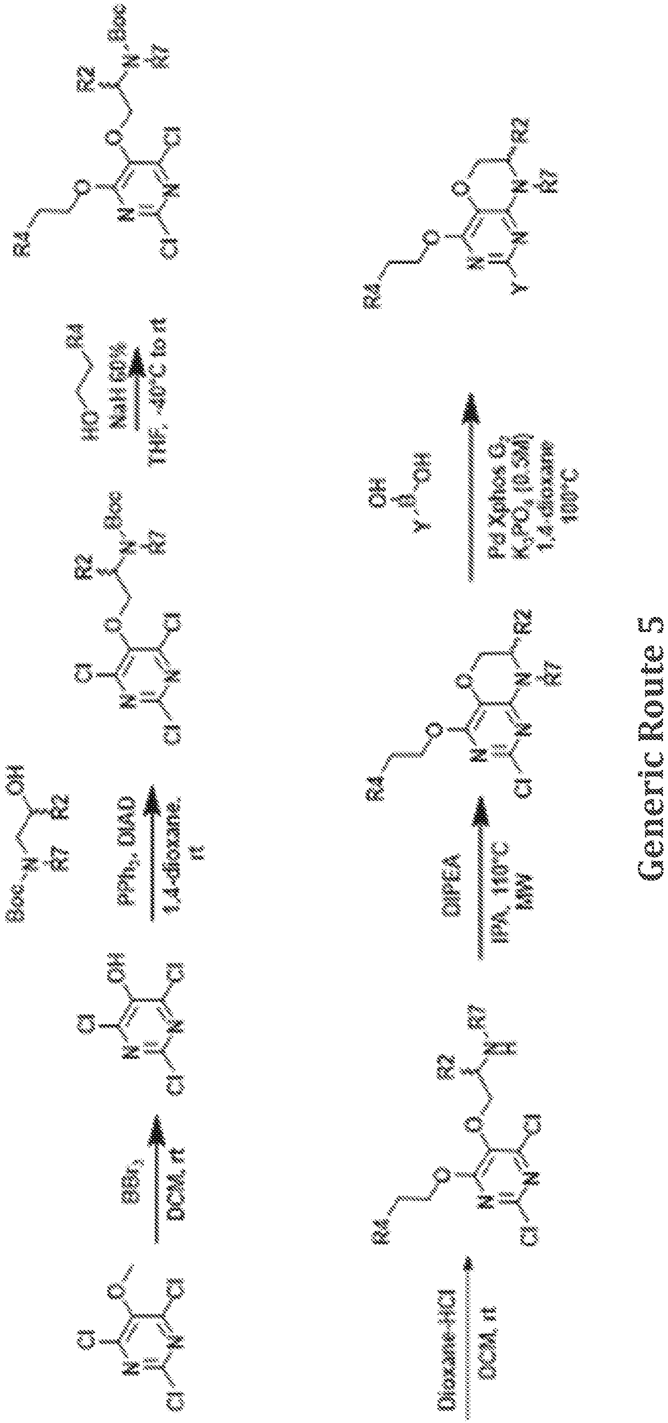

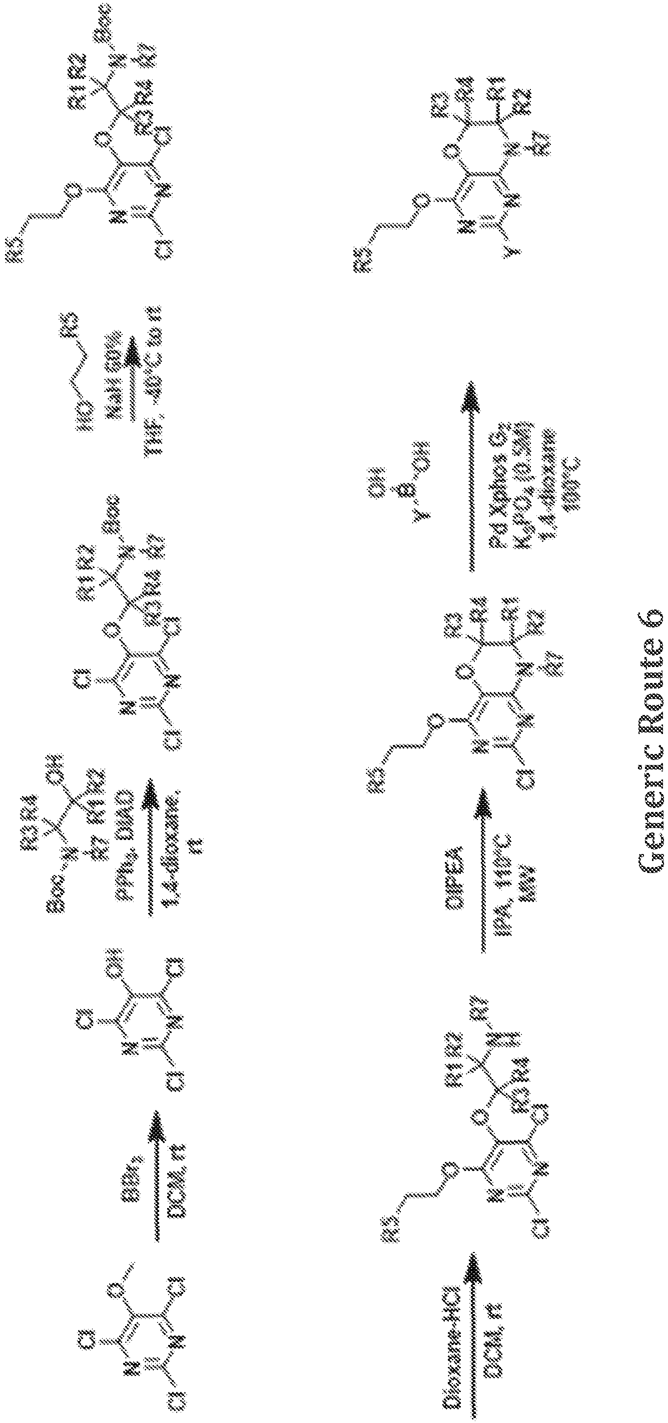

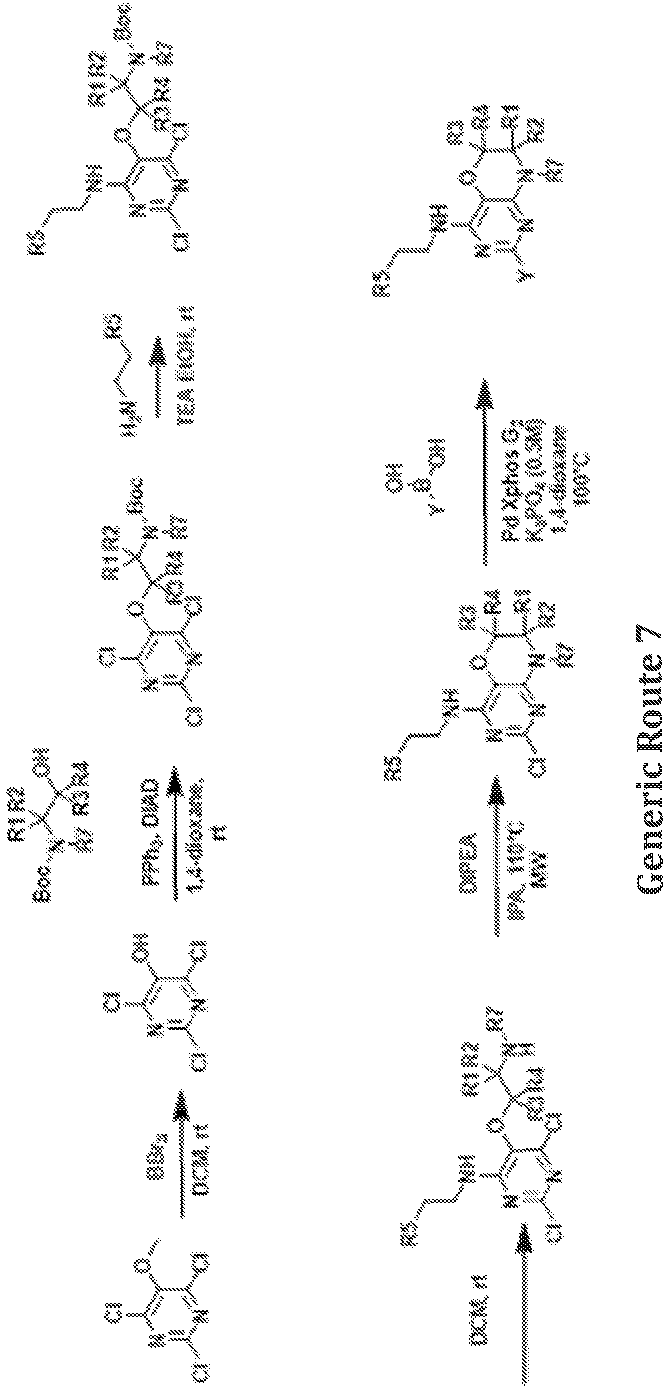

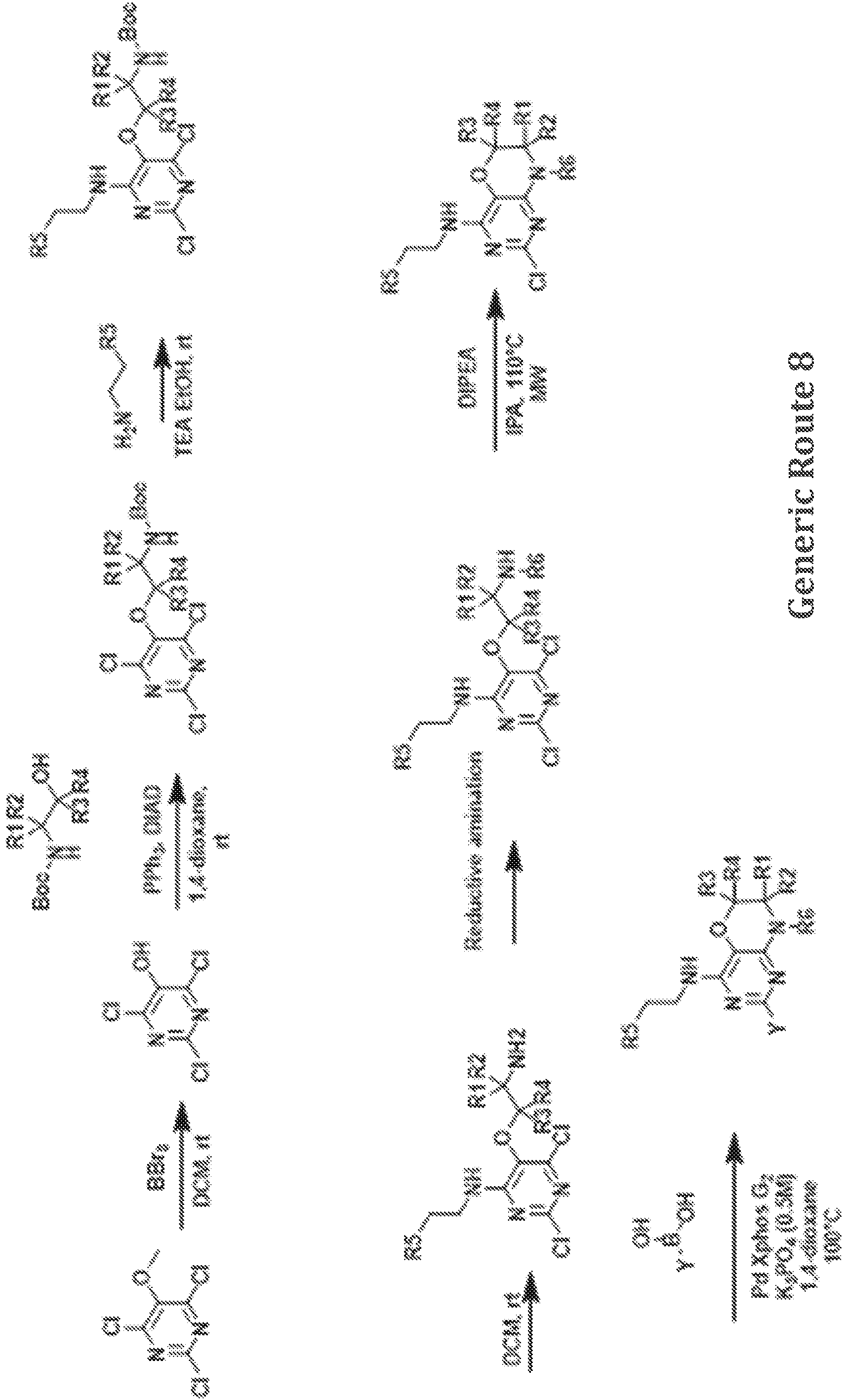

In one embodiment, any one of generic routes 1 to 8 is employed.

In one embodiment, any one of generic routes 1 to 4 is employed. In one embodiment, any one of generic routes 5 to 8 is employed. In one embodiment, any one of generic routes 1, 2 or 5 to 8 is employed. In one embodiment, generic route 1 is employed. In one embodiment, generic route 2 is employed. In one embodiment, generic route 3 is employed. In one embodiment, generic route 4 is employed. In one embodiment, generic route 5 is employed. In one embodiment, generic route 6 is employed. In one embodiment, generic route 7 is employed. In one embodiment, generic route 8 is employed.

Protecting groups may be required to protect chemically sensitive groups during one or more of the reactions described above, to ensure that the process is efficient. Thus, If desired or necessary, intermediate compounds may be protected by the use of conventional protecting groups. Protecting groups and means for their removal are described in "Protective Groups in Organic Synthesis", by Theodora W. Greene and Peter G.M. Wuts, published by John Wiley & Sons Inc; 4^{th} Rev Ed, 2006, ISBN-10: 0471697540.

Examples of salts of compound of the present disclosure include all pharmaceutically acceptable salts, such as, without limitation, acid addition salts of strong mineral acids such as HCl and HBr salts and addition salts of strong organic acids, such as a methansulfonic acid salt.

The present disclosure extends to solvates of the compounds disclosed herein. Examples of solvates include hydrates.

Novel intermediates are an aspect of the invention.

A further aspect of the present disclosure is methods of making the compounds disclosed herein.

Also provided herein a pharmaceutically composition comprising a compound according to the present disclosure and an excipient, diluent or carrier. A thorough discussion of pharmaceutically acceptable carriers is available in Remington's Pharmaceutical Sciences (Mack Publishing Company, N.J. 1991).

The pharmaceutical compositions of this disclosure may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes. Hyposprays may also be used to administer the pharmaceutical compositions of the invention.

In one embodiment the therapeutic compositions may be prepared as injectables, either as liquid solutions or suspensions. Solid forms suitable for solution in, or suspension in, liquid vehicles prior to Injection may also be prepared. Suitable liquids for reconstitution of such solid forms (including lyophilised solids) may be selected from aqueous solutions, for example saline, dextrose or water for injection and the like. In one embodiment the reconstituted liquid formulation is isotonic.

In one embodiment the pharmaceutical composition according to the present disclosure is provided as a tablet or a capsule for oral administration.

### TREATMENT

The present disclosure also extends to methods of treating a patient comprising administering a therapeutically effective amount of a compound of the present disclosure (or a pharmaceutical composition comprising the same), for example for the treatment of cancer.

Also provide is a compound according to the present disclosure (or a pharmaceutical composition comprising the same) for use in treatment, for example for use in the treatment of cancer.

In a further aspect there is provided a compound of the present disclosure (or a pharmaceutical composition comprising the same) for use in the manufacture of a medicament for the treatment of cancer.

In one embodiment the cancer is an epithelial cancer, for example selected from example is selected from liver cancer (such as hepatocellular carcinoma), biliary tract cancer, breast cancer (such as none ER+ breast cancer), prostate cancer, colorectal cancer, ovarian cancer, cervical cancer, lung cancer, gastric cancer, pancreatic, bone cancer, bladder cancer, head and neck cancer, thyroid cancer, skin cancer, renal cancer, and oesophagus cancer, for example gastric cancer.

In one embodiment the cancer is selected from selected from the group comprising hepatocellular carcinoma, cholanglocarcinoma, breast cancer, prostate cancer, colorecetal cancer, ovarian cancer, lung cancer, gastric cancer, pancreatic and oesophagus cancer.

In one embodiment the biliary duct cancer is in a location selected from Intrahepatic bile ducts, left hepatic duct, right hepatic duct, common hepatic duct, cystic duct, common bile duct, Ampulla of Vater and combinations thereof.

In one embodiment the biliary duct cancer is in an Intrahepatic bile duct. In one embodiment the biliary duct cancer is in a left hepatic duct. In one embodiment the biliary duct cancer is in a right hepatic duct. In one embodiment the biliary duct cancer is in a common hepatic duct In one embodiment the biliary duct cancer is in a cystic duct. In one embodiment the biliary duct cancer is in a common bile duct. In one embodiment the biliary duct cancer is in an Ampulla of Vater. In one embodiment the epithelial cancer is a carcinoma.

In one embodiment the treatment according to the disclosure is adjuvant therapy, for example after surgery.

In one embodiment the therapy according to the disclosure is neoadjuvant treatment, for example to shrink a tumour before surgery.

In one embodiment the tumour is a solid tumour. In one embodiment the cancer is a primary cancer, secondary cancer, metastasis or combination thereof. In one embodiment the treatment according to the present disclosure is suitable for the treatment of secondary tumours. In one embodiment the cancer is metastatic cancer. In one embodiment the treatment according to the present disclosure is suitable for the treatment of primary cancer and metastases. In one embodiment the treatment according to the present disclosure is suitable for the treatment of secondary cancer and metastases. In one embodiment the treatment according to the present disclosure is suitable for the treatment of primary cancer, secondary cancer and metastases.

In one embodiment the treatment according to the present disclosure is suitable for the treatment of cancerous cells in a lymph node.

In one embodiment the liver cancer is primary liver cancer. In one embodiment the liver cancer is secondary liver cancer. In one embodiment the liver cancer is stage 1, 2, 3A, 3B, 3C. 4A or 4B.

In one embodiment the gastric cancer is stage 0, I, II, III or IV.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. Generally, a therapeutically effective amount will be from 0.01 mg/kg to 1000 mg/kg, for example 0.1 mg/kg to 500 mg/kg. Pharmaceutical compositions may be conveniently presented In unit dose forms containing a predetermined amount of an active agent of the invention per dose.

### Combination Therapy

In one embodiment the compound of the present disclosure is employed in combination therapy, for example wherein the further therapy is an anticancer therapy.

In one embodiment the anticancer therapy is a chemotherapy.

Chemotherapeutic agent and chemotherapy or cytotoxic agent are employed interchangeably herein unless the context indicates otherwise.

Chemotherapy as employed herein is intended to refer to specific antineoplastic chemical agents or drugs that are "selectively" destructive to malignant cells and tissues, for example alkylating agents, antimetabolites including thymidylate synthase inhibitors, anthracyclines, anti-microtubule agents Including plant alkaloids, topoisomerase inhibitors, parp inhibitors and other antitumour agents. Selectively in this context Is used loosely because of course many of these agents have serious side effects.

The preferred dose may be chosen by the practitioner, based on the nature of the cancer being treated.

Examples of alkylating agents, which may be employed in the method of the present disclosure include an alkylating agent selected from nitrogen mustards, nitrosoureas, tetrazines, aziridines, platins and derivatives, and non-classical alkylating agents.

Platinum containing chemotherapeutic agent (also referred to as platins) includes, for example cisplatin, carboplatin, oxaliplatin, satraplatin, picoplatin, nedaplatin, triplatin and lipoplatin (a liposomal version of cisplatin), in particular cisplatin, carboplatin and oxaliplatin.

The dose for cisplatin ranges from about 20 to about 270 mg/m² depending on the exact cancer. Often the dose is in the range about 70 to about 100mg/m².

Nitrogen mustards include mechlorethamine, cyclophosphamide, melphalan, chlorambucil, ifosfamide and busulfan.

Nitrosoureas Include N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide.

Aziridines include thiotepa, mytomycin and diaziquone (AZQ).

Examples of antimetabolites, which may be employed In the method of the present disclosure, include anti-folates (for example methotrexate and pemetrexed), purine analogues (for example thiopurines, such as azathiopurine, mercaptopurine, thiopurine, fludarabine (including the phosphate form), pentostatin and cladribine), pyrimidine analogues (for example fluoropyrimidines, such as 5-fluorouracil and prodrugs thereof such as capecitabine [Xeloda^{®}]), floxuridine, gemcitabine, cytarabine, decitabine, raltitrexed(tomudex) hydrochloride, cladribine and 6-azauracil.

Examples of anthracyclines, which may be employed in the method of the present disclosure, include daunorubicin (Daunomycin), daunorubicin (liposomal), doxorubicin (Adriamycin), doxorubicin (liposomal), epirubicin, idarubicin, valrubicin (currently used only to treat bladder cancer) and mitoxantrone an anthracycline analog, in particular doxorubicin.

Examples of anti-microtubule agents, which may be employed in the method of the present disclosure, include include vinca alkaloids and taxanes.

Vinca alkaloids include completely natural chemicals, for example vincristine and vinblastine and also semi-synthetic vinca alkaloids, for example vinorelbine, vindesine, and vinflunine

Taxanes include paclitaxel, docetaxel, abraxane, carbazitaxel and derivatives of thereof. Derivatives of taxanes as employed herein includes reformulations of taxanes like taxol, for example in a micellar formulation, derivatives also include chemical derivatives wherein synthetic chemistry is employed to modify a starting material which is a taxane.

Topoisomerase inhibitors, which may be employed in a method of the present disclosure include type I topoisomerase inhibitors, type II topoisomerase inhibitors and type II topoisomerase poisons. Type I Inhibitors include topotecan, irinotecan, indotecan and indimitecan. Type II inhibitors include genistein and ICRF 193 which has the following structure:

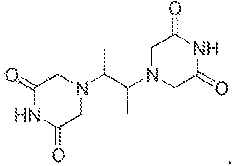

Type II poisons include amsacrine, etoposide, etoposide phosphate, teniposide and doxorubicin and fluoroquinolones.

In one embodiment a combination of chemotherapeutic agents employed is, for example a platin and 5-FU or a prodrug thereof, for example cisplatin or oxaplatin and capecitabine or gemcitabine, such as FOLFOX.

In one embodiment the chemotherapy comprises a combination of chemotherapy agents, in particular cytotoxic chemotherapeutic agents.

In one embodiment the chemotherapy combination comprises a platin, such as cisplatin and fluorouracil or capecitabine.

In one embodiment the chemotherapy combination in capecitabine and oxaliplatin (Xelox).

In one embodiment the chemotherapy is a combination of folinic acid and 5-FU, optionally in combination with oxaliplatin.

In one embodiment the chemotherapy is a combination of folinic acid, 5-FU and irinotecan (FOLFIRI), optionally in combination with oxaliplatin (FOLFIRINOX). The regimen consists of: irinotecan (180 mg/m² IV over 90 minutes) concurrently with folinic acid (400 mg/m² [or 2 x 250 mg/m²] IV over 120 minutes); followed by fluorouracil (400-500 mg/m² IV bolus) then fluorouracil (2400-3000 mg/m² Intravenous infusion over 46 hours). This cycle is typically repeated every two weeks. The dosages shown above may vary from cycle to cycle.

In one embodiment the chemotherapy combination employs a microtubule inhibitor, for example vincristine sulphate, epothilone A, N-[2-[(4-Hydroxyphenyl)amino]-3-pyridinyl]-4-methoxybenzenesulfonamide (ABT-751), a taxol derived chemotherapeutic agent, for example paclitaxel, abraxane, or docetaxel or a combination thereof.

In one embodiment the chemotherapy combination comprises an antimetabolite such as capecitabine (xeloda), fludarabine phosphate, fludarabine (fludara), decitabine, raltitrexed (tomudex), gemcitabine hydrochloride and cladribine.

In one embodiment the anticancer therapy combination employs an mTor inhibitor. Examples of mTor inhibitors Include: everolimus (RAD001), WYE-354, KU-0063794, papamycin (Sirolimus), Temsirolimus, Deforolimus(MK-8669), AZD8055 and BEZ235(NVP-BEZ235).

In one embodiment the anticancer therapy combination employs a MEK inhibitor. Examples of MEK Inhibitors include: AS703026, Cl-1040 (PD184352), AZD6244 (Selumetinib), PD318088, PD0325901, AZD8330, PD98059, U0126-EtOH, BIX 02189 or BIX 02188.

In one embodiment the chemotherapy combination employs an AKT inhibitor. Examples of AKT inhibitors include: MK-2206 and AT7867.

In one embodiment the anticancer therapy employs an aurora kinase inhibitor. Examples of aurora kinase inhibitors include: Aurora A Inhibitor I, VX-680, AZD1152-HQPA (Barasertib), SNS-314 Mesylate, PHA-680632, ZM-447439, CCT129202 and Hesperadin.

In one embodiment the chemotherapy combination employs a p38 inhibitor, for example as disclosed in WO2010/038086, such as N-[4-({4-[3-(3-tert-Butyl-1-p-tolyl-1H-pyrazol-5-yl)ureido]naphthalen-1-yloxy}methyl)pyridin-2-yl]-2-methoxyacetamide.

In one embodiment the combination employs a Bcl-2 inhibitor. Examples of Bcl-2 inhibitors include: obatoclax mesylate, ABT-737, ABT-263(navitoclax) and TW-37.

In one embodiment the chemotherapy combination comprises ganciclovir, which may assist in controlling immune responses and/or tumour vasculation.

In one embodiment the anticancer therapy includes a PARP Inhibitor.

In one embodiment the anticancer therapy includes an inhibitor of cancer metabolism with specific inhibition of the activity of the DHODH enzyme.

In one embodiment the compound of the present disclosure is employed in combination (for example in a combination therapy) with a checkpoint inhibitor. Thus, the present disclosure provides a combination therapy comprising a compound or pharmaceutical composition of the present disclosure, and a checkpoint inhibitor or a combination of checkpoint inhibitors.

In one embodiment the checkpoint inhibitor is selected from the group comprising; PD-1 inhibitor, PD-L1/L2 inhibitor, CTLA-4 inhibitor, checkpoint kinase inhibitor 1 (CHEK1/CHK1), checkpoint kinase inhibitor 2 (CHEK2/ CHK2), Ataxia telangiectasia and Rad3 related (ATR) inhibitor, ataxia-telangiectasia mutated (ATM) inhibitor, Wee1 dual specificity protein kinase (Wee1) inhibitor, Poly ADP Ribose polymerase (PARP) Inhibitor and Myt1 inhibitor.

In one embodiment the checkpoint inhibitor is selected from the group comprising: a PD-1 inhibitor, a PD-L1/L2 inhibitor, a CTLA-4 inhibitor; and a combination thereof. In one embodiment a combination of a PD-1 inhibitor and a PD-L1 inhibitor is employed. In one embodiment a combination of a PD-1 and a CTA-4 inhibitor is employed. In one embodiment, a combination of a PD-L1 and CTA-4 inhibitor is employed. In one embodiment, a combination of a PD-1, PD-L1 and a CTA-4 inhibitor is employed.

In one embodiment, the checkpoint inhibitor is a PD-1 inhibitor. In one embodiment, the PD-1 inhibitor is selected from the group comprising: nivolumab (also known as OPDIVO^{®}, SC4, BMS-936558, MDX-1106, and ONO-4538). pembrolizumab (Merck; also known as KEYTRUDA^{®}, lambrolizumab, and MK-3475), PDR001 (Novartis; also known as spartalizumab), MEDI- 0680 (AstraZeneca; also known as AMP-514), cemiplimab (Regeneron; also known as REGN-2810), JS001 or "toripalimab" (TAlZHOU JUNSHI PHARMA), BGB-A317 ("Tislelizumab;" Belgene), INCSHR1210 (Jiangsu Hengrut Medicine; also known as "camrelizumab,", SHR- 1210), TSR-042 or "dostarlimab" (Tesaro Biopharmaceutical; also known as ANB011), GLS- 010 (Wuxi/Harbin Gloria Pharmaceuticals; also known as WBP3055), STI-1110 (Sorrento Therapeutics), AGEN2034 or "balstilimab" (Agenus), MGA012 or "retifanlimab" (Macrogenics), IBI308 or "sinitillmab" (Innovent), BCD-100 or "bevacizumab" (Biocad), and ITX-4014 (Jounce Therapeutics).

In one embodiment, the checkpoint inhibitor is pembrollzumab. In one embodiment the checkpoint inhibitor is nivolumab. In one embodiment the checkpoint inhibitor is cemiplimab. In one embodiment the checkpoint inhibitor is dostarlimab.

In one embodiment the checkpoint inhibitor is a PD-L1 inhibitor. In one embodiment the PD-L1 inhibitor is selected from the group comprising: atezolizumab (Tecentriq), avelumab (Bavencio), durvalumab (Imfinzi), KN035, CK-301. (Checkpoint Therapeutics), AUNP12 (Aurigene), CA-170 (Aurigen/Curis) and BMS-986189 (BMS).

In one embodiment, the checkpoint inhibitor is atezolizumab. In one embodiment, the checkpoint inhibitor is avelumab. In one embodiment, the checkpoint inhibitor is durvalumab.

In one embodiment, the checkpoint inhibitor is a CTLA-4 inhibitor. In one embodiment the CTLA-4 inhibitor is selected from the group comprising: ipilimumab (Yervoy), tremelimumab

In one embodiment the checkpoint inhibitor is an antibody or binding fragment specific to a checkpoint protein, in particular one disclosed herein, such as PD-1, PD-L1 or CTLA-4.

In one embodiment the checkpoint kinase inhibitor is independently selected from: 3-[(Aminocarbonyl)amino]-5-(3-fluorophenyl)-N-(3S)-3-piperidinyl-2-thiophenecarboxamide hydrochloride, (3R,45)-4-[[2-(5-Fluoro-2-hydroxyphenyl)-6,7-dimethoxy-4-quinazolinyl]amino]-α,α-dimethyl-3-pyrrolidinemethanol dihydrochloride; 4.4'-diacetyldiphenylurea bis(guanylhydrazone) ditosylate; 9-Hydroxy-4-phenyl-pyrrolo[3,4-c]carbazole-1,3(2H,6H)-dione; (R)-α-Amino-N-[5,6-dihydro-2-(1-methyl-1H-pyrazol-4-yl)-6-oxo-1H-pyrrolo[4,3,2-ef][2,3]benzodiazepin-8-yl]-cyclohexaneacetamide; 9,10,11,12-Tetrahydro- 9,12-epoxy-1H-diindolo[1,2,3-fg:3',2',1'-kl]pyrrolo[3,4-1][1,6]benzodiazocine-1,3(2H)-dione; 4'-[5-[[3-[(Cyclopropylamino)methyl)phenyl]amino]-1H-pyrazol-3-yl]-[1,1'-biphenyl]-2,4-diol; and (R)-5-((4-((Morpholin-2-ylmethyl)amino)-5-(trifluommethyl)pyridin-2-yl)amino)pyrazine-2-carbonitrile (CCT245737).

In one embodiment one or more therapies employed in the method herein are metronomic, that is a continuous or frequent treatment with low doses of anticancer drugs, often given concomitant with other methods of therapy.

In one embodiment, there is provided the use of multiple cycles of treatment (such as chemotherapy) for example 2, 3, 4, 5, 6, 7 or 8.

Comprising" in the context of the present specification is intended to mean "including". Where technically appropriate, embodiments of the invention may be combined.

Embodiments are described herein as comprising certain features/elements. The disclosure also extends to separate embodiments consisting or consisting essentially of said features/elements.

Technical references such as patents and applications are incorporated herein by reference.

Any embodiments specifically and explicitly recited herein may form the basis of a disclaimer either alone or in combination with one or more further embodiments.

The invention will now be described with reference to the following examples, which are merely illustrative and should not be construed as limiting the scope of the present invention.

### EXAMPLES

The present compounds can be made by methods analogous to those described in WO2020/039093 and PCT/SG2021/050095 both incorporated herein by reference.

### General method 1

A flask was charged with 2,4,6-trichloropyrimidin-5-ol (1.0 eq), tert-butyl N-(2-hydroxyethyl)carbamate (1.10 eq), triphenylphosphine (1.20 eq) and diisopropyl azodicarboxylate (1.20 eq) in 1,4-dioxane (6 mL), and the resulting mixture was stirred at room temperature for 1 h. The reaction was concentrated to dryness in vacuo to afford a residue, which was purified by automated flash column chromatography over silica (40 g cartridge), eluting with 15% EtOAc In iso-hexane, to give the desired product as a solid.

### General method 2a

A suitable round bottom flask or reacti-vial was charged with aryl halide (1 equiv.), amine (1.1 equiv.), methanol or ethanol (10 mL/mmol) and triethylamine (2 equiv.), and stirred at ambient temperature for 3-16h (reaction monitored by UPLC analysis). The reaction mixture was evaporated to dryness and the resultant residue partitioned between ethyl acetate and water. The organic phase was separated and sequentially washed with saturated bicarbonate solution, water, brine, then dried over sodium sulfate, filtered and evaporated Purification, if required was performed by chromatography or trituration.

### General method 2b

A solution of Sodium hydride, 60% in mineral oil (1.00 eq,) and indol-3-ethanol (1.10 eq,) in THF were stirred for 30min and added at -78C to a solution of appropriate chloropyrimidine (1.00 eq,) in THF. The mixture was stirred overnight leaving the cooling bath dry out over time (slow warm up). The reaction was diluted in DCM and then the mixture was washed with 2.5M citric acid solution and water. The organic phase was concentrated to dryness to give the product Purification, if required was performed by chromatography or trituration.

### General method 3

A solution of Boc-protected amine (1.0 eq) in dichloromethane (10 mL/mmol) was treated with a 4 N solution of hydrogen chloride in 1,4-dioxane (25.0 eq), and the resulting mixture was stirred at ambient temperature for 1 h. The reaction was concentrated and the resulting residue was triturated with diethyl ether (50 mL/mmol), filtered and dried under vacuum to afford the desired product

### General Method 4

A solution of appropriate intermediate (1.0 eq) in iso-propan-2-ol was treated with triethylamine (14 eq), and the resulting mixture was stirred at 110-160 °C for 2-6 h in the microwave (CEM, 200 watt). The reaction was concentrated to dryness to give a solid residue, which was suspended in water with stirring. The mixture was filtered and the solid was dried under vacuum to give the desired product as a solid. Purification, if required was performed by chromatography or trituration.

### General method 5a

A suitable round bottom flask or reacti-vial was charged with aryl halide (1equiv.), aryl boronic acid (1.5-2.0 equiv.), potassium carbonate (1.5-2.0 equiv.), Dioxane/water ([5:1] about 60 vol). Head space was flushed with nitrogen gas, then [1,1'-Bis(diphenylphosphino) ferrocene]dichloropalladium(II) dichloride (0.2-0.3 equiv.) was added. The reaction mixture was heated under nitrogen at 100°C for 2-24 h until complete as determined by UPLC analysis. The reaction mixture was evaporated to dryness. Purification, if required was performed by chromatography or trituration.

### General method 5b

A suitable round bottom flask or reacti-vial was charged with aryl halide (1equiv.), aryl boronic acid (1.5-2.0 equiv.), potassium phosphate tribasic (0.5M aqueous solution, 1.5-2.0 equiv.), Dioxane. Head space was flushed with nitrogen gas, then Chloro(2-dicyclohexylphosphino-2',4',6'-triisopropyl-1,3'-biphenyl)[2-(2'-amino-1,1'-biphenyl)]palladium(II), (0.2-0.3 eq) was added. The reaction mixture was heated under nitrogen at 100 °C for 2-24 h until complete as determined by UPLC analysis. Purification, if required was performed by chromatography or trituration.

### General method 5c

Chloro-aryl (1.00 eq) in Toluene (4 mL) was added (Tri-n-butylstannyl)aryl (1.10 eq) followed by Tetrakis(triphenylphosphine)palladium(0) (0.2 eq). The solution was heated at 110 °C for 1h. Upon completion, the mixture was cooled to rt, the reaction was concentrated to dryness. Purification, if required was performed by chromatography or trituration.

### Intermediates for Example 1

### 1.1 2,4,6-Trichloropyrimidin-5-ol

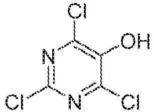

A stirred solution of 2,4,6-trichloro-5-methoxy-pyrimidine (1.05 g, 4.92 mmol, 1.0 eq) In dichloromethane (35 mL) was cooled to 0 °C and treated with a 1 M solution of boron tribromide in dichloromethane (17 mL, 17.2 mmol, 3.5 eq), and the resulting mixture was stirred at ambient temperature for 16 h.

The reaction mixture was cooled to 0 °C and diluted by the sequential addition of methanol (10 mL) and water (50 mL), and the resulting mixture was extracted with dichloromethane (2 × 30 mL). The combined organic extracts were dried (Na₂SO₄), filtered and concentrated in vacuo to afford 2,4,6-trichloropyrimidin-5-ol as an off-white solid (1.01 g, 4.81 mmol, 98% yield).

This material was used directly in the next step without further purification. UPLC-MS analysis (2 min, acidic): rt = 0.79 min, m/z = 196.9/198.9/200.9 [M-H]-, 93% purity.

¹³C NMR (101 MHz, DMSO) δ 149.73, 145.68, 145.64.

### 1.2 tert-butyl N-[2-(2,4,6-trichloropyrimidin-5-yl)oxyethyl]carbamate

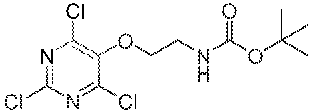

Prepared according to general method 1, using 2,4,6-trichloropyrimidin-5-ol (0.35 g, 1.63 mmol, 1.0 eq) and tert-butyl N-(2-hydroxyethyl)carbamate (0.28 mL, 1.80 mmol, 1.10 eq).

The crude was purified by column chromatography (40 g cartridge) eluting with 15% EtOAc in iso-hexane to afford tert-butyl N-[2-(2,4,6-trichloropyrimidin-5-yl)oxyethyl]carbamate (0.42 g.1.21 mmol, 74.21% yield) as a white solid. UPLC-MS analysis (2 min basic): rt = 1.18 min, no ionisation), 100% purity. ¹H NMR (400 MHz, DMSO-D6) δ 7.03 (t, J = 5.6 Hz, 1H), 4.13 (t, J = 5.7 Hz, 2H), 3.37 - 3.33 (m, 2H), 1.39 (s, 9H).

### 1.3 tert-butyl N-[2-[2,4-dichloro-6-[2-(1H-indol-3-yl)ethylamino]pyrimidin-5-yl]oxyethyl]carbamate

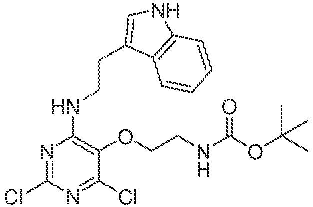

Prepared according to general method 2a, using tert-butyl N-[2-(2.4,6-trichloropyrimidin-5-yl)oxyethyl]carbamate (0.36 g. 1.05 mmol, 1.0 eq) and tryptamine (0.18 g, 1.10 mmol, 1.05 eq). The reaction was concentrated to dryness and the residue was dispersed in water (25 mL).

The resultant suspension was filtered to afford tert-butyl N-[2-[2,4-dichloro-6-[2-(1H-indol-3-yl)ethylamino]pyrimidin-5-yl]oxyethyl]carbamate (500 mg,1.05 mmol, 99.99% yield) as an off-white solid. The product was used in the next step without further purification.
UPLC-MS analysis (2 min basic): rt = 1.25 mins (M+H+ = 466.2/468.3/470.3), 98% purity
¹H NMR (400 MHz, DMSO-D6) δ 10.83 (s, 1H), 8.03 (d, J = 6.2 Hz, 1H), 7.64 (d, J = 7.9 Hz, 1H), 7.35 (dt, J = 8.1, 1.0 Hz, 1H), 7.19 (d, J = 2.3 Hz, 1H), 7.13 - 7.00 (m, 2H), 7.03 - 6.93 (m, 1H), 3.92 (t, J = 5.4 Hz, 2H), 3.62 (q, J = 6.9 Hz, 2H), 3.30 - 3.22 (m, 2H), 2.99 (t, J = 7.6 Hz, 2H), 1.39 (s, 9H).

### 1.4 5-(2-aminoethoxy)-2,6-dichloro-N-[2-(1H-indol-3-yl)ethyl]pyrimidin-4-amine;hydrochloride

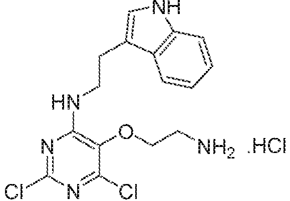

Prepared according to general method 3, tert-butyl N-[2-[2,4-dichloro-6-[2-(1H-indol-3-yl)ethylamino] pyrimidin-5-yl]oxyethyl]carbamate (1.00 eq, 0.50 g, 1.05 mmol) to afford 5-(2-aminoethoxy)-2,6-dichloro-N-[2-(1H-indol-3-yl)ethyl]pyrimidin-4-amine;hydrochloride (0.47 g,1.05 mmol, 99.97% yield) as an off-white solid.

The product was used in the next step without further purification.

UPLC-MS analysis (2 min basic): rt = 1.09 mins (M+H+ = 366.2/368.2/370.2), 92% purity.

¹H NMR (400 MHz, DMSO-D6) δ 10.86 (d, J = 2.4 Hz, 1H), 8.38 (t, J = 6.0 Hz, 1H), 8.30 (s, 3H), 7.67 (d, J = 7.8 Hz, 1H), 7.35 (d, J = 8.1 Hz, 1H), 7.20 (d, J = 2.3 Hz, 1H), 7.14 - 7.04 (m, 1H), 7.04 - 6.94 (m, 1H), 4.14 (t, J = 5.0 Hz, 2H), 3.70 - 3.58 (m, 2H), 3.23 (h, J = 5.5 Hz, 2H), 3.06 - 2.91 (m, 2H).

### 1.5 2-chloro-N-[2-(1H-Indol-3-yl)ethyl]-7.8-dihydro-6H-pyrimido[5,4-b][1,4]oxazin-4-amine

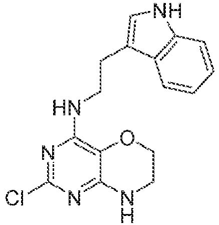

Prepared according to general method 4, using 5-(2-aminoethoxy)-2,6-dichloro-N-[2-(1H-indol-3-yl)ethyllpyrimidin-4-amine;hydrochloride (1.00 eq, 0.25 g, 0.571 mmol) to afford 2-chloro-N-[2-(1H-indol-3-yl)ethyl]-7,8-dihydro-6H-pyrimido[5,4-b][1,4]oxazin-4-amine (0.18 g,0.488 mmol, 85.52% yield) as an off-white solid. The product was used in the next step without further purification.
UPLC-MS analysis (2 min basic): rt = 1.03 mins (M+H+ = 330.2/332.2), 91% purity
¹H NMR(400 MHz, DMSO-D6) δ 10.79 (s, 1H), 7.67 (d, J = 7.9 Hz, 1H), 7.33 (d, J = 7.9 Hz, 1H), 7.22 - 6.91 (m, 4H), 6.61 (s, 1H), 4.07 (s, 2H), 3.60 - 3.48 (m, 2H), 3.39 (s, 2H), 2.90 (t, J = 7.9 Hz, 2H),

### Example 1 2-(5-fluoro-3-pyridyl)-N-[2-(1H-indol-3-yl)ethyl]-7,8-dihydro-6H-pyrimido[5,4-b][1,4]oxazin-4-amine

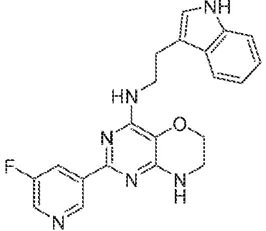

Prepared according to general method 5b, using 2-chloro-N-[2-(1H-indol-3-yl)ethyl]-1,8-dihydro-6H-pyrimldo[5,4-b][1,4]oxazin-4-amine (1.0 eq, 150 mg, 0.414 mmol) and 5-Fluoropyridine-3-boronic acid (2.0 eq, 117 mg, 0.828 mmol). The crude was purified by column chromatography (40 g column) eluting with a gradient of 90 to100% (v/v)

EtOAc in iso-hexane to afford 2-(5-fluoro-3-pyridyl)-N-[2-(1H-indol-3-yl)ethyl]-7,8-dihydro-6H-pyrimido[5,4-b][1,4]oxazin-4-amine (50 mg,0.128 mmol, 30.94% yield) as an off-white solid.

UPLC-MS analysis (4 min basic): rt = 1.79 mins (M+H+ = 391.3), 100% purity.

¹H NMR (400 MHz, DMSO-D6) δ 10.81 (s, 1H), 9.23 (t, J = 1.8 Hz, 1H), 8.59 (d, J = 2.9 Hz, 1H), 8.28 - 8.15 (m, 1H), 7.63 (dd, J = 7.9, 1.1 Hz, 1H), 7.34 (dt, J = 7.9, 0.9 Hz, 1H), 7.20 (d, J = 2.2 Hz, 1H), 7.14 - 7.03 (m, 1H), 7.03 - 6.95 (m, 1H), 6.92 (d. J = 2.6 Hz, 1H), 6.45 (t, J = 5.9 Hz, 1H), 4.25 - 4.08 (m, 2H), 3.80 - 3.62 (m, 2H), 3.45 (q, J = 3.8 Hz, 2H), 3.07 - 2.91 (m, 2H).

¹⁹F NMR (376 MHz, DMSO-D6) δ -128.14.

### Example 2 2-(4-fluorophenyl)-N-[2-(1H-indol-3-yl)ethyl]-7,8-dihydro-6H-pyrimido[5,4-b][1.4]oxazin-4-amine

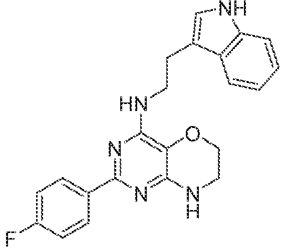

Prepared according to general method 5b, using 2-chloro-N-[2-(1H-indol-3-yl)ethyl]-7,8-dihydro-6H-pyrimido[5,4-b][1,4]oxazin-4-amine (65 mg, 0.18 mmol, 1.0 eq) and 4-fluorobenzenelboronic acid (50 mg, 0.359 mmol, 2.0 eq). The crude was purified column chromatography (20 g column) eluting with a gradient of 30 to 80% (v/v) EtOAc in iso-hexane to afford

2-(4-fluorophenyl)-N-[2-(1H-indol-3-yl)ethyl]-7,8-dihydro-6H-pyrimido[5,4-b][1,4]oxazin-4-amine (33 mg,0.0839 mmol, 46.77% yield) as a pale yellow solid.

UPLC-MS analysis (4 min basic): rt = 2.00 mins (M+H+ = 390.3, M-H- = 388.2), 99% purity.

¹H NMR (400 MHz, DMSO-D6) δ 10.81 (s, 1H), 8.38 - 8.11 (m, 2H), 7.63 (dt, J = 7.8, 1.0 Hz, 1H), 7.35 (dt, J = 8.1, 0.9 Hz, 1H), 7.33 - 7.12 (m, 3H), 7.14 - 7.03 (m, 1H), 7.03 - 6.94 (m, 1H), 6.84 - 6.67 (m, 1 H), 6.27 (t, J = 5.9 Hz, 1H), 4.24 - 4.06 (m, 2H), 3.83 - 3.63 (m, 2H), 3.43 (q, J = 3.7 Hz, 2H), 3.11 - 2.89 (m, 2H), ¹⁹F NMR (376 MHz, DMSO-D6) δ -113.65.

### Example 3 2-(3,5-difluorophenyl)-N-[2-(1H-indol-3-yl)ethyl])-7,8-dihydro-6H-pyrimido[5,4-b][1,4]oxazin-4-amine

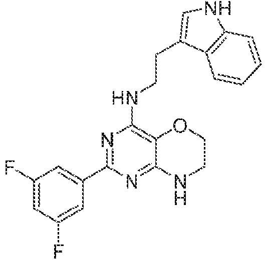

Prepared according to general method 5b, using 2-chloro-N-[2-(1H-indol-3-yl)ethyl]-7,8-dihydro-6H-pyrimido[5,4-b][1,4]oxazin-4-amine (65 mg, 0.18 mmol, 1.0 eq) and 3,5-difluorobenzeneboronic acid (57 mg, 0.36 mmol, 2.0 eq). The crude was purified by column chromatography (20 g cartridge) eluting with a gradient of 30 to 80% (v/v) EtOAc in iso-hexane, to afford

2-(3,5-difluorophenyl)-N-[2-(1H-indol-3-yl)ethyl]-7,8-dihydro-6H-pyrimido[5,4-b][1,4]oxazin-4-amine (9.0 mg,0.0221 mmol, 12.32% yield) as an off-white solid.

UPLC-MS analysis (4 min, basic): rt = 2.14 min, m/z = 408.2 [M+H]+, 100% purity.

¹H NMR (400 MHz, DMSO-D6) δ 10.82 (s, 1H), 7.95 - 7.76 (m, 2H), 7.65 (dd, J = 7.7, 1.1 Hz, 1H), 7.41 - 7.31 (m, 1H), 7.31 - 7.22 (m, 1H), 7.19 (d, J = 2.3 Hz, 1H), 7.12 - 7.04 (m, 1H), 7.02 - 6.93 (m, 1H), 6.93 - 6.84 (m, 1H), 6.42 (t, J = 5.9 Hz, 1H), 4.20 - 4.06 (m, 2H), 3.79 - 3.61 (m, 2H), 3.44 (q, J = 3.7 Hz, 2H), 3.03 - 2.88 (m, 2H). ¹⁹F NMR (376 MHz, DMSO-D6) δ -110.27.

### Intermediates for Example 4

### 4.1 tert-butyl N-[2-[2,4-dichloro-6-[[(3R)-2,3,4,9-tetrahydro-1H-carbazol-3-yl]amino] pyrimidin-5-yl]oxyethyl]carbamate

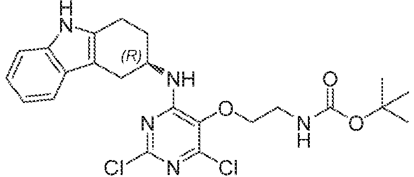

Prepared according to general method 2a, using tert-butyl N-[2-(2,4,6-trichloro pyrimidin-5-yl)oxyethyl]carbamate (0.34 g, 0.99 mmol, 1.0 eq) and (R)-2,3,4,9-Tetra hydro-1H-carbazol-3-amine (203 mg, 1.09 mmol, 1.10 eq) In ethanol (3 mL) at 50 °C.

The reaction was concentrated to dryness in vacuo to give an oily residue, which was dispersed in water (25 mL). The resulting suspension was filtered and the solid. was dried under vacuum to afford tert-butyl N-[2-[2,4-dichloro-6-[[(3R)-2,3.4,9-tetrahydro-1H-carbazol-3-yl]amino]pyrimidin-5-yl]oxyethyl]carbamate (0.33 g,0.693 mmol, 70.33% yield) as an off-white solid. This material was used directly in the next step with further purification.
UPLC-MS analysis (2 min, basic): rt = 1.30 min, m/z = 492.1/494.1/496.1 [M+H]+, 97% purity.
¹H NMR (400 MHz, DMSO-D6) δ 10.75 (s, 1H), 7.90 (d, J = 8.4 Hz, 1H), 7.34 (d, J = 7.8 Hz, 1H), 7.26 (d, J = 7.9 Hz, 1H), 7.17 (d, J = 6.8 Hz, 1H), 7.01 (t, J = 7.6 Hz, 1H], 6.93 (t, J = 7.4 Hz, 1H), 4.33 (s, 1H), 4.00 (d, J = 5.9 Hz, 2H), 3.19 - 2.96 (m, 2H), 2.88 (s, 2H), 2.78 - 2.63 (m, 1H), 2.13 (s, 1H), 2.08 - 1.96 (m, 1H), 1.39 (s, 9H), 1.18 (t, J = 7.4 Hz, 1H),

### 4.2 (3R)-N-[5-(2-aminoethoxy)-2,6-dichloro-pyrimidin-4-yl]-2,3,4,9-tetrahydro-1H-carbazol-3-amine;hydrochloride

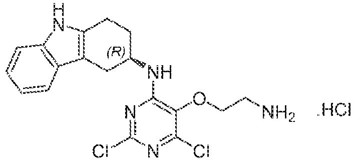

Prepared according to general method 3, using tert-butyl N-[2-[2,4-dichloro-6-[[(3R)-2,3,4,9-tetrahydro-1H-carbazol-3-yl]amino]pyrimidin-5-yl]oxyethyl] carbamate (500 mg, 0.99 mmol, 1.0 eq).

The crude product was dissolved in methanol (3 mL), and the resulting stirred solution was treated with diethyl ether (50 mL). The resulting suspension was filtered and the solid was washed with diethyl ether to afford (3R)-N-[5-(2-aminoethoxy)-2,6-dichloro-pyrimidin-4-yl]-2,3,4,9-tetrahydro-1H-carbazol-3-amine;hydrochloride (0.33 g,0.693 mmol, 70.33% yield) as an off-white solid. This material was used directly in the next step without any further purification.

UPLC-MS analysis (2 min, basic): rt = 1.17 min, m/z = 392.1/394.1/396.1 [M+H]+, 90% purity.

¹H NMR (400 MHz, DMSO-D6) δ 10.77 (s, 1H), 8.26 (s, 3H), 8.07 (d, J = 8.4 Hz, 1H), 7.33 (d, J = 7.7 Hz, 1H), 7.27 (d, J = 8.0 Hz, 1H), 7.01 (t, J = 7.5 Hz, 1H), 6.94 (t, J = 7.4 Hz, 1 H), 4.37 (d, J = 10.6 Hz, 1H), 4.19 (t, J = 5.3 Hz, 2H), 3.31 - 3.22 (m, 2H), 3.02 (dd, J = 14.8, 5.5 Hz, 1H), 2.98 - 2.70 (m, 3H), 2.22 - 2.00 (m, 2H).

### 4.3 2-chloro-N-[(3R)-2,3,4,9-tetrahydro-1H-carbazol-3-yl]-7,8-dihydro-6H-pyrimido[5,4-b][1,4]oxazin-4-amine

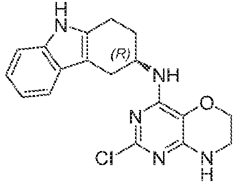

Prepared according to general method 4, using (3R)-N-[5-(2-aminoethoxy)-2,6-dichloro-pyrimidin-4-yl]-2,3,4,9- tetrahydro-1H-carbazol-3-amine;hydrochloride (130 mg, 0.27 mmol, 1.0 eq) to afford 2-chloro-N-[(3R)-1,3,4,9-tetrahydro-1H-carbazol-3-yl]-7,8-dibydro-6H-pyrimido[5,4-b][1,4]oxazin-4-amine(98 mg.0.273 mmol, 99.92% yield) as an off-white solid. This material was used directly in the next step without further purification. UPLC-MS analysis (2 min, basic): rt = 1.08 min, m/z = 356.2/358.2 [M+H]+, 99% purity.

¹H NMR (400 MHz, DMSO-D6) δ 10.70 (s,1H), 7.53 - 7.06 (m,3H), 7.10 - 6.80 (m,2H), 6.42 (d, J = 8.6 Hz, 1H), 4.27 (s,1H), 4.09 (s,2H), 3.40 (s,2H), 3.02 - 2.78 (m,3H), 2.67 - 2.59(m,1H), 2.00 (s,2H).

### Example 4 2-(5-fluoro-3-pyridyl)-N-[(3R)-2,3,4,9-tetrahydro-1H-carbazol-3-yl]-7,8-dihydro-6H-pyrimido[5,4-b][1,4]oxazin-4-amine

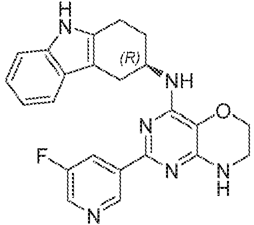

Prepared according to general method 5b, using 2-chloro-N-[(3R)-2,3,4,9-tetrahydro-1H-carbazol-3-yl]-7,8-dihydro-6H-pyrimido[5,4-b][1,4]oxazin-4-amine (95 mg, 0.26 mmol, 1.0 eq) and 5-fluoropyridine-3-boronic acid (74 mg, 0.53 mmol, 2.0 eq). The crude was purified by column chromatography (40 g cartridge) eluting with a gradient of 90 to 100% (v/v) EtOAc in iso-hexane to afford 2-(5-fluoro-3-pyridyl)-N-[(3R)-2,3,4,9-tetrahydro-1H-carbazol-3-yl]-7,8-dihydro-6H-pyrimido [5,4-b][1,4]oxazin-4-amine (48 mg,0.115 mmol, 43.61% yield) as an off-white solid. UPLC-MS analysis (4 min, basic): rt = 1.95 min, m/z = 417.2 [M+11]+, 100% purity.

¹H NMR (400 MHz, DMSO-D6) δ 10.73 (s, 1H), 9.18 (t, J = 1.7 Hz, 1H), 8.56 (d, J = 2.9 Hz, 1H), 8.27 - 8.11 (m, 1H), 7.33 (d, J = 7.7 Hz, 1H), 7.26 (dt, J = 8.0, 1.0 Hz, 1H), 7.08 - 6.85 (m, 3H), 6.17 (d, J = 8.3 Hz, 1H), 4.65 - 4.43 (m, 1H), 4.27 - 4.07 (m, 2H), 3.52 - 3.41 (m, 2H), 3.06 (dd, J = 14.9, 5.3 Hz, 1H), 3.01 - 2.88 (m, 1H), 2.88 - 2.78 (m, 1H), 2.74 - 2.63 (m, 1H), 2.20 - 2.10 (m, 1H), 2.07 - 1.92 (m, 1H). ¹⁹F NMR (376 MHz, DMSO-D6) 6 -128.07.

### Intermediates for Example 5

### 5.1 tert-butyl N-[1-methyl-2-(2,4,6-trichloropyrimidin-5-yl)oxy-ethyl]carbamate

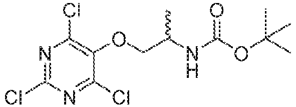

Prepared according to general method 1, using 2,4,6-tri chloropyrimidin-5-ol (0.30 g, 1.43 mmol, 1.0 eq) and N-Boc-2-amino-1-propanol (0.24 mL, 1.57 mmol, 1.10 eq).

The reaction was concentrated to dryness and the residue was purified by automated flash column chromatography over silica (40 g cartridge), eluting with 18-20% EtOAc In iso-hexane, to afford tert-butyl N-[1-methyl-2-(2,4,6-trichloropyrimidin-5-yl)oxy-ethyl]carbamate (208 mg,0.583 mmol, 40.81% yield) as a white solid. UPLC-MS analysis (2 min, basic): rt = 1.26 min, no ionisation, 100% purity. ¹H NMR (400 MHz, DMSO) δ 6.92 (d, J = 7.9 Hz, 1H), 4.01 (q, J = 5.0 Hz, 2H), 3.86 (dd, J = 13.8,7.2 Hz, 1H), 1.38 (s, 9H), 1.18 (dd, J = 9.2, 6.8 Hz, 3H)

### 5.2 tert-butyl N-[2-[2,4-dichloro-6-[2-(1H-indol-3-yl)ethylamino]pyrimidin-5-yl]oxy-1-methyl-ethyl]carbamate

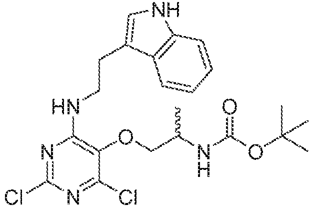

Prepared according to general method 2a, using tert-butyl N-[1-methyl-2-(2,4,6-trichloropyrimidin-5-yl)oxyethyl]carbamate (0.21 g, 0.58 mmol, 1.0 eq) and tryptamine (0.098 g, 0.61 mmol, 1.05 eq). The reaction was concentrated to dryness to give an oily residue, which was suspended in water (25 mL) with stirring.

The resultant suspension was filtered to afford tert-butyl N-[2-[2,4-dichloro-6-[2-(1H-indol-3-yl) ethylamino]pyrimidin-5-yl]oxy-1-methyl-ethyl]carbamate (267 mg,0.478 mmol, 81.95% yield) as an off-white solid. This material was used directly in the next step without further purification. UPLC-MS analysis (2 min, basic): rt = 1.30 min, m/z = 480.2 [M+H]+, 86% purity.

¹H NMR (400 MHz, DMSO-D6) δ 10.87 - 10.82 (m, 1H), 8.01 (s, 1H), 7.63 (d, J = 7.8 Hz, 1H), 7.35 (dt, J = 8.1, 1.0 Hz, 1H), 7.19 (d, J = 2.4 Hz, 1H), 7.12 - 6.94 (m, 3H), 3.87 (d, J = 7.9 Hz, 1H), 3.72 (dt, J = 9.5, 4.8 Hz, 1H), 3.68 - 3.58 (m, 2H), 3.00 (t, J = 7.7 Hz, 2H), 1.40 (s, 8H), 1.17 (t, J = 7.2 Hz, 2H), 1.07 (d, J = 6.0 Hz, 3H).

### 5.3 5-(2-antinopropoxy)-2,6-dichloro-N-[2-(1H-Indol-3-yl)ethyl]pyrimidin-4-amine; HCl

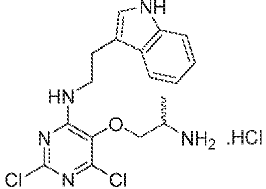

Prepared according to general method 3, using tert-butyl N-[2-[2,4-dichloro-6-[2-(3 H-indol-3-yl)ethylamino] pyrimidin-5-yl]oxy-1-methyl-ethyl]carbamate (0.27 g, 0.56 mmol, 1.0 eq to afford5-(2-aminopropoxy)-2,6-di chloro-N-[2-(1H-indol-3-yl)ethyl]pyrimidin-4-amine; hydrochloride (0.26 g,0.530 mmol, 95.42% yield) as an off-white solid.

This material was used directly in the next step without further purification.

UPLC-MS analysis (2 min, basic): rt = 1.09 min, m/z = 380.2/382.2/384.1 [M+H]+, 85% purity.

¹H NMR (400 MHz, DMSO-D6) δ 10.86 (s, 1H), 8.32 (t, J = 5.7 Hz, 3H), 7.67 (d, J = 7.8 Hz, 1H), 7.39 - 7.28 (m, 1H), 7.21 (d, J = 2.3 Hz, 1H), 7.12 - 7.05 (m, 1H), 7.04 - 6.94 (m, 1H), 4.07 - 3.95 (m, 2H), 3.64 (q, J = 6.7 Hz, 3H), 3.03 - 2.95 (m, 2H), 1.26 (d, J = 6.8 Hz, 3H).

### 5.4 2-chloro-N-[2-(1H-indol-3-yl)ethyl])-7-methyl-7,8-dihydro-6H-pyrimido[5,4-b][1,4]oxazin-4-amine

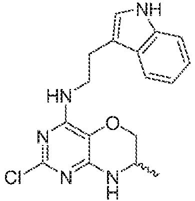

Prepared according to general method 4, using 5-(2-aminopropoxy)-2,6-dichloro-N-[2-(1H-indol-3-yl)ethyl]pyrimidin-4-amine;HCl (0.20 g, 0.48 mmol, 1.0 eq) to afford 2-chloro-N-[2-(1H-indol-3-yl)ethyl]-7-methyl-7,8-dihydro-6H-pyrimido[5,4-b][1,4] oxazin-4-amine (0.17 g,0.500 mmol, 83.4% yield) as an off-white solid. This material was used directly in the next step without further purification.

UPLC-MS analysis (2 min, basic): rt = 1.09 min, m/z = 344.2/346.1 [M+H]+. 100% purity.

¹H NMR (400 MHz, DMSO-D6) δ 10.79 (s, 1H), 7.67 (d, J = 7.9 Hz, 1H), 7.33 (d, J = 8.0 Hz, 1H), 7.26 - 7.14 (m, 2H), 7.07 (t, J = 7.5 Hz, 1H), 6.98 (t, J = 7.4 Hz, 1H), 6.64 (t, J = 5.9 Hz, 1H), 4.19 - 4.03 (m, 1H), 3.74 - 3.41 (m, 4H), 2.90 (t, J = 7.7 Hz, 2H), 1.11 (d, J = 6.3 Hz, 3H).

### Example 5 2-(5-fluoro-3-pyridyl)-N-[2-(1H-indol-3-yl)ethyl]-7-methyl-7,8-dihydro-6H-pyrimido[5,4-b][1,4]oxazin-4-amine

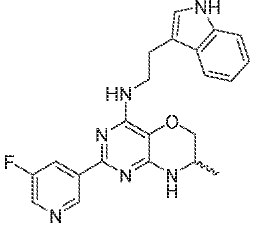

Prepared according to general method 5b, using 2-chloro-N-[2-(1H-indol-3-yl)ethyl]-7-methyl-7,8-dihydro-6H-pyrimido[5,4-b][1,4]oxazin-4-amine (80 mg, 0.23 mmol, 1.0 eq) and 5-fluoropyridine-3-boronic acid (66 mg, 0.47 mmol, 2.0 eq). The crude was purified by column chromatography (20 g cartridge) eluting with a gradient of30 to 70% (v/v)

EtOAc in iso-hexane to afford 2-(5-fluoro-3-pyridyl)-N-[2-(3 H-indol-3-yl)ethyl]- 7-methyl- 7.8-dihydro-6H-pyrimido[5,4-b][1,4]oxazin-4-amine (55 mg,0.136 mmol, 58.44% yield) as an off-white solid. UPLC-MS analysis (4 min, basic): rt = 1.91 min, m/z = 405.2 [M+H]+, 100% purity.

¹H NMR (400 MHz, DMSO-D6) δ 10.81 (s, 1 H), 9.23 (t, J = 1.7 Hz, 1H), 8.59 (d, J = 2.9 Hz, 1H), 8.31 - 8.14 (m, 1H), 7.72 -7.59 (m, 1H), 7.39 - 7.29 (m, 1H), 7.20 (d, J = 2.3 Hz, 1H), 7.11 - 7.04 (m, 1H), 7.04 - 6.93 (m, 2H), 6.48 (t, J = 6.0 Hz, 1H), 4.26 - 4.11 (m, 1H), 3.81 - 3.65 (m, 3H), 3.61 (tt, J = 6.9, 5.5, 3.9 Hz, 1H), 2.99 (dd, J = 8.8, 6.4 Hz, 2H), 1.17 (d, J = 6.4 Hz, 3H).

¹⁹F NMR (376 MHz, DMSO-D6) δ -128.1.2.

Example 5 (51 mg, 0.125 mmol) was submitted to chiral separation to provide two enantiomers

### Example 8 and Example 9.

### Intermediates for Example 6

### 6.1 N-(2-(1H-indol-3-yl)ethyl)-2,6-dichloro-5-(2-(isopropylamino)ethoxy)pyrimidin-4-amine

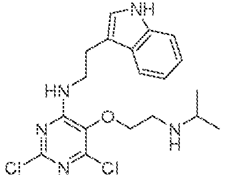

A stirred solution of 5-(2-aminoethoxy)-2,6-dichloro-N-[2-(1H-indol-3-yl)ethyl]pyrimidin-4-amine hydrochloride (134 mg, 0.33 mmol, 1.0 eq), triethylamine (0.046 mL, 0.33 mmol, 1.0 eq), acetic acid (0.038 mL, 0.67 mmol, 2.0 eq) and acetone (0.074 mL, 0.99 mmol, 3.0 eq) in 1.2,dichloroethane (10 mL) was treated with sodium triacetoxyborohydride (106 mg, 0.49 mmol, 1.5 eq), and the resulting mixture was stirred for 4 h. The reaction was quenched by the addition of methanol (1 mL), and the resulting mixture was evaporated to dryness in vacuo. The residue was partitioned between EtOAc (10 mL) and water (10 mL). The organic phase was collected and washed with water (3 x 10 mL) before being dried (Na₂SO₄), filtered and evaporated to dryness to afford 2,6-dichloro-N-[2-(1H-indol-3-yl)ethyl]-5-[2-(isopropylamino)ethoxy]pyrimidin-4-amine (160 mg, 0.314 mmol, 94.45% yield) as a beige oil. This material was used directly in the next step with further purification. UPLC-MS analysis (2 min, basic): rt = 1.28 min, m/z = 408.2/410.2/412.2 [M+H]+. 97% purity. ¹H NMR (400 MHz, DMSO-D6) δ 10.85 (s, 1H), 9.18 (s, 1H), 7.61 (d, J = 7.8 Hz, 1H), 7.35 (dt, J = 8.2, 0.9 Hz, 1H), 7.18 (d, J = 2.3 Hz, 1H), 7.08 (ddd, J = 8.1, 7.0, 1.2 Hz, 1H), 6.99 (ddd, J = 8.0, 6.9, 1.1 Hz, 1H), 3.89 (t, J = 4.9 Hz, 2H), 3.62 (t, J = 7.4 Hz, 2H), 2.98 (t, J = 7.4 Hz, 2H), 2.77 (d, J = 26.7 Hz, 3H), 0.96 (d, J = 6.3 Hz, 6H).

### 6.2 2-chloro-N-[2-(1H-indol-3-yl)ethyl]-8-isopropyl-6,7-dihydropyrimido[5,4-b][1,4]oxazin-4-amine

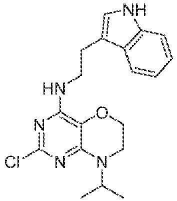

Prepared according to general method 4, using 2,6-dichloro-N-[2-(1H-indol-3-yl)ethyl]-5-[2-(isopropylamino)ethoxy]pyrimidin-4-amine (160 mg, 0.31 mmol, 1.0 eq). The reaction was partitioned between EtOAc (10 mL) and water (10 mL). The aqueous phase was extracted with EtOAc (2 x 10 mL) and the combined organics were dried (Na₂SO₄), filtered and evaporated to dryness to afford 2-chloro-N-[2-(1H-indol-3-yl)ethyl]-8-isopropyl-6,7-dihydropyrimido[5,4-b][1,4]oxazin-4-amine (136 mg,0.344 mmol. 109.47% yield) as a brown oil. This material was used directly in the next step with further purification.

UPLC-MS analysis (basic, 2 min): rt = 1.25 min, m/z = 372.2/374.2 [M+H]+, 94% purity.

¹H NMR (400 MHz, DMSO-D6) δ 10.79 (s, 1H), 7.67 (dt, J = 7.8, 1.0 Hz, 1H), 7.33 (dt, J = 8.1, 1.0 Hz, 1H), 7.16 (d, J = 2.2 Hz, 1H), 7.07 (ddd, J = 8.1. 7.0.1.2 Hz, 1H), 6.98 (ddd, J = 8.0. 7.0.1.1 Hz, 1H), 6.56 (t, J = 5.9 Hz, 1H), 4.73 (hept, J = 6.8 Hz, 1H). 4.09 (dd, J = 5.0, 3.7 Hz, 2H), 3.57 - 3.49 (m, 2H), 3.36 (t, J = 4.4 Hz, 2H), 2.90 (dd, J = 8.8, 6.5 Hz, 2H), 1.10 (d, J = 6.8 Hz, 6H).

### Example 6 2-(5-fluoro-3-pyridyl)-N-[2-(1H-indol-3-yl)ethyl]-8-isopropyl-6,7-dihydropyrimido[5.4-b][1,4]oxazin-4-amine

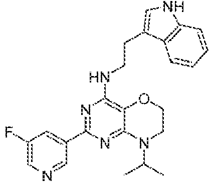

Prepared according to general method 5b, using 2-chloro-N-[2-(1H-indol-3-yl)ethyl]-8-isopropyl-6,7-dihydropyrimido[5,4-b][1.4]oxazin-4-amine (145 mg, 0.37 mmol, 1.0 eq) and 5-fluoro pyridine-3-boronic acid (103 mg, 0.73 mmol, 2.0 eq). The crude was purified by column chromatography (40 g cartridge) eluting with a gradient of 20 to 40% (v/v) EtOAc in iso-hexane to afford 2-(5-fluoro-3-pyridyl)-N-[2-(1H-indol-3-yl)ethyl]-8-tsopropyl-6,7-dihydropyrimido[5,4-b][3,4]oxazin-4-amine (85 mg,0.190 mmol, 51.93% yield) as a beige solid. UPLC-MS analysis (basic, 4 min): rt = 2.29 min, m/z = 433.3 [M+H]+, 97% purity. ¹H NMR (400 MHz, DMSO-D6) δ 10.81 (s, 1H), 9.28 (s, 1H), 8.60 (d, J = 3.0 Hz, 1H), 8.27 (d, J = 10.1 Hz, 1H), 7.63 (d, J = 7.9 Hz, 1H), 7.34 (d. J = 8.1 Hz, 1H), 7.20 (s. 1H), 7.07 (t, J = 7.5 Hz, 1H), 6.97 (t, J = 7.4 Hz, 1H), 6.39 (t, J = 6.0 Hz, 1H), 5.07 (p, J = 6.7 Hz, 1H), 4.18 (t, J = 4.3 Hz, 2H), 3.72 (q, J = 7.1 Hz, 2H), 3.42 (t, J = 4.3 Hz, 2H), 2.98 (t, J = 7.6 Hz, 2H), 1.17 (d, J = 6.7 Hz, 6H). ¹⁹F NMR (376 MHz, DMSO-D6) δ -128.13.

### Example 7 N-[2-(1H-indol-3-yl) ethyl]-2-(2-methylthiazol-5-yl)-7,8-dihydro-6H-pyrimido[5.4-b][1,4]oxazin-4-amine

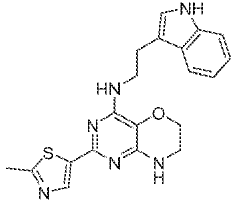

Prepared according to general method 5b, using 2-chloro-N-[2-(1H-indol-3-yl) ethyl]-7,8-dihydro-6H-pyrimido[5,4-b][1,4]oxazin-4-amine (1.00 eq, 200 mg, 0.606 mmol) and 2-methyl-5-(tetramethyl-1,3,2-dioxaborolan-2-yl)-1,3-thiazole (1.50 eq, 205 mg, 0.910 mmol). The crude was purified by column chromatography (40g cartridge) eluting with EtOAc/DCM 70%-90% gradient to afford N-[2-(1H-indol-3-yl)ethyl]-2-(2-methylthlazol-5-yl)-7,8-dihydro-6H-pyrimido[5,4-b][1,4]oxazin-4-amine (98 mg,0.239 mmol, 39.39% yield) as a brown solid.

UPLC-MS analysis (basic, 4min): rt =1.70 min, m/z = 393.0 [M+H]+, 96% purity.

¹H NMR (400 MHz, DMSO-D6) δ 10.81 (s, 1H), 8.02 (s, 1H), 7.66 (d, J = 8.0 Hz, 1H), 7.35 (dt, J = 8.1, 1.0 Hz, 1H), 7.19 (d, J = 2.3 Hz, 1H), 7.08 (m, 1H), 7.00 (m, 1H), 6.87 (s, 1H), 6.36 (t, J = 5.9 Hz, 1H), 4.12 - 4.08 (m, 2H), 3.63 (q, J = 6.8 Hz, 2H), 3.41 (t, J = 3.8 Hz, 2H), 2.99 - 2.92 (m, 2H), 2.64 (s, 3H).

### Example 8 (75)-2-(5-fluoro-3-pyridyl)-N-[2-(1H-indol-3-yl)ethyl]-7-methyl-7,8-dihydro-6H-pyrimido[5,4-b][1,4]oxazin-4-amine

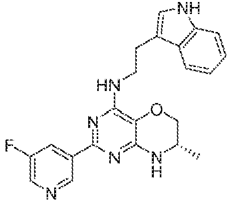

Title compound (17 mg, 0.0420 mmol, 33.66% yield)
Chemical purity: 99.94%, m/z = 405.3
Enantiomeric excess: 98.6
UPLC-MS analysis (6 min, basic, EtOH, 30% NH3): rt = 2.99 min, m/z = 405.3 [M+H]+, 100% purity.
19F NMR (376 MHz, DMSO-D6) δ -128.12

1H NMR (400 MHz, DMSO-D6) δ 10.81 (s, 1H), 9.23 (t, J = 1.7 Hz, 1H), 8.59 (d, J = 2.9 Hz, 1H), 8.22 (m, 1H), 7.63 (d, J = 7.9 Hz, 1H), 7.34 (dt, J = 8.0, 0.9 Hz, 1H), 7.20 (d, J = 2.3 Hz, 1H), 7.07 (m, 1H), 7.02 - 6.93 (m, 2H), 6.48 (t, J = 5.9 Hz, 1H), 4.23 - 4.15 (m, 1H), 3.70 (dd, J = 10.4, 6.8 Hz. 3H), 3.61 (s, 1H), 3.03 - 2.95 (m, 2H), 1.16 (d, J = 6.4 Hz, 3H).

### Example 9 (7R)-2-(5-fluoro-3-pyridyl)-N-[2-(1H-indol-3-yl)ethyl]-7-methyl-7,8-dihydro-6H-pyrimido[5,4-b][1,4]oxazin-4-amine

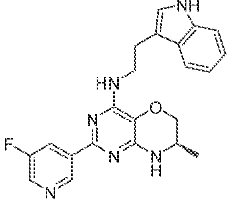

Title compound (17 mg, 0.0420 mmol, 33.66% yield)
Chemical purity: 99.93%, m/z = 405.3
Enantiomeric excess: 95.8
19F NMR (376 MHz, DMSO-D6) δ -128.12.

UPLC-MS analysis (6 min, basic, EtOH, 30% NH3): rt = 3.36 min, m/z = 405.3 [M+H]+, 100% purity. ¹H NMR (400 MHz, DMSO-D6) δ 10.81 (s, 1H), 9.23 (t, J = 1.7 Hz, 1H), 8.59 (d, J = 2.9 Hz, 1H), 8.22 (m, 1H), 7.63 (d, J = 7.8 Hz, 1H), 7.34 (dt, J = 8.2. 0.9 Hz, 1H), 7.20 (d. J = 2.3 Hz, 1H), 7.07 (m, 1H), 7.02 - 6.93 (m, 2H), 6.48 (t, J = 5.9 Hz, 1H), 4.23 - 4.15 (m, 1H), 3.70 (dd, J = 10.4, 6.9 Hz, 3H), 3.61 (d, J = 7.0 Hz, 1H), 3.03 - 2.95 (m, 2H), 1.16 (d, J = 6.4 Hz, 3H).

### Example 10 N-[2-(1H-indol-3-yl)ethyl]-2-(4methylthiazol-5-yl)-7,8-dihydro-6H-pyrimido[5.4-b][1,4]oxazin-4-amine

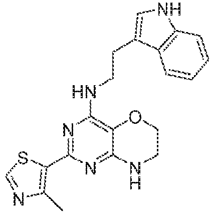

Prepared according to general method 5b, using 2-chloro-N-[2-(1H-indol-3-yl)ethyl]-7,8-dihydro-6H-pyrimido[5,4-b][1,4]oxazin-4-amine (1.00 eq, 95 mg, 0.289 mmol) and 4-Methyl-5-(4,4,5,5-tetramethyl-1,3,2-dioxaborolan-2-yl)-1,3-thiazole (1.50 eq, 98 mg, 0.434 mmol).

The crude was purified by column chromatography (20 g cartridge) eluting with a gradient of EtOAc (0% to 100%) in iso-hexane to afford N-[2-(1H-indol-3-yl)ethyl]-2-(4-methylthiazol-5-yl)-7,8-dihydro-6H-pyrimido[5,4-b][1,4]oxazin-4-amine (70 mg,0.177 mmol, 61.06% yield) as a beige solid. UPLC-MS analysis (4 min, basic): rt = 1.71 min, m/z = 393.3 [M+H]+, 99% purity.

1H NMR (400 MHz, DMSO-D6) δ 10.80 (s, 1H), 8.85 (s, 1H), 7.58 (d, J = 8.0 Hz, 1H), 7.34 (dt, J = 8.0, 0.9 Hz, 1H), 7.16 (d, J = 2.3 Hz, 1H), 7.07 (m, 1H), 6.98 (m, 1H), 6.82 (s, 1H), 6.35 (t, J = 5.9 Hz, 1H), 4.11 (t, J = 4.3 Hz, 2H), 3.70 - 3.60 (m, 2H), 3.41 (d, J = 4.0 Hz, 2H), 3.02 - 2.93 (m, 2H), 2.79 (s, 3H).

### Intermediates for Example 11

### 11.1 tert-butyl N-[2-[2,4-dichloro-6-[2-hydroxyethyl-[2-(1H-indol-3-yl)ethyl]amino) pyrimidin-5-yl]oxyethyl)carbamate

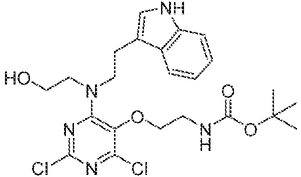

Prepared according to general method 2a, using tert-butyl N-[2-(2,4,6-trichloropyrimidin-5-yl)oxyethyl] carbamate (1.00 eq, 0.40 g, 1.17 mmol) and 2-{[2-(1H-indol-3-yl)ethyl]amino}ethanol (1.29 eq, 0.31 g, 1.50 mmol) to afford tert-butyl N-[2-[2,4-dichloro-6-[2-hydroxyethyl-[2-(1H-indol-3-yl)ethyl]amino]pyrimidin-5-yl]oxyethyl]carbamate (707 mg,1.09 mmol, 93.70% yield) as a white solid.

UPLC-MS analysis (basic, 2min): rt = 1.16 min, m/z = 510.2/512.2/514.2 [M+H]+, 79% purity.

1H NMR (400 MHz, DMSO-D6) δ 10.88 - 10.78 (m, 1H), 7.67 (d, J = 7.8 Hz, 1H), 1.40 - 7.27 (m, 1H), 7.17 (d, J = 2.3 Hz, 1H), 7.13 - 7.05 (m, 1H), 7.05 - 6.93 (m, 2H), 4.81 (t, J = 5.4 Hz, 1H), 3.87 (t, J = 7.8 Hz, 2H), 3.81 (t, J = 5.7 Hz, 2H), 3.70 (d, J = 5.6 Hz, 2H), 3.58 (q, J = 5.6 Hz, 2H), 3.24 (q, J = 5.7 Hz, 2H), 3.07 (t, J = 7.1 Hz, 1H), 2.99 (dd, J = 9.0, 6.6 Hz, 2H), 1.38 (s, 9H).

### 11.2 2-[[5-(2-aminoethoxy)-2,6-dichloro-pyrimidin-4-yl]-[2-(1H-indol-3-yl)ethyl]amino]ethanol;hydrochloride

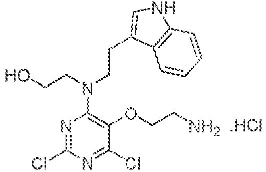

Prepared according to general method 3, using tert-butyl N-[2-[2,4-dichloro-6-[2-hydroxyethyl-[2-(1H-indol-3-yl) ethyl] amino]pyrimidin-5-yl]oxyethyl]carbamate (1.00 eq, 707 mg, 1.09 mmol) to afford 2-[[5-(2-aminoethoxy)-2,6-di chloro-pyrimidin-4-yl]-[2-(1H-indol-3-yl)ethyl]amino]ethanol; hydrochloride (568 mg, 1.27 mmol, 116.26% crude yield) as a yellow solid.

UPLC-MS analysis (basic, 2min): rt = 0.80 min, m/z = 410.3/412.3/414.3 [M+H]+, 100% purity. ¹H NMR (400 MHz, DMSO-D6) δ 10.87 (d, J = 2.3 Hz, 1H), 8.20 (s, 3H), 7.68 (d, J = 7.7 Hz, 1H), 7.34 (dt, J = 8.1, 1.0 Hz, 1H), 7.18 (d, J = 2.4 Hz, 1H), 7.07 (m, 1H), 6.99 (m, 1H), 4.03 (t, J = 5.4 Hz, 2H), 3.85 (t, J = 7.9 Hz, 4H), 3.60 (t, J = 5.7 Hz, 4H), 3.18 - 3.13 (m, 2H), 3.04 - 2.93 (m, 2H), 1.19 (t, J = 7.3 Hz, 1H).

### 11.3 2-[(2-chloro-7,8-dihydro-6H-pyrimido[54-b][1,4]oxazin-4-yl)-[2-(1H-indol-3-yl)ethyl]amino]ethanol

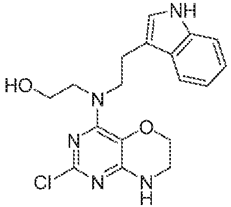

Prepared according to general method 4, using 2-[[5-(2-aminoethoxy)-2,6-dichloro-pyrimidin-4-yl]-[2-(1H-indol-3-yl)ethyl]amino]ethanol;hydrochloride (1.00 eq, 150 mg, 0.336 mmol) to afford2-[(2-chloro-7,8-dihydro-6H-pyrimido[5,4-b][1,4]oxazin-4-yl)-[2-(1H-indol-3-yl)ethyl]aminolethanol (110 mg,0.246 mmol, 73.35% yield) as a beige solid.

UPLC-MS analysis (basic, 2min): rt = 0.97min, m/z = 374.3 / 376.3 [M+H]+, 84% purity.

¹H NMR (400 MHz, DMSO-D6) δ 10.81 (s, 1H), 7.68 (d, J = 7.8 Hz, 1H), 7.39 - 7.28 (m, 2H), 7.12 (d, J = 2.3 Hz, 1H), 7.06 (ddd, J = 8.1, 7.0, 1.2 Hz, 1H), 6.98 (ddd, J = 8.0, 6.9, 1.1 Hz, 1H), 4.71 - 4.64 (m, 1H), 4.01 (t, J = 4.4 Hz, 2H), 3.81 - 3.66 (m, 2H), 3.56 (q, J = 4.6 Hz, 4H), 3.39 (q, J = 4.0 Hz, 2H), 3.00 - 2.85 (m, 2H).

### Example 11 2-(2-(1H-indol-3-yl)ethyl-[2-(2-methylthiazol-5-yl)-7,8-dihydro-6H-pyrimido[5,4-b][1,4]oxazin-4-yl]amino]ethanol

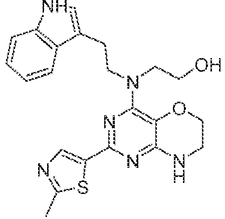

Prepared according to general method 5b, using 2-[(2-chloro-7,8-dihydro-6H-pyrimido[5,4-b][1,4]oxazin-4-yl)-[2-(1H-indol-3-yl)ethyl]amino]ethanol (1 eq, 110 mg, 0.246 mmol) and 2-methyl-5-(tetramethyl-1,3,2-dioxaborolan-2-yl)-1,3-thiazole (1.50 eq, 76 mg, 0.339 mmol). The crude was purified by column chromatography (24g cartridge) eluting with MeOH/EtOAc 0%-10% gradient to afford 2-[2-(1H-indol-3-yl)ethyl-[2-(2-methylthiazol-5-yl)-7,8-dihydro-6H-pyrimido[5,4-b][1,4]oxazin-4-yl]amino]ethanol (38 mg,0.0876 mmol, 38.72% yield) as an orange solid. UPLC-MS analysis (basic, 4min): rt =1.63 min, m/z = 437.1 [M+H]+, 100% purity.

1H NMR (400 MHz, DMSO-D6) δ 10.84 (d, J = 2.3 Hz, 1H), 7.99 (s, 1H), 7.59 (d, J = 7.8 Hz, 1H), 7.34 (dt, J = 8.2, 1.0 Hz, 1H), 7.16 (d. J = 2.3 Hz, 1H), 7.13 - 7.04 (m, 2H), 6.99 (m, 1H), 4.74 - 4.68 (m, 1H), 4.05 (t, J = 4.3 Hz, 2H), 3.84 - 3.76 (m, 2H), 3.62 (q, J = 5.0, 4.1 Hz, 4H), 3.42 (d, J = 4.1 Hz, 2H), 2.99 (dd, J = 9.7, 6.0 Hz, 2H), 2.64 (s, 3H).

### Example 12 2-(2-ethyl-4-methyl-thiazol-5-yl)-N-[2-(1H-ladol-3-yl)ethyl]-7,8-dihydro-6H-pyrimido|5,4-b][1,4]oxazin-4-amine

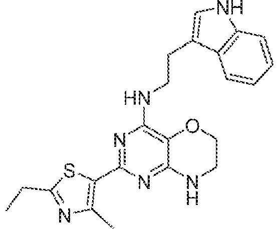

Prepared according to general method 5b, using 2-chloro-N-[2-(1H-indol-3-yl)ethyl]-7,8-dihydro-6H-pyrimido[5.4-b][1,4]oxazin-4-amine (73 mg, 0.220 mmol, 1eq) and 2-Ethyl-4-methyl-5-(4,4,5,5-tetramethyl-1,3,2-dioxaborolan-2-yl)thiazole (1.50 eq, 84 mg, 0.331 mmol). The crude was purified by column chromatography (24g cartridge) eluting with EtOAc/Hex 70%-90% gradient to afford 2-(2-ethyl-4-methyl-thiazol-5-yl)-N-[2-(1H-indol-3-yl)ethyl]-7,8-dihydro-6H-pyrimido[5,4-b][1,4]oxazin-4-amine (68 mg,0.163 mmol, 73.79% yield) as an off-white solid.

UPLC-MS analysis (basic, 4min): rt =1.96 min, m/z = 421.1 [M+H]+, 100% purity.

1H NMR (400 MHz, DMSO-D6) δ 10.82 (s, 1H), 7.59 (d, J = 7.8 Hz, 1H), 7.33 (dt, J = 8.1, 0.9 Hz, 1H), 7.15 (d, J = 2.2 Hz, 1H), 7.07 (m, 1H), 6.97 (m, 1H), 6.78 (d, J = 2.6 Hz, 1H), 6.34 (t, J = 5.9 Hz, 1H), 4.09 (t, J = 4.2 Hz, 2H), 3.62 (q, J = 6.7 Hz, 2H), 3.39 (d, J = 4.7 Hz, 2H), 2.95 (t, J = 7.7 Hz, 2H), 2.88 (q, J = 7.5 Hz, 2H), 2.71 (s, 3H), 1.28 (t, J = 7.5 Hz, 3H).

### Example 13 N-[(3R)-2,3,4,9-tetrahydro-1H-carbazol-3-yl]-2-thiazol-2-yl-7,8-dihydro-6H-pyrimido[5,4-b][1,4]oxazin-4-amine

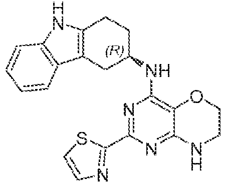

Prepared according to general method 5c, using 2-chloro-N-[(3R)-2,3,4,9-tetrahydro-2 H-carbazol-3-yl]-7,8-dthydro-6H-pyrimido[5,4-b][1,4]oxazin-4-amine (1.00 eq, 107 mg, 0.301 mmol) and 2-(Tri-n-butylstannyl)thiazole (1.10 eq, 0.10 mL, 0.331 mmol).

The crude was purified by column chromatography (12 g cartridge) eluting with a gradient of EtOAc (60% to 100%) in hexane to afford N-[(3R)-2,3,4,9-tetrahydro-1H-carbazol-3-yl]-2-thlazol-2-yl-7,8-dihydro-6H-pyrimido|5,4-b][1,4|oxazin-4-amine (23 mg,0.0579 mmol, 19.25% yield) as a beige solid. UPLC-MS analysis (4 min, basic): rt = 1.84 min, m/z = 405.1 [M+H]+, 100% purity.

1H NMR (400 MHz, DMSO) δ 10.71 (s, 1H), 7.83 (d, J = 3.2 Hz, 1H), 7.67 (d. J = 3.2 Hz, 1H), 7.33 (d, J = 7.7 Hz, 1H), 7.26 (dt, J = 8.1, 0.9 Hz, 1H), 7.06 (d, J = 2.6 Hz, 1H), 7.00 (m, 1H), 6.92 (m, 1H), 6.21 (d, J = 8.3 Hz, 1H), 4.46 (s, 1H), 4.17 (t, J = 4.4 Hz, 2H), 3.45 (d, J = 4.1 Hz, 2H), 3.05 (m, 1H), 2.92 - 2.82 (m, 2H), 2.71 - 2.65 (m, 1H), 2.20 - 1.88 (m, 2H).

### Example 14

### 14.1 tert-butyl N-[2-[2,4-dichloro-6-(1,3,4,5-tetrahydrobenzo[cd]indol-4-ylamino) pyrimidin-5-yl]oxyethyl]carbamate

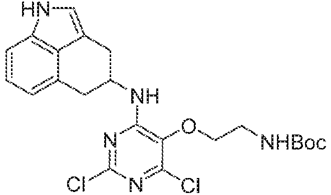

Prepared according to general method 2a, using tert-butyl N-[2-(2,4,6-trichloropyrimidin-5-yl)oxyethyl)carbamate (1.00 eq, 163 mg, 0.477 mmol), 1,3,4,5-tetrahydrobenzo[cd]indol-4-amine (1.29 eq, 106 mg, 0.614 mmol) in Ethanol (2.1679 mL) to afford tert-butyl N-[2-[2,4-dichloro-6-(1,3,4,5-tetrahydrobenzo[cd]indol-4-ylamino)pyrimidin-5-yl]oxyethyl]carbamate (239 mg,0.470 mmol, 98.55% yield) as a beige solid.

UPLC-MS analysis (2 min, basic): rt = 1.26 min, m/z = 478.3/480.3/482.3 [M+H]+, 94% purity.

1H NMR (400 MHz, DMSO-D6) δ 10.71 (d, J = 2.2 Hz, 1H), 7.92 (d, J = 8.6 Hz, 1H), 7.21 - 7.08 (m, 2H), 7.07 - 6.95 (m, 3H), 6.74 (d, J = 7.2 Hz, 1H), 4.42 (dd, J = 9.8, 5.1 Hz, 1H), 4.01 (t, J = 5.3 Hz, 2H), 3.18 - 3.01 (m, 4H), 2.98 - 2.88 (m, 1H), 1.39 (s, 9H).

### 14.2 N-[5-(2-aminoethoxy)-2,6-dichloro-pyrimidin-4-yl]-1,3,4,5-tetrahydrobenzo [cd]indol-4-amine;hydrochloride

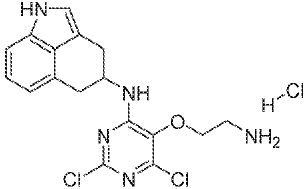

Prepared according to general method 3, using N-[2-[2,4-dichloro-6-(1,3,4,5-tetrahydrobenzo[cd]indol-4-ylamino)pyrimidin-5-yl]oxyethyl]carbamate (1.00 eq, 239 mg, 0.470 mmol) to afford N-[5-(2-aminoethoxy)-2,6-di chloro-pyrimidin-4-yl]- 1,3,4,5-tetrahydro benzo[cd]indol-4-amine;hydrochloride (204 mg,0.493 mmol, 83.85% yield)as
a grey solid. UPLC-MS analysis (2 min, basic): rt = 1.11 min, m/z = 378.1, 381.1, 383.1 [M+H]+, 75% purity. 1H NMR (400 MHz, DMSO-D6) δ 10.72 (d, J = 2.1 Hz, 1H), 8.35 (s, 3H), 8.24 (t, J = 9.4 Hz, 1H), 7.16 (d, J = 8.3 Hz, 1H), 7.04 - 6.95 (m, 2H), 6.74 (d, J = 6.9 Hz, 1H), 4.45 (m, 1H), 4.24 - 4.17 (m, 2H), 3.27 (m, 3H), 3.11 - 2.99 (m, 3H).

### 14.3 2-chloro-N-(1,3,4,5-tetrahydrobenzo[cd]indol-4-yl)-7,8-dihydro-6H-pyrimido[5.4-b][1,4]oxazin-4-amine

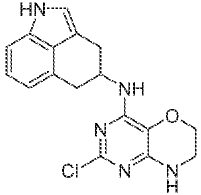

Prepared according to general method 4, using N-[5-(2-aminoethoxy)-2,6-dichloro-pyrimidin-4-yl)-1,3,4,5-tetrahydrobenzo[cd]indol-4-amine;hydrochloride (1.00 eq, 558 mg, 1.13 mmol) to afford 2-chloro-N-(1,3.4,5-tetrahydrobenzo[cd]indol-4-yl)-7,8-dihydro-6H-pyrimido[5,4-b][1,4]oxazin-4-amine (409 mg, 0.958 mmol, 84.69% yield) as a grey solid.

UPLC-MS analysis (2 min, basic): rt = 1.04 min, m/z = 342.2/344.2 [M+H]+, 98% purity.

1H NMR (400 MHz, DMSO-D6) δ 10.65 (s, 1H), 7.17 (d, J = 3.0 Hz, 1H), 7.13 (d, J = 8.0 Hz, 1H), 7.04 - 6.96 (m, 2H), 6.71 (d. J = 7.0 Hz, 1H), 6.34 (d, J = 8.8 Hz, 1H), 4.52 - 4.36 (m, 1H), 4.08 (t, J = 4.3 Hz, 2H), 3.39 (q, J = 3.8 Hz, 2H), 3.01 (q, J = 5.9 Hz, 3H), 2.86 (m, 1H).

### Example 14 2-(2-methylthiazol-5-yl)-N-(1,3,4,5-tetrahydrobenzo[cd]indol-4-yl)-7,8-dibydro-6H-pyrimido[5,4-b][1,4]oxazin-4-amine

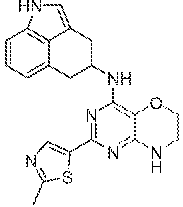

Prepared according to general method 5b, using 2-chloro-N-(1,3,4,5-tetrahydrobenzo[cd]indol-4-yl)-7,8-dihydro-6H-pyrimido[5,4-b][1,4]oxazin-4-amine (1.00 eq, 60 mg, 0.176 mmol) and 2-methyl-5-(tetramethyl-1,3,2-dioxaborolan-2-yl)-1,3-thiazole (1.50 eq, 75 mg, 0.331 mmol). The crude was purified by column chromatography (12 g cartridge) eluting with a gradient of EtOAc (80% to 100%; *v*/*v*) in Heptane to afford 2-(2-methylthiazol-5-yl)-N-(1,3,4,5-tetrahydrobenzo[cd]indol-4-yl)-7,8-di hydro-6H-pyrimido[5,4-b][1,4]oxazin-4-amine (27mg,0.0646 mmol, 36.77% yield) as a beige solid. UPLC-MS analysis (2min, basic): rt =1.80 min; m/z = 405.1 [M+H]+, 100% purity. 1H NMR (400 MHz, DMSO-D6) δ 10.64 (d, J = 2.1 Hz, 1H), 7.90 (s, 1H), 7.14 (d, J = 8.1 Hz, 1H), 7.02 - 6.97 (m, 2H), 6.92 (d, J = 2.6 Hz, 1H), 6.71 (d, J = 6.9 Hz, 1H), 6.00 (d, J = 8.6 Hz, 1H), 4.57 (tt, J = 9.2, 4.6 Hz, 1H), 4.09 (t, J = 4.3 Hz, 2H), 3.42 - 3.36 (m, 2H), 3.15 - 2.99 (m, 3H), 2.95 - 2.83 (m, 1H), 2.57 (s, 3H).

Example 14 title compound (125 mg, 0.309 mmol) was submitted to chiral separation to provide two enantiomers **Example 61** and **Example 62**

### Example 15 N-[2-(1H-indol-3-yl)ethyl]-2-(6-methyl-3-pyridyl)-7,8-dihydro-6H-pyrimido[5,4-b][1,4]oxazin-4-amine

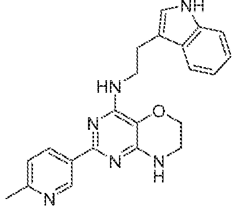

Prepared according to general method 5a, using 2-chloro-N-[2-(1H-indol-3-yl)ethyl]-7,8-dihydro-6H-pyrimido[5,4-b][1m4]oxazin-4-amine (1.00 eq, 90 mg, 0.273 mmol) and 2-Methylpyridine-5-boronic acid (1.50 eq, 56 mg, 0.409 mmol). The crude was purified by column chromatography (20 g cartridge) eluting with a gradient of EtOAc (0% to 100%) in iso-hexane followed by prep HPLC to afford N-[2-(1H-indol-3-yl)ethyl]-2-(6-methyl-3-pyridyl)-7,8-dihydro-6H-pyrimido[5,4-b][1,4]oxazin-4-amine (8.0 mg,0.0205 mmol, 7.51% yield) as a beige solid.

UPLC-MS analysis (6 min, basic): rt = 2.94 min, m/z = 387.1 [M+H]+, 99% purity.

1H NMR (400 MHz, DMSO-D6) δ 10.76 (s, 1H), 9.19 (dd, J = 2.3, 0.8 Hz, 1H), 8.31 (dd, J = 8.1, 2.2 Hz, 1H), 7.58 (d, J = 7.8 Hz, 1H), 7.30 (dd, J = 8.1, 1.0 Hz, 1H), 7.24 (d, J = 8.1 Hz, 1H), 7.15 (d, J = 2.2 Hz, 1H), 7.03 (m, 1H), 6.94 (m, 1H), 6.78 (s, 1H), 6.27 (t, J = 5.9 Hz, 1H), 4.11 - 4.05 (m, 2H), 3.66 (dt, J = 8.3, 6.2 Hz, 2H), 3.38 (q, J = 3.7 Hz, 2H), 2.99 - 2.90 (m, 2H). 3H not observed (Me)

### Example 16 2-(5-fluoro-6-methyl-3-pyridyl)-N-[2-(1H-indol-3-yl)ethyl]-7,8-dihydro-6H-pyrimido[5,4-b][1,4]oxazin-4-amine

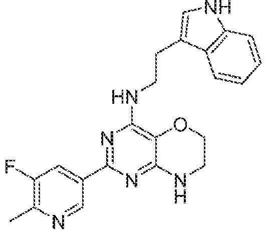

Prepared according to general method 5b, using 2-chloro-N-[2-(1H-indol-3-yl)ethyl]-7,8-dihydro-6H-pyrimido[5,4-b][1,4]oxazin-4-amine (1.00 eq, 90 mg, 0.273 mmol) and 3-Fluoro-2-methyl-5-(4,4,5,5-tetramethyl-1,3,2-dioxaborolan-2-yl)pyridine. The crude was purified by column chromatography (20 g cartridge) eluting with a gradient of EtOAc (0% to 100%) in iso-hexane to afford 2-(5-fluoro-6-methyl-3-pyridyl)-N-[2-(1H-indol- 3-yl)ethyl]-7,8-dihydro-6H-pyrimido[5,4-b][1,4]oxazin-4-amine (8.0 mg,0.0192 mmol, 7.03% yield) as a beige solid.

UPLC-MS analysis (4 min, basic): rt = 1.85 min, m/z = 405.1 [M+H]+, 97% purity.

1H NMR (400 MHz, DMSO-D6) δ 10.76 (s, 1H), 9.04 (t, J = 1.6 Hz, 1H), 8.09 (dd, J = 10.9, 1.7 Hz, 1H), 7.58 (dd, J = 7.8, 1.0 Hz, 1H), 7.30 (dt, J = 8.1, 0.9 Hz, 1H), 7.15 (d, J = 2.4 Hz, 1H), 7.03 (m, 1H), 6.93 (m, 1H), 6.85 (s, 1H), 6.36 (t, J = 5.9 Hz, 1H), 4.09 (t, J = 4.3 Hz, 2H), 3.71 - 3.61 (m, 2H), 3.39 (d, J = 3.9 Hz, 2H), 2.98 - 2.90 (m, 2H). 3Hs not observed.

19F NMR (376 MHz, DMSO-D6) δ 126.00

### Example 17 3-[4-[2-(1H-indol-3-yl)ethylamino]-7,8-dibydro-6H-pyrimido[5,4-b][1,4] oxazin-2-yl]-1H-pyridin-2-one

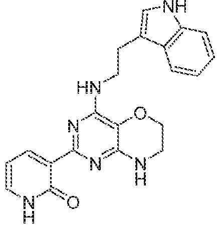

Prepared according to general method 5b, using 2-chloro-N-[2-(1H-indol-3-yl)ethyl]-7,8-dihydro-6H-pyrimido[5,4-b][1,4]oxazin-4-amine (1.00 eq, 150 mg, 0.455 mmol) and (2-oxo-1H-pyridin-3-yl)boronic acid (1.50 eq, 95 mg, 0.682 mmol). The crude was purified by column chromatography (40 g cartridge) eluting with a gradient of EtOAc (0% to 100%; v/v) in iso-Hex to afford 3-[4-[2-(1H-indol-3-yl)ethylamino]-7,8-dihydro-6H-pyrimido[5,4-b][1,4]oxazin-2-yl]- 1H-pyridin-2-one (83 mg,0.214 mmol, 46.98% yield) as a beige solid. Example 17 is subject to keto-enol tautomerism. It may also exist in the following form: 3-(4-((2-(1H-indol-3-yl)ethyl)amino)-7,8-dihydro-6H-pyrimido[5,4-b][1,4]oxazin-2-yl)pyridin-2-ol

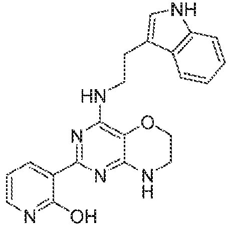

UPLC-MS analysis (2 min, basic): rt = 1.64 min, m/z = 389.1 [M+H]+, 100% purity.

1H NMR (400 MHz, DMSO-D6) δ 10.78 (s, 1H), 8.47 (s, 1H), 8.07 (s, 1H), 7.55 (d, J = 7.9 Hz, 1H), 7.30 (dt, J = 8.0, 0.9 Hz, 2H), 7.17 (d, J = 2.4 Hz, 1H), 7.03 (m, 1H), 6.94 (m, 1H), 6.88 (s, 1H), 6.73 (s, 1H), 4.11 (t, J = 4.3 Hz, 2H), 3.63 (q, J = 6.8 Hz, 2H), 3.43 (d, J = 3.7 Hz, 2H), 3.02 - 2.92 (m, 2H). 1H not observed.

### Example 18 5-[4-[2-(1H-indol-3-yl)ethylamino]-7,8-dihydro-6H-pyrimido[5,4-b][1,4]oxazin-2-yl]-1H-pyridin-2-one

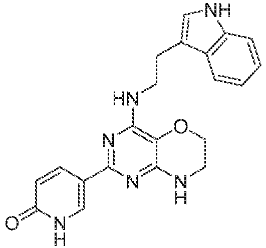

Prepared according to general method 5b, using 2-chloro-N-[2-(1H-indol-3-yl)ethyl]-7,8-dihydro-6H-pyrimido[5,4-b][1,4]oxazin-4-amine (150 mg, 0.455 mmol) and 6-Hydroxypyridine-3-boronic acid pinacol ester (151 mg, 0.682 mmol). The crude was purified by column chromatography (40 g cartridge) eluting with a gradient of EtOAc (0% to 100%; v/v) in iso-Hex to afford 5-[4-[2-(1H-indol-3-yl)ethylamino]-7,8-dihydro-6H-pyrimido[5,4-b][1,4]oxazin-2-yl]-1H-pyridin-2-one (77 mg,0.195 mmol, 42.81% yield) as a yellow solid. Example 18 is subject to keto-enol tautomerism. It may also exist In the following form: 5-(4-((2-(1H-indol-3-yl)ethyl)amino)-7,8-dihydro-6H-pyrimido[5,4-b][1,4]oxazin-2-yl)pyridin-2-ol 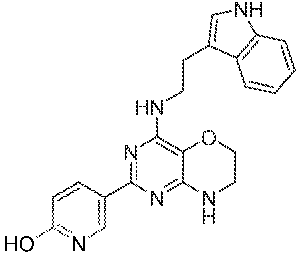 UPLC-MS analysis (2 min, basic): rt = 1.46 min, m/z = 389.1 [M+H]+, 100% purity.

1H NMR analysis 1H NMR (400 MHz, DMSO-D6) δ 10.78 (s, 1H), 8.47 (s, 1H), 8.07 (s, 1H), 7.55 (d, J = 7.9 Hz, 1H), 7.30 (dt, J = 8.0, 0.9 Hz, 2H), 7.17 (d. J = 2.4 Hz, 1H), 7.03 (m, 1H), 6.94 (m, 1H), 6.88 (s, 1H), 6.73 (s, 1H), 4.11 (t, J = 4.3 Hz, 2H). 3.63 (q, J = 6.8 Hz, 2H), 3.43 (d, J = 3.7 Hz, 2H), 3.02 - 2.92 (m, 2H). 1H not observed.

### Example 19 3-[4-[2-(1H-indol-3-yl)ethylamino]-7,8-dihydro-6H-pyrimido[5,4-b][1,4]oxazin-2-yl]-1-methyl-pyridin-2-one

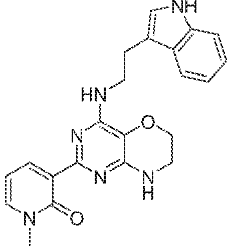

Prepared according to general method 5b, using 2-chloro-N-[2-(1H-indol-3-yl)ethyl]-7,8-dihydro-6H-pyrimido[5,4-b][1,4]oxazin-4-amine (150 mg, 0.455 mmol) and 1-Methyl-2-oxopyridine-3-boronic acid (104 mg, 0.682 mmol). The crude was purified by column chromatography (12 g cartridge) eluting with a gradient of EtOAc (0%-100%v/v) in hexanes to afford 3-[4-[2-(1H-indol-3-yl) ethyl amino]-7,8-dihydro-6H-pyrimido[5,4-b][1,4]oxazin-2-yl]-1-methyl-pyridin-2-one (13 mg,0.0304 mmol, 6.68% yield) as a yellow solid. UPLC-MS analysis (4 min, basic): rt = 1.38 min, m/z = 403.1 [M+H]+, 94% purity. 1H NMR (400 MHz, DMSO-D6) δ 10.70 (s, 1H), 7.70 (d, J = 6.7 Hz, 1H), 7.60 (d, J = 7.9 Hz, 1H), 7.54 (s. 1H), 7.27 (dd, J = 8.2, 1.1 Hz, 1H), 7.10 (d, J = 2.3 Hz, 1H), 6.99 (m, 1H), 6.88 - 6.79 (m, 1H), 6.65 (s, 1H), 6.19 (t, J = 6.8 Hz, 2H), 4.05 (dt, J = 13.1, 4.8 Hz, 2H), 3.51 (q, J = 7.0 Hz, 2H), 3.42 (s, 3H), 3.37 (s, 2H), 2.93 - 2.85 (m, 2H).

### Example 20 N-[2-(6-fluoro-1H-indol-3-yl)ethyl]-2-(6-methyl-3-pyridyl)-7,8-dihydro-6H-pyrimido[5,4-b][1,4]oxazin-4-amine

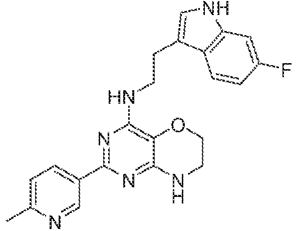

Prepared according to general method 5b, using 2-chloro-N-[2-(6-ftuoro-1H-indol-3-yl)ethyl]-7,8-dihydro-6H-pyrimido[5,4-b][1,4]oxazin-4-amine (140 mg, 0.403 mmol) and 2-Methylpyridine-5-boronic acid (83 mg, 0.604 mmol). The crude was purified by column chromatography (24 g cartridge) eluting with a gradient of EtOAc (0% to 100%; v/v) in iso-hexane followed by prep HPLC to afford N-[2-(6-fluoro-1H-indol-3-yl)ethyl]-2-(6-methyl-3-pyridyl)-7,8-dihydro-6H-pyrimido[5,4-b][1,4]oxazin-4-amine (37 mg,0.0887 mmol, 22.04% yield).

UPLC-MS analysis 6 min, basic): rt = 2.98 min, m/z = 405.1 [M+H]+, 100% purity.

1H NMR (400 MHz, DMSO-D6) δ 10.84 (s, 1H), 9.17 (dd, J = 2.3, 0.8 Hz, 1H), 8.29 (dd, J = 8.0, 2.2 Hz, 1H), 7.54 (dd, J = 8.7, 5.5 Hz, 1H), 7.24 (dt, J = 8.1, 0.7 Hz, 1H), 7.15 (d, J = 2.1 Hz, 1H), 7.06 (dd, J = 10.2, 2.3 Hz, 1H), 6.84 - 6.74 (m, 2H), 6.28 (t, J = 5.9 Hz, 1H), 4.11 - 4.00 (m, 2H), 3.65 (q, J = 6.9 Hz, 2H), 3.39 (t, J = 3.9 Hz, 2H), 2.97 - 2.88 (m, 2H). 3Hs not observed.

19F NMR (376 MHz, DMSO-D6) δ -122.37 - -122.40 (m).

### Example 21 3-[4-[2-(6-fluoro-1H-indol-3-yl)ethylamino]-7,8-dihydro-6H-pyrimido[5,4-b][1,4]axazin-2-yl]-1H-pyridin-2-one

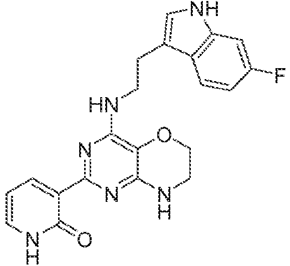

Prepared according to general method 5b, using 2-chloro-N-[2-(6-fluoro-1H-indol-3-yl)ethyl]-7,8-dihydro-6H-pyrimido[5,4-b][1,4]oxazin-4-amine (160 mg, 0.461 mmol) and (2-oxo-1H-pyridin-3-yl)boronic acid (96 mg, 0.691 mmol). The crude was purified by column chromatography (20 g cartridge) eluting with a gradient of EtOAc (0% to 100%; v/v) in iso-hexane to afford 3-[4-[2-(6-fluoro-1H-indol-3-yl)ethylamino]-7,8-dihydro-6H-pyrimido[5,4-b][1,4]oxazin-2-yl]-1H-pyridin-2-one (5.0 mg,0.0122 mmol, 2.64% yield) as a beige solid. Example 21 is subject to keto-enol tautomerism. It may also exist in the following form: 3-(4-((2-(6-fluoro-1H-indol-3-yl)ethyl)amino)-7,8-dihydro-6H-pyrimido[5,4-b][1,4]oxazin-2-yl)pyridin-2-ol

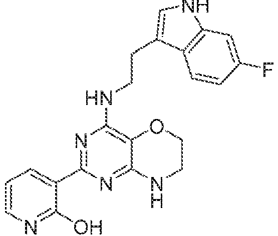

UPLC-MS analysis (6 min, basic): rt = 2.73 min, m/z = 407.1 [M+H]+, 99% purity.

1H NMR (400 MHz, DMSO-D6) δ 10.86 (s, 1H), 8.46 (d, J = 7.8 Hz, 1H), 8.10 (s, 1H), 7.52 (dd, J = 8.6, 5.6 Hz, 1H), 7.30 (s, 1H), 7.16 (s,1H), 7.06 (dd, J = 10.2, 2.4 Hz, 1H), 6.90 (s, 1H), 6.80 (t, J = 9.0 Hz, 1H), 6.70 (s, 1H), 4.11 (t, J = 4.3 Hz, 2H), 3.61 (d, J = 7.5 Hz, 2H), 3.42 (s, 2H), 2.94 (t, J = 7.6 Hz, 2H). 1H not observed. 19F NMR (376 MHz, DMSO-D6) δ -122.36.

### Example 22 2-[2-(difluoromethoxy)-3-pyridyl]-N-[2-(1H-indol-3-yl)ethyl]-7,8-dihydro-6H-pyrimido[5,4-b][1,4]oxazin-4-amine

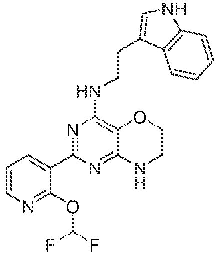

Prepared according to general method 5b, using 2-chloro-N-[2-(1H-indol-3-yl)ethyl]-7,8-dihydro-GH-pyrimido[5,4-b] [1,4]oxazin-4-amine (1.0 eq, 150 mg, 0.414 mmol) and 5-Fluoropyridine-3-boronic acid (2.0 eq, 117 mg, 0.828 mmol). The crude was purified by column chromatography (20 g cartridge) eluting with a gradient of EtOAc (0% to 100%; v/v) in iso-hexane to afford 2-[2-(difluoromethoxy)-3-pyridyl]-N-[2-(1H-indol-3-yl)ethyl]-7,8-dihydro-6H-pyrimido[5,4-b][1,4]oxazin-4-amine (79 mg,0.175 mmol, 57.64% yield) as a beige solid. UPLC-MS analysis (4 min, basic): rt = 1.79 min, m/z = 439.1 [M+H]+, 97% purity. 1H NMR (400 MHz, DMSO-D6) δ 10.70 (s, 1H), 8.23 (dd, J = 4.9, 2.0 Hz, 1H), 8.03 (dd, J = 7.5, 2.0 Hz, 1H), 7.68 (s, 1H), 7.56 - 7.48 (m, 1H), 7.34 - 7.23 (m, 2H), 7.08 (d, J = 2.3 Hz, 1H), 6.99 (m, 1H), 6.85 - 6.76 (m, 2H), 6.30 (t, J = 5.9 Hz, 1H), 4.13 - 4.06 (m, 2H), 3.55 (q, J = 7.0 Hz, 2H), 3.38 (q, J = 3.7 Hz, 2H), 2.94 - 2.86 (m, 2H).

19F NMR (376 MHz, DMSO-D6) δ -86.79, -86.82

### Example 23 N-[2-(1H-indol-3-yl)ethyl]-2-(2-methoxy-5-methyl-3-pyridyl)-7,8-dihydro-6H-pyrimido[5,4-b][1,4]oxazin-4-amine

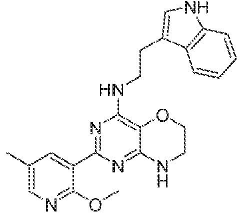

Prepared according to general method 5b, using 2-cbloro-N-[2-(1H-indol-3-yl)ethyl)-7,8-dihydro-6H-pyrimido[5,4-b][1,4]oxazin-4-amine (180 mg, 0.546 mmol) and (2-methoxy-S-methyl-3-pyridyl)boronic acid (137 mg, 0.819 mmol). The crude was purified by column chromatography (20 g cartridge) eluting with a gradient of EtOAc (0% to 100%; v/v) in iso-hexane to afford N-[2-(1H-indol-3-yl)ethyl]-2-(2-methoxy-5-methyl-3-pyridyl)-7,8-dihydro-6H-pyrimido[5,4-b][1,4]oxazin-4-amine (175 mg,0.408 mmol, 74.67% yield) as a beige solid.

UPLC-MS analysis (2 min, basic): rt = 1.07 min, m/z = 417.2, [M+H]+, 98% purity.

1H NMR (400 MHz, DMSO-D6) δ 10.72 (s, 1H), 7.95 (dd, J = 2.5, 0.9 Hz, 1H), 7.62 (dd, J = 2.5, 0.7 Hz, 1H), 7.57 (d, J = 7.9 Hz, 1H), 7.26 (m, 1H), 7.09 (d, J = 2.3 Hz, 1H), 6.99 (m, 1H), 6.82 - 6.71 (m, 2H), 6.25 (t, J = 5.9 Hz, 1H), 4.07 (q, J = 3.7 Hz, 2H), 3.73 (s, 3H), 3.56 - 3.46 (m, 2H), 3.40 - 3.34 (m, 2H), 2.94 - 2.86 (m, 2H), 2.20 (s, 3H).

### Intermediates for Example 24

### 24.1 tert-butyl N-[2-]2,4-dichloro-6-[2-(6-fluoro-1H-indol-3-yl)ethoxy]pyrimidin-5-yl]oxyethyl]carbamate

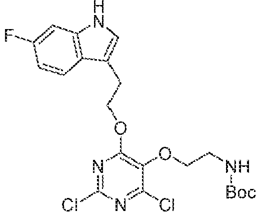

Prepared according to general method 2b, using 2-(6-fluoro-1H-indol-3-yl)ethanol (1.10 eq, 261 mg, 1.46 mmol) and tert-butyl N-[2-(2,4,6-trichloropyrimidin-5-yl)oxyethyl]carbamate (1.00 eq, 454 mg, 1.33 mmol) to afford tert-butyl N-[2-[2,4-dichloro-6-[2-(6-fluoro-1H-indol-3-yl)ethoxy]pyrimidin-5-yl]oxyethyl]carbamate (654 mg,1.09 mmol, 82.25% yield) as a yellow solid.

UPLC-MS analysis (acidic, 2min): rt = 1.31 min, no ionisation, 81% purity.

1H NMR (400 MHz, DMSO-D6) δ 10.96 (s, 1H), 7.63 (dd, J = 8.7, 5.4 Hz, 1H), 7.26 (d, J = 2.3 Hz, 1H), 7.12 (dd, J = 10.2, 2.3 Hz, 1H), 6.92 - 6.77 (m, 2H), 4.62 (t J = 7.0 Hz, 2H), 4.00 (d, J = 5.7 Hz, 2H), 3.26 - 3.12 (m, 4H), 1.37 (d, J = 2.6 Hz, 9H). 19F NMR (376 MHz, DMSO-D6) δ -122.28

### 24.2 2-[2,4-dichloro-6-[2-(6-fluoro-1H-indol-3-yl)ethoxy]pyrimidin-5-yl]oxy ethanamine;hydrochloride

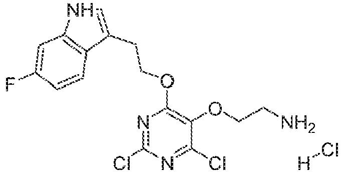

Prepared according to general method 3, using tert-butyl N-[2-[2,4-dichloro-6-[2-(6-fluoro-1H-indol-3-yl)ethoxy]pyrimidin-5-yl]oxyethyl]carbamate (1.00 eq, 854 mg, 1.42 mmol) to afford 2-[2,4-dichloro-6-[2-(6-fluoro-1H-indol-3-yl)ethoxy]pyrimidin-5-yl]oxyethanamine;hydrochloride (484 mg,1.02 mmol, 71.65% yield) as a pink solid.

UPLC-MS analysis (2 min, basic): rt = 1.10 min, m/z = 385.2, 387.2, 389.2 [M+H]+, 89% purity.

1H NMR (400 MHz, DMSO-D6) δ 11.03 - 10.93 (m, 1H), 8.11 (s, 3H), 7.64 (dd, J = 8.7, 5.5 Hz, 1H), 7.28 (d, J = 2.3 Hz, 1H), 7.14 (dd, J = 10.2, 2.4 Hz, 1H), 6.88 (m, 1H), 4.65 (t, J = 7.1 Hz, 2H), 4.18 (t, J = 5.3 Hz, 2H), 3.22 (t, J = 7.1 Hz, 2H), 3.14 (q, J = 5.4 Hz, 2H).

19F NMR (376 MHz, DMSO-D6) δ- 122.19.

### 24.3 2-chloro-4-[2-(6-fluoro-1H-indol-3-yl)ethoxy]-7,8-dihydro-6H-pyrimido[5,4-b][1,4] oxazine

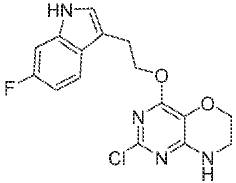

Prepared according to general method 4, using 2-[2,4-dichloro-6-[2-(6-fluoro-1H-indol-3-yl)ethoxy]pyrimidin-5-yl]oxyethanamine;hydrochloride (1.00 eq, 400 mg, 0.949 mmol) to afford 2-chloro-4-[2-(6-fluoro-1H-indol-3-yl)ethoxy]-7,8-dihydro-6H-pyrimido[5,4-b][1,4]oxazine (285 mg, 0.449 mmol, 47.38% yield) as a beige solid.

UPLC-MS analysis (2 min, basic): rt = 1.07 min, m/z = 349.2, 351.2 [M+H]+, 55% purity.

1H NMR (400 MHz, DMSO-D6) δ 10.90 (s, 1H), 7.79 (s, 1H), 7.59 (dd, J = 8.7, 5.4 Hz, 1H), 7.16 (d, J = 2.3 Hz, 1H), 7.11 - 7.06 (m, 1H), 6.83 - 6.75 (m, 1H), 4.39 (t, J = 7.1 Hz, 2H), 4.03 (t, J = 4.3 Hz, 2H), 3.38 (q, J = 3.8 Hz, 2H), 3.04 (t, J = 7.1 Hz, 2H). 19F NMR (376 MHz, DMSO-D6) δ - 122.40.

### Example 24 4-[2-(6-fluoro-1H-indol-3-yl)ethoxy]-2-(2-methylthiazol-5-yl)-7,8-dihydro-6H-pyrimido[5,4-b][1,4]oxazine

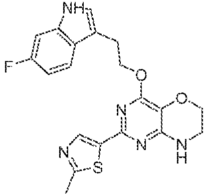

Prepared according to general method 5b, using 2-chloro-4-[2-(6-fluoro-1H-indol-3-yl)ethoxy]-7,8-dihydro-6H-pyrimido[5,4-b][1,4]oxazine (1.00 eq, 140 mg, 0.221 mmol) and 2-methyl-5-(tetramethyl-1,3,2-dioxaborolan-2-yl)-1,3-thiazole (1.50 eq, 75 mg, 0.331 mmol). The crude was purified by column chromatography (12 g cartridge) eluting with a gradient of EtOAc (100%; v/v) in iso-Hexane followed by prep HPLC to obtain 4-[2-(6-fluoro-1H-indol-3-yl)ethoxy]-2-(2-methyl thiazol-5-yl)-7,8-dihydro-6H-pyrimido[5,4-b][1,4]oxazine (5.5 mg,0.0130 mmol, 5.87% yield) as a white solid. UPLC-MS analysis (4 min, basic): rt = 1.78 min, m/z = 412.1 [M+H]+, 97% purity.

1H NMR (400 MHz, DMSO-D6) δ 10.93 (s, 1H), 7.99 (s, 1H), 7.58 (dd, J = 8.7, 5.5 Hz, 1H), 7.51 (d, J = 2.8 Hz, 1H), 7.20 (d, J = 2.3 Hz, 1H), 7.12 - 7.02 (m, 1H), 6.81 (m, 1H), 4.49 (t, J = 7.3 Hz, 2H), 4.05 (t, J = 4.2 Hz, 2H), 3.42 - 3.35 (m, 2H). 3.09 (t, J = 7.2 Hz, 2H), 2.60 (s, 3H).

### Intermediates for Example 25

### 25.1 tert-butyl N-[2-[2,4-dichloro-6-[2-(1H-indol-3-yl)ethoxy] pyrimidin-5-yl]oxyethyl] carbamate

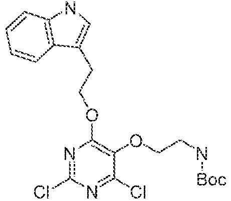

Prepared according to general method 2b, using tert-butyl N-[2-(2,4,6-trichloropyrimidin-5-yl)oxyethyl]carbamate (1.00 eq, 0.36 g, 1.05 mmol) and Tryptophol (1.10 eq, 190 mg, 1.16 mmol). The crude was purified by column chromatography (20 g column) eluting with 20-30% EtOAc in iso-hexane to afford tert-butyl N-[2-[2,4-dichloro-6-[2-(1H-indol-3-yl)ethoxy]pyrimidin-5-yl]oxyethyl]carbamate (0.31 g,0.650 mmol, 61.86% yield) as white solid.

UPLC-MS analysis (acidic, 2min): rt = 1.30 min, no ionisation, 98% purity. 1H NMR (400 MHz, DMSO-D6) δ 10.86 (s, 1H), 7.60 (d, J = 7.8 Hz, 1H), 7.31 (dt, J = 8.1, 1.0 Hz, 1H), 7.22 (d, J = 2.4 Hz, 1H), 7.04 (ddd, J = 8.1, 6.9, 1.2 Hz, 1H), 6.96 (ddd, J = 7.9, 6.9, 1.1 Hz, 1H), 6.82 (t, J = 5.7 Hz, 1H), 4.57 (t, J = 7.1 Hz, 2H), 3.97 (t, J = 5.7 Hz, 2H), 3.16 (dt, J = 7.9, 6.4 Hz, 4H), 1.33 (s, 9H).

### 25.2 2-[2,4-dichloro-6-[2-(1H-indol-3-yl)ethoxy]pyrimidin-5-yl]oxyethanamine; hydrochloride

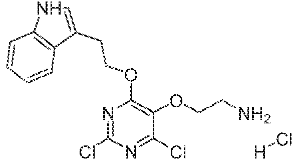

Prepared according to general method 3, using tert-butyl N-[2-[2,4-dichloro-6-[2-(1H-indol-3-yl)ethoxy] pyrimidin-5-yl]oxyethyl]carbamate (1.00 eq, 1.13 g. 2.42 mmol) to afford2-[2,4-dichloro-6-[2-(1H-indol-3-yl)ethoxylpyrimidin-5-yl]oxyethanamine;hydrochloride (0.23g. 0.568 mmol, 85.46% yield) as a grey solid. UPLC-MS analysis: (2 min, acidic): rt = 0.89 min, m/z = 367.2/369.2/371.2 [M+H]+, 97% purity. 1H NMR analysis : 1H NMR (400 MHz, DMSO-D6) δ 10.81 (s, 1H), 8.31 (d, J = 5.7 Hz, 1H), 8.21 (s, 3H), 7.62 (t, J = 6.4 Hz, 1H), 7.30 (t, J = 6.6 Hz, 1H), 7.18 - 7.12 (m, 1H), 7.02 (t, J = 6.7 Hz, 1H), 6.94 (q, J = 6.7 Hz, 1H), 4.08 (d, J = 5.2 Hz, 2H), 3.58 (s, 2H), 3.18 (s, 2H), 2.93 (s, 2H). 19F NMR (376 MHz, DMSO) δ- 122.19.

### 25.3 2-chloro-4-[2-(1H-indol-3-yl)ethoxy]-7,8-dihydro-6H-pyrimido[5,4-b][1,4]oxazine

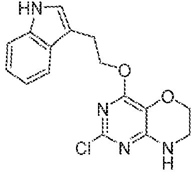

Prepared according to general method 4, using 2-[2,4-dichloro-6-[2-(1H-indol-3-yl)ethoxy]pyrimidin-5-yl]oxyethanamine;hydrochloride (1.00 eq, 140 mg, 0.346 mmol) to afford2-chloro-4-[2-(1H-indol-3-yl)ethoxy]-7,8-dihydro-6H-pyrimido[5,4-b][1,4]oxazine (128 mg.0.348 mmol, 100.43% yield) as an off-white solid.

UPLC-MS analysis: (2 min, basic): rt = 1.10 min, m/z = 331.1/333.1 [M+H]+, 90% purity.

1H NMR analysis : 1H NMR (400 MHz, DMSO-D6) δ 10.83 (s, 1H), 7.80 (s, 1H), 7.59 (d, J = 7.9 Hz, 1H), 7.30 (d, J = 8.1 Hz, 1H), 7.16 (d, J = 2.4 Hz, 1H), 7.03 (t, J = 7.5 Hz, 1H), 6.94 (t, J = 7.5 Hz, 1H), 4.40 (t, J = 7.4 Hz, 2H), 4.03 (s, 2H), 3.38 (s, 2H), 3.06 (t, J = 7.4 Hz, 2H).

### Example 25 3-(4-(2-(1H-indol-3-yl)ethoxy)-7,8-dihydro-6H-pyrimido[5,4-b][1,4]oxazin-2-yl)pyridin-2(1H)-one

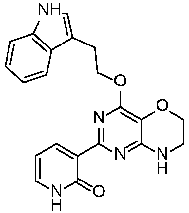

Prepared according to general method 5b, using 2-chloro-4-[2-(1H-indol-3-yl)ethoxy]-7,8-dihydro-6H-pyrimido[5,4-b][1,4]oxazine (1.0 eq, 50 mg, 0.151 mmol) and (2-hydroxy-3-pyridyl)boronic acid (1.5 eq, 31.49 mg, 0.226 mmol). The crude was purified by column chromatography (20 g cartridge) eluting with a gradient of MeOH (10-15%; v/v) in DCM to afford3-[4-[2-(1H-indol-3-yl)ethoxy]-7,8-dihydro-6H-pyrimido [5,4-b][1,4]oxazin-2-yl]pyridin-2-ol (13 mg,0.0308 mmol, 20.39% yield) as a beige solid. Example 25 is subject to keto-enol tautomerism. It may also exist in the following form: 3-(4-(2-(1H-indol-3-yl)ethoxy)-7,8-dihydro-6H-pyrimido[5,4-b][1,4]oxazin-2-yl)pyridin-2-ol

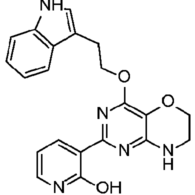

UPLC-MS analysis (4 min, basic): rt= 1.33 min, m/z = 390.3 [M+H]+, 100% purity.

1H NMR (400 MHz, DMSO-D6) δ 13.93 (s, 1H), 10.89 (s, 1H), 8.51 (s, 1H), 8.17 (s, 1H), 8.07 (s, 1H), 7.62 (d, J = 7.9 Hz, 1H), 7.35 (d, J = 8.1 Hz, 1H), 7.25 (s, 1H), 7.07 (t, J = 7.5 Hz, 1H), 6.98 (s, 2H), 4.62 (s, 2H), 4.13 (d, J = 13.8 Hz, 2H), 3.49 (s, 2H), 3.22 - 3.11 (m, 2H).

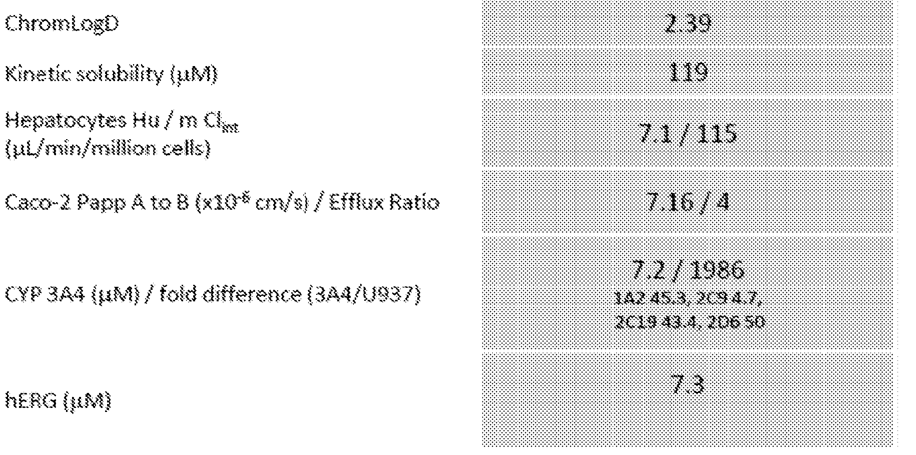

### Intermediates for Example 26

### 26.1 2,6-dichloro-N-[2-(1H-indol-3-yl)ethyl]-5-methoxy-pyrimidin-4-amine

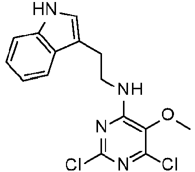

Prepared according to general method 2a, using 2,4,6-trichloro-5-Methoxypyrimidine (1 eq, 1.50 g, 7.03 mmol) and tryptamine (1.15 eq, 1.29 g, 8.08 mmol) in Ethanol (15 mL). Removal of volatiles and trituration in Water/MeOH afforded 2,6-dichloro-N-[2-(1H-indol-3-yl)ethyl]-5-methoxy- pyrimidin-4-amine (2.30 g,6.82 mmol, 97.06% yield).

UPLC-MS analysis (4 min, basic): rt = 1.93 min, m/z = 336.9/338.8 [M+H]+, 97% purity.

1H NMR (400 MHz, DMSO-D6) δ 10.82 (s, 1H), 8.19 (t, *J* = 5.9 Hz, 1H), 7.63 (d, *J* = 7.9, 1.4, 0.7 Hz, 1H), 7.34 (dt, *J* = 8.1, 1.0 Hz, 1H), 7.18 (d, *J* = 2.4 Hz, 1H), 7.07 (td, *J =* 8.1, 7.0, 1.2 Hz, 1H), 6.98 (td, *J* = 8.0, 7.0, 1.1 Hz, 1H), 3.70 (s, 3H), 3.60 (q, 2H), 2.97 (t, 2H).

### 26.2 2,4-dichloro-6-[2-(1H-indol-3-yl)ethylamino]pyrimidin-5-ol

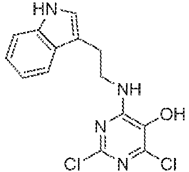

To a solution of 2,6-dichloro-N-[2-(1H-indol-3-yl)ethyl]-5-methoxy-pyrimidin-4-amine (1 eq, 2.30 g, 6.82 mmol) in DCM (120 mL) was added boron tribromide (1.2 eq, 8.2 mL, 8.19 mmol) in DCM (1 M) under a nitrogen atmosphere and the mixture was stirred for 18 hours. Further addition of boron tribromide solution and stirring, were needed to take the reaction to completion as shown by UPLC analysis. The reaction mixture was quenched with MeOH (30 mL) and water (300 mL). Layers were separated and aqueous was extracted with DCM twice and organics were concentrated and absorbed into silica, loaded into a silica packed column and product was eluted using a EtOAc: Hexane mixture to give 2,4-dichloro-6-[2-(1H-indol-3-yl)ethylamino]pyrimidin-5-ol (1.50 g,4.64 mmol, 68.05% yield) as a brown solid.

NOTE: 20% trans-halogenation occurred generating 2-bromo-4-chloro-6-[2-(1H-indol-3-yl)ethylamino]pyrimidin-5-ol. The by product was carried over throughout the synthetic route reacting the same way as its chloro counterpart. UPLC-MS analysis (4 min, acidic): rt = 1.09 min, m/z = 322.9/324.9/326.9 [M-H]-, 99% purity. 20% of brominated regioisomer.

¹H NMR (400 MHz, DMSO-*D*₆) δ 10.81 (s, 1H), 10.16 (s, 1H), 7.78 (t, *J* = 5.9 Hz, 1H), 7.66 (d, 1H), 7.33 (dt, *J* = 8.1, 1.0 Hz, 1H), 7.17 (d, *J* = 2.3, 1.2 Hz, 1H), 7.07 (ddd, *J* = 8.1, 7.0, 1.2 Hz, 1H), 6.98 (ddd, / = 8.0, 7.0, 1.2 Hz, 1H), 3.60 (q, 2H), 2.95 (t, 2H).

### 26.3 tert-butyl N-[(1R)-1-[[2,4-dichloro-6-[2-(1H-indol-3-yl)ethylamino]pyrimidin-5-yl]oxymethyl]-2-methoxy-ethyl]carbamate

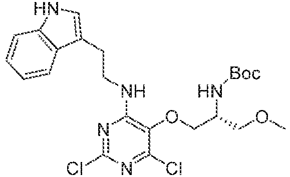

Prepared according to general method 1, using 2,4-dichloro-6-[2-(1H-indol-3-yl)ethylamino]pyrimidin-5-ol (1 eq, 360 mg, 1.11 mmol), and N-Boc-(s)-2-amino-3-methoxy-1-propanol (1.4 eq, 0.32 g, 1.56 mmol). The crude was purified by column chromatography eluting with hexane then 10:1 and 4:1 Hexane:EtOAc to afford tert-butyl N-[(1R)-1-[[2-bromo-4-chloro-6- [2-(1H-indol-3-yl)ethylamino]pyrimidin-5-yl]oxy methyl]-2-methoxy-ethyl]carbamate (100 mg, 0.177 mmol, 15.86% yield) as a brown oil. UPLC-MS analysis (4 min, basic): rt = 2.31 min, m/z = 510.0/512.1/514.2 [M+H]+, 98% purity. 1H NMR (400 MHz, DMSO-D6) δ 10.84 (s, 1H), 8.87 (s, 1H), 7.91 (m, 1H), 7.63 (d, J = 8.1 Hz, 1H), 7.34 (d, J = 8.1, Hz, 1H), 7.18 (s, 1H), 7.11 - 7.02 (m, 1H), 6.98 (t, J = 8.5, 6.0 Hz, 1H), 4.81 - 4.72 (m, 1H), 4.00 - 3.83 (m, 2H), 3.62 (q, *J* = 7.6 Hz, 2H), 3.36 (s, 2H), 3.23 (s, 3H), 2.98 (t, *J* = 7.7 Hz, 2H), 1.39 (s, 9H).

### 26.4 5-[(2R)-2-amino-3-methoxy-propoxy]-2,6-dichloro-N-[2-(1H-indol-3-yl)ethyl] pyrimidin-4-amine;hydrochloride

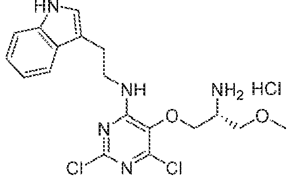

Prepared according to general method 3, using tert-butyl N-[(1R)-1-[[2,4-dichloro-6-[2-(1H-indol-3-yl)ethylamino]pyrimidin-5-yl]oxymethyl]-2-methoxyethyl]carbamate (1 eq, 400 mg, 0.635 mmol) to afford 5-[(2R)-2-amino-3-methoxy-propoxy]-2,6-dichloro-N-[2-(1H-indol-3-yl)ethyl]pyrimidin-4-amine; hydrochloride (330 mg, 0.739 mmol, 90.63% yield).

UPLC-MS analysis (4 min, basic): rt = 1.91 min, m/z = 410.0/412.0/414.0 [M+H]+, 80% purity.

1H NMR (400 MHz, DMSO-D6) δ 10.85 (s, 1H), 8.87 (s, 1H), 8.47 (brs, 3H), 7.66 (d, *J* = 8.1 Hz, 1H), 7.34 (d, *J* = 7.9 Hz, 1H), 7.20 (s, 1H), 7.06 (t, *J* = 7.8 Hz, 1H), 6.99 (t, *J* = 7.5 Hz, 1H), 4.81 - 4.72 (m, 1H), 4.20 - 4.11 (m, 1H), 4.04 (dd, *J* = 10.2, 6.0 Hz, 1H), 3.72 (brs, 1H), 3.67 - 3.58 (m, 3H), 3.32 (s, 3H), 2.98 (t, *J* = 7.9 Hz, 2H).

### 26.5 (7R)-2-chloro-N-[2-(1H-indol-3-yl)ethyl]-7-(methoxymethyl)-7,8-dihydro-6H-pyrimido[5,4-b][1,4]oxazin-4-amine

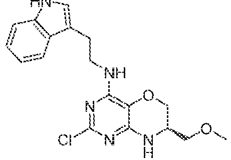

Prepared according to general method 4, using 5-[(2R)-2-amino-3-methoxypropoxy]-2,6-dichloro-N-[2-(1H-indol-3-yl)ethyl]pyrimidin-4-amine;HCl (1 eq, 330 mg, 0.739 mmol) to afford(7R)-2-chloro-N-[2-(1H-indol-3-yl)ethyl]-7-(methoxymethyl)-7,8-dihydro-6H-pyrimido [5,4-b][1,4]oxazin-4-amine (160 mg,0.428 mmol, 57.43% yield).

UPLC-MS analysis (4 min, basic): rt = 1.74 min, m/z = 374/376 [M+H]+, 81% purity.

1H NMR (400 MHz, DMSO-D6) δ 10.80 (s, 1H), 7.65 (d, J = 8.0 Hz, 1H), 7.36 - 7.25 (m, 2H), 7.16 (s, 1H), 7.06 (t, J = 7.5 Hz, 1H), 6.97 (t, J = 7.4 Hz, 1H), 6.75 (t, 1H), 4.81 - 4.72 (m, 1H), 4.04 (d, J = 11.0 Hz, 1H), 3.95 (d, J = 10.5 Hz, 1H), 3.64 (brs, 1H), 3.50 (q, J = 7.7 Hz, 2H), 3.28 (s, 3H), 2.89 (t, *J* = 7.8 Hz, 2H). 1H not observed,

### Example 26 (R)-3-(4-((2-(1H-Indol-3-yl)ethyl)amino)-7-(methoxymethyl)-7,8-dihydro-6H-pyrimido[5,4-b][1,4]oxazin-2-yl)pyridin-2(1H)-one

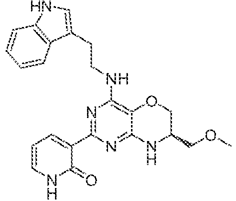

Prepared according to general method 5b, using (7R)-2-chloro-N-[2-(1H-indol-3-yl)ethyl]-7-(methoxymethyl)-7,8-dihydro-6H-pyrimido[5,4-b][1,4]oxazin-4-amine (0.82 eq, 160 mg, 0.428 mmol) and (2-Oxo-1,2-dihydropyridin-3-yl)boronic acid (1.6 eq, 116 mg, 0.838 mmol). The crude was purified by Reversed Phase chromatography (12 g cartridge) eluting with a gradient of MeCN (0% to 100%; v/v) in water (acidic buffer) to afford 3-[(7R)-4-[2-(1H-indol-3-yl)ethylamino]-7-(methoxymethyl)-7,8-dihydro-6H-pyrimido[5,4-b][1,4]oxazin-2-yl]pyridin-2-ol (45 mg,0.0978 mmol, 18.68% yield), 157374-4 as a yellow solid. Example 26 is subject to keto-enol tautomerism. It may also exist in the following form: (R)-3-(4-((2-(1H-indol-3-yl)ethyl)amino)-7-(methoxy methyl)-7,8-dihydro-6H-pyrimido[5,4-b][1,4]oxazin-2-yl)pyridin-2-ol 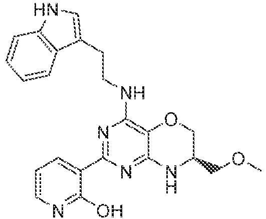 UPLC-MS analysis (4 min, acidic): rt = 1.50 min, m/z = 433.1 [M+H]+, 94% purity. 1H NMR (400 MHz, DMSO-D6) δ 10.87 - 10.81 (m, 1H), 8.53 (d, J = 7.5 Hz, 1H), 8.14 (brs, 1H), 7.58 (d, J = 7.9 Hz, 1H), 7.54 (brs, 1H), 7.34 (d, J = 8.0 Hz, 1H), 7.21 (d, J = 2.3 Hz, 1H), 7.07 (t, J = 8.1, 6.9, Hz, 1H), 7.02 - 6.94 (m, 2H), 6.86 (brs, 1H), 4.08 (m, J = 10.7, 5.9 Hz, 2H), 3.73 (brs, 1H), 3.66 (q, *J* = 7.0 Hz, 2H), 3.46 - 3.36 (m, 2H), 3.31 (m, 4H), 2.99 (t, *J* = 7.6 Hz, 2H).

### Example 27 (R)-3-(4-((2-(1H-indol-3-yl)ethyl)amino)-7-(bydroxymetbyl)-7,8-dihydro-6H-pyrimido[5,4-b][1,4]oxazin-2-yl)pyridin-2(1H)-one

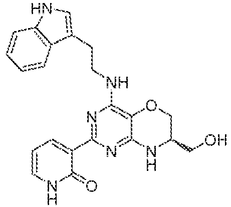

To a solution of 3-[(7R)-4-[2-(1H-indol-3-yl)ethylamino]-7-(methoxymethyl)-7,8-dihydro-6H-pyrimido[5,4-b][1,4]oxazin-2-yl]pyridin-2-ol (1 eq, 30 mg, 0.0694 mmol) in DCM (2 mL) was added boron tribromide solution in DCM 1M (3 eq, 0.21 mL, 0.208 mmol) and the mixture was stirred at ambient temperature for 1 hour.

The reaction mixture was carefully quenched with MeOH (0.5 mL) and then with 1M NaOH (vigorous stir) to pH = 7. The organic layer volume was reduced to dryness and dry loaded into a silica packed column and product was eluted using MeCN, then 1%, 5% and 10% water in MeCN to give crude material which was triturated in diethyl ether (0.5 mL), filtered, dried under suction and then under vacuum for 2 hours at 45 °C, to give 3-[(7R)-7-(hydroxymethyl)-4-[2-(1H-indol-3-yl)ethylamino]-7,8-dihydro-6H-pyrimido[5,4-b][1,4]oxazin-2-yl]pyridin-2-ol (4.0 mg, 0.00908 mmol, 13.09% yield) as a yellow solid. Example 27 is subject to keto-enol tautomerism. It may also exist in the following form: (R)-3-(4-((2-(1H-indol-3-yl)ethyl)amino)-7-(hydroxymethyl)-7,8-dihydro-6H-pyrimido[5,4-b][3,4]oxazin-2-yl)pyridin-2-ol 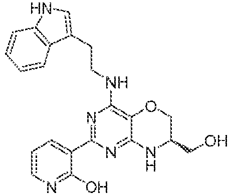 UPLC-MS analysis (4 min, acidic): rt = 1.53 min, m/z = 419.1 [M+H]+, 95% purity. 1H NMR (400 MHz, DMSO-D6) δ 10.84 (s, 1H), 8.53 (d,*J* = 7.6 Hz, 1H), 8.12 (brs, 1H), 7.58 (d, *J* = 7.8 Hz, 1H), 7.46 (brs, 1H), 7.34 (dt, / = 8.1, 0.9 Hz, 1H), 7.21 (d, *J* = 2.3 Hz, 1H), 7.07 (ddd, *J* = 8.1, 6.9, 1.2 Hz, 1H), 6.97 (m, 2H), 6.83 (brs, 1H), 5.02 (t, *J* = 5.1 Hz, 1H), 4.10(qd, *J* = 10.6, 3.2 Hz, 2H), 3.66 (q, *J* = 7.1 Hz, 2H), 3.51 (m, 2H), 3.45 - 3.36 (m, 1H), 2.99 (t, J = 7.6 Hz, 2H).

### Intermediates for Example 28

### 28.1 tert-butyl N-[(1S)-1-(methoxymethyl)-2-(2,4,6-trichloropyrimidin-5-yl)oxyethyl]carbamate

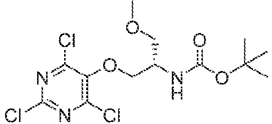

Prepared according to general method 1, using 2,4,6-trichloropyrimidin-5-ol (1.00 eq, 0.30 g, 1.43 mmol) and N-Boc-(R)-2-Amino-3-methoxy-1-propanol (1.10 eq, 0.24 mL, 1.57 mmol) and N-Boc-(R)-2-Amino-3-methoxy-1-propanol (1.10 eq, 0.24 mL, 1.57 mmol).

The crude was purified by column chromatography (40 g column, DCM loading) eluting with 18-20% EtOAc in iso-Hexane to afford tert-butyl N-[(1S)-1-(methoxymethyl)-2-(2,4,6-trichloropyrimidin-5-yl)oxy-ethyl]carbamate (256 mg, 0.636 mmol, 44.47% yield) as a white solid. UPLC-MS analysis (2 min, basic): rt = 1.24 min, no ionisation, 100% purity.

1H NMR (400 MHz, DMSO-D6) δ 6.97 (d, *J* = 8.2 Hz, 1H), 4.19 (dd, *J* = 9.7, 4.8 Hz, 1H), 4.08 (dd, *J* = 9.7, 6.4 Hz, 1H), 4.01 - 3.88 (m, 1H), 3.43 (d. *J* = 6.1 Hz, 2H), 3.28 (s, 3H), 1.39 (s, 9H).

### 28.2 tert-butyl N-[(1S)-1-[[2,4-dichloro-6-[2-(1H-indol-3-yl)ethylamino]pyrimidin-5-yl) oxymethyl]-2-methoxy-ethyl]carbamate

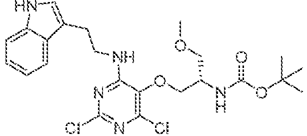

Prepared according to general method 2a, using tert-butyl N-[(1S)-1-(methoxymethyl)-2-(2,4,6-trichloropyrimidin-5-yl)oxy-ethyl]carbamate (1.00 eq, 0.25 g, 0.647 mmol) and Tryptamine (1.05 eq, 0.11 g, 0.679 mmol).

The reaction was concentrated to dryness to give an oily residue, which was suspended in water (25 mL) with stirring. The resultant suspension was filtered and the solid was dried under vacuum to afford tert-butyl N-[(1S)-1-[[2,4-dichloro-6-[2-(1H-indol-3-yl)ethylamino]pyrimidin-5-yl]oxy methyl]-2-methoxy-ethyl]carbamate (297 mg,0.378 mmol, 58.50% yield) as an off-white solid.

UPLC-MS analysis (2 min, basic): rt = 1.31 min, m/z = 510.2/512.2/514.2 [M+H]+, 65% purity. 1H NMR (400 MHz, DMSO-D6) δ 10.84 (s, 1H), 7.91 (s, 1H), 7.64 (d, J = 7.8 Hz, 1H), 7.35 (dt, J = 8.0, 0.9 Hz, 1H), 7.19 (d. J = 2.4 Hz, 1H), 7.12 - 7.03 (m, 2H). 6.99 (ddd, J = 8.0, 7.0, 1.1 Hz, 1H), 3.96 (d, J = 6.4 Hz, 2H), 3.88 (t, J = 6.4 Hz, 1H), 3.63 (q, J = 7.0 Hz, 2H), 3.38 (d, J = 4.3 Hz, 2H), 3.24 (s, 3H), 2.99 (t, J = 7.7 Hz, 2H), 1.40 (s, 9H).

### 28.3 2,6-dichloro-N-[2-(1H-indol-3-yl)ethyl]-5-[(2S)-2-amino-3-methoxy-propoxy] pyrimidin-4-amine;hydrochloride

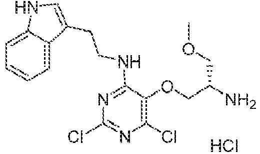

Prepared according to general method 3, using tert-butyl N-[(1S)-1-[[2,4-dichloro-6-[2-(1H-indol-3-yl)ethylamino]pyrimidin-5-ylloxymethyl]-2-methoxy-ethyl]carbamate (1.00 eq, 0.30 g, 0.582 mmol) to afford 2,6-dichloro-N-[2-(1H-indol-3-yl)ethyl]-5-[(2S)-2-amino-3-methoxy-propoxy]pyrimidin-4-amine;hydrochloride (260 mg, 0.559 mmol, 96.01% yield) as a beige solid.

UPLC-MS analysis: (2 min, basic): rt = 1.13 min, m/z = 410.3/412.3/414.3 [M+H]+, 96% purity.

1H NMR (400 MHz, DMSO-D6) δ 10.85 (d, J = 2.1 Hz, 1H), 8.43 (s, 3H), 8.27 (t, J = 6.0 Hz, 1H), 7.67 (d, J = 7.7 Hz, 1 H), 7.35 (dt, J = 8.1.1.0 Hz, 1H), 7.21 (d, J = 2.4 Hz, 1H), 7.08 (ddd, J = 8.2, 7.0, 1.2 Hz, 1H), 7.00 (ddd, J = 8.0, 7.0, 1.1 Hz, 1H), 4.15 (dd, J = 10.2, 4.1 Hz, 1H), 4.05 (dd, J = 10.1, 6.1 Hz, 1H), 3.74 (s, 1H), 3.68 - 3.59 (m, 4H), 3.04 - 2.95 (m, 2H). 3H not observed; underneath water signal.

### 28.4 (7S)-2-chloro-N-[2-(1H-indol-3-yl)ethyl]-7-(methoxymethyl)-7,8-dihydro-6H-pyrimido[5,4-b][1,4]oxazin-4-amine

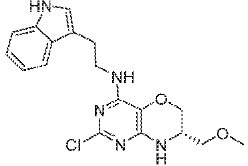

Prepared according to general method 4, using 2,6-dichloro-N-[2-(1H-indol-3-yl)ethyl]-5-[(2S)-2-amino-3-methoxy-propoxy]pyrimidin-4-amine;hydrochloride (1.00 eq. 0.25 g. 0.560 mmol) to afford(7S)-2-chloro-N-[2-(1H-indol-3-yl)ethyl]-7-(methoxymethyl)-7,8-dihydro-6H-pyrimido[5,4-b][1,4]oxazin-4-amine (371 mg, 0.556 mmol, 99.31% yield) as an off-white solid.

UPLCMS (basic, 2min): rt = 1.08 min, m/z = 374.2/376.2 [M+H]+, 96% purity. 1H NMR (400 MHz, DMSO-D6) δ 10.80 (s, 1H), 7.66 (d, J = 7.8 Hz, 1H), 7.33 (dt, J = 8.0, 0.9 Hz, 1H), 7.27 (d, J = 3.2 Hz, 1H), 7.16 (d, J = 2.4 Hz, 1H), 7.07 (ddd, J = 8.2. 7.0. 1.2 Hz, 1H), 6.98 (ddd, J = 7.9. 7.0, 1.1 Hz, 1H), 6.72 (t, J = 5.9 Hz, 1H), 4.05 (dd, J = 10.7, 3.9 Hz, 1H), 3.96 (dd, J = 10.7, 2.9 Hz, 1H), 3.69 - 3.61 (m, 1H), 3.57 - 3.47 (m, 2H), 3.07 (q, J = 7.3 Hz, 2H), 2.95 - 2.86 (m, 2H), 1.19 (d, J = 7.3 Hz, 3H).

### Example 28 (7S)-2-(5-fluoro-3-pyridyl)-N-[2-(1H-indol-3-yl)ethyl]-7-(methoxymethyl)-7,8-dihydro-6H-pyrimido[5,4-b][1,4]oxazin-4-amine

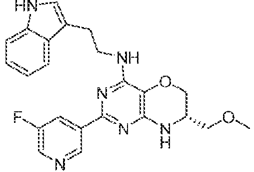

Prepared according to general method 5b, using (7S)-2-chloro-N-[2-(1H-indol-3-yl)ethyl])-7-(methoxymethyl)-7,8-dihydro-6H-pyrimido[5,4-b][3,4]oxazin-4-amine (1.00 eq, 330 mg, 0.883 mmol) and 5-Fluoropyridine-3-boronic Acid (2.00 eq, 249 mg, 1.77 mmol). The crude was purified by column chromatography (20 g column) eluting with a gradient of 30 to 70% (v/v) EtOAc in iso-Hexane to afford (7S)-2-(5-fluoro-3-pyridyl)-N-[2-(1H-indol-3-yl)ethyl]-7-(methoxymethyl)-7,8-dihydro-6H-pyrimido[5,4-b][1,4] oxazin-4-amine (60 mg,0.138 mmol, 15.64% yield) as a brown solid.

UPLC-MS analysis: (4 min, basic): rt = 1.87 min, m/z = 435.3 [M+H]+, 100% purity.

1H NMR (400 MHz, DMSO-D6) δ 10.89 - 10.72 (m, 1H), 9.23 (t, J = 1.8 Hz, 1H), 8.60 (d, J = 2.9 Hz, 1H), 8.29 - 8.15 (m, 1H), 7.63 (d, J = 7.8 Hz, 1H), 7.35 (d, J = 8.1 Hz, 1H), 7.20 (d, J = 2.3 Hz, 1H), 7.11 - 7.01 (m, 2H), 7.02 - 6.92 (m, 1H), 6.56 (t, J = 5.9 Hz, 1H), 4.18 - 3.99 (m, 2H), 3.79 - 3.63 (m, 3H), 3.48 - 3.35 (m, 2H), 3.32 (s, 3H), 3.00 (t, J = 7.6 Hz, 2H).

19F NMR (376 MHz, DMSO-D6) δ -128.09.

### Intermediates for Example 29

### 29.1 tert-butyl N-[(1S)-1-(methoxymethyl)-2-(2,4,6-trichloropyrimidin-5-yl)oxy-ethyl] carbamate

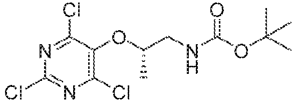

Prepared according to general method 1. using 2,4,6-trichloro pyrimidin-5-ol (1.00 eq, 150 mg, 0.715 mmol) and (R)-1-(Boc-amino)-2-propanol (1.10 eq, 138 mg, 0.786 mmol).

Further addition of DIAD and amino-alcohol and triphenylphosphine repeated and mixture stirred for another 24 hours which took the reaction to completion. The crude was purified by column chromatography eluting with 9:1 and 4:1 Hexane:EtOAc to afford tert-butyl N-[(2S)-2-(2,4.6-trichloropyrimidin-5-yl)oxypropyl]carbamate (150 mg,0.421 mmol, 58.86% yield) as a white solid. UPLC-MS analysis: (4 min, basic): rt = 2.05 min, no ionisation, 93% purity.

1H NMR (400 MHz, DMSO-D6) δ 7.02 (t, J = 5.9 Hz, 1H), 4.62 (q, J = 6.1 Hz, 1H), 3.29 - 3.12 (m, 2H), 1.36 (s, 9H), 1.26 (d. J = 6.3 Hz, 3H).

### 29.2 tert-butyl N-[(2S)-2-[2,4-dichloro-6-[2-(1H-indol-3-yl)ethylamino]pyrimidin-5-yl]oxypropyl]carbamate

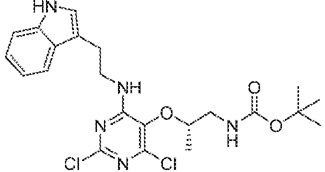

Prepared according to general method 2a. using tert-butyl N-[(2S)-2-(2,4,6-trichloropyrimidin-5-yl)oxypropyl]carbamate (1.00 eq, 150 mg, 0.421 mmol) and Tryptamine (1.05 eq, 71 mg, 0.442 mmol). The reaction was concentrated to dryness to give an oily residue, which was suspended in water (15 mL) with stirring. The resultant suspension was filtered and the solid was dried under vacuum to afford tert-butyl N-[(2S)-2-[2,4-dichloro-6-[2-(1H-indol-3-yl)ethylamino]pyrimidin-5-yl]oxypropyl]carbamate (165 mg,0.343 mmol, 81.66% yield) as a brown solid. UPLC-MS (4 min, basic): rt = 2.21 min, m/z = 480.3/482.3/484.3 [M+H]+, 100% purity. 1H NMR (400 MHz, DMSO-D6) δ 10.82 (d, J = 2.4 Hz, 1H), 7.96 (d, J = 6.3 Hz, 1H), 7.69 - 7.64 (m, 1H), 7.34 (dt, J = 8.1, 1.0 Hz, 1H), 7.17 (d. J = 2.3 Hz, 1H), 7.11 - 6.92 (m, 3H), 4.37 (q, J = 5.9 Hz, 1H), 3.68 - 3.54 (m, J = 6.5 Hz, 2H), 3.17 (t, J = 5.6 Hz, 2H), 2.96 (t, J = 7.7 Hz, 2H), 1.37 (d. J = 2.5 Hz, 9H), 1.16 (d, J = 6.2 Hz, 3H).

### 29.3 2,6-dichloro-N-[2-(1H-indol-3-yl)ethyl]-5-[(1S)-2-amino-1-methyl-ethoxy]pyrimidin-4-amine;hydrochloride

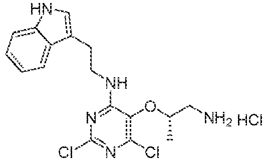

Prepared according to general method 3, using tert-butyl N-[(25)-2-[2,4-dichloro-6-[2-(1H-indol-3-yl)ethylamino]pyrimidin-5-yl)oxypropyl)carbamate (1.00 eq, 200 mg, 0.416 mmol) to afford2,6-dichloro-N-[2-(1H-indol-3-yl)ethyl]-5-[(1S)-2-amino-1-methyl-ethoxy)pyrimidin-4- amine;hydrochloride (173 mg,0.415 mmol, 99.71% yield) as a brown solid.

UPLC-MS analysis (2 min, basic): rt = 1.13 min, m/z = 380.3/382.3/384.3 [M+H] +, 88% purity.

### 29.4 (6S)-2-chloro-N-[2-(1H-Indol-3-yl)ethyl]-6-methyl-7,8-dihydro-6H-pyrimido[5,4-b][1,4]oxazin-4-amine

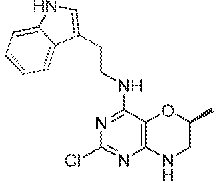

Prepared according to general method 4, using 2,6-dichloro-N-[2-(1H-indol-3-yl)ethyl]-5-[(1S)-2-amino-1-methyl-ethoxy]pyrimidin-4-amine;hydrochloride (1.00 eq, 173 mg, 0.415 mmol). The reaction mixture volume was reduced to dryness and the crude was purified by column chromatography to afford (6S)-2-chloro-N-[2-(1H-indol-3-yl)ethyl]-6- methyl-7,8-dihydro-6H-pyrimido[5,4-b][1,4]oxazin-4-amine (70 mg,0.204 mmol, 49.04% yield) as a white solid.

UPLC-MS analysis (4 min, basic): rt = 1.74 min, m/z = 344.2/346.2 [M+H]+, 100% purity.

1H NMR (400 MHz, DMSO-D6) δ 10.79 (s, 1H), 7.67 (d, J = 7.8 Hz, 1H), 7.33 (dt, J = 8.1, 1.0 Hz, 1H), 7.15 (d, J = 2.3 Hz, 1H), 7.12 - 7.02 (m, 2H), 6.97 (ddd, J = 8.0, 7.0, 1.1 Hz, 1H), 6.48 (t, J = 5.9 Hz, 1H), 4.08 - 3.96 (m, 1H), 3.52 (dt, J = 8.3, 6.4 Hz, 2H), 3.41 (ddd, J = 12.7, 4.2, 2.6 Hz, 1H), 3.03 (ddd, J = 12.6, 7.9, 1.3 Hz, 1H), 2.90 (dd, J = 8.9, 6.4 Hz, 211), 1.29 (d, J = 6.2 Hz, 3H).

### Example 29 (6S)-2-(5-fluoro-3-pyridyl)-N-[2-(1H-indol-3-yl)ethyl]-6-methyl-7,8-dihydro-6H-pyrimido[5,4-b][1,4]oxazin-4-amine

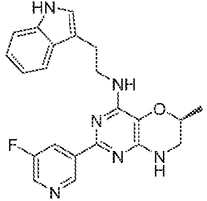

Prepared according to general method 5b, using (6S)-2-chloro-N-[2-(1H-indol-3-yl)ethyl]-6-methyl-7,8-dihydro-6H-pyrimido[5,4-b][1,4]oxazin-4-amine (1.00 eq, 70 mg, 0.204 mmol) and 5-fluoropyridine-3-boronic acid (2.00 eq, 57 mg, 0.407 mmol). The crude was purified by column chromatography eluting with 1:1 and 2:1 EtOAc:Hex to afford (6S)-2-(5-fluoro-3-pyridyl)-N-[2-(1H-indol-3-yl)ethyl]-6-methyl-7,8-dihydro-6H-pyrimido[5,4-b][1,4]oxazin-4-amine (18 mg,0.0441 mmol, 21.64% yield) as a beige solid.

UPLC-MS analysis: (4 min, basic): rt = 1.89 min, m/z = 405.3 [M+H]+, 99% purity.

1H NMR (400 MHz, DMSO-D6) δ 10.81 (s, 1H), 9.22 (t, J = 1.7 Hz, 1H), 8.58 (d, J = 2.9 Hz, 1H), 8.21 (ddd, J = 10.3, 2.9, 1.6 Hz, 1H), 7.67 - 7.62 (m, 1H), 7.34 (dt, J = 8.1, 0.9 Hz, 1H), 7.19 (d, J = 2.3 Hz, 1H), 7.07 (ddd, J = 8.2, 6.9, 1.2 Hz, 1H), 6.97 (ddd, J = 7.9, 7.0, 1.0 Hz, 1H), 6.91 (d, J = 3.6 Hz, 1H), 6.32 (t, J = 6.0 Hz, 1H), 4.15 - 4.05 (m, 1H), 3.71 (q, J = 6.9 Hz, 2H), 3.46 (ddd, J = 12.4, 4.0, 2.6 Hz, 1H), 3.10 (ddd, J = 12.5, 7.8, 1.4 Hz, 1H), 3.03 - 2.95 (m, 2H), 1.33 (d, J = 6.2 Hz, 3H).

19F NMR (376 MHz, DMSO-D6) δ -128.14 (dd, J = 10.4, 1.9 Hz).

### Intermediates for Example 30

### 30.1 tert-butyl N-[(2R)-2-(2,4,6-trichloropyrimidin-5-yl)oxypropyl]carbamate

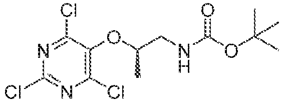

Prepared according to general method 1, using 2,4,6-trichloropyrimidin-5-ol (1.00 eq, 200 mg, 0.9528 mmol) and (S)-1-(Boc-amino)-2-propanol (1.10 eq, 184 mg, 1.047 mmol).

The crude was purified by column chromatography (40 g column) eluting with 18-20% EtOAc in Iso-Hexane to afford tert-butyl N-[(2R)-2-(2,4,6-trichloropyrimidin-5-yl)oxypropyl]carbamate (335 mg,0.705 mmol, 73.94% yield) as a white solid. UPLC-MS analysis: (2 min, basic): rt = 1.24 min, no ionisation, 100% purity. 1H NMR (400 MHz, DMSO-D6) δ 7.01 (t, J = 5.8 Hz, 1H), 4.63 (dt, J = 12.1, 6.3 Hz, 1H), 3.29 - 3.21 (m, 1H), 3.21 - 3.14 (m, 1H), 1.38 (s, 9H), 1.26 (d, J = 6.3 Hz, 3H).

### 30.2 tert-butyl N-[(2R)-2-[2,4-dichloro-6-[2-(1H-Indol-3-yl)ethylamino]pyrimidin-5-yl] oxypropyl)carbamate

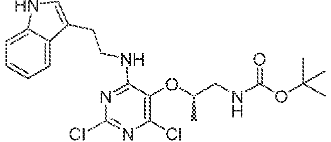

Prepared according to general method 2a, using tert-butyl N-[(2R)-2-(2,4,6-trichloropyrimidin-5-yl)oxypropyl] carbamate (1.00 eq, 335 mg, 0.9393 mmol) and Tryptamine(1.05 eq, 158 mg, 0.9863 mmol) to afford tert-butyl N-[(2R)-2-[2,4-dichloro-6-[2-(1H-indol-3-yl)ethylamino]pyrimidin-5-yl)oxypropyl] carbamate (383 mg,0.551 mmol, 58.61% yield) as an off-white solid.

UPLC-MS (2 min, acidic): rt = 1.28 min, m/z = 480.3/482.3/484.3 [M+H]+, 69% purity.

1H NMR (400 MHz, DMSO-D6) δ 10.82 (s, 1H), 7.95 (s, 1H), 7.67 (s, 1H), 7.37 - 7.31 (m, 1H), 7.17 (d, J = 2.4 Hz, 1H), 7.10 - 7.04 (m, 1H), 7.04 - 6.94 (m, 2H), 4.37 (d, J = 6.0 Hz, 1H), 3.68 - 3.57 (m, 2H), 3.17 (t, J = 5.8 Hz, 2H), 2.96 (t, J = 7.7 Hz, 211), 1.37 (s, 9H), 1.19 - 1.18 (m, 3H).

### 30.3 5-[(1R)-2-amino-1-methyl-ethoxy]-2,6-dichloro-N-[2-(1H-indol-3-yl)ethyl] pyrimidin-4-amine;hydrochloride

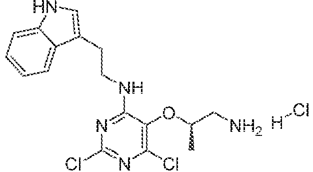

Prepared according to general method 3, using tert-butyl N-((2R)-2-(2,4-dichloro-6-(2-(1H-indol-3-yl)ethylamino] pyrimidin-5-yl]oxypropyl]carbamate (1.00 eq, 383 mg, 0.5505 mmol) to afford 5-[(1R)-2-amino-1-methyl-ethoxy]-2,6-dichloro-N-[2-(1H-indol-3-yl)ethyl]pyrimidin-4-amine;HCl (227 mg,0.387 mmol, 70.25% yield) as a yellow solid. UPLC-MS analysis (2 min, acidic): rt = 0.96 min, m/z = 380.1/382.1/384.1 [M+H] +, 71% purity. 1H NMR (400 MHz, DMSO-D6) δ 10.85 (s, 1H), 8.21 (s, 4H), 7.68 (d, J = 7.8 Hz, 1H), 7.35 (dt, J = 8.0, 0.9 Hz, 1H), 7.19 (d, J = 2.4 Hz, 1H), 7.08 (ddd, J = 8.2, 5.9, 1.3 Hz, 1H), 7.00 (ddd, J = 8.0, 7.0, 1.1 Hz, 1H), 4.83 - 4.68 (m, 1H), 4.70 - 4.57 (m, 1H), 3.63 (d, J = 6.7 Hz, 1H), 3.20 - 3.13 (m, 2H), 2.97 (t, J = 7.7 Hz, 2H), 1.17 (d, J = 6.6 Hz. 3H).

### 30.4 (6R)-2-chloro-N-[2-(1H-indol-3-yl)ethyl]-6-methyl-7,8-dihydro-6H-pyrimido[5,4-b][1,4]oxazin-4-amine

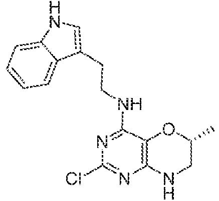

Prepared according to general method 4, using 5-[(1R)-2-amino-2-methyl-ethoxy]-2,6-dichloro-N-[2-(1H-indol-3-yl)ethyl]pyrimidin-4-amine;hydrochloride (1.00 eq, 227 mg, 0.3541 mmol). The reaction mixture volume was reduced to dryness and the crude was purified by column chromatography (40g cartridge) eluting with EtOAc/DCM 0%. 20% to afford (6R)-2-chloro-N-[2-(1H-indol-3-yl)ethyl]-6-methyl -7,8-dihydro-6H-pyrimido[5,4-b][1,4]oxazin-4-amine (71 mg, 0.2065 mmol, . % yield) as a beige solid. UPLC-MS analysis (2 min, basic): rt = 1.09 min, m/z = 344.2/346.2 [M+H]+, 100% purity. 1H NMR (400 MHz, DMSO-D6) δ 10.80 (s, 1H), 7.71 - 7.60 (m, 1H), 7.38 - 7.28 (m, 1H), 7.16 (d, J = 2.3 Hz, 1H), 7.13 - 7.05 (m, 2H), 6.98 (ddd, J = 7.9, 6.9, 1.1 Hz, 1H), 6.49 (t, J = 5.9 Hz, 1H), 4.07 - 3.93 (m, 1H), 3.52 (q, J = 7.0 Hz, 211), 3.42 (ddd, J = 12.5, 4.0, 2.5 Hz, 1H), 3.04 (dd, J = 12.5, 8.0 Hz, 1H), 2.95 - 2.87 (m, 2H), 1.29 (d, J = 6.3 Hz, 3H).

### Example 30 (6R)-2-(5-fluoro-3-pyridyl)-N-[2-(1H-indol-3-yl)ethyl]-6-methyl-7,8-dihydro-6H-pyrimido[5,4-b][1,4]oxazin-4-amine

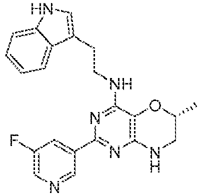

Prepared according to general method 5b, using (6R)-2-chloro-N-[2-(1H-indol-3-yl)ethyl]-6-methyl-7,8-dihydro-6H-pyrimido[5,4-b][1,4]oxazin-4-amine (1.00 eq. 71 mg, 0.2065 mmol) and 5-fluoropyridine-3-boronic acid (1.50 eq, 44 mg, 0.3098 mmol). The crude was purified by column chromatography (12g cartridge) eluting with DCM/EtOAc 60%-80% followed by prep HPLC to afford (6R)-2-(5-fluoro-3-pyridyl)-N-[2-(1H-indol-3-yl)ethyl]-6-methyl-7,8-dihydro-6H-pyrimodo[5,4-b][1,4]oxazin-4-amine (24 mg,0.0592 mmol, 28.69% yield) as a white solid.

UPLC-MS analysis: (4 min, basic): rt = 1.90 min, m/z = 405.0 [M+H]+, 100% purity.

1H NMR (400 MHz, DMSO-D6) δ 10.82 (s, 1H), 9.23 (t, J = 1.7 Hz, 1H), 8.59 (d. J = 2.9 Hz, 1H), 8.22 (ddd, J = 10.3, 2.9, 1.6 Hz, 1H), 7.64 (d. J = 7.9 Hz, 1H), 7.35 (dt, J = 8.2, 1.0 Hz, 1H), 7.20 (d, J = 2.3 Hz, 1H), 7.15 - 7.06 (m, 1H), 6.98 (ddd, J = 8.0, 7.0, 1.1 Hz, 1H), 6.92 (d, J = 3.6 Hz, 1H), 6.33 (t, J = 6.0 Hz, 1H), 4.10 (ddd, J = 7.8, 6.3, 2.5 Hz, 1H), 3.72 (q, J = 6.8 Hz. 2H), 3.47 (ddd, J = 12.4, 4.0, 2.5 Hz, 1H), 3.16 - 3.06 (m, 1H), 3.00 (dd, J = 8.8, 6.4 Hz, 2H), 1.33 (d. J = 6.2 Hz, 3H) 19F NMR (376 MHz, DMSO-D6) δ -128.14

### Intermediates for Example 31

### 31.1 tert-butyl N-[1,1-dimethyl-2-(2,4,6-trichloropyrimidin-5-yl)oxy-ethyl]carbamate

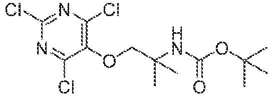

Prepared according to general method 1, using 2,4,6-trichloro pyrimidin-5-ol (1.00 eq, 250 mg, 1.25 mmol) and tert-butyl N-(2-hydroxy-1,1-dimethyl-ethyl)carbamate (1.10 eq, 261 mg, 1.38 mmol). The crude was purified by column chromatography eluting with hexane and 20:1 and 9:1 Hex:EtOAc to afford tert-butyl N-[1,1-dimethyl-2-(2,4,6-trichloropyrimidin-5-yl)oxy-ethyl]carbamate (40 mg, 0.108 mmol, 8.61% yield) as a colourless oil. UPLC-MS analysis: (4 min, basic): rt = 2.15 min, no ionisation, 100% purity.

1H NMR (400 MHz DMSO-D6) δ 7.02 (s, 1H), 3.36 (d, J = 6.6 Hz, 2H), 1.38 (s, 15H).

### 31.2 tert-butyl N-[2-[2,4-dichloro-6-[2-(1H-indol-3-yl)ethylamino]pyrimidin-5-yl]oxy-1,1-dimethyl-ethyl]carbamate

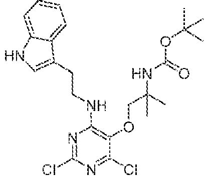

Prepared according to general method 2a, using tert-butyl N-[1,1-dimethyl-2-(2,4,6-trichloro pyrimidin-5-yl)oxy-ethyl]carbamate (1.00 eq, 40 mg, 0.108 mmol) and Tryptamine (1.00 eq, 17 mg, 0.108 mmol) in Ethanol (2 mL). The reaction was concentrated to dryness to give an oily residue, which was suspended in water (10 mL) with stirring.

The resultant suspension was filtered and the solid was dried under vacuum to afford tert-butyl N-[2-[2,4-dichloro-6-[2-(1H-indol-3-yl)ethylamino]pyrimidin-5-yl]oxy-1,1-dimethyl-ethyl)curbamate (64 mg,0.104 mmol, 95.96% yield) as an off-white solid.
UPLC-MS (4 min, basic): rt = 2.31 min, m/z = 494.0/496.0/468.0, [M+H]+, 93% purity
1H NMR (400 MHz, DMSO-D6) δ 10.83 (s, 1H), 8.19 (s, 1H), 7.65 (d, J = 7.7 Hz, 1H), 7.41 - 7.31 (m, 2H), 7.18 (s, 1H), 7.07 (t, J = 7.5 Hz, 1H), 6.98 (t, J = 7.4 Hz, 1H), 3.65 (s, 2H), 3.15 (s, 1H), 3.00 (t, J = 7.8 Hz, 2H), 1.42 (s, 9H), 1.39 (s, 6H). 1H not observed; underneath water peak.

### 31.3 5-(2-amino-2-methyl-propoxy)-2,6-dichloro-N-[2-(1H-indol-3-yl)ethyl]pyrimidin-4-amine

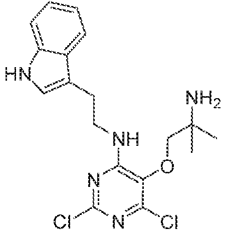

Prepared according to general method 3, using tert-butyl N-[2-[2,4-di chloro-6-[2-(1H-indol-3-yl)ethylamino]pyrimidin-5-yl]oxy-1,1-dt methyl-ethyl]carbamate (1.00 eq, 64 mg, 0.104 mmol). The crude was purified by column chromatography eluting with MeOH (containing 5% ammonia) In EtOAc to afford 5-(2-amino-2-methyl-propoxy)-2,6-dichloro-N-[2-(1H-indol-3-yl)ethyl]pyrimidin-4-amine (14 mg,0.0320 mmol, 30.86% yield) as a white solid.

UPLC-MS analysis (4 min, basic): rt = 2.00 min, m/z = 394.3/396.3/398.3 [M+H] +, 90% purity

### 31.4 2-chloro-N-[2-(1H-indol-3-yl)ethyl)-7,7-dimethyl-6,8-dihydropyrimido[5,4-b][1,4] oxazin-4-amine

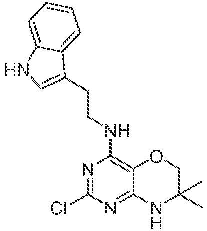

Prepared according to general method 4, using 5-(2-amino-2-methyl-propoxy)-2,6-dichloro-N-[2-(1H-indol-3-yl)ethyl]pyrimidin-4-amine (1.00 eq, 14 mg, 0.0355 mmol) to afford 2-chloro-N-[2-(1H-indol-3-yl)ethyl]-7,7-dimethyl-6.8-dihydropyrimido[S,4-b][1,4]oxazin-4-amine (12 mg,0.0335 mmol, 103.18% yield). UPLC-MS analysis (2 min, basic): rt = 1.11 min, m/z = 358.3/360.3 [M+H]+, 96% purity.

### Example 31 2-(5-fluoro-3-pyridyl)-N-[2-(1H-indol-3-yl)ethyl])-7,7-dimethyl-6,8-dihydro pyrimido[5,4-b][1,4]oxazin-4-amine

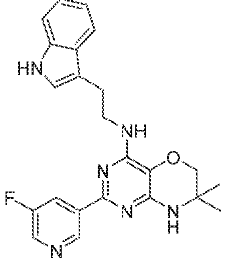

Prepared according to general method 5b, using 2-chloro-N-[2-(1H-indol-3-yl)ethyl]-7,7-dimethyl-6,8-dihydropyrimido[5,4-b][1,4]oxazin-4-amine (1.00 eq, 12 mg, 0.0335 mmol) and 5-fluoropyridine-3-boronic acid (2.00 eq. 9.5 mg, 0.0671 mmol). The crude was purified by column chromatography eluting with 2:1 and 1:1 Hex:EtOAc to afford 2-(5-fluoro-3-pyridyl)-N-[2-(1H-indol-3-yl)ethyl])-7,7-dimethyl-6,8-dihydropyrimido[5,4-b][1,4]oxazin-4-amine (5.0 mg,0.0114 mmol, 33.85% yield) as a white solid.

UPLC-MS analysis: (4 min, basic): rt = 1.17 min, m/z = 419.3 [M+H]+, 95% purity.

1H NMR (400 MHz, DMSO-D6) δ 10.81 (s, 1H), 9.23 (t, J = 1.7 Hz, 1H), 8.58 (d, J = 2.9 Hz, 1H), 8.24 - 8.18 (m, 1H), 7.63 (d, J = 7.9 Hz, 1H), 7.34 (d. J = 8.1 Hz, 1H), 7.17 (d, J = 2.2 Hz, 1H), 7.07 (t, J = 7.6 Hz, 1H), 7.02 - 6.92 (m, 2H), 6.22 (t, J = 6.0 Hz, 1H), 3.70 (q, J = 7.1 Hz, 2H), 3.16 (d, J = 2.6 Hz, 2H), 2.99 (t, J = 7.6 Hz, 2H), 1.28 (s, 6H). 19F NMR (376 MHz, DMSO-D6) δ -128.17 (dd, J = 10.2, 2.0 Hz).

### Intermediates for Example 32

### 32.1 tert-butyl N-[(1R)-2,2,2-trifluoro-1-[(2,4,6-trichloropyrimidin-5-yl)oxymethyl] ethyl]carbamate

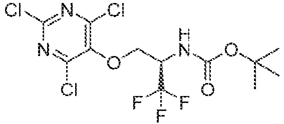

Prepared according to general method 1, using 2,4,6-trichloropyrimidin-5-ol (1.00 eq, 210 mg, 1.05 mmol) and tert-butyl N-[(1R)-2,2,2-trifluoro-1-(hydroxymethyl)ethyl]carbamate (1.10 eq, 265 mg, 1.16 mmol). The crude was purified by column chromatography eluting with hexane and then with 20: and 10:1 Hex:EtOAc to afford tert-butyl N-[(1R)-2,2,2-trifluoro-1-[(2,4,6-trichloropyrimidin-5-yl)oxymethyl]ethyl]carbamate (290 mg,0.706 mmol, 67.07% yield) as a white solid. UPLC-MS analysis: (4 min, basic): rt = 2.16 min, no ionisation, 100% purity.

1H NMR (400 MHz, DMSO-D6) δ 7.84 (d, J = 9.1 Hz, 1H), 4.66 (dt, J = 13.1, 8.3 Hz, 1H), 4.45 (dd, J = 10.6, 4.4 Hz, 1H), 4.26 (dd, J = 10.7, 8.1 Hz, 1H), 1.41 (s, 9H).

19F NMR (376 MHz, DMSO-D6) δ -72.99 (d, J = 8.1 Hz).

### 32.2 tert-butyl N-[(1R)-1-[[2,4-dichloro-6-[2-(1H-indol-3-yl)ethylamino]pyrimidin-5-yl] oxymethyl]-2,2,2-trifluoro-ethyl]carbamate

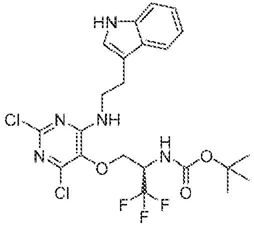

Prepared according to general method 2a, using tert-butyl N-[(1R)-2,2,2-trifluoro-1-[(2,4,6-trichloropyrimidin-5-yl)oxymethyl] ethyl]carbamate (1.00 eq, 290 mg, 0.706 mmol) and Tryptamine (1.00 eq, 113 mg, 0.706 mmol) in Ethanol (5 mL). The reaction was concentrated to dryness to give an oily residue, which was suspended in water (10 mL) with stirring. The resultant suspension was filtered and the solid was dried under vacuum to afford tert-butyl N-[(1R)-1-[[2,4-dichloro-6-[2-(1H-indol-3-yl)ethylamino]pyrimidin-5-yl]oxymethyl]-2,2,2-trifluoro-ethyl]carbamate (500 mg,0.702 mmol, 99.36% yield) as an off white solid. UPLC-MS (4 min, basic): rt = 2.28 min. m/z = 534.0/535.9/537.9, [M+11]+. 100% purity

### 32.3 2,6-dichloro-N-[2-(1H-indol-3-yl)ethyl]-5-[(2R)-2-amino-3,3,3-trifluoro-propoxy] pyrimidin-4-amine;hydrochloride

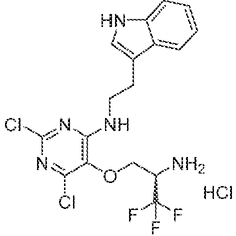

Prepared according to general method 3, using tert-butyl N-[(1 R)-1-([2,4-dichloro-6-[2-(1H-indol-3-yl)ethylamino]pyrimidin-5-yl]oxymethyl]-2,2,2-trlfluoro-ethyl]carbamate to afford 2,6-dichloro-N-[2-(1H-indol-3-yl)ethyl]-5-[(2R)-2-amino-3,3,3-trifluoro-propoxy]pyrimidin-4-amine;hydrochloride (260 mg,0.536 mmol, 57.26% yield) as a purple solid. UPLC-MS analysis (4 min, basic): rt = 2.02 min, m/z = 433.9/435.8/437.9 [M+H] +, 97% purity 1HNMR (400MHz, DMSO-D6) δ 10.85 (s, 1H), 9.77 - 9.71 (m, 3H), 8.61 (t, J = 6.0 Hz, 1H), 7.70 (d, J= 7.8 Hz, 1H), 7.34 (d, J = 8.1 Hz, 1H), 7.21 (d, J = 2.3 Hz, 1H), 7.07 (t, J = 7.5 Hz, 1H), 6.99 (t, J = 7.4 Hz, 1H), 4.88 - 4.74 (m, 1H), 4.44 (dd, J = 11.0, 4.0 Hz, 1H), 3.65 (dt, J = 13.1, 7.0 Hz, 2H), 3.00 (t, J= 7.8 Hz, 2H). 1H not observed; under water peak. 19FNMR (376MHz, DMSO-D6) δ -70.26 (d, J=8.1 Hz).

### 32.4 (7R)-2-chloro-N-[2-(1H-indol-3-yl)ethyl]-7-(trifluoromethyl)-7,8-dihydro-6H-pyrimido[5,4-b][1,4]oxazin-4-amine

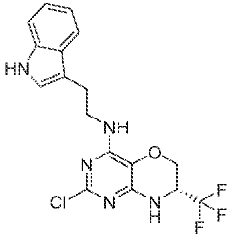

Prepared according to general method 4, using 2,6-dichloro-N-(2-(1H-indol-3-yl)ethyl]-5-[(2R)-2-amino-3,3,3-trifluoro-propoxy] pyrimidin-4-amine;hydrochloride (1.00 eq, 252 mg, 0.536 mmol) at 165 °C. The crude was purified by column chromatography eluting with Hexane:Acetone to afford (7R)-2-chloro-N-[2-(1 H-indol-3-yl) ethyl)-7-(trifluoromethyl)-7,8-dihydro-6H-pyrimido[5,4-b][1,4] oxazin-4-amine (23 mg,0.0578 mmol, 10.79% yield) as a yellow solid.

UPLC-MS analysis (2 min, basic): rt = 1.80 min, m/z = 398.3/400.3, [M+H]+, 99% purity.

1H NMR (400 MHz, DMSO-D6) δ 10.79 (s, 1H), 8.02 (d, J = 5.5 Hz, 1H), 7.65 (d, J = 7.9 Hz, 1H), 7.32 (d, J = 8.1 Hz, 1H), 7.16 (s, 1H), 7.06 (t, J = 7.6 Hz, 1H), 6.97 (t, J = 7.4 Hz, 2H), 4.48 (d. J = 12.2 Hz, 2H), 3.90 (d, J = 12.1 Hz, 1H), 3.52 (q, J = 7.2 Hz, 2H), 2.90 (t, J = 7.9 Hz, 2H).

### Example 32 (7R)-2-(5-fluoro-3-pyridyl)-N-[2-(1H-indol-3-yl)ethyl]-7-(trifluoromethyl)-7,8-dihydro-6H-pyrimido[5,4-b][1,4]oxazin-4-amine

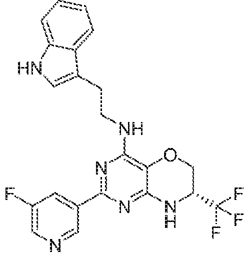

Prepared according to general method 5b, using (7R)-2-chloro-N-[2-(1H-indol-3-yl)ethyl]-7-(trifluoromethyl)-7,8-dihydro-6H-pyrimido [5,4-b][1,4]oxazin-4-amine (1.00 eq, 23 mg, 0.0578 mmol) and 5-fluoropyridine-3-boronic acid (2.00 eq, 16 mg, 0.116 mmol). The crude was purified by column chromatography eluting with 3:1 to 3:2 Hex:EtOAc to afford (7R)-2-(5-fluoro-3-pyridyl)-N-[2-(1H-indol-3-yl)ethyl] 7-(trifluoromethyl)-7,8-dihydro-6H-pyrimido[5,4-b][1,4] oxazin-4-amine (14 mg,0.0305 mmol, 52.82% yield) as a white solid.

UPLC-MS analysis: (4 min, basic): rt = 1.94min, m/z =459.3[M+H]+, 100% purity. 1HNMR (400 MHz, DMSO-D6) δ 10.81 (s, 1H), 9.23 (t, J = 1.7 Hz, 1H), 8.61 (d, J=2.9 Hz, 1H), 8.22 (ddd, J=10.2, 2.9, 1.6 Hz, 1H), 7.83 (d, J = 5.7 Hz, 1H), 7.62 (d, J = 7.8 Hz, 1H), 7.34 (d, J = 1.0 Hz, 1H), 7.19 (d, J = 2.3 Hz, 1H), 7.06 (ddd, J = 8.1, 7.0, 1.2 Hz, 1H), 6.97 (ddd, J = 8.0, 7.0, 1.1 Hz, 1H), 6.77 (t, J = 5.9 Hz, 1H), 4.54 (d, J = 12.1 Hz, 1H), 4.52 - 4.41 (m, 1H), 4.01 - 3.93 (m, 1H), 3.71 (dt, J=10.7, 7.2 Hz, 211), 2.99 (t, J = 7.7 Hz, 2H). 19F NMR (376 MHz, DMSO-D6) δ -73.59 - -73.74 (m), -127.99 (d, J = 10.5 Hz).

### Intermediates for Example 33

### 33.1 2,6-dichloro-N-[2-(1H-indol-3-yl)ethyl]-5-[2-(oxetan-3-ylamino)ethoxy]pyrimidin-4-amine

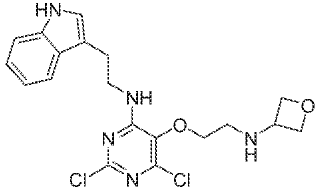

A stirred solution of 5-(2-aminoethoxy)-2,6-dichloro-N-[2-(1H-indol-3-yl)ethyl]pyrimidin-4-amine;hydrochloride (1.00 eq, 150 mg, 0.372 mmol) and 3-Oxetanone (1.30 eq, 0.031 mL, 0.484 mmol) in Methanol (3.7 mL) was treated with Sodium cyanoborohydride (3.00 eq, 70 mg, 1.12 mmol), and the resulting mixture was stirred for 18 h.

After a further addition of 3-Oxetanone (0.01) mL the reaction was stirred at room temperature for 2h. The reaction was quenched by the addition of water (25 mL) and extracted with dichloro methane (2 x 30 mL). The resulting solution was washed sequentially with saturated brine solution (25 mL). The organic phase was then separated and dried (Na2SO4), before being concentration to dryness. The crude was purified by flash column chromatography over silica (40 g column) using iso-Hex/EtOAc 0-100% in 5 CV and then gradient EtOAc/MeOH (product eluted with 10% MeOH) to afford 2,6-dichloro-N-[2-(1H-indol-3-yl)ethyl]-5-[2-(oxetan-3-ylamino) ethoxy]pyrimidin-4-amine (115 mg,0.251 mmol. 67.26% yield) as an off white solid.

UPLC-MS analysis: (2 min, basic): rt = 1.12 min, m/z = 422.1/424.1/426.1 [M+H]+, 100% purity.

1H NMR (400 MHz, DMSO-D6) δ 10.86 (s, 1H), 8.74 (t, J = 5.8 Hz, 1H), 7.69 - 7.53 (m, 1H), 7.36 (dt, J = 8.1, 1.0 Hz, 1H), 7.19 (d, J = 2.4 Hz, 1H), 7.08 (s, 1H), 7.04 - 6.93 (m, 1H), 4.57 (t, J = 6.6 Hz, 2H), 4.27 (t, J = 6.1 Hz, 2H), 3.94 - 3.83 (m, 2H), 3.80 (p, J = 6.5 Hz, 1H), 3.60 (td, J = 8.1, 7.6, 6.0 Hz, 2H), 2.97 (t, J = 7.5 Hz, 2H), 2.88 (s, 1H), 2.70 (t, J = 5.1 Hz, 2H).

### 33.2 2-chloro-N-[2-(1H-Indol-3-yl)ethyl])-8-(oxetan-3-yi)-6,7-dihydropyrimido[5,4-b][1,4] oxazin-4-amine

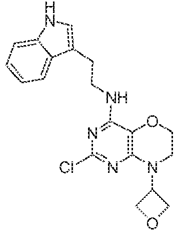

To a solution of 2,6-dichloro-N-[2-(1H-indol-3-yl)ethyl]-5-[2-(oxetan-3-ylamino)ethoxy]pyrimidin-4-amine (1.00 eq, 95 mg, 0.207 mmol) in 1,4-Dioxane (4.1 mL), Potassium tert-butoxide (1.25 eq. 29 mg, 0.259 mmol) was added at room temperature. Upon degassing the solution with N2 flow, Tri-o-tolylphosphin (0.0800 eq, 5.0 mg, 0.0166 mmol) and Tris(dibenzyl ideneacetone)dipalladium(0) (Pd2(dba)3) (0.0400 eq, 7.6 mg, 0.00828 mmol) were added sequentially and stirred at 100°C on a pre-heated plate for 24h. The reaction was diluted with EtOAc and filtered through a celite pad. The filtrate was washed with water then the organic was dried over Na2SO4 and concentrated to dryness. The crude was purified by flash column chromatography over silica (20 g cartridge, DCM loading) eluting with a gradient of EtOAc in iso- Hex (0% to 100% v/v; product eluted with 80% EtOAc) to afford 2-chloro-N-[2-(1H-indol-3-yl)ethyl]-8-(oxetan-3-yl)-6,7-dihydropyrimido[5,4-b][1,4] oxazin-4-amine (15 mg,0.0389 mmol, 18.78% yield) as a yellowish solid.

UPLC-MS analysis: (2 min, acidic): rt = 1.11 min, m/z = 386.0/387.9 [M+H]+, 100% purity.

1H NMR (400 MHz, DMSO-D6) δ 10.80 (s, 1H), 7.66 (d, J = 7.8 Hz, 1H), 7.34 (dt, J = 8.1, 1.0 Hz, 1H), 7.16 (d, J = 2.3 Hz, 1H), 7.13 - 7.02 (m, 1H), 7.04 - 6.93 (m, 1H), 6.78 (t, J = 5.9 Hz, 1H), 5.39 (p, J = 7.1 Hz, 1H), 4.84 - 4.58 (m, 4H), 4.34 - 4.14 (m, 2H), 3.63 (t, J = 4.3 Hz, 2H), 3.58 - 3.41 (m, 211), 3.02 - 2.82 (m, 2H).

### Example 33 N-[2-(1H-indol-3-yl)ethyl]-2-(2-methylthiazol-5-yl)-8-(oxetan-3-yl)-6,7-di hydropyrimido[5,4-b][1,4]oxazin-4-amine

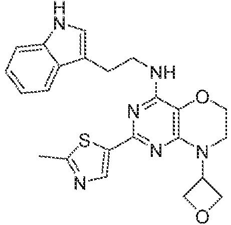

Prepared according to general method 5b using 2-chloro-N-[2-(1H-indol-3-yl)ethyl)-8-(oxetan-3-yl)-6,7-dihydropyrimido(5,4-b][1,4]oxazin-4-amine (1.00 eq, 12 mg, 0.0311 mmol) and 2-methyl-5-(tetramethyl-1,3,2-dioxaborolan-2-yl)-1,3-thiazole (1.50 eq, 11 mg, 0.0467 mmol). The crude was purified by column chromatography (20 g column) eluting with a gradient of 0 to 100% (v/v) EtOAc in iso-Hex to afford N-[2-(1H-indol-3-yl)ethyl]-2-(2-methylthiazol-5-yl)-8-(oxetan-3-yl)-6,7-dihydropyrimido[5,4-b][1,4]oxazin-4-amine (4.5 mg,0.0100 mmol, 32.26% yield) as a grey solid UPLC-MS analysis: (4 min, basic): rt = 1.83 min, m/z = 449.3 [M+H]+, 100% purity. 1HNMR (400 MHz, DMSO-D6) δ 10.80 (s, 1H), 8.06 (s, 1H), 7.70 - 7.60 (m, 1H), 7.35 (dt, J = 8.1, 1.0 Hz, 1H), 7.18 (d, J=2.3 Hz, 1H), 7.12 -7.03 (m, 1H), 7.02 - 6.93 (m, 1H), 6.51 (t, J=5.9 Hz, 1H), 5.25 (p, J = 7.3 Hz, 1H), 4.88 - 4.67 (m, 4H), 4.33 - 4.20 (m, 2H), 3.73 - 3.59 (m, 2H), 3.51 (t, J = 4.3 Hz, 2H), 3.01 - 2.87 (m, 2H), 2.65 (s, 3H).

### Intermediates for Example 34

### 34.1 tert-butyl N-[2-[2,4-dichloro-6-(6,7,8,9-tetrahydro-5H-pyrido[3,2-b]indol-8-ylamino)pyrimidin-5-yl]oxyethyl]carbamate

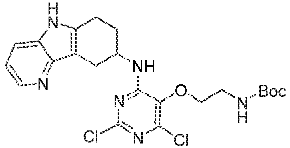

Prepared according to general method 2a, using tert-butyl N-[2-(2,4,6-trichloropyrimidin-5-yl)oxyethyl]carbamate (1.00 eq, 0.30 g, 0.876 mmol) and 6,7,8,9-tetrahydro-5H-pyrido[3,2-b] indol-8-amine (1.10 eq, 0.26 g, 0.963 mmol) (WO202081636) in Ethanol (4.4 mL) at 50° C for 4 h to afford tert-butyl N-[2-[2,4-dichloro-6-(6,7,8,9-tetrahydro-5H-pyrido[3,2-b]indol-8-ylamino)pyrimidin-5-yl]oxyethyl]carbamate (0.52 g,0.843 mmol, 96.29% yield) as a brown solid. UPLC-MS analysis: (2 min, basic): rt = 1.11 min, m/z = 493.3/495.3/497.3 [M+H]+, 89% purity. 1H NMR (400 MHz, DMSO-D6) δ 11.04 (s, 1H), 8.20 (dd, J = 4.6, 1.5 Hz, 1H), 7.93 (d, J = 8.2 Hz, 1H), 7.65 - 7.50 (m, 1H), 7.20 (t, J = 5.9 Hz, 1H), 7.00 (dd, J = 8.1, 4.6 Hz, 1H), 4.44 - 4.21 (m, 1H), 4.09 - 3.94 (m, 211), 3.15 (dd, J = 15.1, 5.4 Hz, 1H), 3.10 - 2.78 (m, 3H), 2.76 - 2.65 (m, 1H), 2.22 - 1.93 (m, 3H), 1.38 (s, 9H).

### 34.2 N-[5-(2-aminoethoxy)-2,6-dichloro-pyrimidin-4-yi]-6,7,8,9-tetrahydro-5H-pyrido[3,2-b]indol-8-amine;dihydrochloride

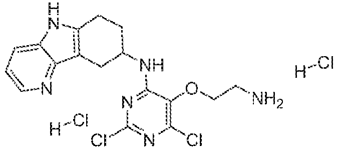

Prepared according to general method 3, using tert-butyl N-[2-[2,4-dichloro-6-(6,7,8,9-tetrahydro-5H-pyrido[3,2-b] indol-8-ylamino)pyrimidin-5-yl]oxyethyl]carbamate (1.00 eq, 0.30 g, 0.486 mmol) to afford N-[5-(2-aminoethoxy) -2,6-dichloro-pyrimidin-4-yl]-6,7,8,9-tetrahydro-5H-pyrido[3,2-b]indol-8-amine;dihydrochloride (0.27 g,0.486 mmol, 100.01% yield) as a brown solid. UPI.C-MS analysis: (2 min, basic): rt = 0.94 min, m/z = 393.3/395.3/397.3 [M+H]+, 90% purity. 1H NMR (400 MHz, DMSO-D6) δ 15.97 (s, 1H), 12.74 (s, 1H), 8.74 - 8.19 (m, 6H), 7.61 - 7.50 (m, 1H), 4.47 (t, J = 7.1 Hz, 1H), 4.33 - 4.15 (m, 2H), 3.34 - 3.18 (m, 3H), 3.15 - 2.94 (m, 3H), 2.35 - 2.24 (m, 1H), 2.18 - 2.06 (m, 1H).

### 34.3 2-chloro-N-(6,7,8,9-tetrahydro-5H-pyrido[3,2-b]indol-8-yl)-7,8-dihydro-6H-pyrimido[5,4-b][1,4]oxazin-4-amine

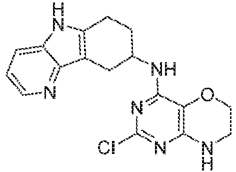

Prepared according to general method 4, using N-[5-(2-amino ethoxy)-2,6-dichloro-pyrimidin-4-yl]-6,7,8,9-tetrahydro-5H-pyrido [3,2-b]indol-8-amine;dihydrochloride (1.0eq, 0.25 g, 0.450mmol) to afford 2-chloro-N-(6,7,8,9-tetrahydro-5H-pyrido[3,2-b]indol-8-yl)-7, 8-dihydro-6H-pyrimido[5,4-b][1,4]oxazin-4-amine (0.17g, 0.412mmol, 91.37% yield) as a brown solid. UPLC-MS analysis: (2 min, basic): rt = 0.86 min, m/z = 357.2/359.2 [M+H]+, 89% purity. 1H NMR (400 MHz, DMSO-D6) δ 10.95 (s, 1H), 8.19 (dd, J = 4.8, 1.4 Hz, 1H), 7.59 (dd, J = 8.0, 1.4 Hz, 1H), 7.16 (s, 1H), 6.99 (dd, J = 8.1, 4.6 Hz, 1H), 6.45 (d, J = 8.3 Hz, 1H), 4.34 - 4.16 (m, 1H), 4.10 (t, J = 4.3 Hz, 2H), 3.45 - 3.36 (m, 2H), 3.04 (dd, J = 15.1, 5.3 Hz, 1H), 2.98 - 2.77 (m, 2H), 2.74 - 2.61 (m, 1H), 2.14 - 1.90 (m, 2H).

### Example 34 2-(2-methylthiazol-5-yl)-N-(6,7,8,9-tetrahydro-5H-pyrido[3,2-b]indol-8-yl)-7,8-dihydro-6H-pyrimido[5,4-b][1,4]oxazin-4-amine

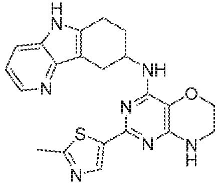

Prepared according to general method 5b, using 2-chloro-N-(6,7,8,9-tetrahydro-5H-pyrido[3,2-b]indol-8-yl)-7,8-dihydro-6H-pyrimido[5,4-b][1,4]oxazin-4-amine (1.00 eq, 90 mg, 0.224 mmol) and 2-methyl-5-(tetramethyl-1,3,2-dioxaborolan-2-yl)-1,3-thiazole (1.50 eq, 76 mg, 0.337 mmol). The crude was purified by column chromatography (20 g column) eluting with a gradient of 0 to 100% (v/v) EtOAc in iso-Hex and then EtOAc/MeOH (0 to 15% MeOH) to afford 2-(2-methylthiazol-5-yl)-N-(6,7,8,9-tetrahydro-5H-pyrido[3,2-b]indol-8-yl)-7,8-dihydro-6H-pyrimido[5,4-b][1,4]oxazin-4-amine (34 mg,0.0786 mmol, 35.02% yield) as a beige solid. UPLC-MS analysis: (4 min, basic): rt = 1.42 min, m/z = 420.1 [M+H]+, 97% purity.

1H NMR (400 MHz, DMSO-D6) δ 10.98 (s, 1H), 8.20 (dd, J = 4.6, 1.5 Hz, 1H), 7.97 (s, 1H), 7.61 (dd, J = 8.1, 1.4 Hz, 1H), 6.99 (dd, J = 8.1, 4.6 Hz, 1H), 6.95 - 6.83 (m, 1H), 6.14 (d. J = 8.1 Hz, 1H), 4.45 - 4.33 (m, 1H), 4.13 (t, J = 4.3 Hz, 2H), 3.48 - 3.38 (m, 2H), 3.09 (dd, J = 15.2, 5.3 Hz, 1H), 3.01 - 2.81 (m, 2H), 2.78 - 2.68 (m, 1H), 2.60 (s, 3H), 2.22 - 2.10 (m, 1H), 2.02 - 1.90 (m, 1H).

### Intermediates for Example 35

### 35.1 (3R)-N-(2,6-dichloro-5-methoxy-pyrimidin-4-yl)-2,3,4,9-tetrahydro-1H-carbazol-3-amine

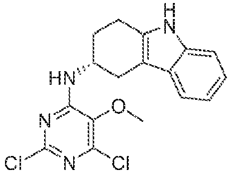

Prepared according to general method 2a, using 2,4,6-trichloro-5-Methoxypyrimidine (1.00 eq, 0.32 mL, 2.34 mmol) and (R)-2,3,4,9-Tetrahydro-1H-carbazol-3-amine (1.05 eq, 0.46 g, 2.46 mmol) in Ethanol (5 mL) to afford (3R)-N-(2,6-dichloro-5-methoxy-pyrimidin-4-yl)-2,3,4,9-tetrahydro-1H-carbazol-3-amine (0.84 g,2.31 mmol, 98.72% yield) as an off-white solid. UPLC-MS (4 min, basic): rt = 2.06 min, m/z = 363.2/365.2/367.2 [M+H]+, 96% purity. 1H NMR (400 MHz, DMSO-D6) δ 10.75 (s. 1H), 8.15 (d, J = 8.3 Hz, 1H), 7.33 (d, J = 7.8 Hz, 1H), 7.25 (d, J = 7.9 Hz, 1H), 7.09 - 6.86 (m, 2H), 4.33 (s, 1H), 3.76 (s, 3H), 3.07 (s, 1H), 2.99 (dd, J = 14.9, 5.3 Hz, 1H), 2.85 (s, 2H), 2.09 - 1.98 (m, 2H).

### 35.2 2,4-dichloro-6-[[(3R)-2,3,4,9-tetrahydro-1H-carbazol-3-yl]amino]pyrimidin-5-ol

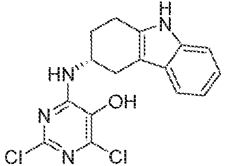

To a solution of (3R)- N-(2,6-dichloro-5-methoxy-pyrimidin-4-yl)-2,3,4,9-tetrahydro-1H-carbazol-3-amine (1.00 eq, 600 mg, 1.65 mmol in DCM (10 mL) was added Boron tribromide (3.00 eq, 5.0 mL, 4.96 mmol) in DCM (1 M) under a nitrogen atmosphere and the mixture was stirred for 18 h. Further addition of boron tribromide solution and stirring, were needed to take the reaction to completion as shown by UPLC analysis. The reaction mixture was quenched with MeOH (10 mL) and water (20 mL). DCM layer was separated, aqueous extracted twice with DCM, organics washed with brine. The organic layer was dried over sodium sulphate, filtered and its volume was reduced to dryness under vacuum at 40 °C in rotatory evaporator obtaining 2,4-dichloro-6-[[(3R)-2,3,4,9-tetrahydro-2 H-carbazol-3-yl]amino]pyrimidin-5-ol (530 mg,1.52 mmol, 91.88% yield) as a red solid. UPLC-MS (4 min, acidic): rt = 0.80/0.82 min, m/z = 349.2/351.2/353.2 [M+H]+, 97% purity. 1H NMR (400 MHz, DMSO-D6) δ 10.75 (s, 1H), 10.21 (s, 1H), 7.64 (d, J = 8.3 Hz, 1H), 7.33 (d, J = 7.7 Hz, 1H), 7.28 - 7.20 (m, 1H), 7.00 (ddd, J = 8.1, 7.0.1.3 Hz, 1H), 6.92 (td, J = 7.4, 7.0, 1.1 Hz, 1H), 4.34 (h, J = 7.3 Hz, 1H), 2.98 (dd, J = 14.9, 5.3 Hz, 1H), 2.83 (tt, J = 11.7, 6.3 Hz, 2H), 2.74 - 2.63 (m, 1H), 2.04 (h, J = 5.1 Hz, 2H).

### 35.3 tert-butyl N-[(1R)-1-[[2,4-dichloro-6-[[(3R)-2,3,4,9-tetrahydro-1H-carbazol-3-yl]amino]pyrimidin-5-yl]oxymethyl]-2-methoxy-ethyl]carbamate

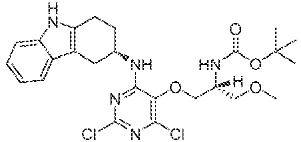

Prepared according to general method 1, using 2,4-dichloro-6-[[(3R)-2,3,4,9-tetrahydro-1H-carbazol-3-yl]amino]pyrimidin-5-of (1.00 eq, 0.75 g, 2.10 mmol) and tert-butyl N-[(15)-1-(hydroxymethyl)-2-methoxy-ethyl]carbamate (1.05 eq, 454 mg, 2.21 mmol). The crude was purified by column chromatography eluting with neat hexane, then 5:1 and 4:1 Hex:EtOAc to afford tert-butyl N-[(1R)-1-[[2,4-dichloro-6-[[(3R)-2,3,4,9-tetrahydro-1H-carbazol-3-yl]amino]pyrimidin -5-yl]oxymethyl]-2-methoxy-ethyl]carbamate (1.10 g,1.93 mmol, 91.58% yield) as a white solid.

UPLC-MS (4 min, basic): rt = 2.33 min, m/z = 536.2/538.2/540.2 [M+H]+, 94% purity.

1H NMR (400 MHz, DMSO-D6) δ 10.76 (s, 1H), 7.72 (d, J = 8.2 Hz, 1H), 7.34 (d, J = 7.7 Hz, 1H), 7.26 (d, J = 8.0 Hz, 1H), 7.13 (d, J = 7.8 Hz, 1H), 7.01 (t, J = 7.5 Hz, 1H), 6.93 (t, J = 7.4 Hz, 1 H), 4.34 (s, 1H), 3.96 (d, J = 8.8 Hz, 3H), 3.41 (d. J = 5.7 Hz, 2H), 3.26 (s, 3H), 3.07 (dd, J = 14.8, 5.3 Hz, 1H), 2.91 (d, J = 15.0 Hz, 2H), 2.78 - 2.59 (m, 1H), 2.16 (d, J = 12.0 Hz, 1H), 2.05 (d, J = 11.8 Hz, 1H), 1.39 (s, 9H).

### 35.4 (3R)-N-[5-[(2R)-2-amino-3-methoxy-propoxy]-2,6-dichloro-pyrimidin-4-yl]-2,3,4,9-tetrahydro-1H-carbazol-3-amine;hydrochloride

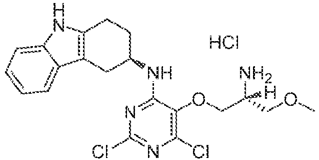

Prepared according to general method 3, using tert-butyl N-[(1R)-1-[[2,4-dichloro-6-[[(3R)-2,3,4,9-tetrahydro-1H-carbazol-3-yl]amino]pyrimidin-5-yl]oxymethyl]-2-methoxyethyl] carbamate (1.00 eq, 1.10 g, 2.04 mmol) to afford (3R)-N-[5-[(2R)-2-amino-3-methoxy-propoxy]-2,6-dichloro-pyrimidin-4-yl]-2,3,4,9-tetrahydro-1H-carbazol-3-amine;hydrochloride (600 mg,1.27 mmol, 62.12% yield) as a white solid. UPLC-MS (2 min, basic): rt = 1.02 min, m/z = 436.1/438.1/440.1 [M+H]+.

1H NMR (400 MHz, DMSO-D6) δ 10.76 (s, 1H), 8.51 (s, 3H), 8.04 (d, J = 8.4 Hz, 1H), 7.33 (d, J = 7.8 Hz, 1H), 7.26 (d, J = 8.0 Hz, 1H), 7.01 (t, J = 7.5 Hz, 1H), 6.93 (t, J = 7.4 Hz, 1H), 4.38 (s, 1H), 4.27 (dd, J = 10.1, 3.9 Hz, 1H), 4.10 (dd, J = 10.1, 5.7 Hz, 1H), 3.76 (s, 1H), 3.66 (d, J = 6.0 Hz. 2H), 3.34 (d, J = 1.6 Hz, 3H), 3.02 (dd, J = 14.9, 5.4 Hz, 1H), 2.94 - 2.75 (m, 3H), 2.10 (s, 2H).

### 35.5 N-[(1R)-1-[[2,4-dichloro-6-[[(3R)-2,3,4,9-tetrahydro-1H-carbazol-3-yl]amino] pyrimidin-5-yl]oxymethyl]-2-methoxy-ethyl]-N',N'-dimethyl-ethane-1,2-diamine

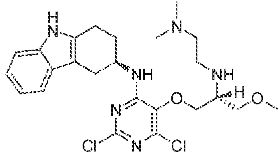

A stirred solution of (3R)-N-[5-[(2R)-2-amino-3-methoxy-pro poxy]-2,6-dichloro-pyrimidin-4-yl}-2,3,4,9-tetrahydro-1H-carbazol-3-amine;hydrochloride (1.00 eq, 350 mg, 0.740 mmol), (Dimethylamino)acetaldehyde sulfite (1:1) (1.10 eq, 138 mg, 0.814 mmol) and Triethylamine (1.00 eq, 0.10 mL, 0.740 mmol) in Methanol (5 mL) was treated with Sodium cyanoborohydride (3.00 eq, 140 mg, 2.22 mmol) and the mixture was stirred at room temperature for 3 days. The reaction was quenched by the addition of water (25 mL) and extracted with dichloromethane (2 x 30 mL). The resulting solution was washed sequentially with saturated brine solution (25 mL). The organic phase was then separated and dried (Na2SO4), before being concentration to dryness. The crude was purified by column chromatography using EtOAc. then 5% and 10% MeOH in EtOAc (ammonia buffer) to afford N-[(1R)-1-[[2,4-dichloro-6-[[(3R)-2,3,4,9-tetrahydro-1H-carbazol-3-yl]amino]pyrimidin-5-yl]oxymethyl]-2-methoxy-ethyl]-N',N'-dimethyl-ethane-1,2-diamine (200 mg,0.370 mmol, 50.05% yield) as a pink solid. UPLC-MS analysis: (4 min, basic): rt = 2.09 min, m/z = 507.3/509.3/511.3 [M+H]+, 94% purity. 1H NMR (400 MHz, DMSO-D6) δ 10.75 (s, 1H), 8.64 (s, 1H), 7.34 (d, J = 7.8 Hz, 1H), 7.26 (d, J = 8.0 Hz, 1H), 7.01 (t, J = 7.6 Hz, 1H), 6.93 (t, J = 7.5 Hz, 1H), 4.33 (s, 1H), 4.03 (q, J = 7.4 Hz, 2H), 3.75 (t, J = 8.5 Hz, 1H), 3.45 - 3.32 (m, 6H), 3.11 (dd, J = 14.9, 5.3 Hz, 1H), 2.99 (s, 1H), 2.86 (d, J = 13.9 Hz, 211), 2.68 (s, 3H), 2.21 (s, 6H), 2.03 (s, 1H), 1.26 - 1.13 (m, 2H).

### 35.6 (7R)-2-chloro-8-[2-(dimethylamino)ethyl]-7-(methoxymethyl)-N-[(3R)-2,3,4,9-tetrahydro-1H-carbazol-3-yl]-6,7-dihydropyrimido[5,4-b][1,4]oxazin-4-amine

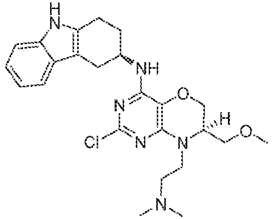

Prepared according to general method 4, using N-[(1R)-1-[[2,4-dichloro-6-[[(3R)-2,3,4,9-tetrahydro-1H-carbazol-3-yl]amino] pyrimidin-5-yl]oxymethyl]-2-methoxy-ethyl]-N',N'-dimethylethane-1,2-diamine (1.00 eq, 200 mg, 0.394 mmol) to afford (7R)-2-chloro-8-[2-(dimethylamino)ethyl]-7-(methoxymethyl)-N-[(3R) -2,3,4,9-tetrahydro-1H- carbazol-3-yl]-6,7-dihydropyrimido [5,4-b][1,4]oxazin-4amine (140 mg,0.282 mmol, 71.65% yield) as a white solid.

UPLC-MS analysis (4 min, basic): rt = 2.04 min, m/z = 471.4/473.4, [M+H]+, 87% purity.

1H NMR (400 MHz, DMSO) δ 10.69 (s, 1H), 7.32 (d, J = 7.7 Hz, 1H), 7.24 (d, J = 7.9 Hz, 1H), 6.99 (t, J = 7.5 Hz, 1H), 6.91 (t, J = 7.4 Hz, 1H), 6.53 (d, J = 8.4 Hz, 1H), 4.31 (d, J = 10.7 Hz, 1H), 4.25 (s, 1H), 3.84 (dq, J = 13.4, 6.7 Hz, 2H), 3.74 (d, J = 10.6 Hz, 1H), 3.45 (dd, J = 9.5, 5.9 Hz, 1H), 3.35 (dd, J = 17.3, 9.0 Hz, 6H), 3.02 - 2.74 (m, 4H), 2.72 - 2.56 (m, 1H), 2.25 (s, 6H), 2.09 - 1.88 (m, 2H).

### Example 35 (7R)-8-[2-(dimethylamino)ethyl]-7-(methoxymethyl)-2-(2-methylthiazol-5-yl)-N-[(3R)-2,3,4,9-tetrahydro-1H-carbazol-3-yl]-6,7-dihydropyrimido[5,4-b][1,4]oxazin-4-amine

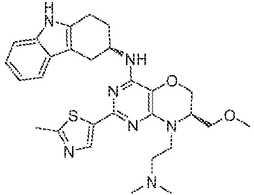

Prepared according to general method 5b using (7R)-2-chloro-8-[2-(dimethylamino)ethyl]-7-(methoxymethyl)-N-[(3R)-2,3,4,9-tetra hydro-1H-carbazol-3-yl]-6,7-dihydropyrimido[5,4-b][1,4] oxazin-4-amine (1.00 eq, 70 mg, 0.149 mmol) and 2-methyl-5-(tetramethyl-1,3,2-dioxaborolan-2-yl)-1,3-thiazole (2.00 eq, 67 mg, 0.297 mmol) to afford (7R)-8-|2- (dimethylamino)ethyl)-7-(methoxymethyl)-2-(2-methylthiazol-5-yl)-N-[(3R)-2,3,4,9-tetrahydro-1H-carbazol-3-yl]-6,7-dihydropyrimido[5,4-b][1,4]oxazin-4-amine (40 mg,0.0712 mmol, 47.91% yield) as a white solid.

UPLC-MS analysis: (4 min, basic): rt = 2.09 min, m/z = 534.4 [M+H]+, 95% purity.

1H NMR (400 MHz, DMSO-D6) δ 10.70 (s, 1H), 7.99 (s, 1H), 7.32 (d, J = 7.7 Hz, 1H), 7.25 (d, J = 7.9 Hz, 1H), 6.99 (ddd, J = 8.1, 7.0, 1.3 Hz, 1H), 6.97 - 6.86 (m, 1H), 6.18 (d. J = 8.2 Hz, 1H), 4.47 - 4.34 (m, 1H), 4.31 (dd, J = 10.7, 1.4 Hz, 1H), 3.98 (dt, J = 13.9.6.9 Hz, 1H), 3.78 (ddd, J = 13.2.9.3.4.2 Hz, 2H), 3.46 (dd, J = 9.4, 5.5 Hz, 1H), 3.38 (dt, J = 9.3, 6.9 Hz, 2H), 3.31 (s, 3H), 3.02 (dd, J = 14.9, 5.3 Hz, 1 H), 2.84 (td, J = 16.2, 15.5, 9.9 Hz, 2H), 2.72 - 2.63 (m. 1 H), 2.60 (s, 3H), 2.22 (s, 611), 2.14 - 2.05 (m, 1H), 2.02 - 1.88 (m, 1H). 2 lis under DMSO signal.

### Example 36 |(7R)-8-[2-(dimethylamino)ethyl]-2-(2-methylthiazol-5-yl)-4-[[(3R)-2,3,4,9-tetrahydro- 1H-carbazol-3-ylJamino}-6,7-dihydropyrimido[5,4-b] [1,4] oxazin-7-yljmethanol

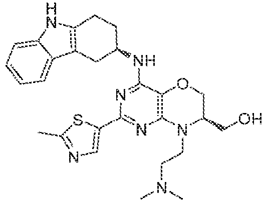

To a solution of (7R)-8-[2-(dimethylamino)ethyl]-7-(methoxymethyl)-2-(2-methylthiazol-5-yl)-N-[(3R)-2,3,4,9-tetrahydro-1H-carbazol-3-yl]-6,7-dihydropyrimido|5,4-b][1,4]oxazin-4-amine (1.00 eq, 18 mg, 0.0320 mmol) in DCM (3 mL) was added boron tribromide solution in DCM 1M (2.00 eq, 0.064 mi. 0.0641 mmol) and the mixture was stirred at ambient temperature for 18 h. Further addition of boron tribromide solution and stirring were needed to take the reaction to completion as shown by UPLC analysis. The reaction mixture was carefully quenched with MeOH (1 mL) and then with water. Layers were separated and aqueous was extracted twice with DCM. Organics were dried over Na2SO4, filtered and concentrated to dryness. The crude was purified by column chromatography eluting with 1% to 5% MeOH in DCM (ammonia buffer) to afford [(7R)-8-[2-(dimethylamino)ethyl]-2-(2-methylthiazol-5-yl)-4-[[(3R)-2.3.4,9-tetrahydro-1H-carbazol-3-yl]amino]-6,7-dihydropyrimido[5,4-b][1.4]oxazin-7-yl]methanol (7.0 mg,0.0129 mmol, 40.36% yield) as a white solid.
UPLC-MS (4 min, acidic): rt = 1.84 min, m/z = 520.4 [M+H]+, 96% purity
1 H NMR (400 MHz, DMSO-D6) δ 10.70 (s, 1H), 7.99 (₅. 1H), 7.32 (d, J = 7.7 Hz, 1H), 7.25 (d, J = 8.1 Hz, 1H), 6.99 (ddd, J = 8.1, 7.0, 1.3 Hz, 1H), 6.96 - 6.87 (m, 1H), 6.11 (d, J = 8.2 Hz, 1H), 5.17 (s, 1H), 4.38 (d, J = 10.3 Hz, 2H), 3.94 (dt, J = 13.7, 6.7 Hz, 1H), 3.76 (d, J = 10.4 Hz, 1H), 3.61 - 3.50 (m, 2H), 3.50 - 3.34 (m, 2H), 3.02 (dd, J = 14.8, 5.3 Hz, 1H), 2.84 (q, J = 15.5, 12.4 Hz, 2H), 2.60 (s, 3H), 2.24 (s, 6H), 2.11 (d. J = 12.1 Hz, 1H), 1.95 (ddt, J = 14.2, 8.9, 4.8 Hz, 1H). 3Hs under solvent signal.

### Intermediates for Example 37

### 37.1 (3R)-N-[5-[2-[2-[tert-butyl(dimethyl)silyl]oxyethylamino]ethoxy]-2,6-dichloro-pyrimidin-4-yl]-2,3,4,9-tetrahydro-1H-carbazol-3-amine

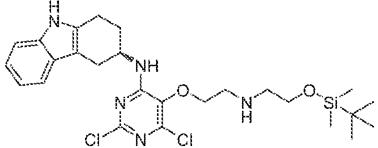

A stirred solution of (3R)-N-[S-(2-aminoethoxy)-2,6-dichloro-pyrimidin-4-yl]-2,3,4,9-tetrahydro-1H-carbazol-3-amine;hydrochloride (1.00 eq. 300 mg, 0.700 mmol) and (tert-Butyldimethyl silyloxy)acetaldehyde (1.30 eq, 0.19 mL, 0.910 mmol) in Methanol (5 mL) was treated with Sodium cyanoborohydride (3.00 eq, 132 mg, 2.10 mmol) and the mixture was stirred at room temperature for 5 h. The reaction was quenched by the addition of water (25 mL) and extracted with dichloromethane (2 x 60 mL). The resulting solution was washed sequentially with saturated brine solution (25 mL). The organic phase was then separated and dried (Na2SO4), before being concentration to dryness. The crude was purified by column chromatography using EtOAc (0% to 100%; v/v) in iso-Hex to afford (3R)-N-[5-[2-[2-[tert-butyl(dimethyl)silyl]oxyethylamino]ethoxy]-2,6-dichloro-pyrimidin-4-yl]-2,3,4,9-tetrahydro-1H-carbazol-3-amine (180 mg,0.180 mmol, 25.70% yield) as a waxy white solid.

UPLC-MS analysis: (2 min, basic): rt = 1.51 min, m/z = 550.3/552.3/554.3 [M+H]+, 55% purity. 1H NMR (400 MHz, DMSO-D6) δ 10.76 (d, J = 4.5 Hz, 1H), 8.87 (s, 1H), 7.34 (d, J = 7.6 Hz, 1H), 7.27 (dt, J = 8.0, 1.0 Hz, 1H), 7.06 - 6.98 (m, 1 H), 6.98 - 6.89 (m, 1 H), 4.31 (s, 1H), 4.05 - 3.94 (m, 2H), 3.72 - 3.58 (m, 2H), 3.58 - 3.43 (m, 1H), 3.14 - 3.04 (m, 1H), 2.99 - 2.83 (m, 4H), 2.75 - 2.63 (m, 3H), 2.21 - 2.11 (m, 1H), 2.06 - 1.95 (m, 1H), 0.93 - 0.76 (m, 9H), 0.12 - -0.10 (m, 6H).

### 37.2 8-[2-[tert-butyl(dimethyl)silyl]oxyethyl]-2-chloro-N-[(3R)-2,3,4,9-tetrahydro-1H-carbazol-3-yl]-6,7-dihydropyrimido[5,4-b][1,4]oxazin-4-amine

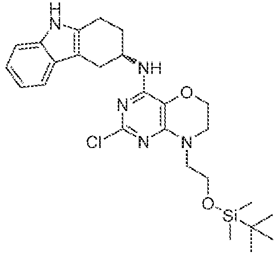

Prepared according to general method 4, using (3R)-N-[5-[2-[2-[tert-butyl(dimethyl)silyl]oxy ethylamino]ethoxy]-2,6-dichloro-pyrimidin-4-yl]-2,3,4,9-tetrahydro-1H-carbazol-3-amine (1.00 eq, 180 mg, 0.180 mmol) to afford 8-[2-[tert-butyl(dimethyl)silyl]oxyethyl]-2-chloro-N-[(3R)-2,3,4,9-tetrahydro-1H-carbazol-3-yl]-6,7-dihydropyrimido[5,4-b][1,4]oxazin-4-amine (130 mg,0.177 mmol, 98.44% yield) as an off-white solid UPLC-MS analysis (2 min, basic): rt = 1.49 min, m/z = 514.3/516.3 [M+H]+, 70% purity. 1H NMR (400 MHz, DMSO-D6) δ 10.65 (s, 1H), 7.27 (d, J = 7.8 Hz, 1H), 7.21 (dd, J = 8.1, 4.9 Hz, 1H), 6.95 (t, J = 7.4 Hz, 1H), 6.88 (t, J = 7.4 Hz, 1H), 6.32 (d, J = 8.6 Hz, 1H), 4.09 (t, J = 4.4 Hz, 2H), 3.71 (t, J = 5.8 Hz, 2H), 3.51 (t, J = 4.3 Hz, 3H), 2.97 - 2.75 (m, 4H), 2.67 - 2.53 (m, 2H), 2.03 - 1.88 (m, 2H), 0.80 (s, 9H), -0.06 (s, 6H).

### 37.3 8-[2-[tert-butyl(dimethyl)silyl]oxyethyl]-2-(2-methylthiazol-5-yl)-N-{(3R)-2,3,4,9-tetrahydro-1H-carbazol-3-yl]-6,7-dihydropyrimido[5,4-b][1,4]oxazin-4-amine

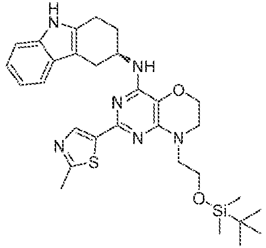

Prepared according to general method 5b using 8-[2-[tert-butyl(dimethyl)silyl]oxyethyl]-2-chloro-N-[(3R)-2,3,4,9-tetrahydro-1H-carbazol-3-yl]-6,7-dihydropyrimido[5,4-b][1,4]oxazin-4-amine (1.00 eq, 120 mg, 0.163 mmol) and 2-methyl-5-(tetramethyl-1,3,2-dioxaborolan-2-yl)-1,3-thiazole (1.50 eq, 55 mg, 0.245 mmol). The crude was purified by column chromatography (20 g column) eluting with a gradient of 0 to 60% (v/v) EtOAc in iso-Hex to afford 8-[2-[tert-butyl(dimethyl)silyl]oxyethyl]-2-(2-methylthiazol-5-yl)-N-[(3R)-2,3,4,9- tetrahydro-1H-carbazol-3-yl]-6,7-dihydropyrimido[5,4-b][1,4]oxazin-4. amine (75 mg,0.124 mmol, 75.60% yield) as a pale yellow oil. UPLC-MS analysis: (2 min, basic): rt = 1.53 min, m/z = 577.4 [M+H]+, 97% purity. 1H NMR (400 MHz, DMSO-D6) δ 10.70 (s, 1H), 7.98 (s, 1H), 7.31 (d. J = 7.7 Hz, 1H), 7.25 (dt, J = 7.9, 0.9 Hz, 1H), 7.06 - 6.95 (m, 1H), 6.94 - 6.85 (m, 1H), 6.03 (d, J = 8.3 Hz, 1H), 4.40 (s, 1H), 4.16 (s, 2H), 3.81 (t, J = 6.0 Hz, 2H), 3.64 (t, J = 6.0 Hz, 2H), 3.57 (t, J = 4.4 Hz, 2H), 3.01 (dd, J = 14.8, 5.3 Hz, 1H), 2.94 - 2.74 (m, 2H), 2.59 (s, 3H), 2.57 - 2.52 (m, 1H), 2.16 - 2.05 (m, 1H), 2.01 - 1.91 (m, 1H), 0.85 (s, 9H), 0.04 (s, 6H).

### Example 37 2-[2-(2-methylthiazol-5-yl)-4-[[(3R)-2,3,4,9-tetrahydro-1H-carbazol-3-yl] amino]-6,7-dihydropyrimido[5,4-b][1,4]oxazin-8-yl]ethanol

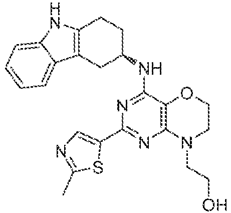

To a solution of 8-[2-(bert-butyl(dimethyl)silyl]oxyethyl]-2-(2-methylthiazol-5-yl)-N-[(3R)-2,3,4,9-tetrahydro-1H-carbazol-3-yl]-6,7-dihydropyrimido[5,4-b][1,4] oxazin-4-amine (1.00 eq, 75 mg, 0.124 mmol) in MeCN (2.4704 mL), 4M solution of Hydrogen chloride in dioxane (30.0 eq, 0.93 mL, 3.71 mmol) was added and the reaction mixture was stirred at room temperature for 15 minutes.

The reaction was concentrated to dryness to give a residue, which was triturated with MTBE (10 mL) and Et2O (5 mL x 3) to afford 2-[2-(2-methylthiazol-5-yl)-4-[[(3R)-2.3,4,9-tetrahydro-1H-carbazol-3-yl]amino]-6,7-dihydropyrimido[5,4-b][3,4]oxazin-8-yl]ethanol (36 mg,0.0778 mmol, 63.01% yield) as a yellow solid. UPLC-MS analysis: (4 min, basic): rt = 1.93 min, m/z = 463.1 [M+H]+, 100% purity. 1H NMR (400 MHz, DMSO-D6) δ 10.76 (s, 1H), 8.12 (s, 1H), 7.36 (d, J = 7.7 Hz, 1H), 7.33 - 7.26 (m, 1H), 7.08 - 6.99 (m, 1H). 6.99 - 6.91 (m, 1H), 6.14 (bs, 1H), 4.48 - 4.40 (m, 1H), 4.21 (t, J = 4.5 Hz, 2H), 3.78 - 3.50 (m, 6H), 3.05 (dd, J = 14.9, 5.2 Hz, 1H), 2.98 - 2.82 (m, 2H), 2.75 - 2.68 (m, 1H), 2.67 (s, 3H), 2.20 - 2.08 (m, 1H), 2.08 - 1.95 (m, 1H). 1H not observed.

### Intermediates for Example 38

### 38.1 tert-butyl 2-chloro-7-[(dimethylamino)methyl]-4-[[(3R)-2,3,4,9-tetrahydro-1H-carbazol-3-y]amino]-6,7-dihydropyrimido[5,4-b][1,4]oxazine-8-carboxylate

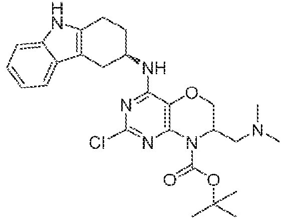

A mixture of 2,4-dichloro-6-[[(3R)-2,3,4,9-tetrahydro-1H-carbazol-3-yl]amino]pyrimidin-5-ol (1.00 eq, 0.35 g, 1.00 mmol), [(2R)-2-(tert-butoxycarbonylamino)-3-(dimethylamino)propyl] methanesulfonate (1.44 eq, 0.45 g, 1.44 mmol) ( Mesylate synthesised from the corresponding N-Boc amino alcohol and used without any purification) and Caesium carbonate (2.00 eq, 0.65 g, 2.00 mmol) in DMF (6.6817 mL) was stirred at 110 °C for 24 h. The mixture was cooled down then poured into crushed ice and water (100 mL). The resultant precipitate was filtered off then dissolved in a mixture DCM/MeOH (9:1), dried over Na2SO4, filtered and concentrated. The crude material was purified by column chromatography eluting with a gradient of EtOAc (0% to 100%; v/v) in iso-Hex and then MeOH (0% to 20%; v/v) in EtOAc to afford tert-butyl 2-chloro-7-[(dimethylamino)methyl]-4-[[(3R)-2,3,4,9-tetrahydro-1H-carbazol-3-yl]amino]-6,7-dihydropyrimido[5,4-b][1,4]oxazine-8-carboxylate (155 mg,0.266 mmol, 26.53% yield) as a brown solid. Note: product isolated as a mixture of diastereoisomers due to racemisation of one of the two stereocenters. UPLC-MS analysis: (2 min, basic): rt = 1.29 min, m/z = 513.3/515.3 [M+H]+, 88% purity. 1H NMR (400 MHz, DMSO-D6) δ 10.71 (s, 1H), 7.36 - 7.27 (m, 2H), 7.27 - 7.23 (m, 1H), 7.02 - 6.97 (m, 1 H), 6.95 - 6.91 (m, 1 H), 4.53 - 4.47 (m, 1 H), 4.39 - 4.26 (m, 1H), 4.06 - 3.98 (m, 1H), 3.03 - 2.94 (m, 2H), 2.87 - 2.78 (m, 2H), 2.41 - 2.31 (m, 1H), 2.27 - 2.17 (m, 7H), 2.11 - 1.93 (m, 3H), 1.49 (s, 9H).

### 38.2 tert-butyl 7-[(dimethylamino)methyl]-2-(2-methylthiazol-5-yl)-4-[[(3R)-2,3,4,9-tetrahydro-1H-carbazol-3-yl]amino]-6,7-dihydropyrimido[5,4-b][1,4]oxazine-8-carboxylate

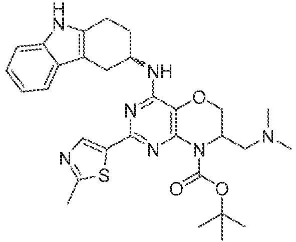

Prepared according to general method 5b using tert-butyl 2-chloro-7-[(dimethylamino)methyl]-4-[[(3R)-2,3,4,9-tetrahydro-1H-carbazol-3-yl]amino]-6,7-dihydropyrimido[5,4-b][1,4]oxazine-8-carboxylate (1.00 eq, 165 mg. 0.212 mmol) and 2-methyl-5- (tetramethyl- 1,3,2-dioxaborolan-2-yl)-1,3-thiazole (1.50 eq, 72 mg, 0.318 mmol). The crude was purified by column chromatography (20 g column) eluting with a gradient of 0 to 100% (v/v) EtOAc in iso-Hex and then 0-20% (v/v) MeOH in EtOAc to afford tert-butyl 7-[(dimethylamino)methyl]-2-(2-methylthiazol-5-yl)-4- [[(3R)-2,3,4,9-tetrahydro-1H-carbazol-3-yl]amino]-6,7-dihydropyrimido[5,4-b](1,4]oxazine-8-carboxylate (90 mg, 0.127 mmol, 59.65% yield) as a yellow oil. Note: product isolated as a mixture of diastereoisomers due to racemisation of one of the two stereocenters in the previous step.

UPLC-MS analysis: (2 min, basic): rt = 1.29/1.30 min, m/z = 576.3 [M+H]+, 81% purity.

### Example 38 7-[(dimethylamino)methyl]-2-(2-methyithiazol-5-yl)-N-[(3R)-2,3,4,9-tetrahydro-1H-carbazol-3-yl]-7,8-dihydro-6H-pyrimido[5,4-b][1,4]oxazin-4-amine

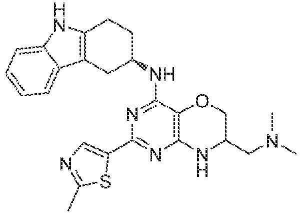

To a solution of tert-butyl 7-|(dimethylamino)methyl]-2-(2-methylthiazol-5-yl)-4-[[(3R)-2,3,4,9-tetrahydro-1H-carbazol-3-yl]amlno]-6,7-dihydropyrimido[5,4-b][1,4]oxazine-8-carboxylate (1.00 eq, 150 mg, 0.229 mmol) in DCM (1.1959 mL), a 4M solution of Hydrogen chloride in dioxane (25.0 eq, 1.4 mL, 5.73 mmol) was added and the reaction mixture was stirred at room temperature for 2h. The reaction was concentrated to dryness to give a residue, which was triturated with Et2O (10 mL) and filtered. The crude was purified by prep HPLC (basic method) to afford 7-[(dimethylamino)methyl]-2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amine (12 mg,0.0241 mmol, 10.50% yield) as an off-white solid. Note: product isolated as a mixture of diastereoisomers due to racemisation of one of the two stereocenters in the previous step. UPLC-MS analysis: (4 min, basic): rt = 1.96/1.97 min, m/z = 476.3 [M+H]+, 97% purity. 1H NMR (400 MHz, DMSO-D6) δ 10.71 (s, 1H), 7.97 (s, 1H), 7.33 (d, J = 7.7 Hz, 1H), 7.29 - 7.20 (m, 1H), 7.06 - 6.97 (m, 1 H), 6.97 - 6.88 (m, 1H), 6.80 - 6.70 (m, 1H), 6.21 (d. J = 8.2 Hz, 1H), 4.48 - 4.34 (m, 1H). 4.12 - 3.96 (m, 2H), 3.68 - 3.56 (m, 1H), 3.07 - 2.97 (m, 1H), 2.97 - 2.78 (m, 2H), 2.72 - 2.63 (m, 1H), 2.60 (s, 3H), 2.42 - 2.26 (m, 2H), 2.20 (s, 6H), 2.15 - 2.06 (m, 1 H), 2.04 - 1.91 (m, 1H).

### Intermediates for Example 39

### 39.1 N-[2-[2,4-dichloro-6-[[(3R)-2,3,4,9-tetrahydro-1H-carbazol-3-yl]amino]pyrimidin-5-yl]oxyethyl]methanesulfonamide

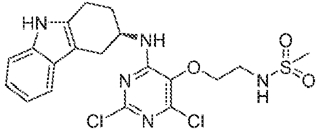

To a solution of (3R)-N-[5-(2-aminoethoxy)-2,6-dichloro-pyrimidin-4-yl]-2,3,4,9-tetrahydro-3 H-carbazol-3-amine, hydrochloride (1.00 eq, 0.30 g, 0.616 mmol) and Triethylamine (2.00 eq, 0.17 mL, 1.23 mmol) in DCM (6.1576 mL), Methanesulfonyl chloride (1.10 eq, 0.052 ml., 0.677 mmol) was added dropwise at at 0 °C and the reaction mixture was left warmed up to room temperature over 3h. The reaction mixture was diluted with DCM (30 mL) then the organic phase was washed with 1 N HCl (3 × 15 mL), dried over Na2SO4, filtered and concentrated to dryness. The crude was purified by column chromatography eluting with a gradient of EtOAc (0% to 100%; v/v) in iso-Hex to afford N-[2-[2,4-dichloro-6-[[(3R)-2,3,4,9-tetrahydro-1H-carbazol-3-yl]amino]pyrimidin-5-yl]oxyethyl]methanesulfonamide (290 mg,0.536 mmol, 87.11% yield) as a viscous pale yellow oil.

UPLC-MS analysis: (2 min, basic): rt = 1.12 min, m/z = 470.2/472.2/474.2 [M+H]+, 100% purity. 1H NMR (400 MHz, DMSO-D6) δ 10.74 (s, 1H), 7.69 (s, 1H), 7.41 (s, 1H), 7.33 (d, J = 7.7 Hz, 1H), 7.25 (dt, J = 8.0, 1.0 Hz, 1H), 7.07 - 6.97 (m, 1H), 6.95 - 6.85 (m, 1H), 4.40 - 4.25 (m, 1H), 4.09 - 4.02 (m, 2H), 3.38 (t; J = 5.5 Hz, 2H), 3.04 (dd, J = 14.8, 5.4 Hz, 1H), 2.96 (s, 3H), 2.92 - 2.79 (m, 2H), 2.76 - 2.64 (m, 1H), 2.17 - 2.07 (m, 1H), 2.08 - 1.99 (m, 1H).

### 39.2 2-chloro-8-methylsulfonyl-N-[(3R)-2,3,4,9-tetrahydro-1H-carbazol-3-yl]-6,7-dihydropyrimido[5,4-b][1,4]oxazin-4-amine

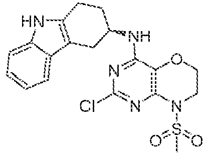

To mixture of N-[2-[2,4-dichloro-6-[[(3R)-2,3,4,9-tetrahydro-1H-carbazol-3-yl]amino]pyrimidin-5-yl]oxyethyl]methanesulfonamide (220 mg, 0.407 mmol) and Potassium phosphate tribasic (121 mg, 0.570 mmol) in 1,4-Dioxane (4.0691 mL), Tris(dibenzylideneacetone) dipalladium(0) (Pd2(dba)3) (7.5 mg, 0.00814 mmol) and 4,5-Bis(diphenylphosphino)-9,9-dimethylxanthene (Xantphos) (14 mg, 0.0244 mmol) were added. The mixture was stirred at 100°C for 24h. The reaction mixture was diluted with EtOAc and filtered through celite then the filtrate was evaporated to dryness. The crude was purified by column chromatography eluting with a gradient of EtOAc (0% to 80%; v/v) in iso-Hex to afford 2-chloro-8-methylsulfonyl-N-[(3R)-2,3,4,9-tetrahydro-1H-carbazol-3-yl]-6,7-dihydropyrimido[5,4-b][1,4]oxazin-4-amine (130 mg,0.291 mmol, 71.42% yield) as an off-white solid.

UPLC-MS analysis: (2 min, basic): rt = 1.14 min, m/z = 434.3/436.3 [M+H]+, 97% purity.

1H NMR (400 MHz, DMSO-D6) δ 10.72 (s, 1H), 7.42 (d, J = 8.4 Hz, 1H), 7.36 - 7.29 (m, 1H), 7.31 - 7.21 (m, 1H), 7.03 - 6.97 (m, 1H), 6.96 - 6.87 (m, 1H), 4.43 - 4.24 (m, 3H), 3.97 - 3.88 (m, 2H), 3.49 (s, 3H), 3.01 - 2.93 (m, 1H), 2.93 - 2.78 (m, 2H), 2.73 - 2.62 (m, 1H), 2.09 - 2.00 (m, 2H).

### Example 39 8-methylsulfonyl-2-(2-methylthiazol-5-yl)-N-[(3R)-2,3,4,9-tetrahydro-1H-carbazol-3-yl]-6,7-dihydropyrimido[5,4-b][1,4]oxazin-4-amine

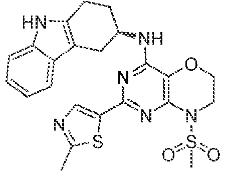

Prepared according to general method 5b using 2-chloro-8-methylsulfonyl-N-[(3R)-2.3,4,9-tetrahydro-1 H-carbazol-3-yl]-6,7-dihydropyrimido[5,4-b][1,4]oxazin-4-amine (115 mg, 0.257 mmol) and 2-methyl-5-(tetramethyl-1,3,2-dioxaborolan-2-yl)-1,3-thiazole (1.50 eq, 87 mg, 0,386 mmol) to afford 8-methylsulfonyl-2-(2-methylthiazol-5-yl)-N-[(3R)-2,3,4,9-tetrahydro- 1H-carbazol-3-yl]-6,7-dihydropyrimido[5,4-b][1,4]oxazin-4-amine (107 mg,0.215 mmol, 83.81% yield) as an off-white solid. UPLC-MS analysis: (4 min, basic): rt = 1.93 min, m/z = 497.2 [M+H]+, 100% purity.

1H NMR (400 MHz, DMSO-D6) δ 10.74 (s, 1H), 8.08 (s, 1H), 7.41 - 7.29 (m, 1H), 7.29 -7.20 (m, 1H), 7.11 - 6.95 (m, 2H), 6.95 - 6.87 (m, 1H), 4.50 (s, 1H), 4.39 - 4.27 (m, 2H), 3.95 (t, J = 4.3 Hz, 2H), 3.59 (s, 3H), 3.03 (dd, J = 14.9, 5.3 Hz, 1H), 2.98 - 2.80 (m, 2H), 2.78 - 2.68 (m, 1H), 2.64 (s, 3H), 2.17 - 2.08 (m, 1H), 2.08 - 1.96 (m, 1H).

### Intermediates for Example 40

### 40.1 tert-butyl N-[2-[2,4-dichloro-6-[2-(2-chloro-1H-indol-3-yl)ethylamino]pyrimidin-5-yl]oxyethyl]carbamate

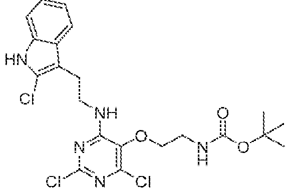

Prepared according to general method 2a, using tert-butyl N-[2-(2,4,6-trichloropyrimidin-5-yl)oxyethyl]carbamate (857 mg, 2.50 mmol) and 2-(2-chloro-2 H-indol-3-yl)ethanamine hydrochloride (578 mg, 2.50 mmol) (synthesised by chlorination of the tryptamine hydrochloride and used without any purification). The reaction was concentrated to dryness and the crude was purified by column chromatography eluting with hexane to 4:1 Hexane:EtOAc to afford tert-butyl N-[2-[2,4-dichloro-6-[2-(2-chloro-1H-indol-3-yl)ethylamino]pyrimidin-5-yl]oxyethyl]carbamate (570 mg,1.14 mmol, 45.53% yield) as a brown solid.

UPLC-MS analysis: (4 min, basic): rt = 2.31 min, m/z = 500.0/502.0/504.0 [M+H]+, 100% purity.

1H NMR (400 MHz, DMSO-D6) δ 11.67 (s, 1H), 8.09 (t, J = 6.0 Hz, 1H), 7.63 - 7.56 (m, 1H), 7.27 (dt, J = 8.1, 1.0 Hz, 1H), 7.15 - 6.99 (m, 3H), 3.87 (t, J = 5.4 Hz, 2H), 3.53 (q, J = 6.9 Hz, 2H), 3.23 (q, J = 5.6 Hz, 2H), 2.99 - 2.91 (m, 2H), 1.37 (s, 9H).

### 40.2 5-(2-aminoethoxy)-2,6-dichloro-N-[2-(2-chloro-1H-indol-3-yl)ethyl]pyrimidin-4-amine;hydrochloride

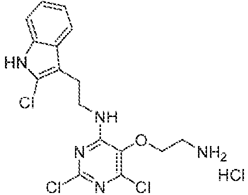

Prepared according to general method 3, using tert-butyl N-[2-[2,4-dichloro-6-(2-(2-chloro-1H-indol-3-yl)ethylamino]pyrimidin-5-yl]oxyethyl]carbamate (570 mg, 1.14 mmol) to afford 5-(2-aminoethoxy)-2,6-dichloro-N-[2-(2-chloro-1H-indol-3-yl)ethyl]pyrimidin-4-amine;hydrochloride (330 mg,0.755 mmol, 66.33% yield) as a yellow solid.

UPLC-MS analysis (4 min, basic): rt = 1.90 **min,** m/z = 400.0/402.0/404.0 [M+H]+, 99% purity.

1H NMR (400 MHz, DMSO-D6) δ 11.70 (s, 1H), 8.36 (t, J = 6.0 Hz, 1H), 8.22 (s. 3H), 7.64 (d, J = 7.8 Hz, 1H), 7.28 (dt, J = 8.1, 0.9 Hz, 1H), 7.14 - 7.10 (m, 1H), 7.10 - 7.00 (m, 1H), 4.09 (t, J = 5.0 Hz, 2H), 3.56 - 3.49 (m, 2H), 3.25 - 3.18 (m, 2H), 3.00 - 2.92 (m, 2H).

### 40.3 2-chloro-N-[2-(2-chloro-1H-indol-3-yl)ethyl]-7,8-dihydro-6H-pyrimido[5,4-b][1,4] oxazin-4-amine

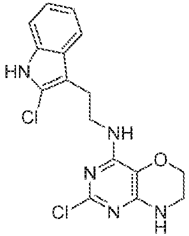

Prepared according to general method 4, using 5-(2-aminoethoxy)-2,6-dichloro-N-[2-(2-chloro-1H-indol-3-yl)ethyl]pyrimidin-4-amine; hydrochloride (370 mg, 0.846 mmol). The reaction mixture volume was reduced to dryness and the crude was purified by column chromatography using 3:1 and 1:1 Hexane:EtOAc to afford 2-chloro-N-[2-(2-chloro-1H-indol-3-yl)ethyl]-7,8-dihydro-6H-pyrimido[5,4-b][1,4]oxazin-4-amine (120 mg,0.329 mmol, 38.93% yield) as a brown solid.

UPLC-MS analysis (4 min, basic): rt = 1.87 **min,** m/z = 364.0/366.0 [M+H]+, 75% purity.

1H NMR (400 MHz, DMSO-D6) δ 11.62 (s, 1H), 7.69 - 7.62 (m, 1H), 7.26 (dt, J = 8.1, 1.0 Hz, 1H), 7.14 (t, J = 2.6 Hz, 1H), 7.10 (ddd, J = 8.2, 7.1, 1.2 Hz, 1H), 7.02 (ddd, J = 8.1, 7.1, 1.1 Hz, 1H), 6.69 (t, J = 6.0 Hz, 1H), 4.07 - 3.98 (m, 2H), 3.47 - 3.35 (m, 4H), 2.91 - 2.83 (m, 2H).

### Example 40 3-(4-((2-(2-chloro-1H-indol-3-yl)ethyl)amino)-7,8-dihydro-6H-pyrimido[5,4-b][1,4]oxazin-2-yl)pyridin-2(1H)-one

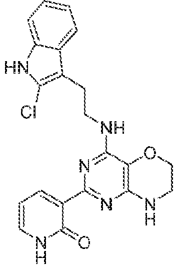

Prepared according to general method 5b, using 2-chloro-N-[2-(2-chloro-1H-indol-3-yl)ethyl]-7,8-dlhydro-6H-pyrimido[5,4-b][1,4]oxazin-4-amine (120 mg, 0.329 mmol) and (2-hydroxy-3-pyridyl)boronic acid (46 mg, 0.329 mmol). The crude was purified by prep HPLC to afford 3-[4-[2-(2-chloro-1 H-indol-3-yl)ethylamino]-7,8-dihydro-6H-pyrimido[S,4-b][1,4]oxazin-2-yl]pyridin-2-ol (3.0 mg,0.00709 mmol, 2.15% yield) as a yellow solid.

### Example 40 is subject to keto-enol tautomerism. It may also exist in the following form: 3-(4-((2-(2-chloro-1H-indol-3-yl)ethyl)amino)-7,8-dihydro-6H-pyrimido[5,4-b][1,4]oxazin-2-yl)pyridin-2-ol

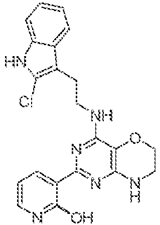

UPLC-MS analysis (4 min. basic): rt = 1.71 min, m/z = 423.1/425.1 [M+H]+, 100% purity.

1H NMR (400 MHz, DMSO-D6) δ 11.64 (s, 1H), 8.51 (m, 1H), 8.22 (s, 1H), 8.12 (m, 1H), 7.56 (d, J = 7.9 Hz, 1H), 7.35 (m, 1H), 7.26 (dt, J = 8.1, 1.0 Hz, 1H), 7.10 (ddd, J = 8.1, 7.1, 1.3 Hz, 1H), 7.08 - 6.98 (m, 1H), 6.91 (m, 1H), 6.78 (m, 1H), 4.13 (t, J = 4.2 Hz, 2H), 3.59 (q, J = 6.9 Hz, 2H), 3.46 (m, J = 4.7 Hz, 2H), 3.01 - 2.92 (m, 2H),

### Intermediates for Example 41

### 41.1 tert-butyl N-[2-[2,4-dichloro-6-[2-(7-fluoro-2-methyl-1H-indol-3-yl)ethyl amino]pyrimidin-5-yl]oxyethyl]carbamate

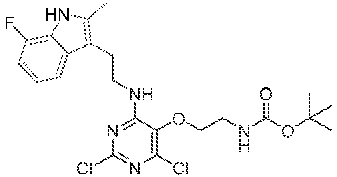

Prepared according to general method 2a, using tert-butyl N-[2-(2,4,6-trichloropyrimidin-5-yl)oxyethyl]carbamate (400 mg, 1.17 mmol) and 2-(7-Fluoro-2-methyl-1H-indol-3-yl)ethanamine oxalate (330 mg, 1.17 mmol). The reaction was concentrated to dryness and the residue was triturated in 20:1 MeCN:MeOH, filtered, washed with diethyl ether to afford tert-butyl N-[2-[2,4-dichloro-6-[2-(7-fluoro-2-methyl-1H-indol-3-yl)ethylamino]pyrimidin-5-yl]oxyethyl]carbamate (500 mg,1.00 mmol, 85.93% yield) as a brown solid. UPLC-MS analysis: (2 min, basic): rt = 1.30 min, m/z = 498.2/500.2/502.2 (M+H]+. 1H NMR (400 MHz, DMSO-D6) δ 11.20 (s, 1H), 8.06 (d, J = 6.4 Hz, 1H), 7.33 (d, J = 7.8 Hz, 1H), 7.08 (s, 1H), 6.90 (td, J = 7.8, 4.8 Hz, 1H), 6.84 - 6.75 (m, 1H), 3.87 (h, J = 6.2 Hz, 2H), 3.47 (q, J = 6.8 Hz, 2H), 3.25 (q, J = 5.3 Hz, 2H), 2.94 - 2.86 (m. 2H), 2.34 (s, 3H), 1.38 (d, J = 2.0 Hz, 9H). 19F NMR (376 MHz, DMSO-D6) δ -134.21.

### 41.2 5-(2-aminoethoxy)-2,6-dichloro-N-[2-(7-fluoro-2-methyl-1H-indo1-3-yl)ethyl] pyrimidin-4-amine

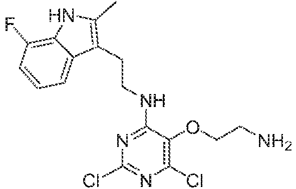

Prepared according to general method 3, using tert-butyl N-[2-[2,4-dichloro-6-[2-(7-fluoro-2-methyl-1H-indol-3-yl)ethyl amino]pyrimidin-S-yl]oxyethyl]carbamate (500 mg, 1.00 mmol). The crude was purified by column chromatography using EtOAc/MeOH (basic buffer, TEA) to afford 5-(2-aminoethoxy)-2,6-dichloro-N-[2-(7-fluoro-2-methyl-1H-indol-3-yl)ethyl]pyrimidin-4-amine (210 mg,0.527 mmol, 52.56% yield) as an off white solid.

UPLC-MS analysis (4 min, basic): rt = 1.96 min, m/z = 398.0/400.0/402.0 [M+H]+, 94% purity. 1H NMR (400 MHz, DMSO-D6) δ 11.21 (s, 1H), 7.34 (d, J = 7.8 Hz, 1H), 6.90 (td, J = 7.8, 4.8 Hz, 1H), 6.80 (dd, J = 11.4, 7.8 Hz, 1H), 3.79 (t, J = 5.0 Hz, 2H), 3.51 - 3.41 (m, 2H), 2.96 - 2.86 (m, 2H), 2.80 (t, J = 5.1 Hz, 2H), 2.34 (s, 3H). 3H not observed.

19F NMR (376 MHz, DMSO-D6) δ -134.18 (dd, J = 11.7, 4.8 Hz).

### 41.3 2-chloro-N-[2-(7-fluoro-2-methyl-1H-indol-3-yl)ethyl]-7,8-dihydro-6H-pyrimido[5,4-b][1,4]oxazin-4-amine

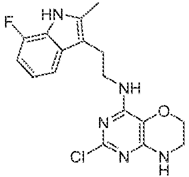

Prepared according to general method 4, using 5-(2-aminoethoxy)-2,6-dichloro-N-[2-(7-fluoro-2-methyl-1H-indol-3-yl)ethyl] pyrimidin-4-amine (192 mg, 0.483 mmol) to afford 2-chloro-N-[2-(7-fluoro-2-methyl-1H-indol-3-yl)ethyl]-7,8-dihydro-6H-pyrimido[5,4- b]|1,4]oxazin-4-amine (170 mg,0.470 mmol, 97.27% yield) as an off-white solid.

UPLC-MS analysis (4 min, basic): rt = 1.75 min, m/z = 362.0/364.0 [M+H]+, 94% purity.

1H NMR (400 MHz, DMSO-D6) δ 11.14 (s, 1 H), 7.39 - 7.33 (m, 1H), 7.11 (s, 1H), 6.88 (td, J = 7.8, 4.9 Hz, 1H), 6.79 (ddd, J = 11.5, 7.8, 0.9 Hz, 1H), 6.61 (t, J = 6.0 Hz, 1H), 4.05 (dd, J = 4.9, 3.7 Hz, 2H), 3.38 (s, 4H), 2.87 - 2.77 (m, 2H), 2.33 (s, 3H).

### Example 41 3-[4-[2-(7-fluoro-2-methyl-1H-indol-3-yl)ethylamino]-7,8-dihydro-6H-pyrimido[5,4-b][1,4]axazin-2-yl]-1H-pyridin-2-one

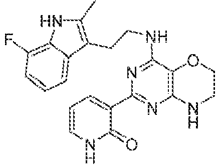

Prepared according to general method 5b, using 2-chloro-N-[2-(7-fluoro-2-methyl-1H-indol-3-yl)ethyl]-7,8-dihydro-6H-pyrimido[5,4-b][1,4]oxazin-4-amine (85 mg, 0.235 mmol) and (2-Oxo-1,2-dihydropyridin-3-yl)boronic acid(1.50 eq, 49 mg, 0.352 mmol). The crude was purified by column chromatography eluting with a gradient of EtOAc (0% to 100%; v/v) in iso-Hex then MeOH (0% to 20%; v/v) in EtOAc to afford 3-[4-[2-(7-fluoro-2-methyl-1H-indol-3-yl)ethylamino]-7,8-dihydro-6H-pyrimido[5,4-b][1,4]oxazin-2-yl]-1H-pyridin-2-one (40 mg, 0.0932 mmol, 39.69% yield) as a pale yellow solid. Example 41 is subject to keto-enol tautomerism. It may also exist in the following form: 3-(4-((2-(7-fluoro-2-methyl-3 H-indol-3-yl)ethyl)amino)-7,8-dihydro-6H- pyrimido[5,4-b][1,4]oxazin-2-yl)pyridin-2-ol 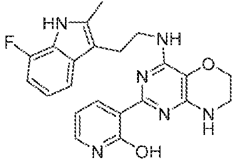 UPLC-MS analysis (4 min, basic): rt = 1.86 min, m/z=421.4 [M+H]+, 98% purity. 1H NMR (400 MHz, DMSO-D6) δ 11.15 (s, 1H), 8.46 (dd, J=7.6, 2.1 Hz, 1H), 8.14 (dd, J=4.8. 2.1 Hz,1H), 7.43 - 7.28 (m,2H), 7.01- 6.86 (m, 2H), 6.85 - 6.69 (m,2H), 4.14 (t, J=4.3 Hz, 2H), 3.54 (q, J =7.0 Hz, 2H), 3.47 (d, J = 4.9 Hz, 2H), 2.91 (t, J=7.6 Hz, 2H),2.34 (s, 3H). 1H not observed. 19F NMR (376 MHz, DMSO-D6) δ -134.27.

### Intermediate for Example 42

### 42.1 tert-butyl N-[2-[2,4-dichloro-6-[2-(5,7-difluoro-2-methyl-1H-indol-3-yl)ethylamino]pyrimidin-5-yl]oxyethyl)carbamate

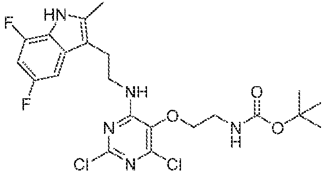

Prepared according to general method 2a. using tert-butyl N-[2-(2,4,6-trichloropyrimidin-5-yl)oxyethyl]carbamate (350 mg, 102 mmol) and 2-(5,7-difluoro-2-methyl-1H-indol-3-yl)ethanamine;hydrochloride (413 mg, 1.02 mmol). The reaction was evaporated then the residue was suspended in water and the resulted precipitate was collected by filtration. The crude was purified by column chromatography eluting with a gradient of EtOAc (0% to 80%; v/v) in iso-Hex to afford tert-butyl N-[2-[2,4-dichloro-6-[2-(5,7-difluoro-2-methyl-1H-indol-3-yl)ethylamino]pyrimidin-5-yl]oxyethyl]carbamate (230 mg,0.343 mmol, 33.57% yield) as an off-white solid.

UPLC-MS analysis (2 min, basic): rt = 1.29 min, m/z = 516.2/518.1/520.1 [M+H]+, 77% purity.

1H NMR (400 MHz, DMSO-D6) δ 11.28 (s, 1H), 8.01 (t, J = 5.9 Hz, 1H), 7.12 (dd, J = 9.6, 2.2 Hz, 1H), 7.09 - 6.99 (m, 1H), 6.85 - 6.73 (m, 1H), 3.84 (t, J = 5.5 Hz, 2H), 3.42 (q, J = 6.8 Hz, 2H), 3.22 (q, J = 5.5 Hz, 2H), 2.82 (t, J = 7.6 Hz, 2H), 2.29 (s, 3H), 1.34 (s, 9H).

19F NMR (376 MHz, DMSO-D6) δ -123.23, -130.75.

### 42.2 5-(2-aminoethoxy)-2,6-dichloro-N-[2-(5,7-difluoro-2-methyl-1H-indol-3-yl)ethyl]pyrimidin-4-amine;hydrochloride

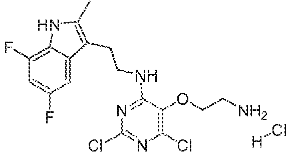

Prepared according to general method 3, using tert-butyl N-[2-[2,4-dichloro-6-[2-(5,7-difluoro-2-methyl-1H-indol-3-yl)ethyl amino]pyrimidin-5-yl]oxyethyl]carbamate (225 mg, 0.336 mmol) to afford 5-(2-aminoethoxy)-2,6-dichloro-N-[2-(5,7-di fluoro-2-methyl-1H-indol-3-yl)ethyl]pyrimidin-4-amine;HCl (210 mg,0.325 mmol, 96.78% yield) as an off-white solid. UPLC-MS analysis (2 min, basic): rt = 1.20 min, m/z = 416.3/418.2/420.2 [M+H]+, 81% purity. 1H NMR (400 MHz, DMSO-D6) δ 11.29 (s, 1H), 8.42 (t, J = 6.0 Hz, 1H), 8.29 (s, 3H), 7.17 (dd, J = 9.5, 2.2 Hz, 1H), 6.86 - 6.72 (m, 1H), 4.08 (t, J = 5.0 Hz, 2H), 3.43 (q, J = 6.6 Hz, 2H), 3.25 - 3.15 (m, 2H), 2.88 - 2.80 (m, 2H), 2.31 (d, J = 6.4 Hz, 3H). 19F NMR (376 MHz, DMSO-D6) δ -123.16 (t, J = 9.5 Hz), -130.72 (d, J = 11.2 Hz).

### 42.3 2-chloro-N-[2-(5,7-difluoro-2-methyl-1H-indol-3-yl)ethyl]-7,8-dihydro-6H-pyrimido [5,4-b][1,4]oxazin-4-amine

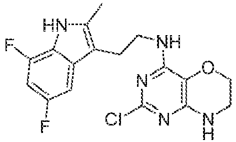

Prepared according to general method 4. using 5-(2-aminoethoxy)-2,6-dichloro-N-(2-(5,7-difluoro-2-methyl-1H-indol-3-yl)ethyl]pyrimidin-4-amine;hydrochloride (190 mg, 0.340 mmol) to afford 2-chloro-N-[2-(5,7-difluoro-2-methyl- 1H-indol-3-yl)ethyl]-7,8-dihydro-6H-pyrimido[5,4-b][1,4]oxazin-4-amine (150 mg,0.336 mmol, 98.75% yield) as a beige solid. UPLC-MS analysis (2 min, basic): rt = 1.21 min, m/z = 380.3/382.2 [M+H]+, 67% purity. 1H NMR (400 MHz, DMSO-D6) δ 11.22 (s, 1 H), 7.16 (dt, J = 9.6, 2.5 Hz, 1H), 7.08 (d, J = 2.5 Hz, 1H), 6.84 - 6.70 (m. 1 H), 6.58 (t, J = 5.7 Hz, 1H), 4.09 - 3.91 (m, 2H), 3.33 (q. J = 3.3 Hz, 2H), 3.30 (d, J = 5.6 Hz, 2H), 2.81 - 2.67 (m, 2H), 2.34 - 2.20 (m, 3H). 19F NMR (376 MHz, DMSO-D6) δ -123.40, - 130.96.

### Example 42 3-[4-[2-(5,7-difluoro-2-methyl-1H-indol-3-yl)ethylamino]-7,8-dihydro-6H-pyrimido[5,4-b][1,4]oxazin-2-yl]-1H-pyridin-2-one

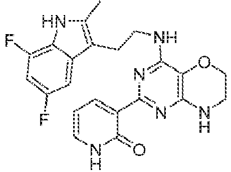

Prepared according to general method 5b, using 2-chloro-N-[2-(5,7-difluoro-2-methyl-1 H-indol-3-yl)ethyl]-7,8-dihydro-6H-pyrimido[5,4-b][1,4]oxazin-4-amine (90 mg, 0.201 mmol) and (2-Oxo-1,2-dihydropyridin-3-yl)boronic acid (1.50 eq, 42 mg, 0.302 mmol). The crude was purified by column chromatography (20 g cartridge) eluting with a gradient of EtOAc (0% to 100%; v/v) in iso-Hex then MeOH (0% to 20%; v/v) in EtOAc to afford 3-[4-[2-(5,7-difluoro-2-methyl-1H-indol-3-yl)ethylamino]-7,8-dihydro-6H-pyrimido(5,4-b][1,4]oxazin-2-yl]-1H-pyridin-2-one (40 mg,0.0912 mmol, 45.29% yield) as a yellow solid. Example 42 is subject to keto-enol tautomerism. It may also exist in the following form: 3-(4-((2-(5,7-difluoro-2-methyl-1H-indol-3-yl)ethyl)amino)-7,8-dihydro-6H-pyrimido[5,4-b][1,4]oxazin-2-**yl)pyridin-2-ol**

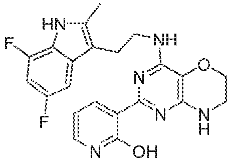

UPLC-MS analysis (4 min, basic): rt = 1.90 min, m/z = 439.3 [M+H]+, 100% purity. 1H NMR (400 MHz, DMSO-D6) δ 11.22 (s, 1H), 8.41 (dd, J = 7.6. 2.1 Hz, 1H), 8.10 (dd, J = 4.8, 2.1 Hz, 1H), 7.31 (s, 1H), 7.08 (dd, J = 9.7, 2.2 Hz, 1H), 6.87 (dd, J = 7.5, 4.8 Hz, 1H), 6.82 - 6.72 (m, 1H), 6.69 (t J = 6.2 Hz, 1H), 4.09 (t, J = 4.2 Hz, 2H), 3.49 (q, J = 7.0 Hz, 2H), 3.42 (s, 2H), 2.83 (t, J = 7.4 Hz, 2H), 2.29 (s, 3H). 1H not observed. 19F NMR (376 MHz, DMSO-D6) δ-123.40, -130.90.

### Intermediates for Example 43

### 43.1 tert-butyl N-[2-[2,4-dichloro-6-[2-[6-(trifluoromethoxy)-1H-indol-3-yl]ethylamino] pyrimidin-5-yl]oxyethyl]carbamate

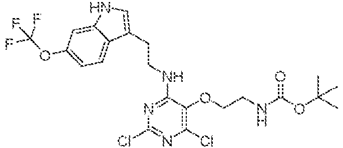

Prepared according to general method 2a, using tert-butyl N-[2-(2,4,6-trichloropyrimidin-5-yl)oxyethyl]carbamate (0.50 g, 1.46 mmol) and 2-[6-(trifluoromethoxy)-1H-indol-3-yl]ethanamine (356 mg, 1.46 mmol).

The solvent was evaporated to dryness under reduced pressure and the crude was purified by column chromatography eluting with a gradient of MeOH (0% to 5%; v/v) in DCM to afford tert-butyl N-[2-[2,4-dichloro-6-[2-[6-(trifluoromethoxy)-1H-indol-3-yl]ethylamino] pyrimidin-5-yl]oxyethyl]carbamate (676 mg,1.07 mmol, 73.22% yield)) as a beige solid.

UPLC-MS analysis (2 min, acidic): rt = 1.33 min, m/z = 550.2/552.2/554.2 [M+H]+, 87% purity.

1H NMR (400 MHz, DMSO-D6) δ 11.09 (s, 1H), 8.05 (s, 1H), 7.72 (d, J = 8.6 Hz, 1H), 7.31 (t, J = 2.6 Hz, 2H), 7.09 (s, 1H), 7.01 - 6.94 (m, 1H), 3.90 (t, J = 5.5 Hz, 2H), 3.61 (q, J = 7.0 Hz, 2H), 3.31 - 3.23 (m, 2H), 2.98 (t, J = 7.5 Hz, 2H), 1.38 (d, J = 1.8 Hz, 9H).

19F NMR (376 MHz, DMSO-d6) δ -56.71.

### 43.2 5-(2-aminoetboxy)-2,6-dichloro-N-[2-16-(trifluorometboxy)-1H-indol-3-yl]ethyl] pyrimidin-4-amine;hydrochloride

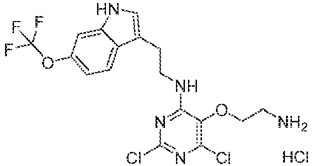

Prepared according to general method 3, using tert-butyl N-[2-[2,4-dichloro-6-[2-[6-(trifluoromethoxy)-1H-indol-3-yl]etihylamino]pyrimidin-5-yl]oxyethyl]carbamate (676 mg, 1.23 mmol) to afford5-(2-aminoethoxy)-2,6-dichloro-N-[2-[6-(trifluoromethoxy)- 1H-indol-3-yl]ethyl]pyrimidin-4-amine;hydrochloride (645 mg,1.22 mmol, 99.26% yield) as a brown solid. UPLC-MS analysis (2 min, acidic): rt = 0.98 min, m/z = 450.2/452.2/454.2 [M+H]+, 92% purity.

1H NMR (400 MHz, DMSO-D6) δ 11.13 (s, 1H), 8.43 (d, J = 35.9 Hz, 4H), 7.76 (d, J = 8.9 Hz, 1H), 7.35 - 7.29 (m, 2H), 6.97 (d, J = 8.6 Hz, 1H), 4.14 (d, J = 5.4 Hz, 2H), 3.61 (d, J = 6.1 Hz, 2H), 3.22 (s, 2H), 2.98 (t, J = 7.5 Hz, 2H). 19F NMR (376 MHz, DMSO-d6) δ -56.70.

### 43.3 2-chloro-N-[2-[6-(trifluoromethoxy)-1H-indol-3-yl]ethyl]-7,8-dihydro-6H-pyrimido [5,4-b][1,4]oxazin-4-amine

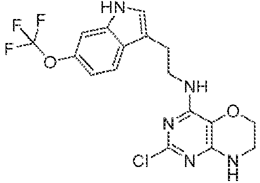

Prepared according to general method 4, using 5-(2-aminoethoxy)-2,6-dichloro-N-[2-[6-(trifluoromethoxy)-1H-indol-3-yl]ethyl]pyrimidin-4-amine;hydrochloride (676 mg, 1.39 mmol). The solvent was evaporated to dryness under reduced pressure and the crude was purified by column chromatography eluting with a gradient of MeOH (0% to 10%; v/v) in DCM to afford 2-chloro-N-[2-[6-(trifluoromethoxy)-1H-indol-3-yl]ethyl]-7,8-dihydro-6H-pyrimido[5,4-b][1,4]oxazin-4-amine (320 mg,0.565 mmol, 56.00% yield) as a brown solid. UPLC-MS analysis (2 min, acidic): rt = 1.15 min, m/z = 414.2/416.2 [M+H]+, 73% purity. 1H NMR (400 MHz, DMSO-D6) δ 11.05 (s, 1H), 7.75 (d, J = 8.6 Hz, 1H), 7.29 (dd, J = 5.8, 2.2 Hz, 2H), 7.12 (s, 1H), 6.95 (ddd, J = 8.7, 2.2, 1.1 Hz, 1H), 6.67 (t, J = 5.9 Hz, 1H), 4.06 (t, J = 4.4 Hz, 2H), 3.50 (q, J = 7.0 Hz, 2H), 3.38 (p. J = 3.7 Hz, 2H), 2.93 - 2.85 (m, 2H). 19F NMR (376 MHz, DMSO-d6) δ -56.70.

### Example 43 3-(4-((2-(6-(trifluoromethoxy)-1H-indol-3-yl)ethyl)amino)-7,8-dihydro-6H-pyrimido[5,4-b][1,4]oxazin-2-yl)pyridin-2(1H)-one

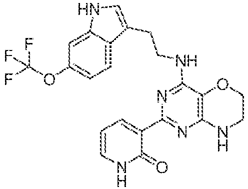

Prepared according to general method 5b, using 2-chloro-N-[2-[6-(trifluoromethoxy)-1H-indol-3-yl]ethyl]-7,8-dihydro-6H-pyrimido[5,4-b][1,4]oxazin-4-amine (1.00 eq, 320 mg, 0.773 mmol) and (2-hydroxy-3-pyridyl)boronic acid (1.50 eq, 161 mg, 1.16 mmol). The crude was purified by column chromatography (20 g cartridge) eluting with a gradient of MeOH (0% to 10%; v/v) in DCM and subsequent prep HPLC (basic method) to afford 3-[4-[2-[6-(trifluoromethoxy)-1H-indol-3-yl]ethylamino]-7,8-dihydro-6H-pyrimido[5,4-b][1,4]oxazin-2-yl]pyridin-2-ol (19 mg,0.0404 mmol, 5.23% yield) as a yellowish solid. Example 43 is subject to keto-enol tautomerism. It may also exist in the following form: 3-(4-((2-(6-(trifluorometboxy)-1H-indol-3-yl)ethyl)amino)-7,8-dihydro-6H-pyrimido[5,4-b)[1,4]oxazin-2-yl)pyridin-2-ol 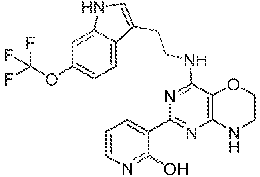 UPLC-MS analysis (4 min, basic): rt = 2.01 min, m/z = 473.3 [M+H]+, 99% purity.

1H NMR (400 MHz, DMSO-D6) δ 11.09 (s, 1H), 8.50 (d, J = 7.5 Hz, 1H), 8.14 (s, 1H), 7.65 (d, J = 8.5 Hz, 1H), 7.42 - 7.25 (m, 3H), 6.96 (d. J = 10.6 Hz, 2H), 6.78 (s, 1H), 4.14 (s. 2H), 3.66 (s, 2H), 3.46 (s, 2H), 3.00 (s, 2H). 1H signal not observed. 19F NMR (376 MHz, DMSO-d6) δ -56.69.

### Intermediates for Example 44

### 44.1 tert-butyl N-methyl-N-[2-(2,4,6-trichloropyrimidin-5-yl)oxyethyl]carbamate

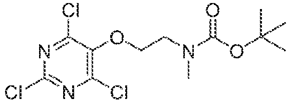

Prepared according to general method 1, using 2,4,6-trichloropyrimidin-5-ol (0.30 g, 1.43 mmol) and tert-butyl N-(2-hydroxyethyl)-N-methyl-carbamate (0.29 mL, 1.71 mmol).

The crude was purified by column chromatography (40 g cartridge) eluting with a gradient of EtOAc (0% to 45%; v/v) in heptane to afford tert-butyl N-methyl-N-[2-(2,4,6-trichloropyrimidin-5-yl)oxyethyl)carbamate (130 mg,0.365 mmol, 25.51% yield) as a colourless oil.

UPLC-MS analysis (2 min, basic): rt = 1.27 min, no ionisation, 100% purity.

1H NMR (400 MHz, DMSO-D6) δ 4.16 (s, 2H), 3.53 (t, J = 5.4 Hz, 2H), 2.85 (s, 3H), 1.35 (s, 9H).

### 44.2 tert-butyl N-[2-[2,4-dichloro-6-[2-(1H-indol-3-yl)ethylamino]pyrimidin-5-yl]oxyethyl]-N-methyl-carbamate

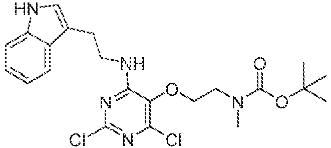

Prepared according to general method 2a, using tert-butyl N-methyl-N-[2-(2,4,6-trichloropyrimidin-5-yl)oxyethyl] carbamate (1.00 eq, 120 mg, 0.336 mmol) and Tryptamine (1.11 eq, 60 mg, 0.374 mmol) in Ethanol (1.8 mL).

The reaction was concentrated to dryness to give an oily residue, which was suspended in water (25 mL) with stirring. The resultant suspension was filtered and the solid was dried under vacuum to afford tert-butyl N-[2-[2,4-dichloro-6-[2-(1H-indol-3-yl)ethylamino]pyrimidin-5-yl]oxyethyl]-N-methyl-carbamate (145 mg,0.284 mmol, 84.32% yield) as a white solid.

UPLC-MS analysis (2 min, basic): rt = 1.30 min, m/z = 480.2/482.2/484.1 [M+H]+, 94% purity.

1H NMR (400 MHz, DMSO-D6) δ 10.81 (s, 1H), 8.05 (d, J = 41.3 Hz, 1H), 7.59 (d, J = 7.8 Hz, 1H), 7.30 (d, J = 8.1 Hz, 1H), 7.15 (d, J = 2.3 Hz, 1H), 7.02 (t, J = 7.4 Hz, 1H), 6.94 (t, J = 7.4 Hz, 1H), 3.91 (d, J = 1 8.9 Hz, 2H), 3.57 (q, J = 7.0 Hz, 2H), 3.46 (s, 2H), 2.94 (d, J = 7.7 Hz, 2H), 2.83 (s, 3H), 1.36 (s, 9H).

### 44.3 2,6-dichloro-N-[2-(1H-indol-3-yl)ethyl]-5-[2-(methylamino)ethoxy]pyrimidin-4-amine;hydrochloride

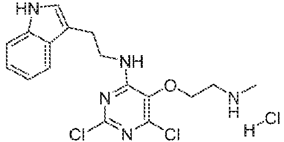

Prepared according to general method 3, using tert-butyl N-[2-[2,4-dichloro-6-[2-(1H-indol-3-yl)ethylamino]pyrimidin-5-yl] oxyethyl]-N-methyl-carbamate (140 mg, 0.274 mmol) to afford 2,6-dichloro-N-[2-(1 H-indol-3-yl)ethyl]-5-[2-(methylamino) ethoxy]pyrimidin-4-amine;hydrochloride (125 mg,0.258 mmol, 94.17% yield) as a beige solid.

UPLC-MS analysis (2 min, basic): rt = 1.20 min, m/z = 380.3/382.2/384.2 [M+H]+, 86% purity.

1 H NMR (400 MHz, DMSO-D6) δ 10.86 (s, 1H), 9.29 (s. 2H), 8.50 (t, J = 6.0 Hz, 1H), 7.68 (d, J = 7.8 Hz, 1H), 7.34 (d, J = 8.2 Hz, 1H), 7.30 - 7.17 (m, 1H), 7.07 (t, J = 7.7 Hz, 1H). 6.99 (q, J = 8.1 Hz, 1H), 4.18 (t, J = 4.9 Hz, 2H), 3.63 (q, J = 7.1 Hz, 2H), 3.12 - 2.95 (m, 4H), 2.60 (t, J = 5.3 Hz, 3H).

### 44.4 2-chloro-N-[2-(1H-indol-3-yl)ethyl]-8-methyl-6,7-dihydropyrimido[5,4-b][1,4] oxazin-4-amine

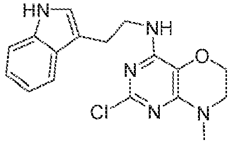

Prepared according to general method 4, using 2,6-dichloro-N-[2-(1H-indol-3-yl)ethyl]-5-[2-(methylamino)ethoxy]pyrimidin-4-amine;hydrochloride (1.00 eq. 120 mg, 0.2476 mmol) to afford 2-chloro-N-[2-(1H-indol-3-yl)ethyl]-8-methyl-6,7-dihydro pyrimido[5,4-b][1,4]oxazin-4-amine (90 mg,0.246 mmol, 99.36% yield) as an off-white solid.

UPLC-MS analysis (2 min, basic): rt = 1.13 min, m/z = 344.1/346.1 [M+H]+, 94% purity.

1H NMR (400 MHz, DMSO-D6) δ 10.75 (s, 1H). 7.62 (d, J = 7.8 Hz, 1H), 7.29 (d, J = 8.1 Hz, 1H), 7.11 (d, J = 2.3 Hz, 1H), 7.07 - 6.96 (m, 1H), 6.96 - 6.89 (m, 1H), 6.55 (t, J = 6.0 Hz, 1H), 4.11 (t, J = 4.4 Hz, 2H), 3.47 (q, J = 7.0 Hz, 2H), 3.38 (t, J = 4.4 Hz, 2H), 2.92 (s, 3H), 2.85 (t, J = 7.7 Hz, 2H).

### Example 44 3-(4-((2-(1H-indol-3-yl)ethyl)amino)-8-methyl-7,8-dihydro-6H-pyrimido[5,4-b][1,4]oxazin-2-yl)pyridin-2(1H)-one

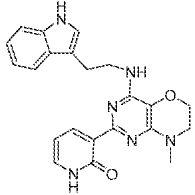

Prepared according to general method 5b, using 2-chloro-N-(2-(1H-indol-3-yl)ethyl]-8-methyl-6,7-dihydropyrimido[5,4-b][1,4]oxazin-4-amine (85 mg, 0.232 mmol) and (2- Oxo-1,2-dihydropyridin-3-yl)boronic acid (48 mg, 0.349 mmol). The crude was purified by column chromatography (20 g cartridge) eluting with a gradient of EtOAc (0-100%; v/v) in iso-Hex then MeOH (0-10%; v/v) in EtOAc to afford 3-(4-(2-(1H-indol-3-yl)ethylamlno)-8-methyl-6,7-dihydropyrimido[5,4-b][1,4]oxazin-2-yl]pyridin-2-ol (24 mg,0.0567 mmol, 24.38% yield) as an off-white solid. Example 44 is subject to keto-enol tautomerism. It may also exist in the following form: 3-(4-((2-(1H-indol-3-yl)ethyl)amino)-8-methyl-7,8-dihydro-6H-pyrimido[5,4-b][1,4]oxazin-2-yl)pyridin-2-ol 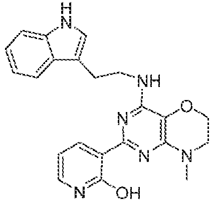 UPLC-MS analysis (4 min, basic): rt = 1.82 min, m/z = 403.4 [M+H]+, 95% purity. 1H NMR (400 MHz, DMSO-D6) δ 10.84 (s, 1H), 8.58 (s, 1H), 8.15 (s, 1H), 7.58 (d, J = 7.9 Hz, 1H), 7.34 (d, J = 8.0 Hz, 1H), 7.21 (d, J = 2.3 Hz, 1H), 7.12 - 7.03 (m, 1H), 7.04 - 6.91 (m, 2H), 6.78 (s, 1H), 4.24 (t, J = 4.4 Hz, 2H), 3.64 (q, J = 7.0 Hz, 2H), 3.51 (s, 2H), 3.11 (s, 3H), 2.99 (t, J = 7.6 Hz, 2H), 1H not observed.

### Example 45 (S)-3-(4-((2-(1H-indol-3-yl)ethyl)amino)-7-(methoxymethyl)-7,8-dihydro-6H-pyrimido[5,4-b][1,4]oxazin-2-yl)pyridin-2(1H)-one

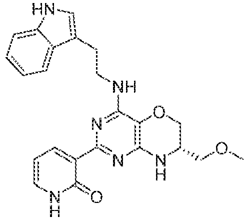

Prepared according to general method 5b, using (7S)-2-chloro-N-[2-(1H-indol-3-yl)ethyl]-7-(methoxymethyl)-7,8-dihydro-6H-pyrimido[5,4-b][1,4]oxazin-4-amine (150 mg, 0.401 mmol) and (2-hydroxy-3-pyridyl)boronic acid (84 mg, 0.602 mmol). The crude was purified by Reversed Phase column chromatography (23 g cartridge) eluting with MeCN in water gradient (5% to 95% acidic buffer) to afford 3-[(7S)-4-[2-(1H-indol-3-yl)ethylamino]-7-(methoxymethyl)-7,8-dihydro-6H-pyrimido[5,4-b][1,4]oxazin-2-yl]pyridin-2-ol (41 mg,0.0920 mmol, 22.92% yield) as a yellow solid. Example 45 is subject to keto-enol tautomerism. It may also exist in the following form:

### (S)-3-(4-((2-(1H-indol-3-yl)ethyl)amino)-7-(methoxymethyl)-7,8-dihydro-6H-pyrimido[5,4-b][1,4]oxazin-2-yl)pyridin-2-ol

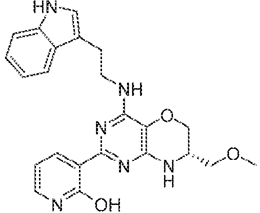

UPLC-MS analysis (4 min, acidic): rt = 1.32 min, m/z = 433.4 [M+H]+, 97% purity. 1H NMR (400 MHz, DMSO-D6) δ 10.84 (s, 1H), 8.53 (s, 1H), 8.19 - 8.06 (m, 1H), 7.58 (d, J = 7.8 Hz, 1H), 7.53 (s, 1H), 7.34 (dd, J = 8.1, 1.0 Hz, 1H), 7.21 (d, J = 2.2 Hz, 1H), 7.10 - 7.03 (m, 1 H), 7.03 -6.90 (m, 2H), 6.85 (s, 1H), 4.18 - 3.99 (m, 2H), 3.73 (s, 1H), 3.66 (q, J = 6.9 Hz, 2H), 3.48 - 3.40 (m, 2H), 2.99 (t, J = 7.6 Hz, 2H). 4Hs not observed.

1H NMR (400 MHz, METHANOL-D4) δ 8.54 (d, J = 8.2 Hz, 1H), 7.95 (d, J = 5.4 Hz, 1H), 7.63 - 7.55 (m, 1H), 7.29 (dq, J = 8.1, 1.1 Hz, 1H), 7.11 (s, 1H), 7.09 - 7.03 (m, 1H), 7.03 - 6.94 (m, 1 H), 6.81 (dd, J = 7.5, 5.5 Hz, 1H), 4.19 - 4.07 (m, 2H), 3.78 (t, J = 7.1 Hz, 3H), 3.52 - 3.43 (m, 2H), 3.40 (s, J = 1.0 Hz, 3H), 3.08 (t, J = 7.2 Hz, 2H).

### Intermediates for Example 46

### 46.1 tert-butyl N-[(1R)-1-(methoxymethyl)-2-(2,4,6-trichloropyrimidin-5-yl)oxy-ethyl]carbamate

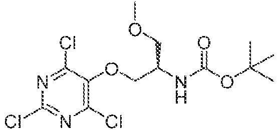

Prepared according to general method 1, using 2,4,6-trichloro pyrimidin-5-ol (1.00 eq, 0.30 g, 1.43 mmol) and tert-butyl N-[(1S)-1-(hydroxymethyl)-2-methoxy-ethyl]carbamate (1.10 eq, 0.32 g, 1.57 mmol). The crude was purified by column chromatography (40 g column) eluting with 18-20% EtOAc in iso-hexane to afford tert-butyl N-[(1R)-3-(methoxymethyl)-2-(2,4,6-trichloropyrimidin-5-yl)oxy-ethyl]carbamate (380 mg,0.983 mmol, 68.77% yield) as a white solid.

UPLC-MS analysis: (2 min, basic): rt = 1.22 min, no ionisation, 100% purity.

1H NMR (400 MHz, DMSO-D6) δ 6.97 (d, J = 8.2 Hz, 1H), 4.19 (dd, J = 9.8, 4.8 Hz, 1H), 4.07 (dd, J = 9.7, 6.4 Hz, 1H), 3.98 - 3.91 (m, 1H), 3.43 (d, J = 6.1 Hz, 2H), 3.28 (s, 3H), 1.39 (s, 9H).

### 46.2 tert-butyl N-[(1R)-1-[[2,4-dichloro-6-[2-(1H-indol-3-yl)ethylamino]pyrimidin-5-yl] oxymethyl]-2-methoxy-ethyl]carbamate

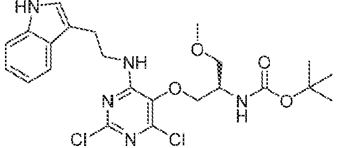

Prepared according to general method 2a, using tert-butyl N-[(1R)-1-(methoxymethyl)-2-(2,4,6- trichloropyrimidin-5-yl)oxy-ethyl]carbamate (1.00 eq, 0.38 g, 0.983 mmol) and Tryptamine (1.05 eq, 0.17 g, 1.03 mmol) in Ethanol (4.2 mL). The reaction was concentrated to dryness to give an oily residue, which was suspended in water (10 mL) with stirring. The resultant suspension was filtered and the solid was dried under vacuum to afford tert-butyl N-[(1R)-1-[[2,4-dichloro-6-[2-(1H-indol-3-yl)ethylamino]pyrimidin-5-yl]oxymethyl]-2-methoxy-ethyl]carbamate (534 mg,0.942 mmol, 95.81% yield) as an off white solid

UPLC-MS analysis (2 min, basic): rt = 1.29 min, m/z =510.3/512.3/514.3 [M+H]+, 95% purity.

1H NMR (400 MHz, DMSO-D6) δ 10.84 (s, 1H), 7.91 (s, 1H), 7.67 - 7.59 (m, 1H), 7.35 (dt, J = 8.2, 1.0 Hz, 1H), 7.19 (d, J = 2.4 Hz, 1H), 7.12 - 7.06 (m, 1H), 7.05 (d, J = 7.7 Hz, 1H), 6.99 (ddd, J = 8.0, 7.0, 1.1 Hz, 1H), 3.96 (d, J = 6.3 Hz, 1H), 3.88 (t, J = 6.5 Hz, 1H), 3.63 (q, J = 6.9 Hz, 2H), 3.38 (s, 2H), 3.24 (s, 3H), 3.09 (q, J = 7.1 Hz, 1H), 2.99 (t, J = 7.6 Hz, 2H), 1.40 (s, 9H).

### 46.3 2,6-dichloro-N-[2-(1H-indol-3-yl)ethyl]-5-[(2R)-2-amino-3-methoxy-propoxy] pyrimidin-4-amine;hydrochloride

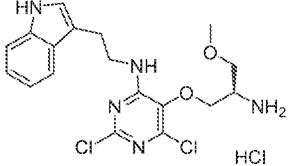

Prepared according to general method 3, using tert-butyl N-[(1R)-1-((2,4-dichloro-6-(2-(1H-indol-3-yl)ethylamino] pyrimidin-5-yl]oxymethyl]-2-methoxy-ethyl]carbamate (1.00 eq, 0.53 g, 1.05 mmol) to afford 5-[(2R)-2-amino-3-methoxy-propoxy]-2,6-dichloro-N-[2-(1H-indol-3-yl)ethyl]pyrimidin-4-amine;hydrochloride (465 mg,0.937 mmol, 89.54% yield) as a beige solid.

UPLC-MS analysis (2 min, basic): rt = 1.13 min, m/z = 410.3/412.3/414.2 [M+H]+, 92% purity.

1H NMR (400 MHz, DMSO-D6) δ 10.85 (s, 1 H), 8.59 (s, 3H), 8.48 (t, J = 6.0 Hz, 1H), 7.68 (d, J = 7.8 Hz, 1H), 7.37 - 7.30 (m, 1H), 7.20 (d, J = 2.3 Hz, 1H), 7.10 -7.03 (m, 1H), 7.02 - 6.95 (m, 1H), 4.21 (d, J = 6.2 Hz, 1H), 4.06 (dd, J = 10.1, 5.6 Hz, 1H), 3.75 - 3.68 (m, 1H), 3.69 - 3.58 (m, 4H), 3.32 (s, 3H), 3.04 - 2.93 (m, 2H),

### 46.4 (7R)-2-chloro-N-[2-(1H-indol-3-yl)ethyl]-7-(methoxymethyl)-7,8-dihydro-6H-pyrimido[5,4-b][1,4]oxazin-4-amine

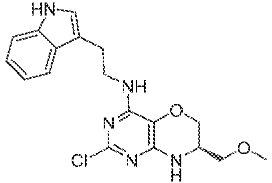

Prepared according to general method 4, using 5-[(2R)-2-amino-3-methoxy-propoxy]-2,6-dichloro-N-[2-(1H-indol-3-yl)ethyl]pyrimidin-4-amine;hydrochloride (1.00 eq, 0.47 g, 0.937 mmol) to afford (7R)-2-chloro-N-[2-(1H-indol-3-yl)ethyl]-7-(methoxymethyl)-7,8-dihydro-6H-pyrimido[5,4-b][1,4]oxazin-4-amine (0.34 g,0.833 mmol, 88.97% yield) as a beige solid.

UPLC-MS analysis (2 min, basic): rt = 1.06 min, m/z = 374.2/376.1 [M+H]+, 93% purity.

1 H NMR (400 MHz, DMSO-D6) δ 10.79 (s, 1H), 7.66 (d, J = 7.8 Hz, 1H), 7.33 (d, J = 8.1 Hz, 1H), 7.27 (d, J = 3.2 Hz, 1H), 7.16 (d, J = 2.3 Hz, 1H), 7.12 - 7.03 (m, 1H), 7.03 - 6.94 (m, 1H), 6.72 (t, J = 5.9 Hz, 1H), 4.04 (dd, J = 10.7, 4.0 Hz, 1H), 3.96 (dd, J = 10.7, 2.9 Hz, 1H), 3.70 - 3.59 (m, 1H), 3.52 (q, J = 6.9 Hz, 2H), 3.38 - 3.33 (m, 2H), 3.29 (s, 3H), 2.90 (t, J = 7.7 Hz, 2H).

### Example 46 (7R)-2-(5-fluoro-3-pyridyl)-N-[2-(1H-indol-3-yl)ethyl]-7-(methoxymethyl)-7,8-dihydro-6H-pyrimido[5,4-b][1,4]oxazin-4-amine

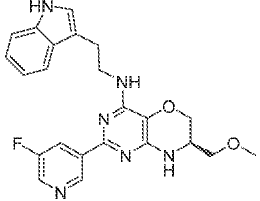

Prepared according to general method 5b, using (7R)-2-chloro-N-[2-(1H-indol-3-yl)ethyl]-7-(methoxymethyl)-7.8-dihydro-6H-pyrimido[5,4-b][1,4]oxazin-4-amine (1.00 eq, 100 mg, 0.249 mmol) and 5-Fluoropyridine-3-boronic Acid (2.00 eq, 70 mg, 0.498 mmol). The crude was purified by column chromatography (20 g column) eluting with a gradient 0 to 100% (v/v) EtOAc in iso-Hex to afford (7R)-2-(5-fluoro-3-pyridyl)-N-[2-(1H-indol-3-yl)ethyl]-7-(methoxymethyl)-7,8-dihydro-6H-pyrimido[5,4-b][1,4]oxazin-4-amine (62 mg,0.143 mmol, 57.36% yield) as an off-white solid. UPLC-MS analysis (4 min, basic): rt = 1.86 min, m/z = 435.4 [M+H]+, 100% purity.

1H NMR (400 MHz, DMSO-D6) δ 10.81 (s, 1H). 9.23 (t, J = 1.8 Hz, 1H), 8.60 (d, J = 2.9 Hz, 1H), 8.30 - 8.16 (m, 1H), 7.63 (d, J = 7.8 Hz, 1H), 7.34 (dt, J = 8.1, 1.0 Hz, 1H), 7.20 (d, J = 2.3 Hz, 1H), 7.15 - 7.03 (m. 2H), 7.03 - 6.93 (m, 1H), 6.56 (t, J = 5.9 Hz, 1H), 4.13 - 4.01 (m, 2H), 3.78 - 3.65 (m, 3H), 3.45 - 3.35 (m, 2H), 3.32 (s, 3H), 2.99 (dd, J = 8.9, 6.4 Hz, 2H). 19F NMR (376 MHz, DMSO-D6) δ -128.10.

### Intermediates for Example 47

### 47.1 tert-butyl N-[(1R)-1-[[24-dichloro-6-[2-(6-fluoro-1H-indol-3-yl)ethylamino] pyrimidin-5-yl]oxymethyl]-2-methoxy-ethyl]carbamate

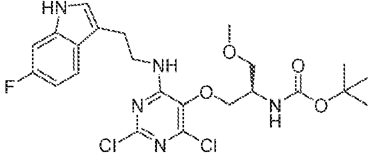

Prepared according to general method 2a, using tert-butyl N-[(1R)-1-(methoxymethyl)-2-(2,4,6-trichloro pyrimidin-5-yl)oxy-ethyl]carbamate (1.00 eq, 250 mg, 0.647 mmol) and 2-(6-fluoro-1H-indol-3-yl)ethanamine (1.10 eq, 127 mg, 0.714 mmol) in Ethanol (4.3 mL).

The reaction was concentrated to dryness to give an oily residue, which was suspended in water (50 mL) with stirring. The resultant suspension was filtered and the solid was dried under vacuum to afford tert-butyl N-[(1R)-1-[[2,4-dichloro-6-[2-(6-fluoro-1H-indol-3-yl)ethylamino)pyrimidin-5-yl]oxymethyl]-2-methoxy-ethyl]carbamate (290 mg,0.472 mmol, 73.00% yield) as an white solid. UPLC-MS analysis (2 min, basic): rt = 1.29 min, m/z = 529.3/531.3/533.3 [M+H]+, 86% purity.

1H NMR (400 MHz, DMSO-D6) δ 10.88 (s, 1H), 7.87 (s, 1H), 7.58 (dd, J = 8.6, 5.5 Hz, 1H), 7.14 (d, J = 2.3 Hz, 1H), 7.07 (dd, J = 10.2, 2.3 Hz, 1H), 7.02 - 6.91 (m, 1 H), 6.80 (ddd, J = 9.8, 8.6, 2.4 Hz, 1H), 3.94 - 3.78 (m, 3H), 3.70 - 3.39 (m, 2H), 3.38 - 3.30 (m, 2H), 3.21 (d, J = 15.4 Hz, 3H), 2.92 (t, J = 7.6 Hz, 2H), 1.34 (d, J = 0.8 Hz, 9H).

### 47.2 2,6-dichloro-N-[2-(6-fluoro-1H-indol-3-yl)ethyl]-5-[(2R)-2-amino-3-methoxy-propoxy]pyrimidin-4-amine

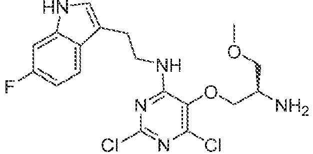

Prepared according to general method 3, using tert-butyl N-[(1R)-1-[[2,4-dichloro-6-[2-(6-fluoro-1H-indol-3-yl)ethylamino]pyrimidin-5-yl]oxymethyl]-2-methoxyethyl]carbamate (220 mg, 0.416 mmol).

The reaction was concentrated and the crude was purified by column chromatography using EtOAc/MeOH (1% in TEA) to afford 2,6-dichloro-N-[2-(6-fluoro-1H-indol-3-yl)ethyl]-5-[(2R)-2-amino-3-methoxy-propoxy]pyrimidin-4-amine (70 mg,0.159 mmol, 38.08% yield) as an off-white.

UPLC-MS analysis (2 min, basic): rt = 1.18 min, m/z = 428.0/430.0/432.0 [M+H]+, 97% purity.

1H NMR (400 MHz, DMSO-D6) δ 10.87 (s, 1H), 7.56 (dd, J = 8.7, 5.5 Hz, 1H), 7.15 (d, J = 2.3 Hz, 1H), 7.07 (ddd, J = 10.2, 2.4, 0.5 Hz, 1H), 6.80 (ddd, J = 9.8, 8.6, 2.4 Hz, 1H), 4.05 (q, J = 5.3 Hz, 2H), 3.88 (dd, J = 9.9, 3.7 Hz, 1H), 3.61 - 3.49 (m, 3H), 3.22 (s, 1H), 3.20 (s, 4H), 3.01 (dd, J = 13.5, 7.1 Hz, 1H), 2.92 (t, J = 7.4 Hz, 2H). 1H peak is not observed.

### 47.3 (7R)-2-chloro-N-[2-(6-fluoro-1H-indol-3-yl)ethyl]-7-(methoxymethyl)-7,8-dihydro-6H-pyrimido[5,4-b][1,4]oxazin-4-amine

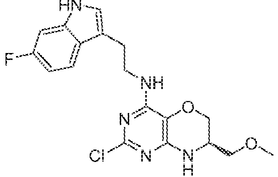

Prepared according to general method 4, using 5-[(2R)-2-amino-3-methoxy-propoxy]-2,6-dichloro-N-[2-(6-fluoro-1H-indol-3-yl) ethyl]pyrimidin-4-amine (70 mg, 0.163 mmol) to afford (7R)-2-chloro-N-[2-(6-fluoro-1H-indol-3-yl)ethyl]-7-(methoxymethyl)-7,8-dihydro-6H-pyrimido[5,4-b][1,4]oxazin-4-amine (50 mg,0.115 mmol, 70.27% yield) as beige solid. Note reaction required multiple microwave irradiation cycles for a total of 13h at 140°C.

UPLC-MS analysis (2 min, basic): rt = 1.07 min, m/z = 392.3/394.3 [M+H]+, 90% purity.

1H NMR (400 MHz, DMSO-D6) δ 10.83 (s, 1H), 7.61 (dd, J = 8.6, 5.6 Hz, 1H), 7.24 (d, J = 3.2 Hz, 1H), 7.12 (d, J = 2.2 Hz, 1H), 7.05 (dd, J = 10.2, 2.4 Hz, 1H), 6.82 - 6.75 (m, 1H), 6.71 (t, J = 5.9 Hz, 1H), 4.03 - 3.85 (m, 3H), 3.60 (s, 2H), 3.44 (dd, J = 8.5, 6.3 Hz, 2H), 3.25 (s, 3H), 2.87 - 2.79 (m, 2H).

### Example 47 (R)-3-(4-((2-(6-fluoro-1H-indol-3-yl)ethyl)amino)-7-(methoxymethyl)-7,8-dihydro-6H-pyrimido[5,4-b][1,4]oxazin-2-yl)pyridin-2(1H)-one

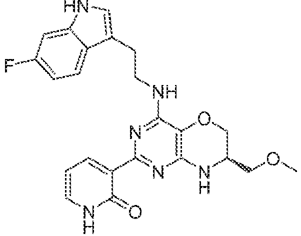

Prepared according to general method 5b, using (7R)-2-chloro-N-[2-(6-fluoro-1H-indol-3-yl)ethyl]-7-(methoxymethyl)-7,8-dihydro-6H-pyrimido[5.4-b][1,4]oxazin-4-amine (50 mg, 0.128 mmol) and (2-hydroxy-3-pyridyl)boronic acid (27 mg, 0.191 mmol). The crude was purified by Reversed Phase column chromatography (23 g cartridge) eluting with MeCN in water gradient (5% to 95% acidic buffer) to afford 3-[(7R)-4-[2-(6-fluoro-1H-indol-3-yl)ethylamino]-7-(methoxymethyl)-7,8-dihydro-6H-pyrimido [5,4-b][1,4]oxazin-2-yl]pyridin-2-ol (11 mg,0.0232 mmol, 18.18% yield) as a yellow solid.

### Example 47 Is subject to keto-enol tautomerism. It may also exist in the following form: (R)-3-(4-((2-(6-fluoro-1H-indol-3-yl)ethyl)amino)-7-(methoxymethyl)-7,8-dihydro-6H-pyrimido[5,4-b][1,4]oxazin-2-yl)pyridin-2-ol

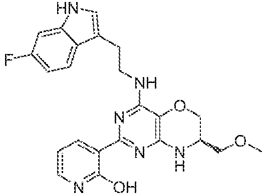

UPLC-MS analysis (4 min, acidic): rt = 1.34 min, m/z = 451.3 [M+H]+, 95% purity. 1H NMR (400 MHz, DMSO-D6) δ 10.91 (s, 1H), 8.47 (brs, 1H), 8.16 (s, 1H), 8.11 (brs, 1H), 7.55 (dd, J = 8.7, 5.5 Hz, 1 H), 7.51 (brs, 1 H), 7.21 (d, J = 2.2 Hz, 1H), 7.10 (dd, J = 10.2, 2.4 Hz, 1H), 6.92 (brs, 1H), 6.89 - 6.78 (m, 2H), 4.15 - 4.01 (m, 2H), 3.73 (m, 1H), 3.65 (q, J = 7.0 Hz, 2H), 3.45 - 3.35 (m, 2H), 3.31 (s, 3H), 3.02 - 2.93 (t, 2H), 19F NMR (376 MHz, DMSO-D6) δ -122.36 (td, J = 10.0, 5.5 Hz).

### Intermediates for Example 48

### 48.1 tert-butyl N-[(1R)-1-[[2,4-dichloro-6-(2-(1H-indol-3-yl)ethoxy]pyridin-5-yl) oxymethyl]-2-methoxy-ethyl]carbamate

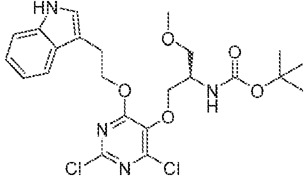

Prepared according to general method 2b, using tert-butyl N-[(1R)-1-(methoxymethyl)-2-(2,4,6-trichloropyrimidin-5-yl) oxy-ethyl]carbamate (590 mg, 1.53 mmol) and Tryptophol (246 mg, 1.53 mmol) at -40°C. The crude was purified by column chromatography eluting with 8:1 to 3:1 hexane:EtOAc to afford tert-butyl N-[(1R)-1-[[2,4-dichloro-6-[2-(1H-indol-3-yl)ethoxy]pyrimidin-5-yl]oxymethyl]-2-methoxy-ethyl]carbamate (350 mg,0.684 mmol, 44.85% yield) as a white solid. UPLC-MS analysis (4 min, basic): rt = 2.28 min, no ionisation, 98% purity.

1H NMR (400 MHz, DMSO-D6) δ 10.89 (s, 1H), 7.63 (d, J = 7.9 Hz, 1H), 7.35 (dq, J = 8.1, 0.9 Hz, 1H), 7.25 (d, J = 2.4 Hz, 1H), 7.08 (ddd, J = 8.2, 6.6, 1.2 Hz, 1H), 7.00 (ddt, J = 7.8, 6.9, 0.8 Hz, 1H), 6.79 (d, J = 8.2 Hz, 1H), 4.62 (t, J = 7.1 Hz, 2H), 4.08 (dd, J = 10.2, 5.2 Hz, 1H), 4.03 - 3.91 (m, 1H), 3.90 - 3.81 (m, 1H), 3.33 (d, J = 6.1 Hz, 2H), 3.22 (d, J = 7.4 Hz, SH), 1.37 (s, 9H).

### 48.2 (2R)-1-[2,4-dichloro-6-[2-(1H-indol-3-yl)ethoxy]pyrimidin-5-yl]oxy-3-methoxy-propan-2-amine;hydrochloride

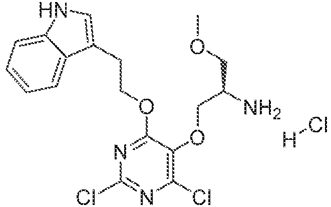

Prepared according to general method 3, using tert-butyl N-[(1R)-1-[[2,4-dichloro-6-[2-(1H-indol-3-yl)ethoxy] pyrimidin-5-yl]oxymethyl]-2-methoxy-ethyl]carbamate (1.00 eq, 350 mg, 0.657 mmol) to afford (2R)-1-[2,4-dichloro-6-[2-(1H-indol-3-yl)ethoxy]pyrimidin-5-yl]oxy-3-methoxy-propan-2-amine;hydrochloride (210 mg,0.422 mmol, 64.25% yield) as a purple solid. UPLC-MS analysis (2 min, basic): rt = 1.13 min, m/z = 411.2/413.1/415.2 [M+H]+, 90% purity. 1H NMR (400 MHz, DMSO-D6) δ 10.95 (s, 1H), 8.39 (s, 3H), 7.63 (d, J = 7.8 Hz, 1H), 7.36 (d, J = 8.1 Hz, 1H), 7.28 (d, J = 2.4 Hz, 1H), 7.09 (t, J = 7.5 Hz, 1H), 7.00 (t, J = 7.4 Hz, 1H), 4.66 (t, J = 7.1 Hz, 2H), 4.16 (q, J = 2.4 Hz, 2H), 3.57 (s, 2H), 3.29 (s, 3H), 3.23 (t, J = 7.1 Hz, 2H). 1H not observed, likely overlapped with water peak.

### 48.3 (7R)-2-chloro-4-[2-(1H-indol-3-yl)ethoxy]-7-(methoxymethyl)-7,8-dihydro-6H-pyrimido([5,4-b][1,4]oxazine

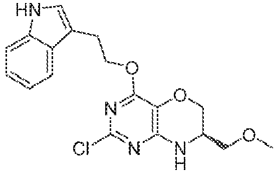

Prepared according to general method 4, using (2R)-1-[2,4-dichloro-6-[2-(1H-indol-3-yl)ethoxy]pyrimidin-5-yl]oxy-3-methoxy-propan-2-amine;hydrochloride (180 mg, 0.442 mmol) to afford (7R)-2-chloro-4-[2-(1H-indol-3-yl)ethoxy]-7-(methoxy methyl)- 7,8-dihydro-6H-pyrimido[5,4-b][1,4]oxazine (150 mg,0.344 mmol, 85.61 % yield) as an off-white solid.

UPLC-MS analysis (2 min, basic): rt = 1.09 min, m/z = 375.2/377.1 [M+H]+, 86% purity.

1H NMR (400 MHz, DMSO-D6) δ 10.83 (s, 1H), 7.97 (d, J = 3.2 Hz, 1H), 7.59 (d, J = 7.9 Hz, 1H), 7.30 (d, J = 8.1 Hz, 1H), 7.17 (d, J = 2.3 Hz, 1H), 7.03 (t, J = 7.5 Hz, 1H), 6.94 (t, J = 7.4 Hz, 1H), 4.40 (t, J = 7.3 Hz, 2H), 4.06 - 3.86 (m, 2H), 3.65 (s, 1H), 3.25 (s, 3H), 3.07 (t, J = 7.3 Hz, 2H). 2Hs not observed; underneath water signal.

### Example 48 3-[(7R)-4-[2-(1H-indol-3-yl)ethoxy]-7-(methoxymethyl)-7,8-dihydro-6H-pyrimido[5,4-b][1,4]oxazin-2-yl]-1H-pyridin-2-one

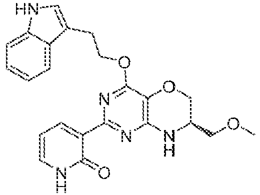

Prepared according to general method 5b, using (7R)-2-chloro-4-[2-(1H-indol-3-yl)ethoxy]-7-(methoxymethyl)-7,8-dihydro-6H-pyrimido[5,4-b][3,4]oxazine (100 mg, 0.229 mmol) and (2-Oxo-1,2-dihydropyridin-3-yl)boronic acid (48 mg, 0.344 mmol). The crude material was purified by column chromatography (20 g cartridge) eluting with a gradient of EtOAc (0-100%; v/v) in iso-Hex then MeOH (0-10%; v/v) in EtOAc followed by Reversed Phase column chromatography (12 g cartridge) eluting with a gradient of MeCN (0.19% NH3) (0% to 95%; v/v) in water (0.1% NH3) to afford 3-[(7R)-4-[2-(1H-indol-3-yl)ethoxy]-7-(methoxymethyl)-7,8-dihydro-6H-pyrimido[5,4-b][1,4]oxazin-2-yl]-1H-pyridin- 2-one (47 mg,0.105 mmol, 45.84% yield) as an off-white solid Example 48 is subject to keto-enol tautomerism. It may also exist in the following form: (R)-3-(4-(2-(1H-indol-3-yl)ethoxy)-7-(methoxymethyl)-7,8-dihydro-6H-pyrimido[5,4-b][1,4]oxazin-2-yl)pyridin-2-ol 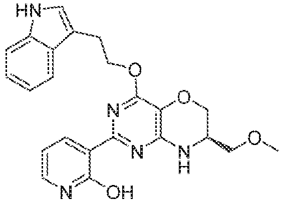 UPLC-MS analysis (4 min, basic): rt = 1.64 min, m/z = 343.3 [M+H]+, 97% purity. 1H NMR (400 MHz, DMSO-D6) δ 13.86 (s, 1H), 10.89 (s, 1H), 8.51 (s, 1H), 8.28 (s, 1H), 8.17 (s, 1H), 7.61 (d, J = 7.9 Hz, 1H), 7.35 (d, J = 8.1 Hz, 1H), 7.25 (s, 1H), 7.07 (t, J = 7.5 Hz, 1H), 6.98 (s, 2H), 4.63 (s, 2H), 4.08 (s, 2H), 3.79 (s, 1H), 3.42 (d, J = 6.3 Hz, 2H), 3.18 (s, 2H), 2.54 (s, 3H).

### Example 49 (7R)-2-(3,5-difluorophenyl)-N-[2-(1H-indol-3-yl)ethyl]-7-(methoxymethyl)-7,8-dihydro-6H-pyrimido|5,4-b][1.4]oxazin-4-amine

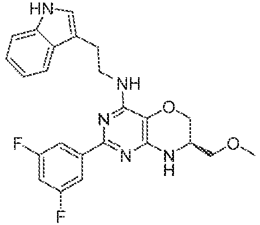

Prepared according to general method 5b, using (7R)-2-chloro-N-[2-(1H-indol-3-yl)ethyl])-7-(methoxymethyl)-7,8-dihydro-6H-pyrimido[5,4-b][3,4]oxazin-4-amine (1.00 eq, 150 mg, 0.373 mmol) and 3,5-Difluorobenzeneboronic acid (1.00 eq, 59 mg, 0.373 mmol). The crude was purified by column chromatography (40 g column) eluting with a gradient of 0 to 100% (v/v) EtOAc in iso- Hex to afford (7R)-2-(3,5-difluorophenyl)-N-[2-(1H-indol-3-yl)ethyl]-7-(methoxymethyl)-7,8-dihydro-6H-pyrimido[5,4-b][1,4]oxazin-4-amine (58 mg,0.128 mmol, 34.43% yield) as a yellow solid.

UPLC-MS analysis (4 min, basic): rt = 2.18 min, m/z = 452.4 [M+H]+, 100% purity.

1H NMR (400 MHz, DMSO-D6) **δ** 10.82 (s, 1H), 7.90 - 7.77 (m, 2H), 7.72 - 7.58 (m, 1H), 7.35 (dt, J = 8.1, 1.0 Hz, 1H), 7.26 (tt, J = 9.0, 2.5 Hz, 1H), 7.20 (d, J = 2.3 Hz, 1H), 7.14 - 7.03 (m, 1H), 7.03 - 6.91 (m, 2H), 6.53 (t, J = 5.9 Hz, 1H), 4.18 - 3.99 (m, 2H), 3.82 - 3.59 (m, 3H), 3.47 - 3.35 (m, 2H), 3.31 (s, 3H), 3.04 - 2.94 (m, 2H). 19F NMR (376 MHz, DMSO-D6) δ -110.24.

### Example 50 2-(3-fluorophenyl)-N-[2-(1H-indol-3-yl)ethyl]-7,8-dihydro-6H-pyrimido[5,4-b][1,4]oxazin-4-amine

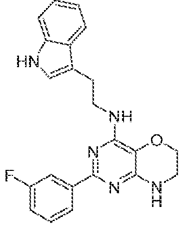

Prepared according to general method 5a, using 2-chloro-N-[2-(1H-indol-3-yl)ethyl]-7,8-dihydro-6H-pyrimido[5,4-b)[1,4]oxazin-4-amine (1.00 eq, 75 mg, 0.227 mmol) and 3-Fluorobenzenelboronic acid (1.50 eq, 48 mg, 0.341 mmol). The reaction mixture volume was reduced to dryness under vacuum and the crude was purified by Reversed Phase column chromatography eluting with MeCN +0.1%NH3 / water +0.1% NH3 to afford 2-(3-fluorophenyl)-N-[2-(1H-indol-3-yl)ethyl]-7.8-dihydro-6H-pyrimido[5,4-b][1,4]oxazin-4-amine (17 mg,0.0423 mmol, 18.62% yield) was obtained a beige solid.

UPLC-MS analysis (4 min, basic): rt = 2.01 min, m/z = 390.3 [M+H]+, 97% purity.

1H NMR (400 MHz, DMSO-D6) δ 10.81 (s, 1H), 8.07 (dt, J = 7.9, 1.3 Hz, 1H), 8.02 - 7.88 (m, 1H), 7.65 (d, J = 7.8 Hz, 1H), 7.46 (td, J = 8.1, 6.1 Hz, 1H), 7.39 - 7.31 (m, 1H), 7.28 - 7.15 (m, 2H), 7.13 - 7.03 (m, 1H), 7.03 - 6.94 (m, 1H), 6.82 (d, J = 2.8 Hz, 1H), 6.34 (t, J = 6.0 Hz, 1H), 4.14 (t, J = 4.3 Hz, 2H), 3.71 (q, J = 7.0 Hz, 2H), 3.52 - 3.38 (m, 2H), 3.06 - 2.92 (m, 2H).

19F NMR (376 MHz, DMSO-D6) δ -113.94.

### Example 51 2-(2-methylthiazol-5-y)-N-[(3R)-2,3,4,9-tetrahydro-1H-carbazol-3-yl]-7,8-dihydro-6H-pyrimido[5,4-b][1.4]oxazin-4-amine

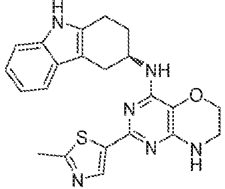

Prepared according to general method 5b, using 2-chloro-N-[(3R)-2,3,4,9-tetrahydro-1 H-carbazol-3-yl]-7,8-dihydro-6H-pyrimido[5,4-b][1,4]oxazin-4-amine (1.00 eq. 70 mg, 0.197 mmol) and 2-methyl-5-(tetramethyl-1,3,2-dioxaborolan-2-yl)-1,3-thiazole (1.50 eq, 66 mg, 0.295 mmol). The crude was purified by column chromatography (20 g column) eluting with a gradient of 0 to 100% (v/v) EtOAc in iso-Hex to afford 2-(2-methylthiazol-5-yl)-N-[(3R)-2,3,4,9-tetrahydro- 1H-carbazol-3-yl]-7,8-dihydro-6H-pyrimido[5,4-b] [1,4]oxazin-4-amine (60 mg,0.143 mmol, 72.87% yield) as an off-white solid.

UPLC-MS analysis (4 min, basic): rt = 2.23 min, m/z = 419.2 [M+H]+, 100% purity.

1H NMR (400 MHz, DMSO-D6) δ 10.72 (s, 1H), 7.96 (s, 1H), 7.33 (d, J = 7.7 Hz, 1H), 7.26 (dt, J = 8.0, 1.0 Hz, 1H), 7.03 - 6.95 (m, 1 H), 6.95 - 6.85 (m, 2H), 6.10 (d, J = 8.2 Hz, 1H), 4.49 - 4.33 (m, 1 H), 4.20 - 4.05 (m, 2H), 3.51 - 3.35 (m, 2H), 3.02 (dd, J = 14.9, 5.3 Hz, 1H), 2.95 - 2.76 (m, 2H), 2.72 - 2.62 (m, 1H), 2.60 (s, 3H), 2.17 - 2.06 (m, 1 H), 2.04 - 1.92 (m, 1H).

### Example 52 2-(2,4-dimethylthiazol-5-yl)-N-[2-(1H-indol-3-yl)ethyl}-7,8-dihydro-6H-pyrimido[5,4-b][1,4]oxazin-4-amine

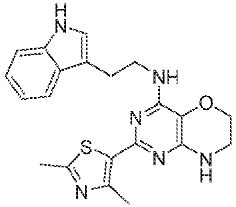

Prepared according to general method 5b, using 2-chloro-N-[2-(1H-indol-3-yl)ethyl]-7,8-dihydro-δH-pyrimido[5,4-b] [1,4]oxazin-4-amine (1.00 eq, 100 mg, 0.303 mmol) and 2,4-dimethyl-5-(4,4,5,5-tetramethyl-1,3,2-dioxaborolan-2-yl)-1,3-thiazole (1.50 eq, 109 mg, 0.455 mmol). The crude was purified by column chromatography (24 g column), eluting with a gradient of 0 to 100% (v/v) EtOAc in iso- Hex (product eluted with 95-100% EtOAc) to afford 2-(2,4-dimethylthiazol-5-yl)-N-[2-(1H-indol-3-yl)ethyl]-7,8-dihydro-6H-pyrimido[5,4-b][1,4]oxazin-4-amine (87 mg,0.214 mmol, 70.58% yield) as an off-white solid. UPLC-MS analysis (4 min, basic): rt = 1.77 min, m/z = 407.3 [M+H]+, 100% purity. 1H NMR (400 MHz, DMSO-D6) δ 10.81 (s, 1H), 7.60 - 7.55 (m, 1H), 7.35 - 7.31 (m, 1H), 7.15 (d, J = 2.3 Hz, 1H), 7.09 - 7.03 (m, 1H), 7.00 - 6.93 (m, 1H), 6.79 - 6.75 (m, 1H), 6.32 (t, J = 5.9 Hz, 1H), 4.13 - 4.06 (m, 2H), 3.62 (q, J = 6.8 Hz, 2H), 3.44 - 3.35 (m, 2H), 3.00 - 2.90 (m, 2H), 2.70 (s, 3H), 2.55 (s, 3H).

### Intermediates for Example 53

### 53.1 tert-butyl N-[(1R)-1-[[2,4-dlcbloro-6-[[(3R)-2,3,4,9-tetrabydro-1H-carbazol-3-yl]amino]pyrimidin-5-yl]axymethyl]-2-methoxy-ethyl]carbamate

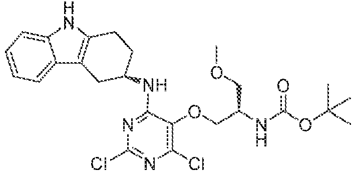

Prepared according to general method 2a, using tert-butyl N-[(1R)-1-(methoxymethyl)-2-(2,4,6-trichloro pyrimidin-5-yl)oxy-ethyl]carbamate (1.00 eq, 0.20 g, 0.476 mmol) and (R)-2,3,4,9-Tetrahydro-1H-carbazol-3-amine (1.10 eq, 0.097 g, 0.523 mmol) in Ethanol (3.4483 mL) at 50 °C. The reaction was concentrated to dryness to give an oily residue, which was dispersed in water (20 mL). The resultant suspension was filtered to afford tert-butyl N-[(1R)-1-[[2,4-dichloro-6-[[(3R)-2,3,4,9-tetrahydro-1H-carbazol-3-yl]amino]pyrimidin-5-yl]oxymethyl]-2-methoxy-ethyl]carbamate (0.26 g,0.461 mmol, 96.89% yield) as an off-white solid.

UPLC-MS analysis (2 min, basic): rt = 1.35 min, m/z = 536.1 /538.1/540.1 [M+H]+, 97% purity.

1H NMR (400 MHz, DMSO-D6) δ 10.74 (s, 1H), 7.70 (d, J = 8.0 Hz, 1H), 7.33 (d, J = 7.8 Hz, 1H), 7.25 (dt, J = 8.0, 0.9 Hz, 1H), 7.10 (d, J = 8.0 Hz, 1H), 7.05 - 6.96 (m, 1H), 6.96 - 6.87 (m, 1H), 4.40 - 4.27 (m, 1H), 4.11 - 4.01 (m, 1H), 3.98 - 3.90 (m, 1 H), 3.40 (d, J = 5.7 Hz, 2H), 3.26 (s, 3H), 3.06 (dd, J = 14.9, 5.3 Hz, 1H), 2.94 - 2.79 (m, 2H), 2.76 - 2.66 (m, 1H), 2.21 - 2.10 (m, 1H), 2.09 - 1.93 (m, 1H), 1.39 (s, 9H), 1.17 - 1.02 (m, 1H).

### 53.2 (3R)-N-[5-[(2R)-2-amino-3-methoxy-propoxy]-2,6-dichloro-pyrimidin-4-yl]-2,3 4,9-tetrahydro-1H-carbazol-3-amine;hydrochloride

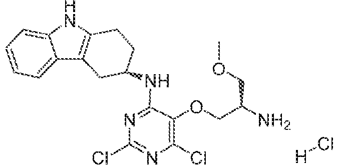

Prepared according to general method 3, using tert-butyl N-[(1R)-1-[[2,4-dichloro-6-[[(3R)-2,3,4,9-tetrahydro-1H-carbazol-3-yl]amino]pyrimidin-5-yl]oxymethyl]-2-methoxy-ethyl]carbamate(1.00 eq, 0.25 g, 0.452 mmol) to afford (3R)-N-[5-[(2R)-2-amino-3-methoxy-propoxy]-2,6-dichloro-pyrimidin-4-yl]-2,3,4,9-tetrahydro-1H-carbazol-3-amine;hydrochloride (0.23 g,0.452 mmol, 100.08% yield) as a beige solid.

UPLC-MS analysis (2 min, basic): rt = 1.21 min, m/z = 436.3.0/438.2/440.3 [M+H]+, 93% purity.

1H NMR (400 MHz, DMSO-D6) δ 10.77 (s, 1H), 8.57 (s, 3H), 8.12 (d, J = 8.4 Hz, 1H), 7.33 (d, J = 7.8 Hz, 1H), 7.29 - 7.21 (m, 1H), 7.05 - 6.96 (m, 1H), 6.93 (td, J = 1.4, 1.1 Hz, 1H), 4.45 - 4.33 (m, 1H), 4.29 (dd. J = 10.2, 4.0 Hz, 1H), 4.14 - 4.06 (m, 1H), 3.80 - 3.71 (m, 1H), 3.70 - 3.65 (m, 2H), 3.34 (s, 3H), 3.11 - 2.95 (m, 2H), 2.95 - 2.76 (m, 3H), 2.12 - 2.04 (m, 1H).

### 53.4 (7R)-2-chloro-7-(methoxymethyl)-N-[(3R)-2,3,4,9-tetrahydro-1H-carbazol-3-yl]-7,8-dihydro-6H-pyrimido[5,4-b][1.4]oxazin-4-amine

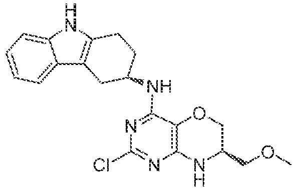

Prepared according to general method 4, using (3R)-N-[5-[(2R)-2-amino-3-methoxy-propoxy]-2,6-dichloro-pyrimidin-4-yl]-2,3,4,9-tetrahydro-1H-carbazol-3-amine;HCl (1.00 eq, 200 mg, 0.393 mmol) to afford(7R)-2-chloro-7-(methoxy methyl). N-[(3R)-2,3,4,9-tetrahydro-1H-carbazol-3-yl]-7,8-dihydro-6H-pyrimido[5,4-b][1,4]oxazin-4-amine (0.16 g.0.384 mmol, 97.64% yield) as a beige solid.

UPLC-MS analysis (2 min, basic): rt = 1.12 min, m/z = 400.3/402.3 [M+H]+, 96% purity.

1H NMR (400 MHz, DMSO-D6) δ 10.70 (s, 1H), 7.41 - 7.27 (m, 2H), 7.24 (d, J = 7.9 Hz, 1H), 6.99 (t, J = 7.4 Hz, 1H), 6.91 (t, J = 7.4 Hz, 1H), 6.58 (d, J = 8.5 Hz, 1H), 4.30 - 4.17 (m, 1H), 4.12 - 3.93 (m, 2H), 3.65 (s, 1H), 3.38 - 3.34 (m, 1H), 3.29 (s, 3H), 2.94 (dd, J = 14.9, 5.3 Hz, 1H), 2.88 - 2.76 (m, 2H), 2.69 - 2.56 (m, 1H), 2.07 - 1.90 (m, 2H), 1.11 - 1.00 (m, 1H).

### Example 53 (7R)-7-(methexymethyl)-2-(2-methylthlazol-5-yl)-N-[(3R)-2,3,4,9-tetrahydro-1H-carbazol-3-yl]-7,8-dihydro-6H-pyrimido[5,4-b][1,4]oxazin-4-amine

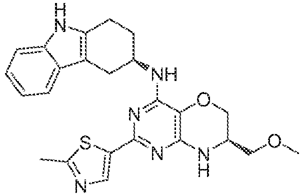

Prepared according to general method 5b, using (7R)-2-chloro-7-(methoxymethyl)-N-[(3R)-2,3,4,9-tetrahydro-2 H-carbazol-3-yl]-7,8-dihydro-6H-pyrimido[5,4-b][1,4]ox-azin-4-amine (1.00 eq, 90 mg, 0.216 mmol) and 2-methyl-5-(tetramethyl-1,3,2-dioxaborolan-2-yl)-1,3-thiazole (1.50 eq, 73 mg, 0.324 mmol).

The crude was purified by flash column chromatography (20 g column), eluting with a gradient of 0 to 100% (v/v) EtOAc in iso-Hex and then EtOAc/MeOH (product eluted with 5-7% MeOH) to afford (7R)-7-(methoxymethyl)-2-(2-methylthiazol-5-yl)-N-[(3R)-2,3,4,9-tetrahydro-1H-carbazol-3-yl]-7,8-dihydro-6H-pyrimido[5,4-b][1.4]oxazin-4-amine (48 mg,0.104 mmol, 48.03% yield) as an off-white solid.

UPLC-MS analysis (4 min, basic): rt = 1.93 min, m/z = 463.4 [M+H]+, 98% purity.

1H NMR (400 MHz, DMSO-D6) δ 10.74 (s, 1H), 7.96 (s, 1H), 7.33 (d, J = 7.6 Hz, 1H), 7.30 - 7.20 (m, 1H), 7.07 (d. J = 3.1 Hz, 1H), 7.03 - 6.96 (m, 1H), 6.92 (td, J = 7.5, 1.1 Hz. 1 H), 6.30 (d, J = 8.2 Hz, 1H), 4.40 (s, 1H), 4.12 - 3.98 (m, 2H), 3.73 - 3.62 (m, 1H), 3.44 - 3.36 (m, 2H), 3.30 (s, 3H), 3.01 (dd, J = 15.0, 5.3 Hz, 1H), 2.87 (d, J = 21.8 Hz, 2H), 2.66 (s, 1H), 2.60 (s, 3H), 2.10 (d, J = 12.4 Hz, 1H), 2.02 - 1.89 (m, 1H).

### Example 54 [(7R)-2-(2-methylthlazol-5-yl)-4-[[(3R)-2,3,4,9-tetrahydro-1H-carbazol-3-yl]amino]-7,8-dydro-6H-pyrimido[5,4-b][1,4]oxazin-7-yl]methanol

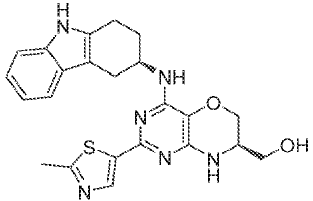

(7R)-7-(methoxymethyl)-2-(2-methylthiazol-5-yl)-N-[(3R)-2,3,4,9-tetrahydro-1H-carbazol-3-yl]-7,8-dihydro-6H-pyrimido[5,4-b]|1,4]oxazin-4-amine (1.00 eq, 23 mg, 0.0497 mmol) in DCM (2 mL) was added boron tribromide solution in DCM 1M (1.00 eq, 0.050 mL, 0.0497 mmol) and the mixture was stirred at ambient temperature for 24 h. The reaction mixture was carefully quenched with MeOH (0.5 mL) and evaporated. The crude was purified by column chromatography over silica eluting with a gradient of MeOH (0% to 5%; v/v) in DCM to afford [(7R)-2-(2-methylthiazol-5-yl)-4-[[(3R)-2.3,4,9-tetrahydro-1H-carbazol-3-yl]amino]-7,8-dihydro-6H-pyrimido[5,4-b][1,4]oxazin-7-yl]methanol (9.0 mg,0.0187 mmol, 37.53% yield) as a white solid UPLC-MS analysis (4 min, basic): rt = 1.67 min, m/z = 449.4 [M+H]+, 93% purity.

1H NMR (400 MHz, DMSO-D6) δ 10.70 (s, 1H), 7.95 (s, 1H), 7.32 (d, J = 7.7 Hz, 1H), 7.25 (dt, J = 8.0, 0.9 Hz, 1H), 6.99 (ddd, J = 8.1, 7.0, 1.3 Hz, 1H), 6.95 - 6.88 (m, 2H), 6.17 (d, J = 8.2 Hz, 1H), 4.93 (t, I = 5.3 Hz, 1H), 4.40 (s, 1H), 4.07 (d, J = 3.3 Hz, 2H), 3.56 - 3.45 (m, 2H), 3.42 - 3.33 (m, 1H), 3.01 (dd, J = 14.9.5.3 Hz, 1H), 2.84 (td, J = 16.2, 10.0 Hz, 2H), 2.71 - 2.61 (m, 1H), 2.59 (s, 3H), 2.14 - 2.07 (m, 1H), 1.96 (qd, J = 10.8, 5.9 Hz, 1H).

### Example 55 N-[2-(1H-indol-3-yl)ethyl)-2-(1-methyltriazol-4-yl)-7,8-dihydro-6H-pyrimido(5,4-b][1,4]oxazin-4-amine

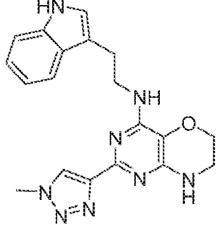

Prepared according to general method 5b, using 2-chloro-N-[2-(1H-indol-3-yl)ethyl]-7,8-dihydro-6H-pyrimido[5,4-b][1,4]oxazin-4-amine (1.00 eq, 200 mg, 0.606 mmol) and 1-methyl-4-(4,4,5,5-tetramethyl-1,3,2-dioxaborolan-2-yl)-1H-1,2,3-triazole (1.00 eq, 126 mg, 0.606 mmol). The reaction mixture was concentrated, and the product was precipitated in a mixture DCM/EtOAc. The crude was purified by Reversed Phase chromatography (16g cartridge) eluting with MeCN/water 0%-95% gradient followed by prep HPLC (basic method) to afford N-[2-(1H-indol-3-yl)ethyl]-2-(1-methyltriazol-4-yl)-7,8-dihydro-6H-pyrimido[5,4-b][1,4]oxazin-4-amine (2.3 mg, 0.00599 mmol, 0.98% yield) as a white solid. UPLC-MS analysis (6 min, basic): rt = 2.41 min, m/z = 377.1 [M+H]+, 98% purity.

1H NMR (400 MHz, MeOD) δ 7.98 (s, 1H), 7.61 (d. J = 7.9 Hz, 1H), 7.30 (d, J = 8.1 Hz, 1 H), 7.07 (d, J = 7.3 Hz, 2H), 7.01 (t, J = 7.5 Hz, 1H), 4.15 (t, J = 4.5 Hz, 2H), 4.11 (s, 3H), 3.81 (t, J = 7.1 Hz, 2H), 3.50 (t, J = 4.3 Hz, 2H), 3.03 (t, J = 7.1 Hz, 2H).

### Example 56 (7R)-2-(4-fluorophenyl)-N-[2-(1H-indol-3-yl)ethyl]-7-(methoxymethyl)-7,8-dibydro-6H-pyrimido[5,4-b][1,4]oxazin-4-amine

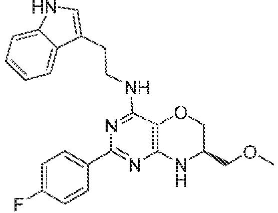

Prepared according to general method 5b, using (7R)-2-chloro-N-[2-(1H-indol-3-yl)ethyl]-7-(methoxymethyl)-7,8-dihydro-6H-pyrimido[5,4-b][1,4]oxazin-4-amine (55 mg, 0.137 mmol) and 4-Fluorobenzenelboronic acid (38 mg, 0.274 mmol). The reaction mixture volume was reduced to dryness under vacuum. The crude was purified by column chromatography using 4:1 and 2:1 Hexane:EtOAc to afford (7R)-2-(4-fluorophenyl)-N-[2-(1H-indol-3-yl)ethyl]-7-(methoxymethyl)-7,8-dihydro-6H-pyrimido[5,4-b][1,4]oxazin-4-amine (22 mg,0.0508 mmol, 37.09% yield) as a white solid. UPLC-MS analysis (4 min, basic): rt = 2.07 min, m/z =434.4 [M+H]+, 100% purity.

1H NMR (400 MHz, DMSO-D6) δ 10.80 (s, 1H), 8.29 - 8.19 (m, 2H), 7.61 (d, J = 7.8 Hz, 1H), 7.34 (dt,

J = 8.1, 1.0 Hz, 1H), 7.27 - 7.16 (m, 3H), 7.07 (ddd, ) = 8.1, 7.0,1.2 Hz, 1H), 6.98 (ddd, J = 7.9, 7.0, 1.1 Hz, 1H), 6.87 (d, J = 3.1 Hz, 1H), 6.37 (t, J = 5.9 Hz, 1H), 4.05 (qd, J = 10.6, 3.5 Hz, 2H), 3.69 (dt, J = 8.9, 6.5 Hz, 3H), 3.45 - 3.33 (m, 2H), 3.31 (s, 3H), 3.03 - 2.94 (m, 2H),

19F NMR (376 MHz, DMSO-D6) δ -113.49 - -113.60 (m)

### Example 57 (7R)-2-(3-fluorophenyl)-N-[2-(1H-indol-3-yl)ethyl]-7-(methoxymethyl)-7,8-dihydro-6H-pyrimido[5,4-b][1,4]oxazin-4-amine

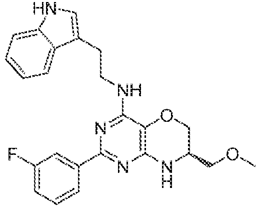

Prepared according to general method 5b, using (7R)-2-chloro-N-[2-(1H-indol-3-yl)ethyl]-7-(methoxymethyl)-7,8-dihydro-6H-pyrimido[5,4-b][1,4]oxazin-4-amine (50 mg, 0.0976 mmol) and 3-Fluorobenzenelboronic acid (27 mg, 0.195 mmol). The reaction mixture volume was reduced to dryness under vacuum. The crude was purified by column chromatography using 4:1 and 2:1 Hexane:EtOAc to afford (7R)-2-(3-fluorophenyl)-N-[2-(1H-indol-3-yl)ethyl]-7-(methoxymethyl)-7,8-dihydro-6H-pyrimido [5,4-b][1,4]oxazin-4-amine (16 mg,0.0365 mmol, 37.43% yield) as an off-white solid.

UPLC-MS analysis (4 min, basic): rt = 2.09 min, m/z = 434.4 [M+H]+, 99% purity.

1H NMR (400 MHz, DMSO-D6) δ 10.81 (s, 1H), 8.06 (dt, J = 7.8, 1.2 Hz, 1H), 7.94 (ddd, J = 11.0, 2.8, 1.4 Hz, 1H), 7.64 (d, J = 7.8 Hz, 1H), 7.45 (td, J = 8.0, 6.1 Hz, 1H), 7.34 (dt, J = 8.1, 0.9 Hz, 1H), 7.28 - 7.16 (m, 2H), 7.07 (ddd, J = 8.1, 6.9, 1.2 Hz, 1H), 6.97 (ddd, J = 8.0, 7.0, 1.1 Hz, 1H), 6.92 (d, J = 3.0 Hz, 1H), 6.44 (t, J = 5.9 Hz, 1H), 4.06 (qd, J = 10.6, 3.5 Hz, 2H), 3.74 - 3.64 (m, 3H), 3.46 - 3.32 (m, 2H), 3.31 (s, 3H), 2.99 (dd, J = 8.9, 6.4 Hz, 2H).

19F NMR (376 MHz, DMSO-D6) δ -113.90 (ddd, J = 10.9, 8.6, 6.1 Hz).

### Example 58 5-[4-[2-(1H-indol-3-yl)ethylamino]-7,8-dihydro-6H-pyrimido[5.4-b][1,4]oxaxin-2-yl]-1-methyl-pyridin-2-one

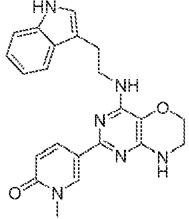

Prepared according to general method 5b, using 2-chloro-N-[2-(1H-indol-3-yl)ethyl)-7,8-dihydro-6H-pyrimido[5,4-b][1,4)oxazin-4-amine (150 mg, 0.455 mmol) and 1-Methyl-5-(4,4,5,5-tetramethyl-1,3,2-dioxaborolan-2-yl)pyridin-2(1H)-one (160 mg, 0.682 mmol). The crude was purified by column chromatography (40 g cartridge) eluting with a gradient of EtOAc (0% to 100%; v/v) in iso-Hex then MeOH (0% to 15%; v/v) in EtOAc to afford 5-[4-[2-(1H-indol-3-yl)ethylamino]-7,8-dihydro-6H-pyrimido[5,4-b][1,4]oxazin-2-yl]-1-methyl-pyridin-2-one (110 mg,0.273 mmol, 60.09% yield) as a beige solid.

UPLC-MS analysis (4 min, basic): rt = 1.54 min, m/z = 403.1 [M+H]+, 100% purity.

1H NMR (400 MHz, DMSO-D6) δ 10.80 (s, 1H), 8.35 (d, J = 2.5 Hz, 1H), 8.16 (dd, J = 9.5, 2.4 Hz, 1H), 7.60 (d, J = 7.8 Hz, 1H), 7.39 - 7.30 (m, 1H), 7.19 (d, J = 2.3 Hz, 1H), 7.07 (dd, J = 8.1, 6.9 Hz, 1H), 7.03 - 6.91 (m, 1H), 6.66 (t, J = 2.5 Hz, 1H), 6.41 (d, J = 9.5 Hz, 1H), 6.21 (t, J = 6.0 Hz, 1H), 4.09 (t, J = 4.3 Hz, 2H), 3.67 (q, J = 7.0 Hz, 2H), 3.49 (s, 3H), 3.41 (q, J = 3.0 Hz, 2H), 2.96 (t, J = 7.6 Hz, 2H).

### Example 59 (7R)-N-[2-(1H-indol-3-yl)ethyl]-7-(methoxymethyl)-2-(2-methylthiazol-5-yl)-7,8-dihydro-6H-pyrimido[5,4-b][1,4]oxazin-4-amine

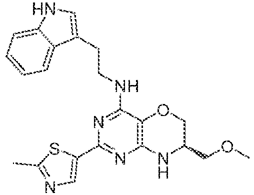

Prepared according to general method 5b, using (7R)-2-chloro-N-[2-(1H-indol-3-yl)ethyl]-7-(methoxymethyl)-7,8-dihydro-6H-pyrimido[5,4-b][1,4]oxazin-4-amine (300 mg, 0.746 mmol) and 2-methyl-5-(tetramethyl-1,3,2-dioxaborolan-2-yl)-1,3-thiazole (218 mg, 0.970 mmol). The reaction mixture was concentrated to dryness under vacuum. The crude was purified column chromatography eluting with 3:1 and 1:1 hexane:EtOAc to afford (7R)-N-[2-(1H-indol-3-yl)ethyl]-7-(methoxy methyl)-2-(2-methylthiazol-5-yl)-7,8-dihydro-6H-pyrimido[5,4-b][1,4]oxazin-4-amine (157 mg,0.360 mmol, 48.19% yield) as a white solid.

UPLC-MS analysis (2 min, basic): rt = 1.09 min, m/z = 437.1 [M+H]+, 100% purity.

1H NMR (400 MHz, DMSO-D6) δ 10.80 (s, 1H), 8.02 (s, 1H), 7.65 (ddt, J = 7.8, 1.3, 0.7 Hz, 1.11). 7.34 (dt, J = 8.1, 0.9 Hz, 1H), 7.19 (d, J = 2.3 Hz, 1H), 7.07 (ddd, J = 8.2, 7.0, 1.2 Hz, 1H), 7.03 - 6.95 (m, 2H), 6.45 (t, J = 5.9 Hz, 1H), 4.03 (qd, J = 10.6, 3.5 Hz, 2H), 3.70 - 3.57 (m, 3H), 3.38 (d, J = 7.7 Hz, 2H), 3.29 (s, 3H), 2.95 (dd, J = 9.0. 6.4 Hz, 2H), 2.64 (s, 3H).

### Example 60 [(7R)-4-[2-(1H-indol-3-yl)ethylamino]-2-(2-methylthiazol-5-yl)-7,8-dihydro-6H-pyrimido[5,4-b][1,4]oxazin-7-yl]methanol

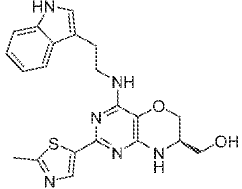

To (7R)-N-[2-(1H-indol-3-yl)ethyl]-7-(methoxymethyl)-2-(2-methylthiazol-5-yl)-7,8-dihydro-6H-pyrimido[5,4-b][1,4]oxazin-4-amine (140 mg, 0.321 mmol) in DCM (4 mL) was added boron tribromide solution in DCM 1M (0.38 mL, 0.385 mmol) and the mixture was stirred at ambient temperature for 3 h. The reaction mixture was carefully quenched with MeOH(1 mL) and water (4 mL) then extracted with DCM twice and the organics were dried over Na2SO4, filtered and concentrated to dryness. The crude was purified by Reversed Phase column chromatography (4 g column) eluting with MeCN in water gradient (5% to 95% basic buffer) to afford [(7R)-4-[2-(1H-indol-3-yl)ethylamino]-2-(2-methylthiazol-5-yl)-7,8-dihydro-6H-pyrimido[5,4-b][1,4]oxazin-7-yl]methanol (22 mg,0.0521 mmol, 16.24% yield) as an off white solid.

UPLC-MS analysis (4 min, basic): rt = 1.56 min, m/z = 423.1 [M+H]+, 100% purity.

1H NMR (400 MHz, DMSO-D6) δ 10.80 (d, J = 2.4 Hz, 1H), 8.03 (s, 1H), 7.65 (ddt, J = 7.8, 1.3, 0.7 Hz, 1H), 7.34 (dt, J = 8.1, 0.9 Hz, 1H), 7.18 (d, J = 2.3 Hz, 1H), 7.07 (ddd, J = 8.2, 7.0,1.3 Hz, 1H), 6.99 (ddd, J = 7.9. 7.0, 1.1 Hz, 1H), 6.91 (s, 1H), 6.47 (s, 1H), 4.11 - 3.99 (m, 2H), 3.62 (s, 2H), 3.54 - 3.42 (m, 2H), 3.40 - 3.31 (m, 1H), 2.95 (dd, J = 9.0, 6.4 Hz, 2H), 2.64 (s, 3H). 1H not observed

### Example 61 2-(2-methylthiazol-5-yl)-N-[(4R)-1,3,4,5-tetrahydrobenzo[cd]indol-4-yl]-7,8-dihydro-6H-pyrimido[5,4-b][1,4]oxazin-4-amine

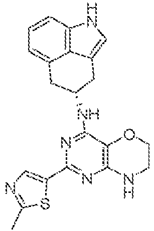

arbitrary assignment has been made: 2-(2-methylthiazol-5-yl)-N-[(4R)-1,3,4,5-tetrahydrobenzo[cd]indol-4-yl]-7,8-dihydro-6H-pyrimido[5,4-b][1,4]oxazin-4-amine (29 mg, 0.0717 mmol, 7.49% yield). Chemical purity: 99.51%, m/z = 405.21
Enantiomeric excess: 100. UPLC-MS analysis (4 min, basic): rt = 1.75 min, m/z = 405.1 [M+H]+, 100% purity.

1H NMR (400 MHz, DMSO-D6) δ 10.60 (s, 1H), 7.87 (s, 1H), 7.10 (d, J = 8.1 Hz, 1H), 7.01 - 6.91 (m, 3H), 6.88 (s, 1H), 6.67 (d, J = 7.0 Hz, 1H), 5.96 (d, J = 8.6 Hz, 1H), 4.53 (dt, J = 9.0, 4.5 Hz, 1H), 4.05 (t, J = 4.1 Hz, 2H), 3.35 (d, J = 4.2 Hz, 2H), 3.03 (dt, J = 12.3, 7.4 Hz, 3H), 2.85 (dd, J = 15.0, 9.6 Hz, 1H), 2.54 (s, 3H). Arbitrarily assigned to enantiomer R

### Example 62 2-(2-methylthlazol-5-yl)-N-[(4S)-1,3,4,5-tetrahydrobenzo[cd]indol-4-yl]-7,8-dihydro-6H-pyrimido[5,4-b][1,4]oxazin-4-amine

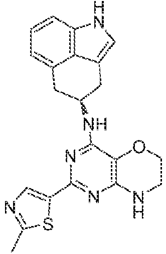

arbitrary assignment has been made:
2-(2-methylthiazol-5-yl)-N-[(4S)-1,3,4,5-tetrahydrobenzo[cd]indol-4-yl]-7,8-dihydro-6H-pyrimido[5,4-b][1,4]oxazin-4-amine (36 mg, 0.0881 mmol, 9.20% yield). Chemical purity: 98.75%, m/z = 405.21
Enantiomeric excess: 97.0.

UPLC-MS analysis (4 min, basic): rt = 1.75 min, m/z = 405.1 [M+H]+, 100% purity.

1H NMR (400 MHz, DMSO-D6) δ 10.60 (s, 1H), 7.87 (s, 1H), 7.10 (d, J = 8.1 Hz, 1H), 6.98 - 6.93 (m, 2H), 6.88 (s, 1H), 6.71 - 6.60 (m, 1H), 5.96 (d, J = 8.6 Hz, 1H), 4.53 (dt, J = 9.0, 4.5 Hz, 1H), 4.05 (t, J = 4.2 Hz, 2H), 3.35 (d, J = 4.2 Hz, 2H), 3.09 - 2.95 (m, 3H), 2.86 (dd, J = 15.4, 9.5 Hz, 1H), 2.54 (s, 3H).

Arbitrarily assigned to enantiomer S

### Example 63 3-[4-[[(3R)-2,3,4,9-tetrahydro-1H-carbazol-3-yl]amino]-7,8-dihydro-6H-pyrimido[5,4-b][1,4]oxazin-2-yl]-1H-pyridin-2-one

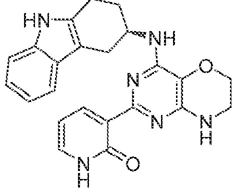

Prepared according to general method 5b, using 2-chloro-N-[(3R)-2,3,4,9-tetrahydro-1H-carbazol-3-yl)-7,8-dihydro-6H-pyrimido[5,4-b][1,4]oxazin-4-amine (75 mg, 0.198 mmol) and (2-Oxo-1,2-dihydro pyridin-3-yl)boronic acid (1.50 eq, 41 mg, 0.297 mmol). The crude was purified by column chromatography (20 g cartridge) eluting with a gradient of EtOAc (0% to 100%; v/v) in iso-Hex then MeOH (0% to 15%; v/v) in EtOAc to afford 3-[4-[[(3R)-2,3,4,9-tetrahydro-1H-carbazol-3-yl]amino]-7,8-dihydra-6H-pyrimido[5,4-b][1,4]oxazin-2-yl]-1H-pyridin-2-one (24 mg,0.0579 mmol, 29.23% yield) as a yellow solid. Example 63 is subject to keto-enol tautomerism. It may also exist in the following form: (R)-3-(4-((2,3.4,9-tetrahydro- 1H-carbazol-3-yl)amino)-7.8-dihydro-6H-pyrimido(5,4-b][1,4]oxazin-2-yl)pyridin-2-ol

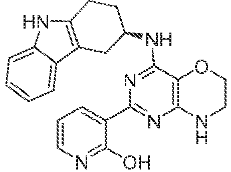

UPLC-MS analysis (4 min, basic): rt = 1.76 min, m/z = 415.2 [M+H] +, 100% purity. 1H NMR (400 MHz, DMSO-D6) δ 10.73 (s, 1H), 8.53 - 8.46 (m, 1H), 8.11 (dd, J = 4.8, 2.1 Hz, 1H), 7.37 (d, J = 7.4 Hz, 1H), 7.33 (d, J = 7.7 Hz, 1H), 7.28 - 7.24 (m, 1H), 7.03 - 6.97 (m, 1H), 6.94 - 6.89 (m, 2H), 6.60 - 6.49 (m, 1H), 4.38 (s, 1H), 4.18 (s, 2H), 3.48 (s, 2H), 3.03 (dd, J = 14.9, 5.3 Hz, 1H), 2.87 (q, J = 13.3 Hz, 2H), 2.74 (dd, J = 14.9, 9.5 Hz, 1H), 2.20 - 2.08 (m, 1H), 2.06 - 1.92 (m, 1H). 1H not observed.

### Example 64 N-[2-(1H-indol-3-yl)ethyl)-2-(2-methoxy-3-pyridyl)-7,8-dihydro-6H-pyrimido[5,4-b][1,4]oxazin-4-amine

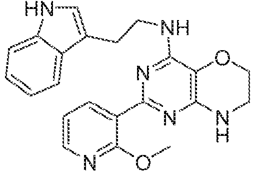

Prepared according to general method 5b, using 2-chloro-N-[2-(1H-indol-3-yl)ethyl]-7,8-dihydro-6H-pyrimido[5,4-b][1,4]oxazin-4-amine (80 mg, 0.243 mmol) and (2-methoxy-3-pyridyl)boronic acid (1.50 eq, 56 mg, 0.364 mmol). Additional amounts of Boronic acid, catalyst and ligand were added and the reaction was stirred at 100 °C for 24h. The crude was purified by column chromatography (40 g cartridge)eluting with a gradient of EtOAc (0% to 100%; v/v) in iso-Hex then MeOH (0% to 5%; v/v) in EtOAc to afford N-[2-(1H-indol-3-yI)ethyl]-2-(2-methoxy-3-pyridyl)-7,8-dihydro-6H-pyrimido[5,4-b] [1,4]oxazin-4-amine (45 mg,0.112 mmol, 46.09% yield) as a beige solid.

UPLC-MS analysis (4 min, basic): rt = 1.62 min, m/z = 403.1 [M+H]+, 100% purity.

1H NMR (400 MHz, DMSO-D6) δ 10.73 (s, 1H), 8.18 (dd, J = 4.9, 2.0 Hz, 1H), 7.81 (dd, J = 7.3, 2.0 Hz, 1H), 7.62 - 7.57 (m, 1H), 7.30 (dt, J = 8.1, 0.9 Hz, 1H), 7.13 (d, J = 2.3 Hz, 1H), 7.06 - 7.00 (m, 2H), 6.86 - 6.80 (m, 1H), 6.79 - 6.74 (m, 1H), 6.27 (t, J = 5.9 Hz, 1H), 4.17 - 4.08 (m, 2H), 3.81 (s, 3H), 3.61 - 3.51 (m, 2H), 3.45 - 3.35 (m, 2H), 2.98 - 2.89 (m, 2H).

### Example 65 2-(5-fluoro-2-methoxy-3-pyridyl)-N-[(3R)-2,3,4,9-tetrahydro-1H-carbazol-3-yl]-7,8-dihydro-6H-pyrimido[5,4-b][1,4]oxazin-4-amine

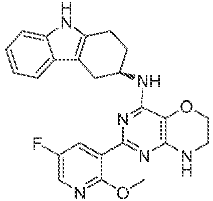

Prepared according to general method 5b, using 2-chloro-N-[(3R)-2,3,4,9-tetrahydro-1H-carbazol-3-yl]-7,8-dihydro-6H-pyrimido[5,4-b][1,4]oxazin-4-amine (130 mg, 0.329 mmol) and 5-Fluoro-2-methox ypyridine-3-boronic acid (84 mg, 0.493 mmol). The crude material was purified by column chromatography (20 g cartridge) eluting with a gradient of EtOAc (0% to 100%; v/v) in iso-Hex then MeOH (0% to 10%; v/v) in EtOAc to afford 2-(5-fluoro-2-methoxy-3-pyridyl)-N-[(3R)-2,3,4,9-tetrahydro-1H-carbazol-3-yl]-7,8-dihydro-6H-pyrimido[5,4-b][1,4]oxazin-4-amine (112 mg,0.251 mmol, 76.29% yield) as a yellow solid. UPLC-MS analysis (4 min, basic): rt = 1.89 min, m/z = 447.2 [M+H]+, 100% purity.

1H NMR (400 MHz, DMSO-D6) δ 10.64 (s, 1H), 8.09 (d, J = 3.2 Hz, 1H), 7.77 - 7.65 (m, 1H), 7.28 (d, J = 7.7 Hz, 1H), 7.19 (dt, J = 8.0, 1.0 Hz, 1H), 7.01 - 6.91 (m, 1H), 6.91 - 6.81 (m, 2H), 5.99 (d, J = 8.2 Hz, 1H), 4.35 - 4.21 (m, 1H), 4.10 (t, J = 4.4 Hz, 2H), 3.76 (s, 3H), 3.37 (d, J = 6.1 Hz, 2H), 3.00 (dd, J = 14.9, 5.3 Hz, 1H), 2.83 - 2.72 (m, 2H), 2.58 (dd, J = 15.0, 9.1 Hz, 1H), 2.13 - 2.02 (m, 1H), 2.01 - 1.87 (m, 1H). 19F NMR (376 MHz, DMSO-D6) δ -139.81.

### Intermediates for Example 66

### 66.1 2-(2-benzyloxy-5-fluoro-3-pyridyl)-N-(2-(1H-indol-3-yl)ethyl]-7,8-dihydro-6H-pyrimido[5,4-b][1,4]oxazin-4-amine

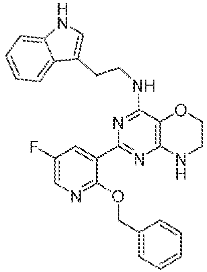

Prepared according to general method 5b, using 2-chloro-N-[2-(1H-indol-3-yl)ethyl]-7,8-dihydro-6H-pyrimido[5,4-b][1,4]oxazin-4-amine (120 mg, 0.364 mmol) and 2-(Benzyloxy)-5-fluoropyridin-3-ylboronic acid (135 mg, 0.546 mmol). The crude material was purified by column chromatography (20 g cartridge) eluting with a gradient of EtOAc (0% to 100%; v/v) in iso-Hex then MeOH (0% to 15%; v/v) in EtOAc to afford 2-(2-benzyloxy-5-fluoro-3-pyridyl)-N-[2-(1H-indol- 3-yl)ethyl]-7,8-dihydro-5H-pyrimido[5,4-b][1,4]oxazin-4-amine(105 mg,0.190 mmol, 52.30% yield) as an off-white solid.

UPLC-MS analysis (4 min, basic): rt = 2.20 min, m/z = 497.2 [M+H]+, 97% purity.

1H NMR (400 MHz, DMSO-D6) δ 10.71 (s, 1H), 8.14 (d, J = 3.1 Hz, 1H), 7.76 (dd, J = 8.6, 3.1 Hz, 1H), 7.55 (d, J = 7.9 Hz, 1H), 7.38 - 7.33 (m, 2H), 7.26 (dt, J = 8.1, 1.0 Hz, 1H), 7.21 - 7.13 (m, 3H), 7.06 (d, J = 2.4 Hz, 1H), 6.98 (ddd, J = 8.1, 6.9, 1.2 Hz, 1H), 6.82 - 6.73 (m, 2H), 6.27 (t, J = 5.9 Hz, 1H), 5.31 (s, 2H), 4.09 (t, J = 4.3 Hz, 2H), 3.53 (q, J = 6.9 Hz, 2H), 3.38 (q, J = 3.7 Hz, 2H), 2.91 - 2.82 (m, 2H). 19F NMR (376 MHz, DMSO-D6) δ -139.38.

### Example 66 3-(4-((2-(1H-indol-3-yl)ethyl)amino)-7,8-dihydro-6H-pyrimido[5,4-b][1,4] oxazin-2-yl)-5-fluoropyridin-2(1H)-one

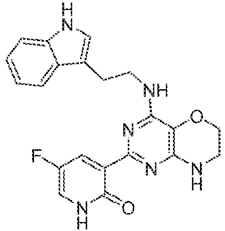

To a solution of 2-(2-benzyloxy-5-fluoro-3-pyridyl)-N-[2-(1H-indol-3-yl)ethyl]-7,8-dihydro-6H-pyrimido[5,4-b][1,4]oxazin-4-amine (95 mg, 0.191 mmol) in Ethanol (3.8265 mL), Palladium on Activated Carbon (10% Pd) (30 mg, 0.191 mmol) was added and the reaction was stirred at room temperature under molecular hydrogen atmosphere for 18h. The suspension was filtered through celite and the filtrate was evaporated to dryness. The crude material was purified by column chromatography (20 g cartridge) eluting with a gradient of EtOAc (0% to 100%; v/v) in iso-Hex to afford 5-fluoro-3-[4-[2-(1H-indol-3-yl)ethylamino]-7,8-dihydro-6H-pyrimido[5,4-b][1,4]oxazin-2-yl]pyrdin-2-ol (49 mg,0.119 mmol, 62.39% yield) as an off-white solid Example 66 is subject to keto-enol tautomerism. It may also exist in the following form: 3-(4-((2-(1H-indol-3-yl)ethyl)amino)-7,8-dlhydro-6H-pyrlmldo[5,4-b][1,4]oxazin-2-yl)-5-fluoropyridin-2-ol

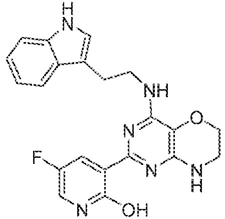

UPLC-MS analysis (4 min, basic): rt = 1.79 min, m/z =407.3 [M+H]+, 99% purity.

1H NMR (400 MHz, DMSO-D6) δ 10.84 (s, 1H), 8.30 (dd, J = 9.1, 3.3 Hz, 1H), 8.15 (d, J = 3.2 Hz. 1H), 7.60 (dd, J = 7.8. 1.1 Hz, 1H), 7.42 (d, J = 2.7 Hz, 1H), 7.34 (dt, J = 8.1, 0.9 Hz, 1H), 7.20 (d, J = 2.4 Hz, 1H), 7.11 - 7.04 (m, 1H), 7.02 - 6.94 (m, 1H), 6.84 (t, J = 5.9 Hz, 1H), 4.21- 4.11 (m, 2H), 3.72 - 3.60 (m, 2H), 3.47 (q, J = 3.8 Hz, 2H), 3.04 - 2.93 (m, 2H), 1H not observed.

19F NMR (376 MHz, DMSO-D6) δ -139.71.

### Example 67 2-(5-fluoro-2-methoxy-3-pyridyl)-N-[2-(1H-indol-3-yl)ethyl]-7,8-dihydro-6H-pyrimido[5,4-b][1,4]oxazin-4-amine

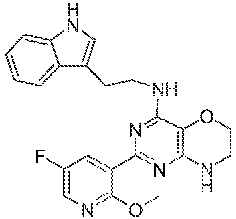

Prepared according to general method 5b, using 2-chloro-N-[2-(1H-indol-3-yl)ethyl]-7,8-dihydro-6H-pyrimido[5,4-b][1,4]oxazin-4-amine (85 mg, 0.232 mmol) and 5-Fluoro-2-methoxypyridine-3-boronic acid (59 mg, 0.348 mmol) The crude material was purified by column chromatography (20 g cartridge) eluting with a gradient of EtOAc (0% to 100%; v/v) in iso-Hex then MeOH (0% to 10%; v/v) in EtOAc to afford 2-(5-fluoro-2-methoxy-3-pyridyl)-N-[2-(1H-indol-3-yl)ethyl]-7,8-dihydro-6H-pyrimido[5,4-b][1,4]oxazin-4-amine (65 mg,0.155 mmol, 66.65% yield) as an off-white solid.

UPLC-MS analysis (4 min, basic): rt = 1.77 min, m/z = 421.3 [M+H]+, 99% purity.

1H NMR (400 MHz, DMSO-D6) δ 10.82 - 10.68 (m, 1H), 8.18 (dd, J = 3.1, 1.0 Hz, 1H), 7.80 - 7.71 (m, 1H), 7.60 (d, J = 7.9 Hz, 1H), 7.30 (dt, J = 8.1, 0.9 Hz, 1H), 7.13 (dd, J = 2.3, 1.0 Hz, 1H), 7.09 - 6.98 (m, 1H), 6.91 - 6.77 (m, 2H), 6.38 (t, J = 5.9 Hz, 1H), 4.12 (t, J = 4.3 Hz, 2H), 3.80 (d, J = 0.9 Hz, 3H), 3.56 (q, J = 6.8 Hz, 2H), 3.41 (s, 2H), 2.99 - 2.87 (m, 2H).

19F NMR (376 MHz, DMSO-D6) δ -139.90 (d, J = 0.9 Hz).

### Example 68 3-(4-((2-(1H-indol-3-yl)ethyl)amino)-7,8-dihydro-6H-pyrimido[5,4-b][1,4]oxazin-2-yl)-5-methylpyridin-2(1H)-one

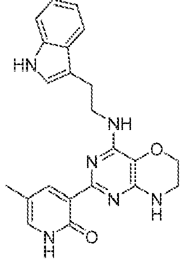

To N-[2-(1H-indol-3-yl)ethyl]-2-(2-methoxy-5-methyl-3-pyridyl)-7,8-dihydro-6H-pyrimido[5,4-b][1,4]oxazin-4-amine (80 mg, 0.192 mmol) in DCM (2 mL) was added boron tribromide solution in DCM 1M (0.091 mL, 0.960 mmol) and the mixture was stirred at ambient temperature overnight The reaction mixture was carefully quenched with MeOH (1 mL) and concentrated to dryness. The crude was purified by column chromatography (20 g cartridge) eluting with a gradient of EtOAc (0% to 100%; v/v) in iso-Hexane followed by prep HPLC to afford the desired product 3-[4-[2-(1H-indol-3-yl)ethylamino]-7,8-dihydro-6H-pyrimido[5,4-b][ 1,4]oxazin-2-yl]-5.methyl- pyridin-2-ol (30 mg,0.0745 mmol, 38.81 % yield) as a white solid. Example 68 is subject to keto-enol tautomerism. It may also exist in the following form:

### 3-(4-((2-(1H-indol-3-yl)ethyl)amino)-7,8-dihydro-6H-pyrimido[5,4-b][1,4]oxazin-2-yl)-5-methylpyridin-2-ol

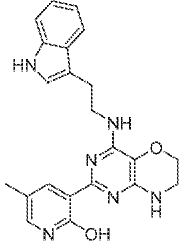

UPLC-MS analysis (6 min, basic): rt = 3.10 min, m/z = 403.2 [M+H]+, 100% purity.

1H NMR (400 MHz, DMSO-D6) δ 10.82 (s, 1H), 8.34 (d, J = 2.6 Hz, 1H), 7.96 -7.90 (m, 1H), 7.57 (d, J = 7.9 Hz, 1H), 7.31 (d, J = 8.0 Hz, 2H), 7.20 - 7.14 (m, 1 H), 7.03 (ddt, J = 8.2, 6.9, 1.4 Hz, 1H), 6.99 -6.90 (m, 1H), 6.73 (t, J = 5.8 Hz, 1H), 4.10 (d, J = 4.5 Hz, 2H), 3.61 (q, J = 7.1 Hz, 2H), 3.42 (s, 2H), 2.96 (t, J = 7.8 Hz, 2H), 2.20 (s, 3H). 1H not observed.

### Example 69 3-(4-((2-(1H-indol-3-yl)ethyl)amino)-7,8-dihydro-6H-pyrimido[5,4-b]|1,4] oxazin-2-yl)-5-(trifluoromethyl)pyridin-2(1H)-one

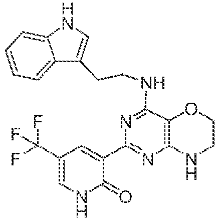

N-[2-(1H-indol-3-yl)ethyl]-2-[2-methoxy-5-(trifluoromethyl)-3-pyridyl]-7,8-dihydro-6H-pyrimido[5,4-b][5,4-b]oxazin-4-amine (70 mg, 0.149 mmol) was treated with Hydrogen chloride 4N in dioxane (5.0 mL, 20.0 mmol) and the mixture was heated at 70°C for 18h. The reaction was concentrated to dryness and the residue was purified by column chromatography (12 g column) eluting with 15-30% Methanol in DCM to afford 3-[4-[2-(1H-indol-3-yl)ethylamino]-7,8-dihydro-6H-prymido[5,4-b][1,4]oxazin-2-yl]-5-(trifluoromethyl)pyridin-2-ol (39 mg,0.0836 mmol, 56.22% yield) as a yellow solid. Example 69 is subject to keto-enol tautomerism. It may also exist in the following form:

### 3-(4-((2-(1H-indol-3-yl)ethyl)amino)-7,8-dihydro-6H-pyrimido[5,4-b][1,4]oxazin-2-yl)-5-(trifluoromethyl)pyridin-2-ol

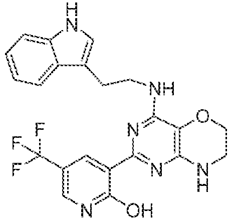

UPLC-MS analysis (4 min, basic): rt = 1.50 min, m/z = 457.3 [M+H]+, 100 % purity.

NMR (400 MHz, DMSO-D6) δ 10.84 (s, 1H), 8.69 (s, 3H), 8.49 (s, 1H), 7.59 (s, 1H), 7.53 (d, J = 7.9 Hz, 1H), 7.30 (d, J = 8.1 Hz, 1H), 7.14 (d, J = 2.3 Hz, 1H), 7.02 (ddd, J = 10.7, 6.0, 2.5 Hz, 2H), 6.93 (td, J = 7.4, 1.1 Hz, 1H), 4.13 (t, J = 4.3 Hz, 2H), 3.62 (q, J = 7.0 Hz, 2H), 3.46 (d, J = 4.7 Hz, 2H), 3.00 - 2.92 (m, 2H). 1H not observed.

19F NMR (376 MHz, DMSO-D6) δ -59.66.

### Intermediates for Example 70

### 70.1 2-(2-benzyloxy-5-fluoro-3-pyridyl)-N-[2-(6-fluoro-1H-indol-3-yl)ethyl]-7,8-dihydro-6H-pyrimido[5,4-b][1,4|orazin-4-amine

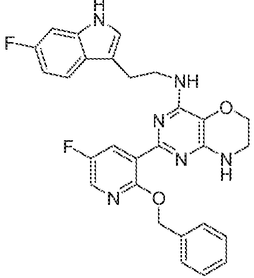

Prepared according to general method 5b, using 2-chloro-N-[2-(6-fluoro-1H-indol-3-yl)ethyl]-7,8-dihydro-6H-pyrimido[5,4-b][1,4]oxazin-4-amine (100 mg, 0.288 mmol) and 2-(Benzyloxy)-5-fluoropyridin-3-ylboronic acid (107 mg, 0.431 mmol). The crude was purified by column chromatography (20 g cartridge) eluting with a gradient of EtOAc (0% to 100%; v/v) in iso-Hex to afford2-(2-benzyloxy-5-fluoro-3-pyridyl)-N-[2-(6-fluoro-1H-indol-3-yl)ethyl]- 7,8-dihydro-6H-pyrimido[5,4-b][1,4]oxazin-4-amine (80 mg,0.140 mmol, 48.66% yield) as a yellowish solid.

UPLC-MS analysis (2 min, basic): rt = 1.23 min, m/z = 515.3 [M+H]+, 94% purity.

1H NMR (400 MHz, DMSO-D6) δ 10.79 (s, 1H), 8.14 (dd, J = 3.1, 1.8 Hz, 1H), 7.81 - 7.70 (m, 1H), 7.52 (dd, J = 8.4, 6.0 Hz, 1H), 7.38 - 7.32 (m, 2H), 7.19 - 7.13 (m, 3H), 7.07 - 6.99 (m, 2H), 6.76 (s, 1H), 6.63 - 6.56 (m, 1H), 6.30 (t, J = 5.7 Hz, 1H), 5.30 (s, 2H), 4.08 (s, 2H), 3.51 (d, J = 7.6 Hz, 2H), 3.38 (s, 2H), 2.84 (t, J = 7.6 Hz, 2H). 19F NMR (376 MHz, DMSO-D6) δ -139.36.

### Example 70 5-fluoro-3-(4-((2-(6-fluoro-1H-indol-3-yl)ethyl)amino)-7,8-dihydro-6H-pyrimido[5,4-b][1,4]oxazin-2-yl)pyridin-2(1H)-one

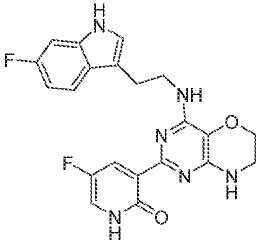

To a solution of 2-(2-benzyloxy-5-fluoro-3-pyridyl)-N-[2-(6-fluoro-1H-indol-3-yl)ethyl]-7,8-dihydro-6H-pyrimido[5,4b][1,4]oxazin-4-amine (90 mg, 0.157 mmol) in Ethanol (3.1485 mL), Palladium on Activated Carbon paste (5% Pd) (60 mg, 0.157 mmol) was added and the reaction was stirred at room temperature under molecular hydrogen atmosphere for 18h. The suspension was filtered through celite and the filtrate was evaporated to dryness.

The crude material was triturated with DCM/MeOH (1:1, 2 mL) to afford 5-fluoro-3-[4-[2-(6-fluoro-1H-indol-3-yl)ethylamino]-7,8-dibydro-6H-pyrimido[5,4-b][1,4]oxazin-2-yl]pyridin-2-ol (25 mg,0.0577 mmol, 36.67% yield) as an off-white solid. Example 70 is subject to keto-enol tautomerism. It may also exist in the following form: 5-fluoro-3-(4-((2-(6-fluoro-1H-indol-3-yl)ethyl)amino)-7,8-dihydro-6H-pyrimido[5,4-b][1,4]oxazin-2-yl)pyridin-2-ol

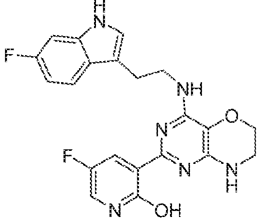

UPLC-MS analysis (4 min, basic): rt = 1.85 min, m/z = 425.3 [M+H]+, 99% purity.

1H NMR (400 MHz, DMSO-D6) δ 10.91 (s, 1H), 8.25 (dd, J = 9.0, 3.2 Hz, 1H), 8.14 (d, J = 3.2 Hz, 1H), 7.56 (dd, J = 8.7, 5.5 Hz, 1H), 7.42 (d, J = 3.0 Hz, 1H), 7.20 (d, J = 2.3 Hz, 1H), 7.10 (dd, J = 10.2, 2.3 Hz, 1H), 6.88 - 6.77 (m, 2H), 4.16 (t, J = 4.2 Hz, 2H), 3.65 (q, J = 7.0 Hz, 2H), 3.51 - 3.42 (m. 2H), 3.02- 2.92 (m. 2H). 1H not observed

19F NMR (376 MHz, DMSO-D6) δ -122.36, -139.73.

### Intermediates for Example 71

### 71.1 2-(2-benzyloxy-5-fluoro-3-pyridyl)-N-[(3R)-2,3,4,9-tetrahydro-1H-carbazol-3-yl]-7,8-dihydro-6H-pyrimido[5,4-b][1,4]oxazin-4-amine

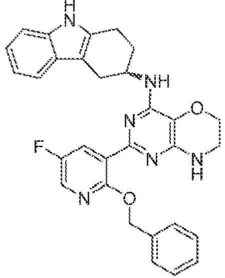

Prepared according to general method 5b, using 2-chloro-N-[(3R)-2,3,4,9-tetrahydro-1H-carbazol-3-yi]-7,8-dihydro-6H-pyrimido[5,4-b][1,4]oxazin-4-amine (80 mg, 0.202 mmol) and 2-(Benzyloxy)-5-fluoro pyridin-3-ylboronic acid (75 mg, 0.304 mmol). The crude material was purified by column chromatography (20 g cartridge) eluting with a gradient of EtOAc (0% to 100%; v/v) In iso-Hex to afford the desired product 2-(2-benzyloxy-5-fluoro-3-pyridyl)-N-[(3R)-2,3,4,9-tetrahydro-1H-carbazol-3-yl)-7,8-dihydro-6H-pyrimido[5,4-b][1,4]oxazin-4-amine (50 mg,0.0957 mmol, 47.28% yield) as a yellow solid.

UPLC-MS analysis (4 min, basic): rt = 2.29 min, m/z = 523.2 [M+H]+, 97% purity.

1H NMR (400 MHz, DMSO-D6) δ 10.62 (s, 1H), 8.08 (d, J = 3.1 Hz, 1H), 7.74 (dd, J = 8.6, 3.1 Hz, 1H), 7.37 (d, J = 1.7 Hz, 1H), 7.36 - 7.34 (m, 1H), 7.23 (d, J = 7.6 Hz, 1H), 7.20 - 7.10 (m, 4H), (1.93 (ddd, J = 8.1, 7.1, 1.3 Hz, 1H), 6.85 (ddd, J = 8.0, 7.0, 1.1 Hz, 1 H), 6.80 (s, 1 H), 5.99 (d, J = 8.5 Hz, 1H), 5.30 (s, 2H), 4.36 (s, 1H), 4.10 (s, 2H), 3.46 - 3.35 (m, 2H), 2.90 (dd, J = 14.9.5.3 Hz, 1H), 2.69 (s, 2H), 2.58 (dd, J = 15.1, 9.2 Hz, 1H), 1.95 (s, 2H). 19F NMR (376 MHz, DMSO-D6) δ -139.32.

### Example 71 (R)-5-fluoro-3-(4-((2,3,4,9-tetrahydro-1H-carbazol-3-yl)amino)-7,8-dihydro-6H-pyrimido[5,4-b][1,4]oxazin-2-yl)pyridin-2(1H)-one

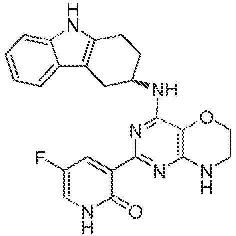

To a solution of 2-(2-benzyloxy-5-fluoro-3-pyridyl)-N-[(3R)-2,3,4,9-tetrahydro- 1H-carbazol-3-yl]-7,8-dihydro-6H-pyrimido[5,4-b][1,4|oxazin-4-amine (45 mg, 0.0775 mmol) in Ethanol (3.4445 mL), Palladium on Activated Carbon paste (5% Pd) (30 mg, 0.0775 mmol) was added and the reaction was stirred at room temperature under molecular hydrogen atmosphere for 18h. The suspension was filtered through celite and the filtrate was evaporated to dryness.

The crude material was triturated with DCM/MeOH (1:1, 2 mL) to afford 5-fluoro-3-[4-[[(3R)-2,3,4,9-tetrahydro-1H-carbazol-3-yl]amino)-7,8-dihydro-6H-pyrimido[5,4-b][1,4]oxazin-2-yl]pyridin-2-ol (13 mg,0.0301 mmol, 38.79% yield) as an off-white solid. Example 71 Is subject to keto-enol tautomerism. It may also exist in the following form: (R)-5-fluoro-3-(4-((2,3,4,9-tetrahydro-1H-carbazol-3-yl)amino)-7,8-dihydro-6H-pyrimido[5,4-b][1,4]oxazin-2-yl)pyridin-2-ol

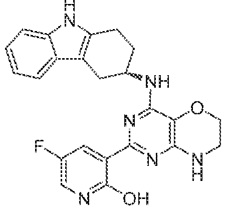

UPLC-MS analysis (4 min, basic): rt = 1.97 min, m/z = 433.3 [M+H]+, 100% purity. 1H NMR (400 MHz, DMSO-D6) δ 10.71 (s, 1H), 8.32 - 8.19 (m, 1H), 8.08 (d, J = 3.2 Hz, 1H), 7.43 (s, 1H), 7.29 (d, J = 7.8 Hz, 1H), 7.22 (d, J = 8.0 Hz, 1H), 6.96 (t, J = 7.5 Hz, 1H), 6.88 (t, J = 7.4 Hz, 1H), 6.62 (d, J = 8.3 Hz, 1H), 4.34 (s, 1H), 4.15 (s, 2H), 3.45 (s, 2H), 3.05 - 2.64 (m, 4H), 2.17 - 2.05 (m, 1H), 2.04 - 1.88 (m, 1H). 1H not observed 19F NMR (376 MHz, DMSO-D6) δ -139.61.

### Example 72 3-(4-((2-(1H-indol-3-yl)ethyl)amino)-7,8-dihydro-6H-pyrimido[5,4-b][1,4] oxazin-2-yl)-5-chloropyridin-2(1H)-one

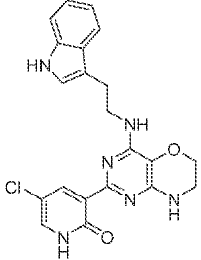

To 2-(5-chloro-2-methoxy-3-pyridyl)-N-[2-(1H-indol-3-yl)ethyl])-7,8-dihydro-6H-pyrimido[5,4-b][1,4)oxazin-4-amine (80 mg, 0.183 mmol) in DCM (10 mL) was added boron tribromide solution in DCM 1M (0.087 mL, 0.916 mmol) and the mixture was stirred at ambient temperature overnight. The reaction mixture was carefully quenched with MeOH (2 mL) and concentrated to dryness. The crude was purified by column chromatography eluting with a gradient of EtOAc (0% to 100%; v/v) in iso-Hexane to afford 5-chloro-3-[4-[2-(1H-indol-3-yl)ethylamino]-7,8 **-dihydro-6H-pyrimido[5,4-b][1,4]oxazin-2-yl]pyridin-2-ol (18 mg,0.0404 mmol, 22.08% yield) as a** yellow solid. Example 72 is subject to keto-enol tautomerism. It may also exist in the following form: 3-(4-((2-(1H-indol-3-yl)ethyl)amino)-7,8-dihydro-6H-pyrimido[5,4-b][1,4]oxazin-2-yl)-5-chloropyridin-2-ol

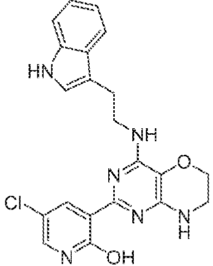

UPLC-MS analysis (4 min, basic): rt = 1.93 min, m/z = 423.3/425.3 [M+H]+, 95% purity.

1H NMR (400 MHz, DMSO-D6) δ 10.81 (s, 1H), 8.47 (d, J = 2.8 Hz, 1H), 8.15 (d, J = 2.8 Hz, 1H), 7.56 (dt, J = 7.7, 0.9 Hz, 1H), 7.42 (s, 1H), 7.30 (dt, J = 8.0, 1.0 Hz, 1H), 7.16 (d, J = 2.3 Hz, 1H), 7.03 (ddd, J = 8.1, 7.0, 1.3 Hz, 1H), 6.96 (ddd, J = 8.0. 7.0. 1.1 Hz, 1H), 6.84 (t, J = 5.9 Hz, 1H), 4.12 (t, J = 4.2 Hz, 2H), 3.66 - 3.57 (m, 2H), 3.46 - 3.41 (m, 2H), 2.99 - 2.91 (m, 2H). 1H not observed.

### Intermediate for Example 73

### 73.1 N-[2-(1H-indol-3-yl)ethyl]-2-(2-methoxy-6-methyl-3-pyridyl)-7,8-dihydro-6H-pyrimido[5,4-b][1,4]oxazin-4-amine

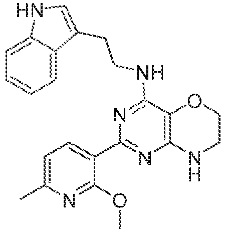

Prepared according to general method 5b, 2-chloro-N-[2-(1H-indol-3-yl)ethyl]-7,8-dihydro-6H-pyrimido[5,4-b][1,4]oxazin-4-amine (1.00 eq, 150 mg, 0.455 mmol) and 2-Methoxy-6-methylpyridine-3-boronic acid (1.50 eq, 114 mg, 0.682 mmol). The crude material was purified by column chromatography (20 g cartridge) eluting with a gradient of EtOAc (50% to 100%; v/v) in hexane to afford the desired product

N-[2-(1H-indol-3-yl)ethyl]-2-(2-methoxy-6-methyl-3-pyridyl)-7.8-dihydro-6H-pyrimido[5,4-b][1,4]oxazin-4-amine (76 mg,0.168 mmol, 37.01% yield) as a beige solid.

UPLC-MS analysis (2 min, basic): rt = 1.17 min, m/z = 417.4 [M+H]+, 92% purity.

1 H NMR (400 MHz, DMSO-D6) δ 10.75 (s, 1H), 7.72 (dd, J = 7.3, 5.0 Hz, 1H), 7.60 (d, J = 7.7 Hz, 1H), 7.34 - 7.28 (m, 1H), 7.13 (s, 1H), 7.03 (s, 1H), 6.86 (d, J = 8.6 Hz, 2H), 6.75 (s, 1H), 6.25 (d. J = 5.5 Hz, 1H), 4.11 (s, 2H), 3.79 (d, J = 4.4 Hz, 3H), 3.55 (s,2H), 3.40 (s,2H), 2.94 (s, 2H), 2.43 (d, J=4.0 Hz, 3H).

### Example 73 3-[4-[2-(1H-indol-3-yl)ethylamino]-7,8-dihydro-6H-pyrimido[5,4-b][1,4] exazin-2-yl]-6-methyl-1H-pyridin-2-one

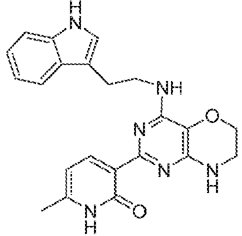

N-[2-(1H-indol-3-yl)ethyl]-2-(2-methoxy-6-methyl-3-pyridyl)-7,8-dihydro-6H-pyrimido[5,4-b][1,4]oxazin-4-amine (76 mg, 0.183 mmol) was treated with Hydrogen chloride 4N in dioxane (6.1 mL, 24.6 mmol) and the mixture was heated at 70°C for 18h. The reaction was concentrated to dryness and the residue was purified by column chromatography (12 g cartridge) eluting with EtOAc to afford the desired product 3-[4-[2-(1H-indol-3-yl)ethylamino]-7,8-dihydro-6H-pyrimido[5,4-b][1,4]oxazin-2-yl]-6-methyl-1 H-pyridin-2-one (55 mg,0.132 mmol, 72.23% yield) as a yellow solid. Example 73 is subject to keto-enol tautomerism. It may also exist in the following form: 3-(4-((2-(1H-indol-3-yl)ethyl)amino)-7,8-dihydro-6H-pyrimido[5,4-b](1,4)oxazin-2-yl)-6-methylpyridin-2-ol

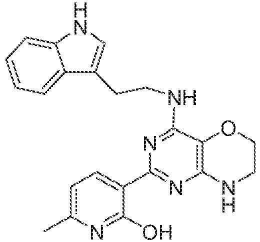

UPLC-MS analysis (4 min, basic): rt = 1.81 min, m/z = 403.4 [M+H]+, 96% purity.

1H NMR 0.12 (400 MHz, DMSO-D6) δ 10.84 (s, 1H), 8.44 (d. J = 7.7 Hz, 1H), 8.14 (s, 1H), 7.57 (d, J = 8.0 Hz, 2H), 7.33 (d, J = 8.1 Hz, 1H), 7.20 (s, 1H), 7.13 - 6.94 (m, 2H), 6.62 (d, J = 7.7 Hz, 1H), 4.18 (s, 2H), 3.71 (s, 2H), 3.52 (s, 2H), 3.00 (s, 2H), 2.38 (s, 3H), 1H not observed.

### Example 74 2-(2-amino-3-pyridyl)-N-[2-(1H-indol-3-yl)ethyl]-7,8-dihydro-6H-pyrimido [5,4-b][1,4]oxazin-4-amine

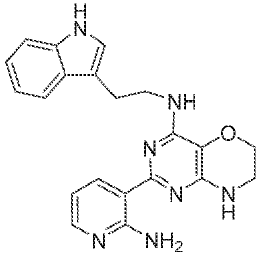

Prepared according to general method 5b, 2-chloro-N-[2-(1H-indol-3-yl)ethyl]-7,8-dihydro-6H-pyrimido[5,4-b][1,4]oxazin-4-amine (100 mg, 0.294 mmol) and 3-(4,4,5,5-tetramethyl-1,3,2-dioxaborolan-2-yl)pyridin-2-amine (97 mg, 0.441 mmol). The crude material was purified by column chromatography (20 g cartridge) eluting with a gradient of MeOH (5% to 10%; v/v) in DCM followed by prep HPLC (basic method) to afford title compound(52 mg,0.129 mmol, 43.80% yield) as a yellow solid.

UPLC-MS analysis (4 min, basic): rt = 1.66 min, m/z = 388.3 [M+H]+, 96% purity.

1H NMR (400 MHr, DMSO-D6) δ 10.83 (s, 1H), 8.78 (dd, J = 7.6, 1.8 Hz, 1H), 8.05 (dd, J = 5.8, 1.8 Hz, 1H), 7.56 (d, J = 7.8 Hz, 1H), 7.33 (dd, J = 8.1, 1.0 Hz, 1H), 7.20 (d, J = 2.3 Hz, 1H), 7.13 (s, 1H), 7.10 - 7.03 (m, 1 H), 7.02 - 6.96 (m, 1 H), 6.89 (dd, J = 7.7, 5.8 Hz, 1H), 6.57 (t, J = 5.9 Hz, 1H), 4.20 - 4.09 (m, 2H), 3.66 (q, J = 6.9 Hz, 2H), 3.46 (s, 2H), 2.98 (t, J = 7.5 Hz, 2H). 2 Hs not observed.

### In vitro assay 1: AhR Antagonism in U937 cells (Promega P450-Glo^{™} Assay)

AhR antagonism was assessed in U937 cells (myeloid lineage cell line derived from a human histiocytic lymphoma). Ligand binds the AhR in the cytoplasm, and the AhR-ligand complex translocates to the nucleus and forms a heterodimer with AhR nuclear translocator (Arnt). This complex binds the xenobiotic response element (XRE) in the 5' upstream region of the CYP1A1 promoter, enhancing CYP1A1 expression. CYP1A1 activity is subsequently determined by assessing the conversion of Luciferin-CEE to luciferin, which in turn reacts with luciferase to produce light The amount of light produced is directly proportional to cytochrome P450 activity.

U937 cells in Ultraculture serum free media (Lonza) were plated at 100,000 cells per well in a round bottom 96 well tissue culture plate. Seven concentrations of test compound (final [DMSO] 1%) were added and incubated for 10 minutes before the addition of 300µM KYNA. The plates were then placed in an Incubator at 37°C, ≥ 85% humidity, 5% CO₂ for 24hrs. After aspiration of the supernatant the CYP1A1 substrate Luciferin-CEE ([Final] 83µM) was added and incubated for 3 hrs before the reaction was stopped by adding luciferin detection reagent and luminescence was read after 20 minutes.

### In vitro assay 2: CYP1A1 inhibition assay

The direct CYP1A1 inhibitory activity of test compounds was also assessed using the Promega P450-Glo^{™} assay system. Seven concentrations of test compound were added to a ½ area white 96 well plate. Cypex CYP1A1 bactosomes ([final] 0.5pmol) and CYP1A1 substrate Luciferin-CEE ([final] 30µM) were prepared in 0.1M potassium phosphate buffer and incubated with test compounds at 37°C for 5 minutes. 0.2mM NADPH was then added to the plates and incubated at 37°C, for 10 minutes. The reaction was stopped by adding luciferin detection reagent and luminescence was read after 20 minutes.

***In vitro* assay 3: AhR Antagonism in human peripheral blood mononuclear cells (PBMCs)** - **Inhibition of Interleukin-22 (IL-22) release.**

PBMCs were isolated from human peripheral blood using Lymphoprep^{™} and diluted to 1 x 10⁶ cells per ml in RPMI media containing 10% foetal bovine serum, 1% penicillin-streptomycin and 1% non-essential amino acids. PBMCs were subsequently activated with 1µl per 100,000 cells of a CD3/CD28 agonist mixture (human T Cell TransAct^{™}(Miltenyl Biotec)) and then plated at 100,000 cells per well in a round bottom 96 well tissue culture plate. One hour after stimulation seven concentrations of each test compound or vehicle (final [DMSO) 0.2%) were added. The plates were then placed in an incubator at 37°C, ≥ 85% humidity, 5% CO₂ for 72hrs before the media was removed and stored at -20°C until cytokine analysis. IL-22 was measured using human IL-22 DuoSet ELISA (R&D systems) according to the manufacturer's instructions. The results of the *in vitro* assays are shown in Table 1 below.

**Table 1 - Results of in vitro assays**

| **EG** | **hPBMC Inhibition of CD3/CD28 Induced IL-22 release:av. pIC50** | **U937 Antagonist pIC50-VAF347: av. pIC50** | ***EG*** | **hPBMC Inhibition of CD3/CD28 induced IL-22 release:av. pIC50** | **U937 Antagonist pIC50-VAF347: av. pIC50** |
|---|---|---|---|---|---|
| 1 | 8.72 | 9.14 | 38 | 7.05 | 7.18 |
| 2 | 7.25 | 7.6 | 39 | | 7.05 |
| 3 | 8.4 | 8.45 | 40 | | 7.82 |
| 4 | 7.08 | 7.79 | 41 | | 7.71 |
| 5 | 9.02 | 8.73 | 42 | | 6.68 |
| 6 | 8.87 | 8.11 | 43 | | 8.15 |
| 7 | 8.02 | 8.04 | 44 | > 8.81 | 7.99 |
| 8 | 8.98 | 8.95 | 45 | > 8.99 | 8.42 |
| 9 | 9.18 | 9.29 | 46 | > 8.87 | 9.12 |
| 10 | 8.09 | 8.34 | 47 | | 8.49 |
| 11 | 6.28 | 7.04 | 48 | 8.79 | 8.36 |
| 12 | 6.85 | 7.05 | 49 | 8.16 | 8.47 |
| 13 | <5.75 | 6.64 | 50 | 7.65 | 8.26 |
| 14 | 7 | 7.13 | 51 | | 6.69 |
| 15 | | 7.05 | 52 | | 7.75 |
| 16 | | 7.48 | 53 | 6.12 | 7.09 |
| 17 | 8.78 | 8.42 | 54 | 6.3 | 6.64 |
| 18 | | 6.35 | 55 | < 5.70 | 6.12 |
| 19 | 7.61 | 7.29 | 56 | 7.73 | 7.92 |
| 20 | 7.36 | 7.04 | 57 | 8.7 | 8.51 |
| 21 | 8.84 | 8.56 | 58 | | 6.46 |
| 22 | <5.70 | 6.91 | 59 | 8.49 | 8.42 |
| 23 | | 6.34 | 60 | 8.13 | 8.37 |
| 24 | 8.54 | 8.58 | 61 | 5.9 | 6.47 |
| 25 | 8.77 | 8.57 | 62 | 6.88 | 7.3 |
| 26 | 9.14 | 8.32 | 63 | 6.8 | 7.03 |
| 27 | 8.67 | 8.31 | 64 | 6.84 | 7.21 |
| 28 | 9.21 | 9.29 | 65 | | < 6.09 |
| 29 | 8.52 | 8.66 | 66 | 9.49 | 8.99 |
| 30 | 8.4 | 8.26 | 67 | | 7.18 |
| 31 | | 7.79 | 68 | | 7.48 |
| 32 | 7.84 | 8.6 | 69 | | 7.09 |
| 33 | 7.87 | 7.85 | 70 | 9.19 | 8.8 |
| 34 | < 5.70 | < 6.12 | 71 | 7.16 | 6.9 |
| 35 | 6.01 | < 6.00 | 72 | | 7.83 |
| 36 | 5.97 | < 6.00 | 73 | | 6.73 |
| 37 | 6.91 | 6.6 | 74 | 8.54 | 7.92 |

## Claims

1. A compound of formula **(I)** 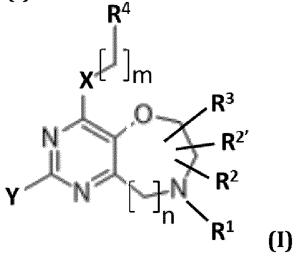 wherein:
Y is phenyl or a 3 to 6 membered ring optionally comprising 1, 2, or 3 heteroatoms selected from N, O and S, said phenyl or ring substituted with R⁵ and R⁶;
R¹ is H, C₁₋₃ alkyl, (-CH₂)pCN, -COC₁₋₃ alkyl, -CO(CH₂)qNR⁷R⁸, -SO₂C₁₋₃ alkyl, -SO₂NR⁷R⁸, -(CH₂)qPh, C₃₋₅ cycloalkyl, C₁₋₃ alkyl bearing 1 to 6 halogen groups, C₁₋₃ alkyl bearing one or more independently selected from OR^{Y} and -NR⁷R⁸, -(CH₂)p' OC₁₋₃alkyl substituted with 1 to 6 halogen groups, -C₀₋₃alkyleneC(O)C₀₋₃ alkyl wherein the alkyl is or bears at least one group selected from OR^{Y} and -NR⁷R⁸, Oxetane, Z, -C(O)(CH₂)qZ, -C(O)O(CH₂)qZ, - C(O)O(CH₂)pPh, -C(O)(CH₂)qZ', -C(O)O(CH₂)qZ';
R² is H or C₁₋₃ alkyl, C₃₋₅ cycloalkyl, halogen and C₁₋₃ alkyl optionally bearing one or more groups independently selected from OR^{Y}, halogen and -NR⁷R⁸;
R^{2'} is H, C₁₋₃ alkyl or halogen;
R³ is H or C₁₋₃ alkyl;
R⁴ is a 9 to 13 membered heterocycle with at least one heteroatom selected from N, O and S, with substituents R⁹, R^{9'} and R¹⁰;
R⁵ is H, oxo, hydroxy, halogen, CN, C₁₋₃ alkyl, C₃₋₅ cycloalkyl, -OC₁₋₃ alkyl, -(O)₀₋₁C₁₋₃ alkyl bearing 1 to 6 halogen groups, C₁₋₃ alkyl bearing one or more OR^{Y} groups, -C(O)C₁₋₃alkylNR⁷R⁸, -SO₂C₁₋₃alkyl, -SO₂ NR⁷R⁸, or NR⁷R⁸;
R⁶ is H, oxo, hydroxy, halogen, CN, C₁₋₃ alkyl, -C(O)C₁₋₃alkylNR⁷R⁸, -SO₂C₁₋₃ alkyl, or -SO₂ NR⁷R⁸,
R⁷ is H, C₁₋₃ alkyl or -C(O)OR^{Y};
R⁸ is H or C₁₋₃ alkyl;
R⁹ is H, hydroxy, halogen, CN, C₁₋₃ alkyl, C₁₋₃ alkoxy, C₁₋₃ alkyl bearing 1 to 6 halogen groups, C₁₋₃ alkyl bearing one or more OR^{Y} groups,
C₃₋₅cycloalkyl, -(CH₂)qOC₁₋₃alkyl substituted with 1 to 6 halogen groups, -CO C₁₋₃ alkylNR⁷R⁸, -SO₂C₁₋₃ alkyl, or -SO₂ NR⁷R⁸;
R^{9'} is H, hydroxy, halogen, CN, C₁₋₃ alkyl, C₁₋₃ alkoxy, C₁₋₃ alkyl bearing 1 to 6 halo groups, C₁₋₃ alkyl bearing one or more hydroxy groups;CO(CH₂)q NR⁷R⁸, -SO₂C₁₋₃ alkyl, or -SO₂ NR⁷R⁸;
R¹⁰ is H, hydroxy, halogen, CN, C₁₋₃ alkyl, -C(O)C₁₋₃ alkyl NR⁷R⁸; -SO₂C₁₋₃ alkyl, or -SO₂ NR⁷R⁸;
R¹¹ is H, C₁₋₃alkyl or C₂₋₆hydroxyalkyl;
R^{Y} is H or C₁₋₃ alkyl;
X is CH₂, S, -SO₂, NR¹¹ or O;
Z' is a 5 or 6 membered heteroaryl with at least one heteroatom selected from N, O and S, wherein said heteroaryl optionally bears one, two or three substituents selected from hydroxy, halogen, CN, C₁₋₃ alkyl;
Z is a 3 to 6 membered ring optionally comprising 1, 2, or 3 heteroatoms selected from N, O and S bearing R⁵ and R⁶ optionally bears one, two or three substituents selected from hydroxy, halogen, CN, C₁₋₃ alkyl;
n is 0 or 1;
n' is 0 or 1;
m is 0, 1, 2 or 3
p is an integer 1, 2 or 3;
p' is an integer 2 or 3;
q is 0, 1, 2 or 3,
or a pharmaceutically acceptable salt thereof.

2. A compound of formula **(I)** according to claim 1, wherein Y is a 5 or 6 membered nitrogen containing ring.

3. A compound of formula **(I)** according to claim 1 or 2, wherein the ring is independently selected from pyrimidine, pyridine, triazole and thiazole.

4. A compound of formula **(I)** according to claim 3, wherein R⁵ is located at position 5 on the Y group.

5. A compound according to any one of claims 1 to 4, wherein n is 0.

6. A compound according to claim 1 of formula **(II)** 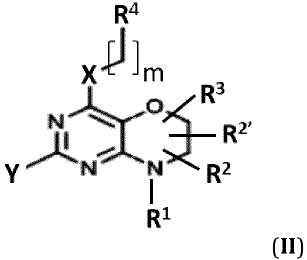 wherein X, R¹, R², R³, R⁴, and m are defined above for compounds of formula **(I)** or a pharmaceutically acceptable salt thereof.

7. A compound according to any one of claims 1 to 6, wherein R¹ is independently selected from hydrogen, -C₁₋₃ alkyl, oxetane, -CH₂CH₂OH, -CH₂CH₂N(CH₃)₂, and -SO₂CH₃.

8. A compound according to any one of claims 1 to 7, wherein R² is independently selected from H, methyl, -CH₂OCH₃, -CH₂OH, CF₃, -CH₂N(CH₃)₂.

9. A compound according to any one of claims 1 to 8, wherein R^{2'} is methyl or H.

10. A compound according to any one of claims 1 to 9, wherein R⁴ is a 9 or 13 membered partially saturated or aromatic heterocycle selected from indoline, indole, isoindole, indolizine, indazole, benzimidazole, azaindole, pyrazolopyrimidine, purine, benzofuran, isobenzofuran, benzothiophene, benzoisoxazole, benzoisothiazole, benzoxazole, benzothiadiazole, adenine, guanine, tetrahydroquinoline, dihydroisoquinoline, quinoline, isoquinoline, quinolizine, quinoxaline, phthalazine, cinnoline, naphthyridine, pyridopyrimidine, pyridopyrazine, pteridine, chromene, isochromene, chromenone, benzoxazine, quinolinone, isoquinolinone, dibenzofuran, carbazole, acridine, phenothiazine, and 2,3,4,9-tetrahydro-1H-carbazole.

11. A compound according to any one of claims 1 to 10, wherein R⁵ is independently selected from the group comprising oxo, methyl, ethyl, -CF₃, -OCF₃, -OCH₃ and halogen.

12. A compound according to any one of claims 1 to 11, wherein R⁶ is independently selected from methyl, ethyl, -OCH₃, and H.

13. A compound according to any one of the claims 1 to 12, wherein:
(i) R⁹ is independently selected from H, methyl, OCF₃ and - C₃₋₅cycloalkyl, and/or
(ii) R^{9'} is independently selected from H, F and methyl, and/or
(iii) R¹⁰ is Independently selected from H, F and methyl.

14. A compound according to any one of claims 1 to 13, wherein m is 2.

15. A compound according to any one of claims 1 to 14, independently selected from:
2-(5-fluoro-3-pyridyl)-N-[2-(1H-indol-3-yl)ethyl]-7,8-dihydro-6H-pyrimido[5,4-b][1,4] oxazin-4-amine, 2-(4-fluorophenyl)-N-[2-(1H-indol-3-yl)ethyl]-7,8-dihydro-6H-pyrimido [5,4-b][1,4]oxazin-4-amine; 2-(3,5-difluorophenyl)-N-[2-(1H-indol-3-yl)ethyl]-7,8-di hydro-6H-pyrimido[5,4-b][1,4]oxazin-4-amine; 2-(5-fluoro-3-pyridyl)-N-[(3R)-2,3,4,9-tetrahydro-1H-carbazol-3-yl]-7,8-dihydro-6H-pyrimido[5,4-b][1,4]oxazin-4-amine; 2-(5-fluoro-3-pyridyl)-N-[2-(1H-indol-3-yl)ethyl]-7-methyl-7,8-dihydro-6H-pyrimido[5,4-b][1,4]oxazin-4-amine; 2-(5-fluoro-3-pyridyl)-N-[2-(1H-indol-3-yl)ethyl]-8-isopropyl-6,7-dihydropyrimido[5,4-b][1,4]oxazin-4-amine; N-[2-(1H-indol-3-yl) ethyl]-2-(2-methyl thiazol-5-yl)-7,8-dihydro-6H-pyrimido[5,4-b][1,4]oxazin-4-amine; (7S)-2-(5-fluoro-3-pyridyl)-N-[2-(1H-indol-3-yl)ethyl]-7-methyl-7,8-dihydro-6H-pyrimido[5,4-b][1,4]oxazin-4-amine; (7R)-2-(5-fluoro-3-pyridyl)-N-[2-(1H-indol-3-yl)ethyl]-7-methyl-7,8-dihydro-6H-pyrimido[5,4-b][1,4]oxazin-4-amine; N-[2-(1H-indol-3-yl)ethyl]-2-(4-methylthiazol-5-yl)-7,8-dihydro-6H-pyrimido[5,4-b][1,4]oxazin-4-amine; 2-[2-(1H-indol-3-yl)ethyl-[2-(2-methylthiazol-5-yl)-7,8-dihydro-6H-pyrimido[5,4-b][1,4]oxazin-4-yl]amino]ethanol; 2-(2-ethyl-4-methyl-thiazol-5-yl)-N-[2-(1H-indol-3-yl)ethyl]-7,8-dihydro-6H-pyrimido[5,4-b][1,4]oxazin-4-amine; N-[(3R)-2,3,4,9-tetrahydro-1H-carbazol-3-yl]-2-thiazol-2-yl-7,8-dihydro-6H-pyrimido[5,4-b][1,4]oxazin-4-amine; 2-(2-methylthiazol-5-yl)-N-(1,3,4,5-tetrahydrobenzo[cd]indol-4-yl)-7,8-dihydro-6H-pyrimido[5,4-b] [1,4] oxazin-4-amine, N-[2-(1H-indol-3-yl)ethyl]-2-(6-methyl-3-pyridyl)-7,8-dihydro-6H-pyrimido[5,4-b][1,4] oxazin-4-amine; 2-(5-fluoro-6-methyl-3-pyridyl)-N-[2-(1H-indol-3-yl)ethyl]-7,8-dihydro-6H-pyrimido[5,4-b][1,4]oxazin-4-amine; 3-[4-[2-(1H-indol-3-yl)ethylamino]-7,8-dihydro-6H-pyrimido[5,4-b][1,4]oxazin-2-yl]-1H-pyridin-2-one; 3-(4-((2-(1H-indol-3-yl)ethyl) amino)-7,8-dihydro-6H-pyrimido[5,4-b][1,4]oxazin-2-yl)pyridin-2-ol; 5-[4-[2-(1H-indol-3-yl)ethylamino]-7,8-dihydro-6H-pyrimido[5,4-b][1,4]oxazin-2-yl]-1H-pyridin-2-one; 5-(4-((2-(1H-indol-3-yl)ethyl)amino)-7,8-dihydro-6H-pyrimido[5,4-b][1,4]oxazin-2-yl)pyridin-2-ol; 3-[4-[2-(1H-indol-3-yl)ethylamino]-7,8-dihydro-6H-pyrimido[5,4-b][1,4]oxazin-2-yl]-1-methyl-pyridin-2-one; N-[2-(6-fluoro-1H-indol-3-yl)ethyl]-2-(6-methyl-3-pyridyl)-7,8-dihydro-6H-pyrimido[5,4-b][1,4]oxazin-4-amine; 3-[4-[2-(6-fluoro-1H-indol-3-yl) ethylamino]-7,8-dihydro-6H-pyrimido[5,4-b][1,4]oxazin-2-yl]-1H-pyridin-2-one; 3-(4-((2-(6-fluoro-1H-indol-3-yl)ethyl)amino)-7,8-dihydro-6H-pyrimido[5,4-b][1,4]oxazin-2-yl) pyridin-2-ol; 2-[2-(difluoromethoxy)-3-pyridyl]-N-[2-(1H-indol-3-yl)ethyl]-7,8-dihydro-6H-pyrimido[5,4-b][1,4]oxazin-4-amine; N-[2-(1H-indol-3-yl)ethyl]-2-(2-methoxy-5-methyl-3-pyridyl)-7,8-dihydro-6H-pyrimido[5,4-b][1,4]oxazin-4-amine, 4-[2-(6-fluoro-1H-indol-3-yl)ethoxy]-2-(2-methylthiazol-5-yl)-7,8-dihydro-6H-pyrimido[5,4-b][1,4] oxazine; 3-(4-(2-(1H-indol-3-yl)ethoxy)-7,8-dihydro-6H-pyrimido[5,4-b][1,4]oxazin-2-yl)pyridin-2(1H)-one; 3-(4-(2-(1H-indol-3-yl)ethoxy)-7,8-dihydro-6H-pyrimido[5,4-b][1,4]oxazin-2-yl)pyridin-2-ol; (R)-3-(4-((2-(1H-indol-3-yl)ethyl)amino)-7-(methoxy methyl)-7,8-dihydro-6H-pyrimido[5,4-b][1,4]oxazin-2-yl)pyridin-2(1H)-one; (R)-3-(4-((2-(1H-indol-3-yl)ethyl)amino)-7-(methoxymethyl)-7,8-dihydro-6H-pyrimido[5,4b][1,4] oxazin-2-yl)pyridin-2-ol; (R)-3-(4-((2-(1H-indol-3-yl)ethyl)amino)-7-(hydroxymethyl)-7,8-dihydro-6H-pyrimido[5,4-b][1,4]oxazin-2-yl)pyridin-2(1H)-one; (R)-3-(4-((2-(1H-indol-3-yl)ethyl)amino)-7-(hydroxymethyl)-7,8-dihydro-6H-pyrimido[5,4-b] [1,4] oxazin-2-yl)pyridin-2-ol; (7S)-2-(5-fluoro-3-pyridyl)-N-[2-(1H-indol-3-yl)ethyl]-7-(methoxy methyl)-7,8-dihydro-6H-pyrimido[5,4-b][1,4]oxazin-4-amine; (6S)-2-(5-fluoro-3-pyridyl)-N-[2-(1H-indol-3-yl)ethyl]-6-methyl-7,8-dihydro-6H-pyrimido[5,4-b][1,4]oxazin-4-amine; (6R)-2-(5-fluoro-3-pyridyl)-N-[2-(1H-indol-3-yl)ethyl]-6-methyl-7,8-dihydro-6H-pyrimido[5,4-b][1,4]oxazin-4-amine, 2-(5-fluoro-3-pyridyl)-N-[2-(1H-indol-3-yl)ethyl]-7,7-dimethyl-6,8-dihydropyrimido[5,4-b][1,4]oxazin-4-amine, (7R)-2-(5-fluoro-3-pyridyl)-N-[2-(1H-indol-3-yl)ethyl]-7-(trifluoromethyl)-7,8-dihydro-6H-pyrimido[5,4-b][1,4]oxazin-4-amine, N-[2-(1H-indol-3-yl)ethyl]-2-(2-methylthiazol-5-yl)-8-(oxetan-3-yl)-6,7-dihydropyrimido[5,4-b][1,4]oxazin-4-amine; 2-(2-methylthiazol-5-yl)-N-(6,7,8,9-tetrahydro-5H-pyrido[3,2-b]indol-8-yl)-7,8-dihydro-6H-pyrimido[5,4-b][1,4]oxazin-4-amine; (7R)-8-[2-(dimethylamino)ethyl]-7-(methoxymethyl)-2-(2-methylthiazol-5-yl)-N-[(3R)-2,3,4,9-tetrahydro-1H-carbazol-3-yl]-6,7-dihydropyrimido[5,4-b][1,4]oxazin-4-amine; (7R)-8-[2-(dimethylamino)ethyl]-2-(2-methylthiazol-5-yl)-4-[[(3R)-2,3,4,9-tetrahydro-1H-carbazol-3-yl]amino]-6,7-dihydropyrimido[5,4-b][1,4]oxazin-7-yl]methanol; 2-[2-(2-methylthiazol-5-yl)-4-[[(3R)-2,3,4,9-tetrahydro-1H-carbazol-3-yl]amino]-6,7-dihydropyrimido[5,4-b][1,4]oxazin-8-yl]ethanol; 7-[(dimethylamino) methyl]-2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amine; 8-methylsulfonyl-2-(2-methylthiazol-5-yl)-N-[(3R)-2,3,4,9-tetrahydro-1H-carbazol-3-yl]-6,7-dihydropyrimido[5,4-b][1,4]oxazin-4-amine; 3-(4-((2-(2-chloro-1H-indol-3-yl)ethyl)amino)-7,8-dihydro-6H-pyrimido[5,4-b][1,4]oxazin-2-yl)pyridin-2(1H)-one; 3-(4-((2-(2-chloro-1H-indol-3-yl)ethyl)amino)-7,8-dihydro-6H-pyrimido[5,4-b][1,4]oxazin-2-yl)pyridin-2-ol; 3-[4-[2-(7-fluoro-2-methyl-1H-indol-3-yl)ethylamino]-7,8-dihydro-6H-pyrimido[5,4-b][1,4]oxazin-2-yl]-1H-pyridin-2-one; 3-(4-((2-(7-fluoro-2-methyl-1H-indol-3-yl)ethyl)amino)-7,8-dihydro-6H-pyrimido[5,4-b][1,4]oxazin-2-yl)pyridin-2-ol; 3-[4-[2-(5,7-difluoro-2-methyl-1H-indol-3-yl)ethylamino]-7,8-dihydro-6H-pyrimido[5,4-b][1,4]oxazin-2-yl]-1H-pyridin-2-one; 3-(4-((2-(5,7-difluoro-2-methyl-1H-indol-3-yl)ethyl)amino)-7,8-dihydro-6H-pyrimido[5,4-b][1,4]oxazin-2-yl)pyridin-2-ol; 3-(4-((2-(6-(trifluoromethoxy)-1H-indol-3-yl)ethyl)amino)-7,8-dihydro-6H-pyrimido[5,4-b][1,4]oxazin-2-yl)pyridin-2(1H)-one; **3-(4-**((2-(6-(trifluoromethoxy)-1H-indol-3-yl)ethyl)amino)-7,8-dihydro-6H-pyrimido[5,4-b][1,4]oxazin-2-yl)pyridin-2-ol; 3-(4-((2-(1H-indol-3-yl)ethyl)amino)-8-methyl-7,8-dihydro-6H-pyrimido[5,4-b][1,4]oxazin-2-yl)pyridin-2(1H)-one; 3-(4-((2-(1H-indol-3-yl)ethyl)amino)-8-methyl-7,8-dihydro-6H-pyrimido[5,4-b][1,4]oxazin-2-yl)pyridin-2-ol, (S)-3-(4-((2-(1H-indol-3-yl)ethyl)amino)-7-(methoxymethyl)-7,8-dihydro-6H-pyrimido [5,4-b][1,4]oxazin-2-yl)pyridin-2(1H)-one; (S)-3-(4-((2-(1H-indol-3-yl)ethyl)amino)-7-(methoxymethyl)-7,8-dihydro-6H-pyrimido[5,4-b][1,4]oxazin-2-yl)pyridin-2-ol; (7R)-2-(5-fluoro-3-pyridyl)-N-[2-(1H-indol-3-yl)ethyl]-7-(methoxymethyl)-7,8-dihydro-6H-pyrimido[5,4-b][1,4]oxazin-4-amine; (R)-3-(4-((2-(6-fluoro-1H-indol-3-yl)ethyl)amino)-7-(methoxymethyl)-7,8-dihydro-6H-pyrimido[5,4-b][1,4]oxazin-2-yl)pyridin-2(1H)-one; (R)-3-(4-((2-(6-fluoro-1H-indol-3-yl)ethyl)amino)-7-(methoxymethyl)-7,8-dithydro-6H-pyrimido[5,4-b][1,4]oxazin-2-yl)pyridin-2-ol; 3-[(7R)-4-[2-(1H-indol-3-yl)ethoxy]-7-(methoxymethyl)-7,8-dihydro-6H-pyrimido[5,4-b][1,4]oxazin-2-yl]-1H-pyridin-2-one; (R)-3-(4-(2-(1H-indol-3-yl)ethoxy)-7-(methoxymethyl)-7,8-dihydro-6H-pyrimido[5,4-b][1,4]oxazin-2-yl)pyridin-2-ol; (7R)-2-(3,5-difluorophenyl)-N-[2-(1H-indol-3-yl)ethyl]-7-(methoxymethyl)-7,8-dihydro-6H-pyrimido[5,4-b][1,4]oxazin-4-amine, 2-(3-fluoro phenyl)-N-[2-(1H-indol-3-yl)ethyl]-7,8-dihydro-6H-pyrimido[5,4-b][1,4]oxazin-4-amine, 2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amine, 2-(2,4-dimethylthiazol-5-yl)-N-[2-(1H-indol-3-yl)ethyl]-7,8-dihydro-6H-pyrimido[5,4-b][1,4]oxazin-4-amine; (7R)-7-(methoxymethyl)-2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amine; [(7R)-2-(2-methylthiazol-5-yl)-4-[[(3R)-2,3,4,9-tetrahydro-1H-carbazol-3-yl]amino]-7,8-dihydro-6H-pyrimido[5,4-b][1,4]oxazin-7-yl]methanol; N-[2-(1H-indol-3-yl)ethyl]-2-(1-methyltriazol-4-yl)-7,8-dihydro-6H-pyrimido[5,4-b][1,4]oxazin-4-amine, (7R)-2-(4-fluorophenyl)-N-[2-(1H-indol-3-yl)ethyl]-7-(methoxymethyl)-7,8-dihydro-6H-pyrimido[5,4-b][1,4]oxazin-4-amine; (7R)-2-(3-fluorophenyl)-N-[2-(1H-indol-3-yl)ethyl]-7-(methoxymethyl)-7,8-dihydro-6H-pyrimido [5,4-b][1,4]oxazin-4-amine; 5-[4-[2-(1H-indol-3-yl)ethylamino]-7,8-dihydro-6H-pyrimido[5,4b][1,4]oxazin-2-yl]-1-methyl-pyridin-2-one; (7R)-N-[2-(1H-indol-3-yl)ethyl]-7-(methoxymethyl)-2-(2-methylthiazol-5-yl)-7,8-dihydro-6H-pyrimido[5,4-b][1,4]oxazin-4-amine; (7R)-4-[2-(1H-indol-3-yl)ethylamino]-2-(2-methylthiazol-5-yl)-7,8-dihydro-6H-pyrimido[5,4-b][1,4]oxazin-7-yl]methanol; 2-(2-methylthiazol-5-yl)-N-[(4R)-1,3,4,5-tetrahydrobenzo[cd]indol-4-yl]-7,8-dihydro-6 H-pyrimido[5,4-b] [1,4] oxazin-4-amine; 2-(2-methylthiazol-5-yl)-N-[(4S)-1,3,4,5-tetrahydrobenzo[cd]indol-4-yl]-7,8-dihydro-6H-pyrimido[5,4-b][1,4]oxazin-4-amine; 3-[4-[[(3R)-2,3,4,9-tetrahydro-1H-carbazol-3-yl]amino]-7,8-dihydro-6H-pyrimido[5,4-b][1,4]oxazin-2-yl]-1H-pyridin-2-one, (R)-3-(4-((2,3,4,9-tetrahydro-1H-carbazol-3-yl)amino)-7,8-dihydro-6 H-pyrimido[5,4-b][1,4]oxazin-2-yl)pyridin-2-ol; N-[2-(1H-indol-3-yl)ethyl]-2-(2-methoxy-3-pyridyl)-7,8-dihydro-6H-pyrimido[5,4-b][1,4]oxazin-4-amine; 2-(5-fluoro-2-methoxy-3-pyridyl)-N-[(3R)-2,3,4,9-tetrahydro-1H-carbazol-3-yl]-7,8-dihydro-6H-pyrimido[5,4-b][1,4]oxazin-4-amine; 3-(4-((2-(1H-indol-3-yl)ethyl)amino)-7,8-dihydro-6H-pyrimido[5,4-b][1,4]oxazin-2-yl)-5-fluoropyridin-2(1H)-one; 3-(4-((2-(1H-indol-3-yl)ethyl)amino)-7,8-dihydro-6H-pyrimido[5,4-b][1,4]oxazin-2-yl)-5-fluoropyridin-2-ol; 2-(5-fluoro-2-methoxy-3-pyridyl)-N-[2-(1H-indol-3-yl)ethyl]-7,8-dihydro-6H-pyrimido[5,4-b][1,4]oxazin-4-amine; 3-(4-((2-(1H-indol-3-yl)ethyl)amino)-7,8-dihydro-6H-pyrimido[5,4-b][1,4]oxazin-2-yl)-5-methylpyridin-2(1H)-one; 3-(4-((2-(1H-indol-3-yl)ethyl)amino)-7,8-dihydro-6H-pyrimido [5,4-b][1,4]oxazin-2-yl)-5-methylpyridin-2-ol; 3-(4-((2-(1H-indol-3-yl)ethyl)amino)-7,8-dihydro-6H-pyrimido[5,4-b][1,4]oxazin-2-yl)-5-(trifluoromethyl)pyridin-2(1H)-one; 3-(4-((2-(1H-indol-3-yl)ethyl)amino)-7,8-dihydro-6H-pyrimido[5,4-b][1,4]oxazin-2-yl)-5-(trifluoromethyl)pyridin-2-ol; 5-fluoro-3-(4-((2-(6-fluoro-1H-indol-3-yl)ethyl)amino)-7,8-dihydro-6H-pyrimido[5,4-b][1,4]oxazin-2-yl)pyridin-2(1H)-one; 5-fluoro-3-(4-((2-(6-fluoro-1H-indol-3-yl)ethyl)amino)-7,8-dihydro-6H-pyrimido[5,4-b][1,4]oxazin-2-yl)pyridin-2-ol; (R)-S-fluoro-3-(4-((2,3,4,9-tetrahydro-1H-carbazol-3-yl)amino)-7,8-dihydro-6H-pyrimido[5,4-b][1,4]oxazin-2-yl)pyridin-2(1H)-one; (R)-S-fluoro-3-(4-((2,3,4,9-tetrahydro-1H-carbazol-3-yl)amino)-7,8-dihydro-6H-pyrimido[5,4-b][1,4]oxazin-2-yl)pyridin-2-ol; 3-(4-((2-(1H-indol-3-yl)ethyl)amino)-7,8-dihydro-6H-pyrimido[5,4-b][1,4]oxazin-2-yl)-5-chloropyridin-2(1H)-one; 3-(4-((2-(1H-indol-3-yl)ethyl)amino)-7,8-dihydro-6H-pyrimido[5,4-b][1,4]oxazin-2-yl)-5-chloropyridin-2-ol; 3-[4-[2-(1H-indol-3-yl)ethylamino]-7,8-dihydro-6H-pyrimido[5,4-b] [1,4] oxazin-2-yl]-6-methyl- 1H-pyridin-2-one; 3-(4-((2-(1H-indol-3-yl)ethyl)amino)-7,8-dihydro-6H-pyrimido[5,4-b][1,4]oxazin-2-yl)-6-methylpyridin-2-ol, and 2-(2-amino-3-pyridyl)-N-[2-(1H-indol-3-yl)ethyl]-7,8-di hydro-6H-pyrimido[5,4-b][1,4]oxazin-4-amine, or a pharmaceutically acceptable salt thereof.

16. A compound according to any one of claims 1 to 15, wherein the compound is 3-(4-(2-(1H-indol-3-yl)ethoxy)-7,8-dihydro-6H-pyrimido[5,4-b][1,4]oxazin-2-yl)pyridin-2(1H)-one or a pharmaceutically acceptable salt thereof.

17. A pharmaceutical composition comprising a compound according to any one of claims 1 to 16, and a pharmaceutically acceptable excipient, diluent or carrier.

18. A compound according to any one of claims 1 to 16 or a composition according to claim 17, for use in treatment

19. A compound according to any one of claims 1 to 16 or a composition according to claim 17 for use in the treatment of cancer.

20. A method of preparing a compound according to any one of claims 1 to 16, comprising the steps selected from one of the following generic routes:
(a) 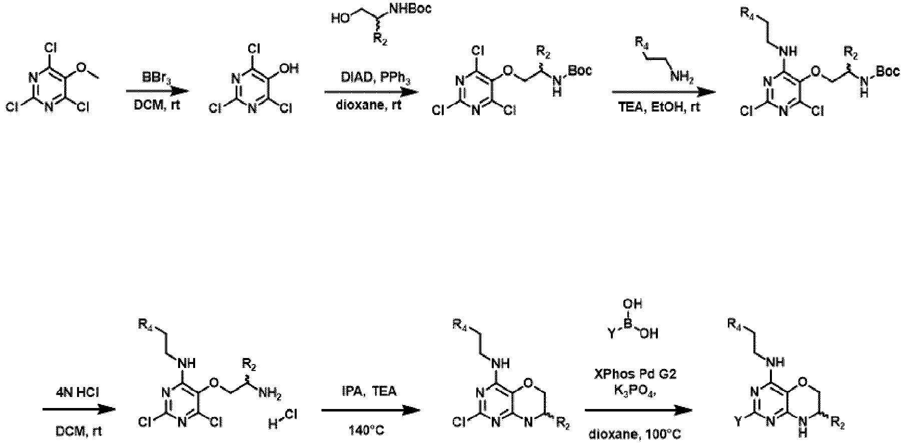 ,wherein R2, R4 and Y are as defined in claim 1 and B is boron;
(b) 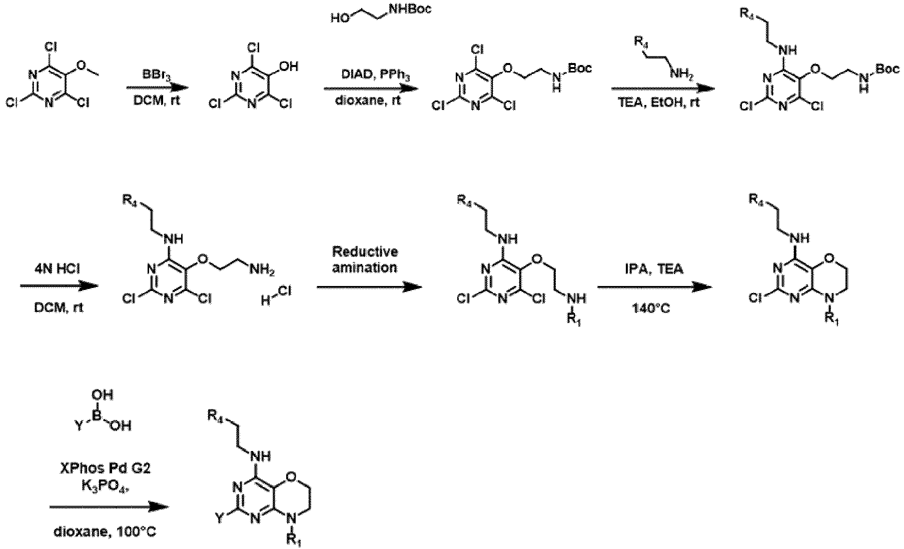 , wherein R₄, R₁ and Y are as defined in claim 1, and B is boron;
(c) 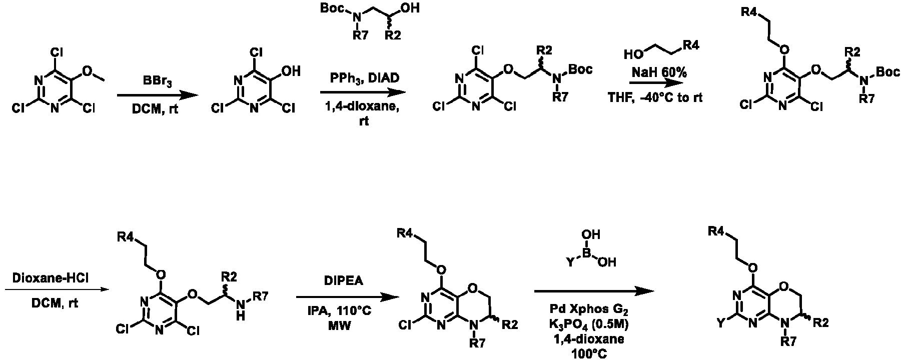 ,wherein R2, R4, R7 and Y are as defined in claim 1, and B is boron;
(d) 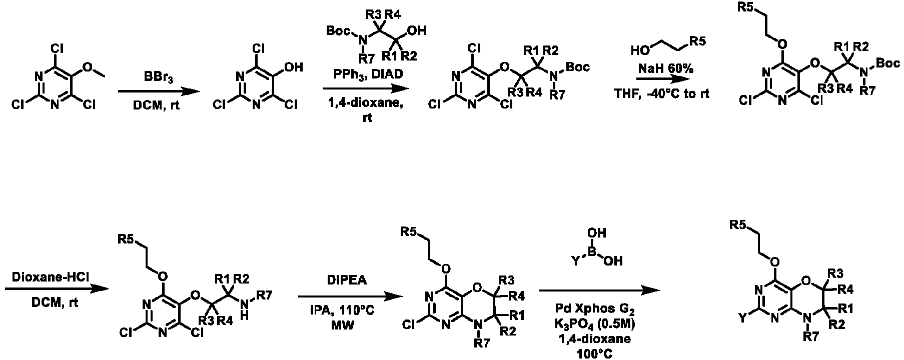 , wherein R1, R2, R3, R4, R5, R7 and Y are as defined in claim 1, and B is boron;
(e) 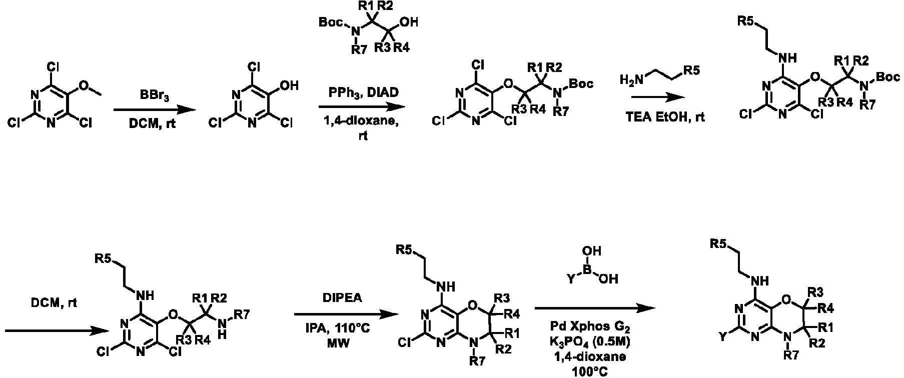 , wherein R1, R2, R3, R4, R5, R7and Y are as defined in claim 1, and B is boron
(f) 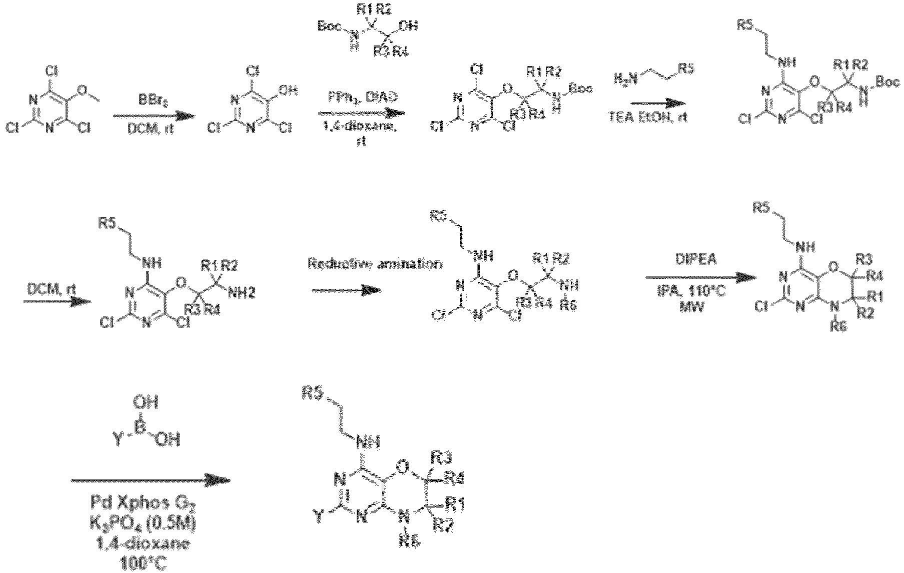 , wherein R1, R2, R3, R4, R5, R6 and Y are as defined in claim 1, and B is boron;
(g) 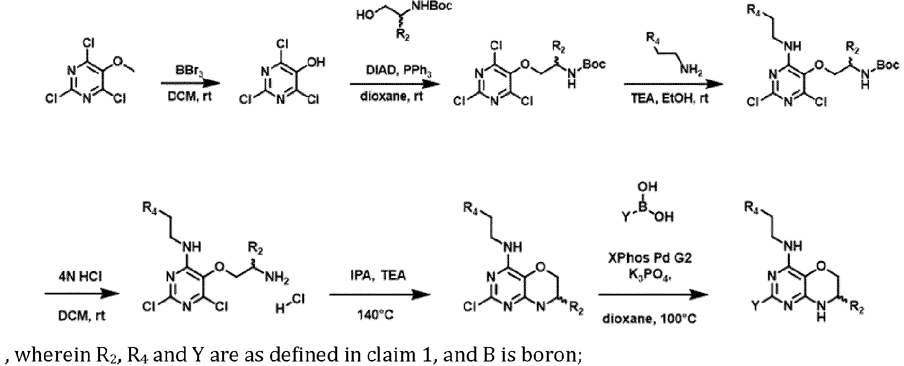
(h) 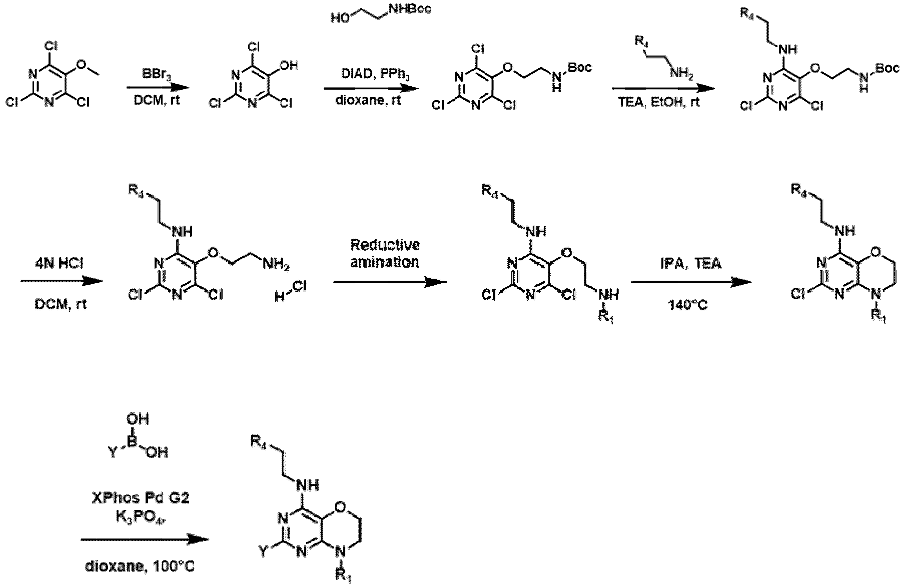 , wherein R₁, R₄, and Y are as defined in claim 1, and B is boron;
(i) 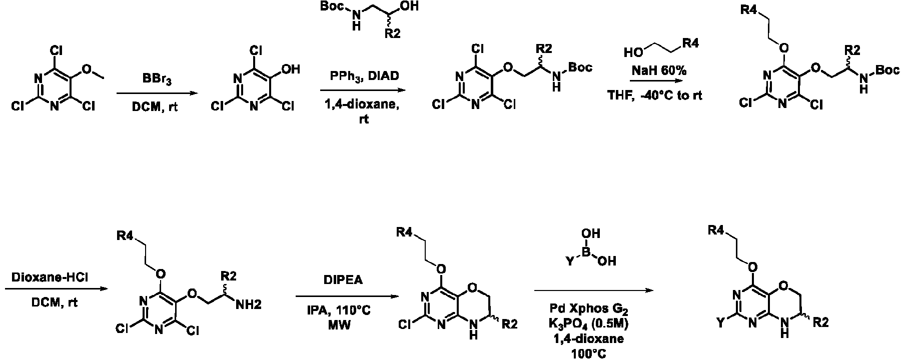 , wherein R₂, R₄, and Y are as defined in claim 1, and B is boron;
(j) 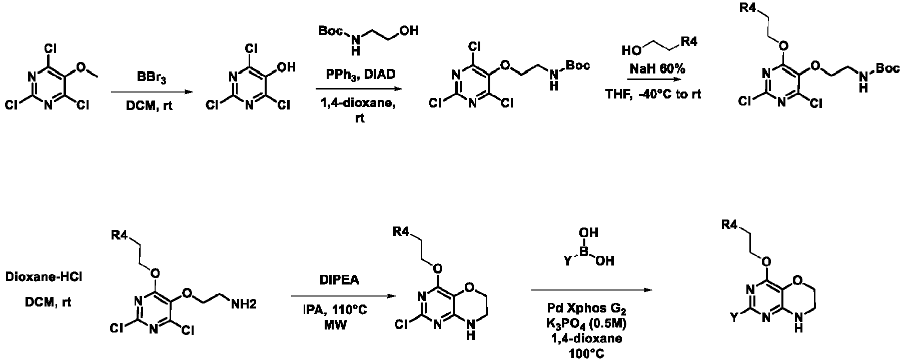 wherein R4 and Y are as defined in claim 1, and B is boron;
(k) 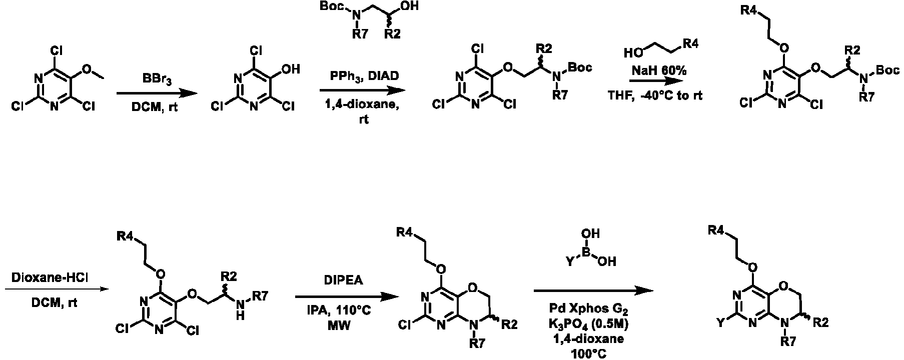 wherein R2, R4, R7 and Y are as defined in claim 1, and B is boron;
(l) 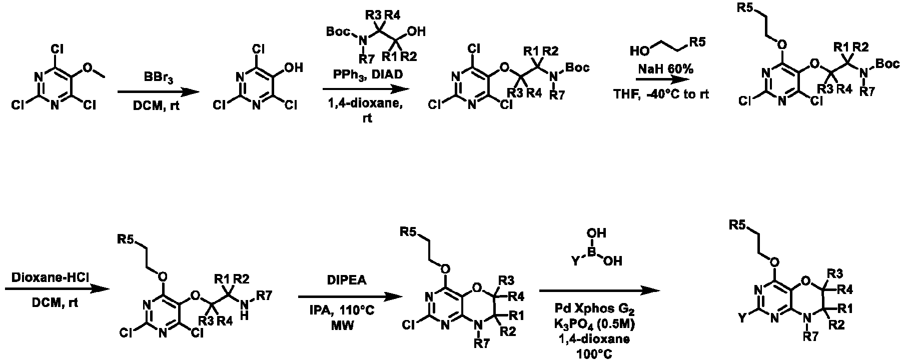 wherein R1, R2, R3, R4, R5, R7 and Y are as defined in claim 1, and B is boron;
(m) 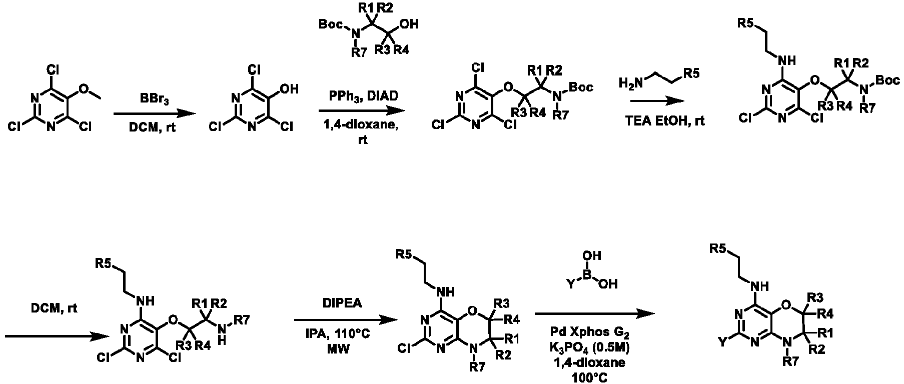 , wherein R1, R2, R3, R4, R5, R7 and Y are as defined in claim 1, and B is boron; OR
(n) 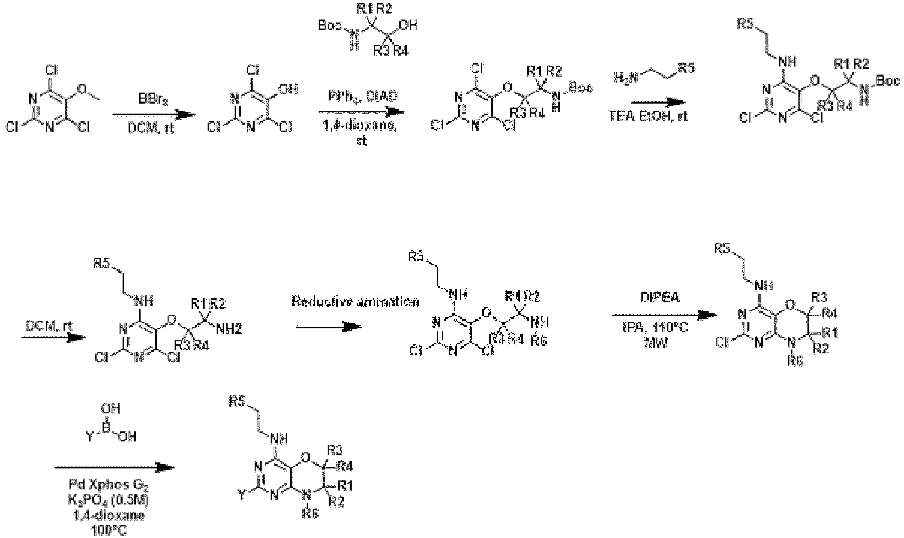 ,wherein R1, R2, R3, R4, R5, R6 and Y are as defined in claim 1, and B is boron.

## Patentansprüche

1. Verbindung der Formel (I) 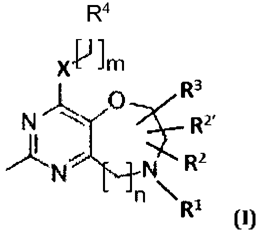 wobei:
Y Phenyl oder ein 3- bis 6-gliedriger Ring ist, der optional 1, 2 oder 3 Heteroatome umfasst, die aus N, O und S ausgewählt sind, wobei das Phenyl oder der Ring mit R⁵ und R⁶ substituiert Ist;
R¹ H, C₁₋₃-Alkyl, (-CH2)pCN, -COC₁₋₃-Alkyl, -CO(CH₂)qNR⁷R⁸, -SO₂C₁-₃-Alkyl, -SO₂NR⁷R⁸, - (CH₂)qPh, C₃₋₅-Cycloalkyl, C₁₋₃-Alkyl, das 1 bis 6 Halogengruppen trägt, C₁₋₃-Alkyl, das eine oder mehrere trägt, die unabhängig voneinander aus OR^{Y} und -NR⁷R⁸ ausgewählt sind, - (CH₂)p'OC₁₋₃ -Alkyl, das mit 1 bis 6 Halogengruppen substituiert ist, -C₀₋₃-AlkylenC(O)C₀₋₃-Alkyl ist, wobei das Alkyl eine Gruppe ist oder mindestens eine Gruppe trägt, die aus OR^{Y} und -NR⁷R⁸, Oxetan, Z, -C(O)(CH₂)qZ, -C(O)O(CH₂)qZ, - C(O)O(CH₂)pPh, -C(O)(CH₂)qZ', - C(O)O(CH₂)qZ' ausgewählt ist;
R² H oder C₁₋₃-Alkyl, C₃₋₅-Cycloalkyl, Halogen und C₁₋₃-Alkyl ist, das optional eine oder mehrere Gruppen trägt, die unabhängig aus OR^{Y}, Halogen und -NR⁷R⁸ ausgewählt sind;
R²' H, C₁₋₃-Alkyl oder Halogen ist;
R³ H oder C₁₋₃-Alkyl ist;
R⁴ ein 9- bis 13-gliedriger Heterocyclus mit mindestens einem Heteroatom, ausgewählt aus N, O und S mit Substituenten R⁹, R⁹' und R¹⁰ ist;
R⁵ H, Oxo, Hydroxy, Halogen, CN, C₁₋₃-Alkyl, C₃₋₅-Cycloalkyl, -OC₁₋₃-Alkyl, -(O)₀₋₁C₁₋₃-Alkyl, das 1 bis 6 Halogengruppen trägt, C₁₋₃-Alkyl, das eine oder mehrere OR^{Y}-Gruppen trägt, -C(O)C₁₋₃-AlkylNR⁷R⁸, -SO₂C₁₋₃-Alkyl, -SO₂ NR⁷R⁸ oder NR⁷R⁸ ist;
R⁶ H, Oxo, Hydroxy, Halogen, CN, C₁₋₃-Alkyl, -C(O)C₁₋₃-AlkylNR⁷R⁸ Ist; -SO₂C₁₋₃-Alkyl oder -SO₂ NR⁷R⁸ ist;
R⁷ H, C₁₋₃-Alkyl oder -C(O)OR^{Y} ist;
R⁸ H oder C₁₋₃-Alkyl ist;
R⁹ H, Hydroxy, Halogen, CN, C₁₋₃-Alkyl, C₁₋₃-Alkoxy, C₁₋₃-Alkyl, das 1 bis 6 Halogengruppen trägt, C₁₋₃-Alkyl, das eine oder mehrere OR^{Y}-Gruppen trägt;
C₃₋₅-Cycloalkyl, -(CH₂)qOC₁₋₃-Alkyl, das mit 1 bis 6 Halogengruppen substituiert ist, -CO C₁₋₃-AlkylNR⁷R⁸, -SO₂C₁₋₃-Alkyl oder -SO₂ NR⁷R⁸ ist;
R⁹' H, Hydroxy, Halogen, CN, C₁₋₃-Alkyl, C₁₋₃-Alkoxy, C₁₋₃-Alkyl, das 1 bis 6 Halogengruppen trägt, C₁₋₃-Alkyl, das eine oder mehrere Hydroxygruppen trägt,-CO (CH₂)qNR⁷R⁸, -SO₂C₁₋₃-Alkyl oder -SO₂ NR⁷R⁸ ist;
R¹⁰ H, Hydroxy, Halogen, CN, C₁₋₃-Alkyl, -C(O)C₁₋₃-Alkyl NR⁷R⁸; -SO₂C₁₋₃-Alkyl oder -SO₂ NR⁷R⁸ ist;
R¹¹ H, C₁₋₃-Alkyl oder C₂-₆-Hydroxyalkyl ist;
R^{Y} H oder C₁₋₃-Alkyl ist;
X CH₂, S,-SO₂, NR¹¹ oder O ist;
Z' ein 5- oder 6-gliedriges Heteroaryl mit mindestens einem Heteroatom, ausgewählt aus N, O und S, wobei das Heteroaryl gegebenenfalls einen, zwei oder drei Substituenten trägt, ausgewählt aus Hydroxy, Halogen, CN, C₁₋₃-Alkyl ist;
Z ein 3- bis 6-gliedriger Ring ist, der gegebenenfalls 1, 2 oder 3 Heteroatome, ausgewählt aus N, O und S, enthält, wobei R⁵ und R⁶ gegebenenfalls einen, zwei oder drei Substituenten, ausgewählt aus Hydroxy, Halogen, CN, C₁₋₃-Alkyl, tragen;
n 0 oder 1 Ist;
n' 0 oder 1 Ist;
m 0, 1, 2 oder 3 ist;
p eine ganze Zahl 1, 2 oder 3 ist;
p' eine ganze Zeit 2 oder 3 Ist;
q 0, 1, 2 oder 3 ist; oder ein pharmazeutisch verträgliches Salz davon.

2. Verbindung der Formel (I) nach Anspruch 1, wobei Y ein 5- oder 6-gliedriger stickstoffhaltiger Ring ist.

3. Verbindung der Formel (I) nach Anspruch 1 oder 2, wobei der Ring unabhängig voneinander aus Pyrimidin, Pyridin, Triazol und Thiazol ausgewählt ist.

4. Verbindung der Formel (I) nach Anspruch 3, wobei sich R⁵ an Position 5 an der Y-Gruppe befindet.

5. Verbindung nach einem der Ansprüche 1 bis 4, wobei n 0 Ist.

6. Verbindung nach Anspruch 1 der Formel (II), wobei X, R¹, R², R³, R⁴ und m wie oben für Verbindungen der Formel (I) definiert sind, oder ein pharmazeutisch verträgliches Salz davon.

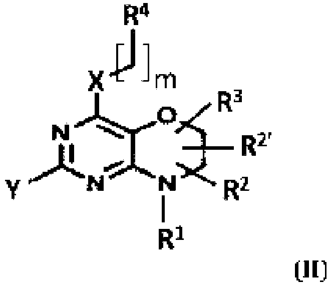

7. Verbindung nach einem der Ansprüche 1 bis 6, wobei R¹ unabhängig ausgewählt ist aus Wasserstoff, -C₁₋₃-Alkyl, Oxetan, -CH₂CH₂OH, -CH₂CH₂N(CH₃)₂ und -SO₂CH₃.

8. Verbindung nach einem der Ansprüche 1 bis 7, wobei R² unabhängig ausgewählt ist aus Wasserstoff, Methyl, -CH₂OCH₃, -CH₂OH, CF₃, -CH₂N(CH₃)₂.

9. Verbindung nach einem der Ansprüche 1 bis 8, wobei R2' Methyl oder Wasserstoff ist.

10. Verbindung nach einem der Ansprüche 1 bis 9, wobei R⁴ ein 9- oder 13-gliedriger teilweise gesättigter oder aromatischer Heterozyklus ist, ausgewählt aus Indolin, Indol, Isoindol, Indolizin, Indazol, Benzimidazol, Azaindol, Pyrazolopyrimidin, Purin, Benzofuran, Isobenzofuran, Benzothiophen, Benzoisoxazol, Benzoisothiazol, Benzoxazol, Benzothiadiazol, Adenin, Guanin, Tetrahydrochinolin, Dihydroisochinolin, Chinolin, Isochinolin, Chinolizin, Chinoxalin, Phthalazin, Cinnolin, Naphthyridin, Pyridopyrimidin, Pyridopyrazin, Pteridin, Chromen, Isochromen, Chromenon, Benzoxazin, Chinolinon, Isochinolinon, Dibenzofuran, Carbazol, Acridin, Phenothiazin, und 2,3,4,9-tetrahydro-1H-carbazol.

11. Verbindung nach einem der Ansprüche 1 bis 10, wobei R⁵ unabhängig aus der Gruppe ausgewählt ist, die Oxo, Methyl, Ethyl, -CF₃, -OCF₃, -OCH₃ und Halogen umfasst.

12. Verbindung nach einem der Ansprüche 1 bis 11, wobei R⁶ unabhängig aus Methyl, Ethyl, - OCH₃ und H ausgewählt Ist.

13. Verbindung nach einem der Ansprüche 1 bis 12, wobei
i. R⁹ unabhängig aus H, Methyl, OCF₃ und -C₃₋₅-Cycloalkyl ausgewählt ist, und/oder
ii. R^{9'} unabhängig aus H, F und Methyl ausgewählt ist, und/oder
iii. R¹⁰ unabhängig aus H, F und Methyl ausgewählt ist.

14. Verbindung nach einem der Ansprüche 1 bis 13, wobei m 2 ist.

15. Verbindung nach einem der Ansprüche 1 bis 14, die unabhängig ausgewählt ist aus:
2-(5-fluoro-3-pyridyl)-N-[2-(1H-indol-3-yl)ethyl]-7,8-dihydro-6H-pyrimido[5,4-b][1,4] oxazin-4-amin; 2-(4-fluorophenyl)-N-[2-(1H-indol-3-yl)ethyl]-7,8-dihydro-6H-pyrimido [5,4-b][1,4]oxazin-4-amin; 2-(3,5-difluorophenyl)-N-[2-(1H-indol-3-yl)ethyl]-7,8-dihydro-6H-pyrimido[5,4-b][1,4]oxazin-4-amin; 2-(5-fluoro-3-pyridyl)-N-[(3R)-2,3,4,9-tetrahydro-1H-carbazol-3-yl]-7,8-dihydro-6H-pyrimido[5,4-b][1,4]oxazin-4-amin; 2-(5-fluoro-3-pyridyl)-N-[2-(1H-indol-3-yl)ethyl]-7-methyl-7,8-dihydro-6H-pyrimido[5,4-b][1,4]oxazin-4-amin; 2-(5-fluoro-3-pyridyl)-N-[2-(1H-indol-3-yl)ethyl]-8-isopropyl-6,7-dihydropyrimido[5,4-b][1,4]oxazin-4-amin; N-[2-(1H-indol-3-yl) ethyl]-2-(2-methyl thiazol-5-yl)-7,8-dihydro-6H-pyrimido[5,4-b][1,4]oxazin-4-amin; (7S)-2-(5-fluoro-3-pyridyl)-N-[2-(1H-indol-3-yl)ethyl-7-methyl-7,8-dihydro-6H-pyrimido[5,4-b][1,4]oxazin-4-amin; (7R)-2-(5-fluoro-3-pyridyl)-N-[2-(1H-Indol-3-yl)ethyl]-7-methyl-7,8-dihydro-6H-pyrimido[5,4-b][1,4]oxazin-4-amin; N-[2-(1H-indol-3-yl)ethyl]-2-(4-methylthiazol-5-yl)-7,8-dihydro-6H-pyrimido[5,4-b][1,4]oxazin-4-amin; 2-[2-(1H-indol-3-yl)ethyl-[2-(2-methylthiazol-5-yl)-7,8-dihydro-6H-pyrimido[5,4-b][1,4]oxazin-4-yl]amino]ethanol; 2-(2-ethyl-4-methyl-thiazol-5-yl)-N-[2-(1H-indol-3-yl)ethyl]-7,8-dihydro-6H-pyrimido[5,4-b][1,4]oxazin-4-amin; N-[(3R)-2,3,4,9-tetrahydro-1H-carbazol-3-yl]-2-thiazol-2-yl-7,8-dihydro-6H-pyrimido[5,4-b][1,4]oxazin-4-amin; 2-(2-Methylthiazol-5-yl)-N-(I,3,4,5-tetrahydrobenzo[cd]indol-4-yl)-7,8-dihydro-6H-pyrimido[5,4-b][1,4]oxazin-4-amin, N-[2-(1H-indol-3-yl)ethyl]-2-(6-methyl-3-pyridyl)-7, 8-dihydro-6H-pyrimido[5,4-b][1,4]oxazin-4-amin; 2-(5-fluoro-6-methyl-3-pyridyl)-N-[2-(1H-indol-3-yl)ethyl]-7,8-dihydro-6H-pyrimido[5,4-b][1,4]oxazin-4-amin; 3-[4-[2-(1H-indol-3-yl)ethylamino]-7,8-dihydro-6H-pyrimido[5,4-b][1,4]oxazin-2-yl]-1H-pyridin-2-on; 3-(4-((2-(1H-indol-3-yl)ethyl)amino)-7,8-dihydro-6H-pyrimido[5,4-b][1,4]oxazin-2-yl)pyridin-2-ol; 5-[4-[2-(1H-indol-3-yl)ethylamino]-7,8-dihydro-6H-pyrimido[5,4-b][1,4]oxazin-2-yl]-1H-pyridin-2-on; 5-(4-[(2-(1H-Indol-3-yl)ethyl)amino)-7,8-dihydro-6H-pyrimido[5,4-b][1,4]oxazin-2-yl)pyridin-2-ol; 3-[4-[2-(1H-Indol-3-yl)ethylamino]-7,8-dihydro-6H-pyrimido[5,4-b][1,4]oxazin-2-yl]-I-methyl-pyridin-2-on; N-[2-(6-fluoro-1H-indol-3-yl)ethyl]-2-(6-methyl-3-pyridyl)-7,8-dihydro-6H-pyrimido[5,4-b][1,4]oxazin-4-amin; 3-[4-[2-(6-fluoro-1H-indol-3-yl)ethylamino]-7,8-dihydro-6H-pyrimido[5,4-b][1,4]oxazin-2-yl]-1H-pyridin-2-on; 3-(4-((2-(6-fluoro-1H-indol-3-yl)ethyl)amino)-7,8-dihydro-6H-pyrimido[5,4-b][1,4]oxazin-2-yl)-pyridin-2-ol; 2-[2-(difluoromethoxy)-3-pyridyl]-N-[2-(1H-indol-3-yl)ethyl]-7,8-dihydro-6H-pyrimido[5,4-b][1,4]oxazin-4-amin; N-[2-(1H-indol-3-yl)ethyl]-2-(2-methoxy-5-methyl-3-pyridyl)-7,8-dihydro-6H-pyrimido[5,4-b][1,4]oxazin-4-amin; 4-[2-(6-fluoro-1H-Indol-3-yl)ethoxy]-2-(2-methylthiazol-5-yl)-7,8-dihydro-6H-pyrimido[5,4-b][1,4]oxazin; 3-(4-(2-(1H-indol-3-yl)ethoxy)-7,8-dihydro-6H-pyrimido[5,4-b][1,4]oxazin-2-yl)pyridin-2(1H)-on; 3-(4-(2-(1H-indol-3-yl)ethoxy)-7,8-dihydro-6H-pyrimido[5,4-b][1,4]oxazin-2-yl)pyridin-2-ol; (R)-3-(4-((2-(1H-indol-3-yl)ethyl)amino)-7-(methoxymethyl)-7,8-dihydro-6H-pyrimido[5,4-b][1,4]oxazin-2-yl)pyridin-2(1H)-on; (R)-3-(4-((2-(1H-indol-3-yl)ethyl)amino)-7-(methoxymethyl)-7,8-dihydro-6H-pyrimido[5,4-b][1,4]oxazin-2-yl)pyridin-2-ol; (R)-3-(4-((2-(1H-indol-3-yl)ethyl)amino)-7-(hydroxymethyl)-7,8-dihydro-6H-pyrimido[5,4-b][1,4]oxazin-2-yl)pyridin-2(1H)-on; (R)-3-(4-((2-(1H-indol-3-yl)ethyl)amino)-7-(hydroxymethyl)-7,8-dihydro-6H-pyrimido[5,4-b][1,4]oxazin-2-yl)pyridin-2-ol; (7S)-2-(5-fluoro-3-pyridyl)-N-[2-(1H-indol-3-yl)ethyl]-7-(methoxy methyl)-7,8-dihydro-6H-pyrimido[5,4-b][1,4]oxazin-4-amin; (6S)-2-(5-fluoro-3-pyridyl)-N-[2-(1H-indol-3-yl)ethyl]-6-methyl-7,8-dihydro-6H-pyrimido[5,4-b][1,4]oxazin-4-amin; (6R)-2-(5-fluoro-3-pyridyl)-N-[2-(1H-indol-3-yl)ethyl]-6-methyl-7,8-dihydro-6H-pyrimido[5,4-b][1,4]oxazin-4-amin; 2-(5-fluoro-3-pyridyl)-N-[2-(1H-Indol-3-yl)ethyl]-7,7-dimethyl-6,8-dihydropyrimido[5,4-b][1,4]oxazin-4-amin; (7R)-2-(5-fluoro-3-pyridyl)-N-[2-(1H-indol-3-yl)ethyl]-7-(trifluoromethyl)-7,8-dihydro-6H-pyrimido[5,4-b][1,4]oxazin-4-amin; N-[2-(1H-indol-3-yl)ethyl]-2-(2-methylthiazol-5-yl)-8-(oxetan-3-yl]-6,7-dihydropyrimido[5,4-b][1,4]oxazin-4-amin; 2-(2-methylthiazol-5-yl)-N-(6,7,8,9-tetrahydro-5H-pyrido[3,2-b]indol-8-yl)-7,8-dihydro-6H-pyrimido[5,4-b][1,4]oxazin-4-amin; (7R)-8-[2-(dimethylamino)ethy]-7-(methoxymethyl)-2-(2-methylthiazol-5-yl)-N-[[3R)-2,3,4,9-tetrahydro-1H-carbazol-3-yl]-6,7-dihydropyrimido[5,4-b][1,4]oxazin-4-amin; (7R)-8-[2-(dimethylamino)ethy]-2-(2-methylthiazol-5-yl)-4-[[(3R)-2,3,4,9-tetrahydro-1H-carbazol-3-yl]amino]-6,7-dihydropyrimido[5,4-b][1,4]oxazin-7-yl]methanol; 2-[2-(2-methylthiazol-5-yl)-4-[[(3R)-2,3,4,9-tetrahydro-1H-carbazol-3-yl]amino]-6,7-dihydropyrimido[5,4-b][1,4]oxazin-8-yl]ethanol; 7-[(dimethylamino)methyl]-2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amin; 8-methylsulfonyl-2-(2-methylthiazol-5-yl)-N-[(3R)-2,3,4,9-tetrahydro-1H-carbazol-3-yl]-6,7-dihydropyrimido[5,4-b][1,4]oxazin-4-amin; 3-(4-((2-(2-chloro-1H-indol-3-yl)ethyl)amino)-7,8-dihydro-6H-pyrimido[5,4-b][1,4]oxazin-2-yl)pyridin-2(1H)-on; 3-(4-((2-(2-Chlor-1H-indol-3-yl)ethyl)amino)-7,8-dihydro-6H-pyrimido[5,4-b][1,4]oxazin-2-yl)pyridin-2-ol; 3-[4-[2-(7-fluoro-2-methyl-1H-indol-3-yl)ehylamino]-7,8-dihydro-6H-pyrimido[5,4-b][1,4]oxazin-2-yl]-1H-pyridin-2-on; 3-(4-((2-(7-Fluor-2-methyl-1H-indol-3-yl)ethyl)amino)-7,8-dihydro-6H-pyrimido[5,4-b][1,4]oxazin-2-yl)pyridin-2-ol; 3-[4-[2-(5,7-difluoro-2-methyl-1H-indol-3-yl)ethylamino]-7,8-dihydro-6H-pyrimido[5,4-b][1,4]oxazin-2-yl]-1H-pyridin-2-on; 3-(4-((2-(5,7-difluoro-2-methyl-1H-indol-3-yl)ethyl)amino)-7,8-dihydro-6H-pyrimido[5,4-b][1,4]oxazin-2-yl)pyridin-2-ol; 3-(4-((2-(6-(trifluoromethoxy)-1H-indol-3-yl)ethyl)amino)-7,8-dihydro-6H-pyrimido[5,4-b][1,4]oxazin-2-yl)pyridin-2(1H)-on; 3-(4-[(2-(6-(trifluoromethoxy)-1H-indol-3-yl)ethyl)amino)-7,8-dihydro-6H-pyrimido [5,4-b][1,4]oxazin-2-yl)pyridin-2-ol; 3-(4-((2-(1H-indol-3-yl)ethyl)amino)-8-methyl-7,8-dihydro-6H-pyrimido[5,4-b][1,4]oxazin-2-yl)pyridin-2(1H)-on; 3-(4-((2-(1H-indol-3-yl)ethyl)amino)-8-methyl-7,8-dihydro-6H-pyrimido[5,4-b][1,4]oxazin-2-yl)pyridin-2-ol, (S)-3-(4-((2-(1H-indol-3-yl)ethyl)amino)-7-(methoxymethyl)-7,8-dihydro-6H-pyrimido[5,4-b][1,4]oxazin-2-yl)pyridin-2(1H)-on; (S)-3-(4-((2-(1H-indol-3-yl)ethyl)amino)-7-(methoxymethyl)-7,8-dihydro-6H-pyrimido[5,4][1,4]oxazin-2-yl)pyridin-2-ol; (7R)-2-(5-fluor-3-pyridyl)-N-[2-(1H-indol-3-yl)ethyl-7-(methoxymethyl)-7,8-dihydro-6H-pyrimido[5,4-b][1,4]oxazin-4-amin; (R)-3-(4-((2-(6-Fluor-1H-indol-3-yl)ethyl)amino)-7-[methoxymethyl)-7,8-dihydro-6H-pyrimido[5,4-b][1,4]oxazin-2-yl)pyridin-2(1H)-on; (R)-3-(4-((2-(6-Fluor-1H-indol-3-yl)ethyl)amino)-7-(methoxymethyl)-7,8-dihydro-6H-pyrimido[5,4-b][1,4]oxazin-2-yl)pyridin-2-ol; 3-[(7R)-4-[2-(1H-indol-3-yl)ethoxy]-7-[methoxymethyl)-7,8-dihydro-6H-pyrimido[5,4-b][1,4]oxazin-2-yl]-1H-pyridin-2-on; (R)-3-(4-(2-(1H-indol-3-yl)ethoxy)-7-(methoxymethyl)-7,8-dihydro-6H-pyrimido[5,4-b][1,4]oxazin-2-yl)pyridin-2-ol; (7R)-2-(3,5-Difluorphenyl)-N-[2-(1H-indol-3-yl)ethyl]-7-(methoxymethyl)-7,8-dihydro-6H pyrimido[5,4-b][1,4]oxazin-4-amin; 2-(3-fluoro phenyl)-N-[2-(1H-indol-3-yl)ethyl]-7,8-dihydro-6H-pyrimido[5,4-b][1,4]oxazin-4-amin; 2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amin; 2-(2,4-dimethylthlazol-5-yl)-N-[2-(1H-indol-3-yl)ethyl]-7,8-dihydro-6H-pyrimido[5,4-b][1,4]oxazin-4-amin; (7R)-7-(methoxymethyl)-2-(2-methylthiazol-5-yl)-N-[(3R)-2,3,4,9-tetrahydro-1H-carbazol-3-yl]-7,8-dihydro-6H-pyrimido[5,4-b][1,4]oxazin-4-amin; [(7R)-2-(2-methylthiazol-5-yl)-4-[[(3R)-2,3,4,9-tetrahydro-1H-carbazol-3-yl]amino]-7,8-dihydro-6H-pyrimido[5,4-b][1,4]oxazin-7-yl]methanol; N-[2-(1H-indol-3-yl)ethyl]-2-(I-methyltriazol-4-yl)-7,8-dihydro-6H-pyrimido[5,4-b][1,4]oxazin-4-amin; (7R)-2-(4-fluorophenyl)-N-[2-(1H-indol-3-yl)ethyl]-7-(methoxymethyl)-7,8-dihydro-6H-pyrimido[5,4-b][1,4]oxazin-4-amin; (7R)-2-(3-Fluorphenyl)-N-[2-(1H-indol-3-yl)ethyl]-7-(methoxymethyl)-7,8-dihydro-6H-pyrimido [5,4-b][1,4]oxazin-4-amin; 5-[4-[2-(1H-indol-3-yl)ethylamino]-7,8-dihydro-6H-pyrimido[5,4-b][1,4]oxazin-2-yl]-I-methyl-pyridin-2-on; (7R)-N-[2-(1H-indol-3-yl)ethyl]-7-(methoxymethyl)-2-(2-methylthiazol-5-yl)-7,8-dihydro-6H-pyrimido[5,4-b][1,4]oxazin-4-amin; (7R)-4-[2-(1H-indol-3-yl)ethylamino]-2-(2-methylthiazol-5-yl)-7,8-dihydro-6H-pyrimido[5,4-b][1,4]oxazin-7-yl]methanol; 2-(2-methylthiazol-5-yl)-N-[(4R)-I,3,4,5-tetrahydrobenzo[cd]indol-4-yl]-7,8-dihydro-6H-pyrimido[5,4-b][1,4]oxazin-4-amin; **2-**(2-methylthiazol-5-yl)-N-[(4S)-I,3,4,5-tetrahydrobenzo[cd]indol-4-yl]-7,8-dihydro-6H-pyrimido[5,4-b][1,4]oxazin-4-amin; 3-[4-[(3R)-2,3,4,9-tetrahydro-1H-carbazol-3-yl]amino]-7,8-dihydro-6H-pyrimido[5,4-b][1,4]oxazin-2-yl]-1H-pyridin-2-on, (R)-3-(4-((2,3,4,9-tetrahydro-1H-carbazol-3-yl)amino)-7,8-dihydro-6H-pyrimido [5,4-b][1,4]oxazin-2-yl)pyridin-2-ol; N-[2-(1H-indol-3-yl)ethyl]-2-(2-methoxy-3-pyridyl)-7,8-dihydro-6H-pyrimido[5,4-b][1,4]oxazin-4-amin; 2-(5-fluoro-2-methoxy-3-pyridyl)-N-[(3R)-2,3,4,9-tetrahydro-1H-carbazol-3-yl]-7,8-dihydro-6H-pyrimido[5,4-b][1,4]oxazin-4-amin; 3-(4-((2-(1H-indol-3-yl)ethyl)amino)-7,8-dihydro-6H-pyrimido[5,4-b][1,4]oxazin-2-yl)-5-fluoropyridin-2(1H)-on; 3-(4-((2-(1H-indol-3-yl)ethyl)amino)-7,8-dihydro-6H-pyrimido[5,4-b][1,4]oxazin-2-yl)-5-fluoropyridin-2-ol; 2-(5-fluoro-2-methoxy-3-pyridyl)-N-[2-(1H-indol-3-yl)ethyl]-7,8-dihydro-6H-pyrimido[5,4-b][1,4]oxazin-4-amin; 3-(4-((2-[1H-indol-3-yl)ethyl)amino)-7,8-dihydro-6H-pyrimido[5,4-b][1,4]oxazin-2-yl)-5-methylpyridin-2(1H)-on; 3-(4-((2-(1H-Indol-3-yl)ethyl)amino)-7,8-dihydro-6H-pyrimido [5,4-b][1,4]oxazin-2-yl)-5-methylpyridin-2-ol; 3-(4-((2-[1H-indol-3-yl)ethyl)amino)-7,8-dihydro-6H-pyrimido[5,4-b][1,4]oxazin-2-yl)-5-(trifluormethyl)pyridin-2 (1H)-on; 3-(4-((2-(1H-indol-3-yl)ethyl]amino]-7,8-dihydro-6H-pyrimido[5,4-b][1,4]oxazin-2-yl]-5-[trifluoromethyl]pyridin-2-ol; 5-fluoro-3-(4-((2-(6-fluoro-1H-indol-3-yl]ethyl]amino]-7,8-dihydro-6H-pyrimido[5,4-b][1,4]oxazin-2-yl]pyridin-2(1H)-on; 5-fluoro-3-(4-((2-(6-fluor-1H-indol-3-yl)ethyl)amino)-7,8-dihydro-6H-pyrimido[5,4-b][1,4]oxazin-2-yl]pyridin-2-ol; (R)-5-fluor-3-(4-((2,3,4,9-tetrahydro-1H-carbazol-3-yl]amino]-7,8-dihydro-6H-pyrimido[5,4-b][1,4]oxazin-2-yl]pyridin-2(1H)-on; (R)-5-fluoro-3-(4-[(2,3,4,9-tetrahydro-1H-carbazol-3-yl]amino]-7,8-dihydro-6H-pyrimido[5,4-b][1,4]oxazin-2-yl)pyridin-2-ol; 3-(4-((2-(1H-indol-3-yl)ethyl]amino]-7,8-dihydro-6H-pyrimido[5,4-b][1,4]oxazin-2-yl)-5-chloropyridin-2(1H)-on; 3-(4-((2-[1H-indol-3-yl)ethyl]amino]-7,8-dihydro-6H-pyrimido[5,4-b][1,4]oxazin-2-yl)-5-chloropyridin-2-ol; 3-[4-[2-(1H-indol-3-yl)ethylamino]-7,8-dihydro-6H-pyrimido[5,4-b][1,4]oxazin-2-yl]-6-methyl-1H-pyridin-2-on; 3-(4-((2-(1H-indol-3-yl)ethyl]amino]-7,8-dihydro-6H-pyrimido[5,4-b][1,4]oxazin-2-yl)-6-methylpyridin-2-ol und 2-(2-amino-3-pyridyl)-N-[2-(1H-indol-3-yl)ethyl]-7,8-dihydro-6H-pyrimido[5,4-b][1,4]oxazin-4-amin oder ein pharmazeutisch verträgliches Salz davon.

16. Verbindung nach einem der Ansprüche 1 bis 15, wobei die Verbindung 3-(4-(2-(1H-indol-3-yl)ethoxy)-7,8-dihydro-6H-pyrimido[5,4-b][1,4]oxazin-2-yl)pyridin-2(1H)-on oder ein pharmazeutisch verträgliches Salz davon ist.

17. Pharmazeutische Zusammensetzung, umfassend eine Verbindung nach einem der Ansprüche 1 bis 16 und einen Hilfsstoff, ein Verdünnungsmittel oder einen Träger, die pharmazeutisch verträglich sind.

18. Verbindung nach einem der Ansprüche 1 bis 16 oder Zusammensetzung nach Anspruch 17 zur Verwendung bei der Behandlung.

19. Verbindung nach einem der Ansprüche 1 bis 16 oder Zusammensetzung nach Anspruch 17 zur Verwendung bei der Behandlung von Krebs.

20. Verfahren zum Herstellen einer Verbindung nach einem der Ansprüche 1 bis 16, umfassend die Schritte, die aus einem der folgenden generischen Verfahrenswegen ausgewählt sind:
(a) 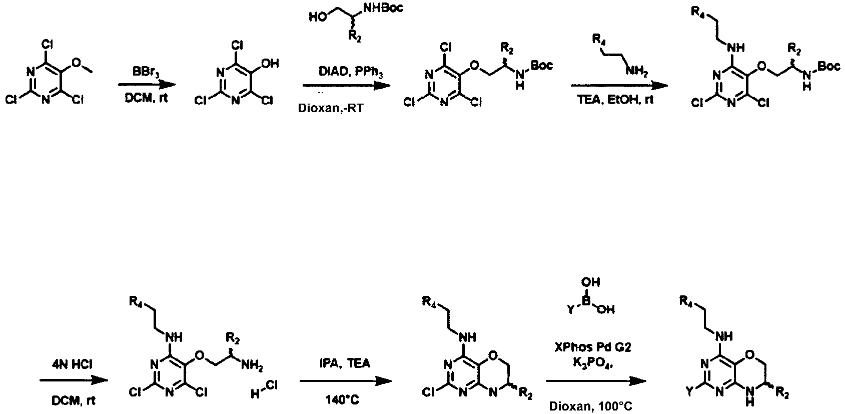 , wobei R2, R4 und Y wie in Anspruch 1 definiert sind und B Bor ist;
(b) 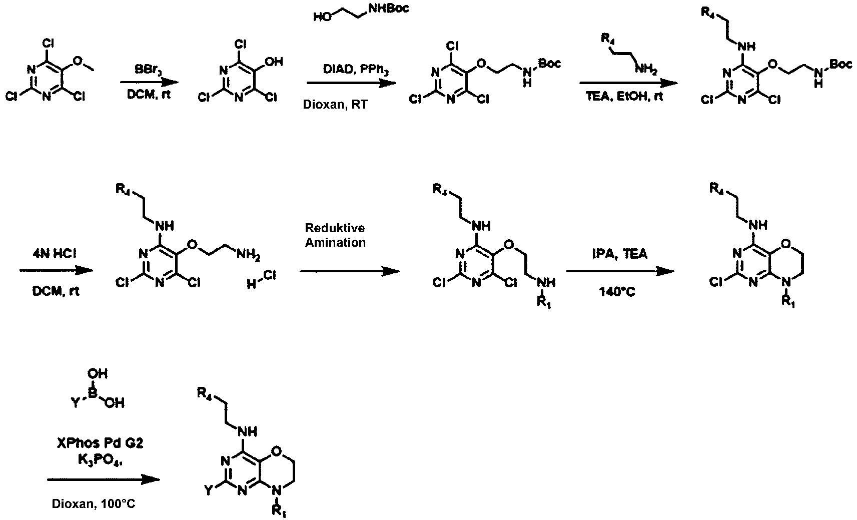 , wobei R₄, R₁ und Y wie in Anspruch 1 definiert sind und B Bor ist;
(c) 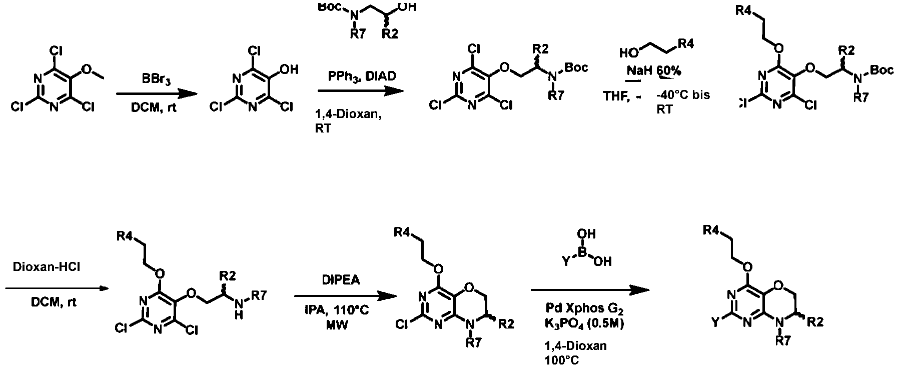 , wobei R2, R4, R7 und Y wie in Anspruch 1 definiert sind und B Bor ist;
(d) 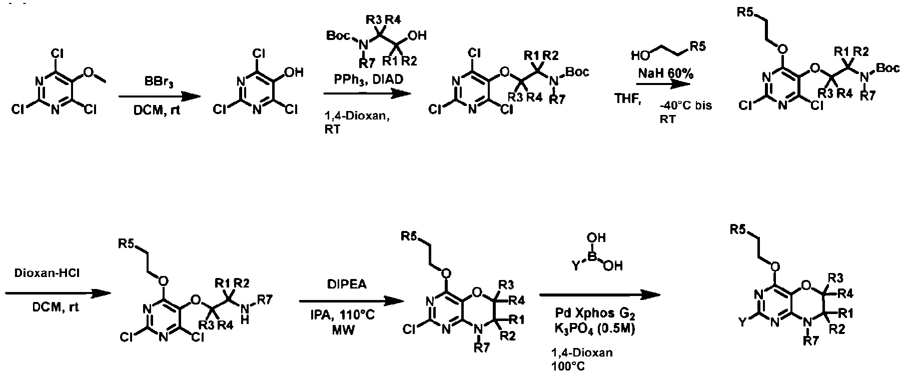 , wobei R1, R2, R3, R4, R5, R7 und Y wie In Anspruch 1 definiert sind und B Bor Ist;
(e) 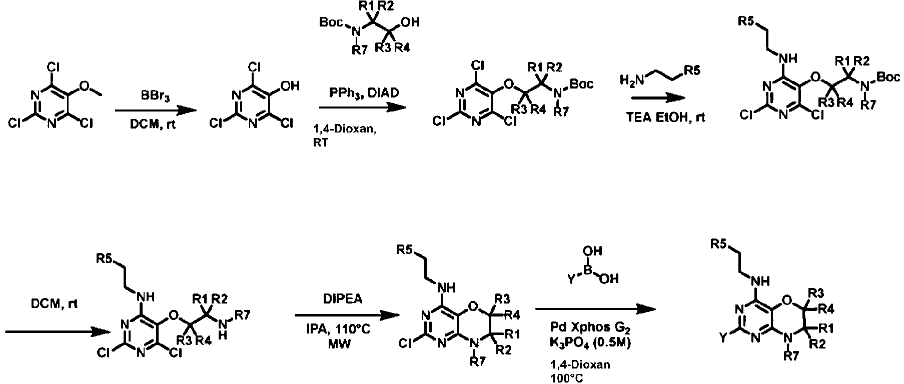 , wobei R1, R2, R3, R4, R5, R7 und Y wie in Anspruch 1 definiert sind und B Bor Ist;
(f) 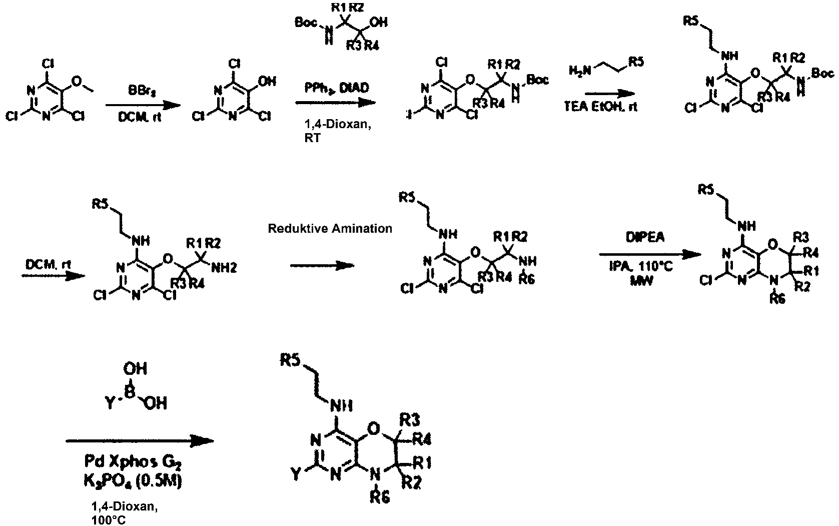 , wobei R1, R2, R3, R4, R5, R6 und Y wie in Anspruch 1 definiert sind und B Bor ist;
(g) 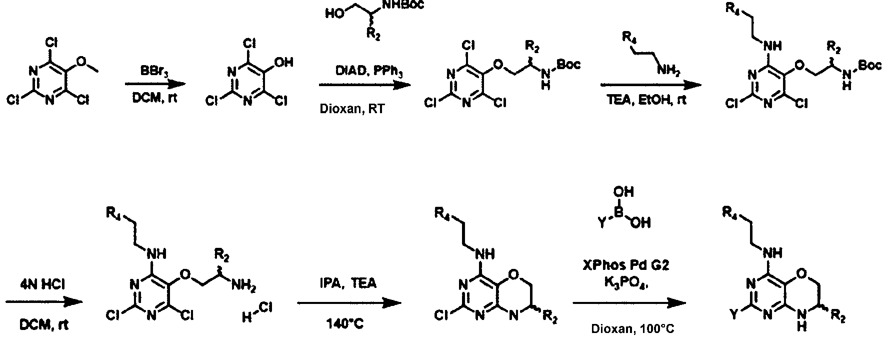 , wobei R₂, R₄ und Y wie in Anspruch 1 definiert sind und B Bor Ist;
(h) 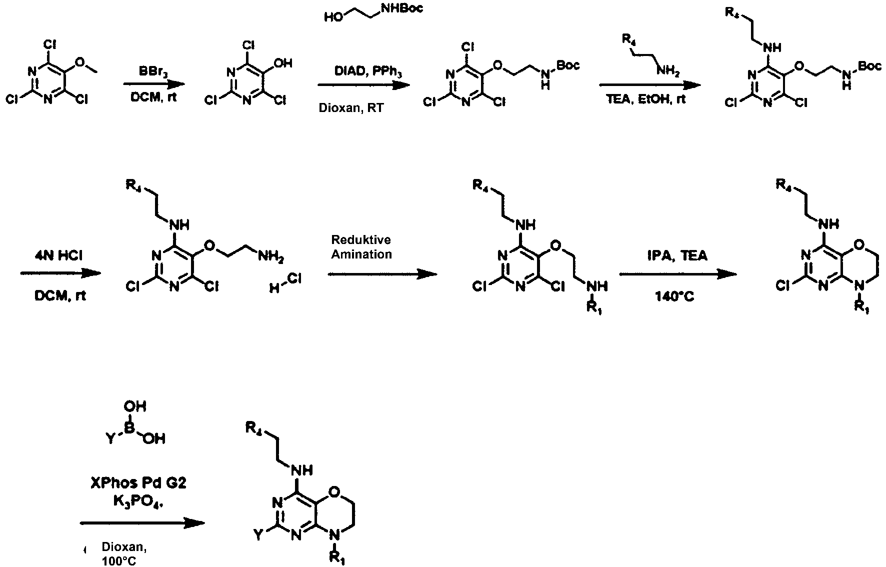 , wobei R₁, R₄ und Y wie in Anspruch 1 definiert sind und B Bor ist;
(i) 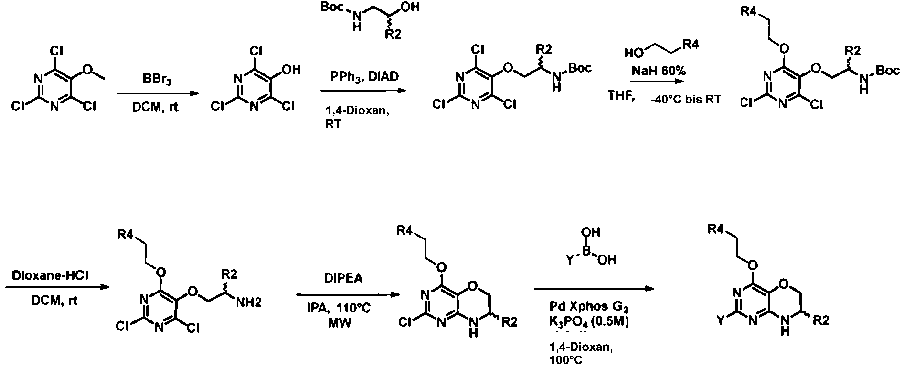 , wobei R₂, R₄ und Y wie in Anspruch 1 definiert sind und B Bor ist;
(j) 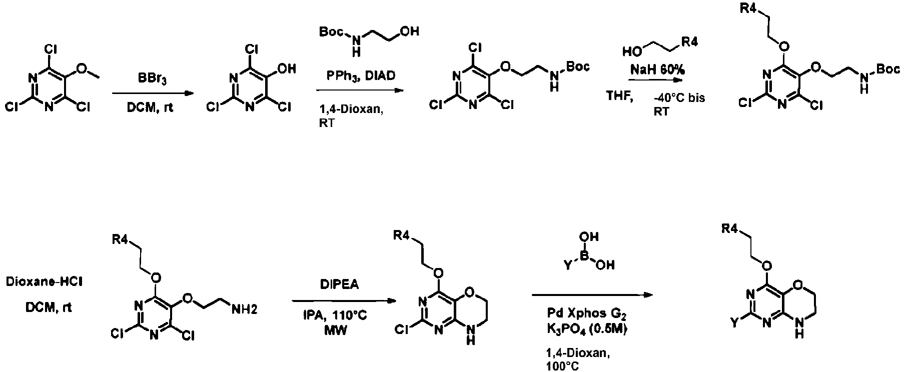 , wobei R4 und Y wie in Anspruch 1 definiert sind und B Bor ist;
(k) 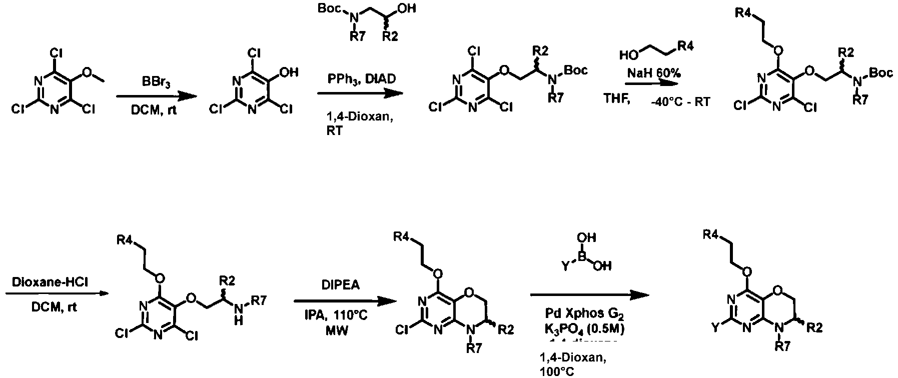 , wobei R2, R4, R7 und Y wie in Anspruch 1 definiert sind und B Bor ist;
(l) 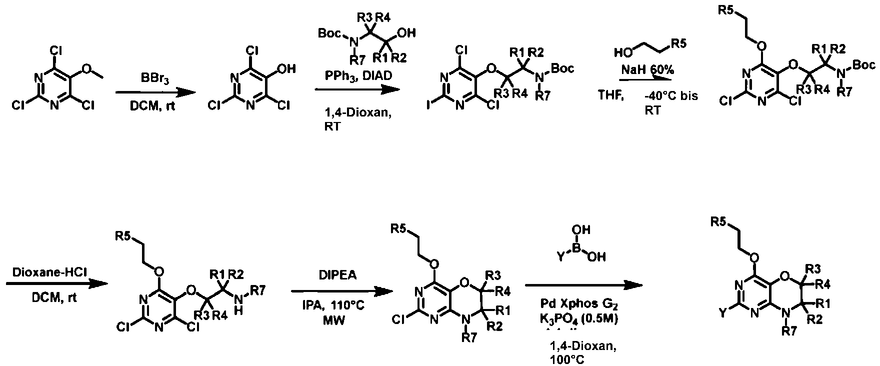 **,** wobei R1, R2, R3, R4, R5, R7 und Y wie in Anspruch 1 definiert sind und B Bor ist;
(m) 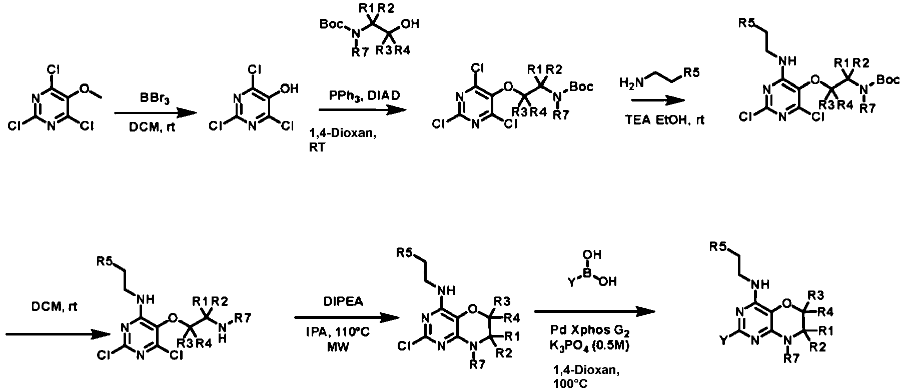 , wobei R1, R2, R3, R4, R5, R7 und Y wie in Anspruch 1 definiert sind und B Bor ist; ODER
(n) 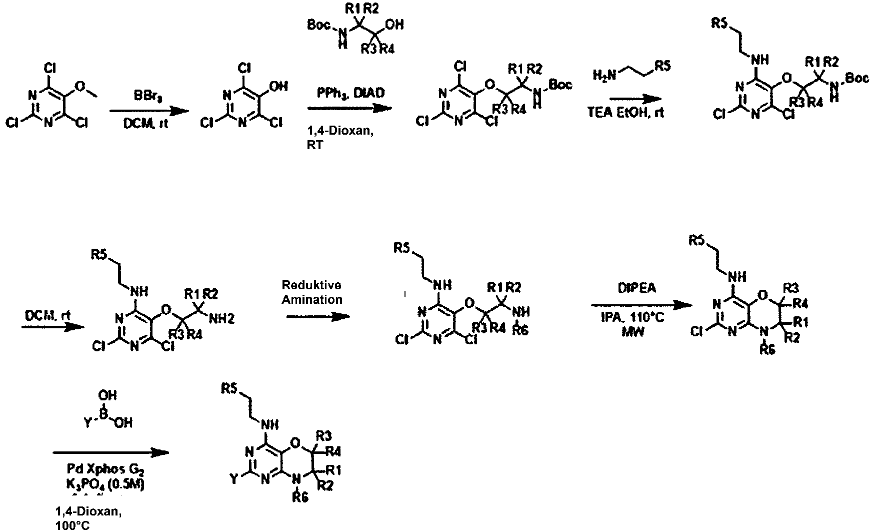 , wobei R1, R2, R3, R4, R5, R6 und Y wie in Anspruch 1 definiert sind und B Bor ist.

## Revendications

1. Composé de formule (**I**) 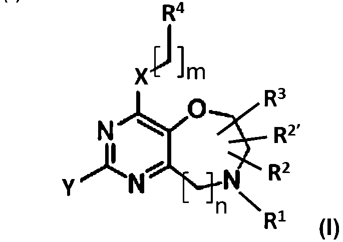 dans laquelle :
Y représente un groupe phényle ou un noyau tri- à hexagonal comprenant facultativement 1, 2 ou 3 hétéroatomes choisis parmi N, O et S, ledit groupe phényle ou noyau étant substitué par R⁵ et R⁶ ;
R¹ représente H, un groupe alkyle en C₁ à C₃, (-CH₂)pCN, -CO-alkyle en C₁ à C₃, -CO(CH₂)qNR⁷R⁸, -SO₂-alkyle en C₁ à C₃, -SO₂NR⁷R⁸, -(CH₂)qPh. cycloalkyle en C₃ à C₅, alkyle en C₁ à C₃ portant 1 à 6 groupes halogène, alkyle en C₁ à C₃ portant un ou plusieurs groupes indépendamment choisis parmi OR^{Y} et -NR⁷R⁸, -(CH₂)p'O-alkyle en C₁ à C₃ substitué par 1 à 6 groupes halogène, -alkylène en C₀ à C₃-CO-alkyle en C₀ à C₃ dans lequel le groupe alkyle représente ou porte au moins un groupe choisi parmi OR^{Y} et -NR⁷R⁸, oxétane, Z, -C(O)(CH₂)qZ, -C(O)O(CH₂)qZ, -C(O)O(CH₂)pPh, -C(O)(CH₂)qZ', -C(O)O(CH₂)qZ' ;
R² représente H ou un groupe alkyle en C₁ à C₃, cycloalkyle en C₃ à C₅, halogène et alkyle en C₁ à C₃ portant facultativement un ou plusieurs groupes indépendamment choisis parmi OR^{Y}, halogène et -NR⁷R⁸;
R^{2'} représente H, un groupe alkyle en C₁ à C₃ ou halogène ;
R³ représente H ou un groupe alkyle en C₁ à C₃ ;
R⁴ représente un hétérocycle ennéa- à tridécagonal contenant au moins un hétéroatome choisi parmi N, O et S, avec des substituants R⁹, R^{9'} et R¹⁰ ;
R⁵ représente H, un groupe oxo, hydroxy, halogène, CN, alkyle en C₁ à C₃, cycloalkyle en C₃ à C₅, -O-alkyle en C₁ à C₃, -(O)₀₋₁alkyle en C₁ à C₃ portant 1 à 6 groupes halogène, alkyle en C₁ à C₃ portant un ou plusieurs groupes OR^{Y}, -C(O)-alkyle en C₁ à C₃NR⁷R⁸, -SO₂alkyle en C₁ à C₃, -SO₂NR⁷R⁸ ou NR⁷R⁸ ;
R⁶ représente H, un groupe oxo, hydroxy, halogène, CN, alkyle en C₁ à C₃, -C(O)alkyle en C₁ à C₃NR⁷R⁸, -SO₂alkyle en C₁ à C₃ ou -SO₂ NR⁷R⁸,
R⁷ représente H, un groupe alkyle en C₁ à C₃ ou C(O)OR^{Y} ;
R⁸ représente H ou un groupe alkyle en C₁ à C₃ ;
R⁹ représente H, un groupe hydroxy, halogène, CN, alkyle en C₁ à C₃, alkoxy en C₁ à C₃, alkyle en C₁ à C₃ portant 1 à 6 groupes halogène, alkyle en C₁ à C₃ portant un ou plusieurs groupes OR^{Y}, cycloalkyle en C₃ à C₅, -(CH₂)qOalkyle en C₁ à C₃ substitué par 1 à 6 groupes halogène, -CO-alkyle en C₁ à C₃NR⁷R⁸, -SO₂alkyle en C₁ à C₃ ou -SO₂NR⁷R⁸;
R^{9'} représente H, un groupe hydroxy, halogène, CN, alkyle en C₁ à C₃, alkoxy en C₁ à C₃, alkyle en C₁ à C₃ portant 1 à 6 groupes halogène, alkyle en C₁ à C₃ portant un ou plusieurs groupes hydroxy, -CO(CH₂)qNR⁷R⁸, -SO₂alkyle en C₁ à C₃ ou -SO₂NR⁷R⁸ ;
R¹⁰ représente H, un groupe hydroxy, halogène, CN, alkyle en C₁ à C₃, -C(O)alkyie en C₁ à C₃NR⁷R⁸ ; -SO₂alkyle en C₁ à C₃ ou -SO₂NR⁷R⁸ ;
R^{11'} représente H, un groupe alkyle en C₁ à C₃ ou hydroxyalkyle en C₂ à C₆ ;
R^{Y} représente H ou un groupe alkyle en C₁ à C₃ ;
X représente un groupe CH₂, S, -SO₂, NR¹¹ ou O ;
*Z* représente un groupe hétéroaryle penta- ou hexagonal avec au moins un hétéroatome choisi parmi N, O et S, ledit groupe hétéroaryle portant facultativement un, deux ou trois substituants choisis parmi les groupes hydroxy, halogène, CN, alkyle en C₁ à C₃ ;
Z représente un noyau tri- à hexagonal comprenant facultativement 1, 2 ou 3 hétéroatomes choisi parmi N, O et S portant R⁵ et R⁶ porte facultativement un, deux ou trois substituants choisis parmi les groupes hydroxy, halogène, CN, alkyle en C₁ à C₃ ;
n vaut 0 à 1 ;
n' vaut 0 à 1 ;
m vaut 0, 1, 2 ou 3 ;
p est un nombre entier 1, 2 ou 3 ;
p' est un nombre entier 2 ou 3 ;
q vaut 0, 1, 2 ou 3 ;
ou un de ses sels pharmaceutiquement acceptables.

2. Composé de formule (I) selon la revendication 1, dans lequel Y est un noyau azoté penta- ou hexagonal.

3. Composé de formule (I) selon la revendication 1 ou 2, dans lequel le noyau est choisi indépendamment parmi les groupes pyrimidine, pyridine, triazole et thiazole.

4. Composé de formule (I) selon la revendication 3, dans lequel R⁵ est situé en position 5 sur le groupe Y.

5. Composé selon l'une quelconque des revendications 1 à 4, dans lequel n vaut 0.

6. Composé selon la revendication 1, de formule **(II) :** 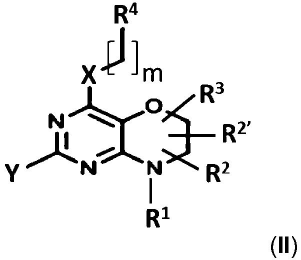 dans laquelle X, R¹, R², R³, R⁴ et m sont définis ci-dessus pour les composés de formule **(I)** ou un de ses sels pharmaceutiquement acceptables.

7. Composé selon l'une quelconque des revendications 1 à 6, dans lequel R' est indépendamment choisi parmi l'hydrogène, un groupe alkyle en C₁ à C₃, oxétane, -CH₂CH₂OH, -CH₂CH₂N(CH₃)₂ et -SO₂CH₃.

8. Composé selon l'une quelconque des revendications 1 à 7, dans lequel R² est indépendamment choisi parmi H, un groupe méthyle, -CH₂OCH₃, -CH₂OH, CF₃, -CH₂N(CH₃)₂.

9. Composé selon l'une quelconque des revendications 1 à 8, dans lequel R^{2'} représente un groupe méthyle ou H.

10. Composé selon l'une quelconque des revendications 1 à 9, dans lequel R⁴ représente un hétérocycle partiellement saturé ou aromatique ennéa- à tridécagonal choisi parmi l'indoline, l'indole, l'isoindole, l'indolizine, l'indazole, le benzlmldazole, l'azaindole, la pyrazolopyrimidine, la purine, le benzofurane, l'isobenzofurane, le benzothiophène, le benzoisoxazole, le benzoisothiazole, le benzoxazole, le benzothladlazole, l'adénine, la guanine, la tétrahydroquinoléine, la dihydroisoquinoléine, la quinoléine, l'isoquinoléine, la quinolizine, la quinoxaline, la phtalazine, la cinnoline, la naphthyridine, la pyridopyrimidine, la pyridopyrazine, la ptéridine, le chromène, l'isochromène, la chroménone, la benzoxazine, la quinolinone, l'isoquinolinone, le dibenzofuranne, le carbazole, l'acridine, la phénothiazine, et le 2,3,4,9-tétrahydro-1H-carbazole.

11. Composé selon l'une quelconque des revendications 1 à 10, dans lequel R⁵ est Indépendamment choisi dans le groupe consistant en un groupe oxo, méthyle, éthyle, -CF₃, -OCF₃, -OCH₃ et halogène.

12. Composé selon l'une quelconque des revendications 1 à 11, dans lequel R⁶ est choisi indépendamment parmi un groupe méthyle, éthyl, -OCH₃ et H.

13. Composé selon l'une quelconque des revendications 1 à 12, dans lequel :
(i) R⁹ est Indépendamment choisi parmi H, un groupe méthyle, OCF₃ et -cycloalkyle en C₃ à Cs, et/ou
(ii) R^{9'} est indépendamment choisi parmi H, F et un groupe méthyle, et/ou
(iii) R¹⁰ est Indépendamment choisi parmi H, F et un groupe méthyle.

14. Composé selon l'une quelconque des revendications 1 à 13, dans lequel m vaut 2.

15. Composé selon l'une quelconque des revendications 1 à 14, Indépendamment choisi parmi :
la 2-(5-fluoro-3-pyridyl)-N-[2-(1H-indole-3-yl)éthyl}-7,8-dihydro-6H-pyrimido[5,4-b][1,4]-oxazine-4-amine ; la 2-(4-fluorophényl)-N-{2-(1H-indole-3-yl)éthyl]-7,8-dihydro-6H-pyrimido[5,4-b][1,4]oxazine-4-amine ; la 2-(3,5-difluorophényl)}-N-2-(1H-indole-3-yl)éthyl]-7,8-dihydro-6H-pyrimido[5,½][1,4]oxazine-4-amine ; la 2-(5-fluoro-3-pyridyl)-N-[(3R)-2,3,4,9-tétrahydro-1H-carbazole-3-yi]-7,8-dihydro-6H-pyrimido[5,4-b][1,4]oxazine-4-amine ; la 2-(5-fluoro-3-pyridyl)-N-[2-(1H-indole-3-yl)éthyl]-7-méthyl-7,8-dlhydro-6H-pyrimido[5,4-b][1,4]oxazine4-amine ; la 2-(5-fluoro-3-pyridyl)-N-[2-(1H-indole-3-yl)éthyl]-8-isopropyl-6,7-dihydropyrimido-[5,4-b][1,4]oxazine-4-amine ; la N-[2-(1H-indole-3-yl) éthyl]-2-(2-méthylthlazole-5-yl)-7,8-dihydro-8H-pyrimido[5,4-b][1,4]oxazine-4-amine ; la (7S)-2-(5-fluoro-3-pyridyl)-N-[2-(1H-indole-3-yl)éthyl-7-méthyl-7,8-dihydro-6H-pyrimido[5,4-b][1,4]oxazine-4-amine ; la (7R)-2-(5-fluoro-3-pyridyl)-N-[2-(1H-Indole-3-yl)éthyl]-7-méthyl-7,8-dihydro-6H-pyrimido[5,4-b][1,4]oxazine-4-amine ; la N-[2-(1H-indole-3-yl)éthyl}-2-(4-méthyithiazole-5-yl)-7,8-dihydro-6H-pyrimido[5,4-b][1,4]oxazine-4-amine ; le 2-[2-(1H-indole-3-yl)éthyl-[2-(2-méthyithiazole-5-y1)-7,8-dihydro-6H-pyrimido[5,4-b][1,4]oxazine-4-yl]amino]éthanol ; la 2-(2-éthyl-4-méthyl-thiazole-5-yl)-N-2-(1H-indole-3-yl)éthyl]-7,8-dihydro-6H-pyrimido[5,4-b][1,4]oxazine-4-amine ; la N-[(3R)-2,3,4,9-tétrahydro-1H-carbazole-3-yi]-2-thiazole-2-yl-7,8-dihydro-6H-pyrimido[5,4-b][1,4]oxazine4-amine ; la 2-(2-méthylthiazole-5-yl)-N-(1,3,4,5-tétrahydrobenzo[cd]indole-4-yl)-7,8-dihydro-6H-pyrimidol5,4-b][1,4]oxazine-4-amine, la N-[2-1H-indole-3-yl)éthyl]-2-(6-méthyl-3-pyridyl)-7,8-dihydro-6H-pyrimido[5,4-b][1,4]oxazine-4-amine ; la 2-(5-fluoro-6-méthyl-3-pyridyl)-N-[2-(1H-indole-3-yl)éthyl]7,8-dihydro-6H-pyrimido-[5,4-b][1,4]oxazine-4-amine ; la 3-[4-[2-(1H-indole-3-yl)éthylamino]-7,8-dihydro-6H-pyrimido[5,4-b][1,4]oxazine-2-yl-1H-pyridine-2-one ; le 3-(4-((2(1H-indole-3-yl)éthyl) amino)-7,8-dihydro-6H-pyrimido[5,4-b][1,4]oxazine-2-yl)pyridine-2-ol ; la 5-[4-[2-(1H-indole-3-yl)éthylamino]-7,8-dihydro-6H-pyrimido[5,4-b][1,4]oxazine-2-yl-1H-pyridine-2-one ; le 5-(4-((2-(1H-indole-3-yl)éthyl)amino)-7,8-dihydro-6H-pyrimido[5,4-b][1,4]oxazine-2-yl)pyridine-2-ol ; la 3-[4-[2-(1H-indole-3-yl)éthylamino]-7,8-dihydro-6H-pyrimido[5,4-b][1,4]oxazine-2-yl]-1-méthyl-pyridine-2-one ; la N-[2-(6-fluoro-1H-indole-3-yl)éthyl]-2-(6-méthyl-3-pyridyl)-7,8-dihydro-6H-pyrimido[5,4-b][1,4]oxazine-4-amine ; la 3-[4-[2-(6-fluoro-1H-indole-3-yl) éthylamino]-7,8-dihydro-6H-pyrimido[5,4-b][1,4]oxazine-2-yl]-1H-pyridine-2-one ; le 3-(4-((2-(6-fluoro-1H-indole-3-yl)éthyl)-amino)-7,8-dihydro-6H-pyrimido[5,4-b][1,4]oxazine-2-yl)pyridine-2-ol ; la 2-[2-(difluoromethoxy)-3-pyridyg-N-[2-(1½Indole-3-yl)óthyg-7,8-dihydro-6H-pyrimido[5,½][1,4]oxazine-4-amine ; la N-[2-(1H-indole-3-yl)éthyl]-2-(2-méthoxy-5-méthyl-3-pyridyl)-7,8-dihydro-6H-pyrimido[5,4-b][1,4]-oxazine-4-amine ; la 4-[2-(6-fluoro-1H-indole-3-yl)éthoxy]-2-2-méthyithiazole-5-yl)-7,8-dihydro-6H-pyrimido[5,4-b][1,4]oxazine ; la 3-(4-(2-(1H-indole-3-yl)éthoxy)-7,8-dihydro-6H-pyrimido[5,4-b][1,4]oxazine-2-yl)pyridine-2(1H)-one ; le 3-(4-(2-(1H-indole-3-yl)éthoxy)-7,8-dihydro-6H-pyrimido[5,4-b][1,4]oxazine-2-yl)pyridine-2-ol ; la (R)-3-(4-((2-(1H-indole-3-yl)éthyl)amino)-7-(méthoxyméthyl)-7,8-dihydro-6H-pyrimido[5,4-b][1,4]oxazine-2-yl)pyridine-2(1H)-one ; le (R)-3-(4-((2-1H-indole-3-yl)éthyl)amino)-7-(méthoxyméthyl)-7,8-dihydro-6H-pyrimido[5,4-b][1,4] oxazine-2-yl)pyridine-2-ol ; la (R)-3-4((2-(1H-indole-3-yl)éthyl)amino)-7-(hydroxyméthyl)-7,8-dihydro-6H-pyrimido[5,4-b][1,4]oxazine-2-yl)pyridine-2(1H)-one; le (R)-3-(4-((2-(1H-indole-3-yl)éthyl)amino)-7-(hydroxyméthyl)-7,8-dihydro-6H-pyrimido[5,4-b][1,4]oxazine-2-yl)pyridine-2-ol ; la (7S)-2-(5-fluoro-3-pyridyl)-N-[2-(1H-indole-3-yl)éthyl]-7-(méthoxyméthyl)-7,8-dihydro-6H-pyrimido[5,4-b][1,4]oxazine-4-amine ; la (6S)-2-(5-fluoro-3-pyrldyl)-N-[2-(1H-indole-3-yl)éthyl]-6-méthyl-7,8-dihydro-6H-pyrimido[5,4-b][1,4]oxazine-4-amine ; la (6R)-2-(5-fluoro-3-pyridyl)-N-[2-(1H-indole-3-yl)éthyl]-6-méthyl-7,8-dihydro-6H-pyrimido[5,4-b][1,4]oxazine-4-amine ; la 2-(5-fluoro-3-pyridyl)-N-[2-(1H-indole-3-yl)éthyl]-7,7-diméthyl-6,8-dihydropyrimido[5,4-b][1,4]oxazine-4-amine ; la (7R)-2-(5-fluoro-3-pyridyl)-N-[2-(1H-indole-3-yl)éthyl]-7-(trifluorométhyl)-7,8-dihydro-6H-pyrimido[5,4-b][1,4]oxazine-4-amine ; la N-[2-(1H-indole-3-yl)éthyl]-2-(2-méthylthiazole-5-yl)-8-(oxétane-3-yl)-6,7-dihydropyrimido[5,4-b][1,4]oxazine-4-amine ; la 2-2-méthylthiazole-5-yl)-N-(6,7,8,9-tétrahydro-5H-pyrido[3,2-b]indole-8-yl)-7,8-dihydro-6H-pyrimido[5,4-b][1,4]oxazine-4-amine ; la (7R)-8-[2-(diméthylamino)éthyl]-7-(méthoxyméthyl)-2-(2-méthyithiazole-5-yl)-N-[(3R)-2,3,4,9-tétrahydro-1H-carbazole-3-yl]-6,7-dihydropyrimido[5,4-b][1,4]oxazine-4-amine ; le (7R)-8-[2-(diméthylamino)éthyl]-2-(2-méthylthiazole-5-yl)-4[(3R)-2,3,4,9-tétrahydro-1H-carbazole-3-yl]amino]-6,7-dihydropyrimido[5,4][1,4]oxazine-7-yl]methanol ; le 2-[2-(2-méthylthlazole-5-yl)-4[[(3R)-2,3,4,9-tétrahydro-1H-carbazole-3-yl]amino]-6,7-dihydropyrimidol[5,4-b][1,4]oxazine-8-yl]ethanol; la 7-[(diméthylamino)méthyi]-2-(2-méthylthiazole-5-yl)-N-[(3R)-2,3,4,9-tétrahydro-1H-carbazole-3-yl]-7,8-dihydro-6H-pyrimido[5,4-b][1,4]oxazine-4-amine ; la 8-méthylsulfonyl-2-(2-méthylthiazole-5-yl)-N-[(3R)-2,3,4,9-tétrahydro-1H-carbazole-3-yl]-6,7-dihydropyrimido[5,4-b]-[1,4]oxazine-4-amine ; la 3-(4-((2-(2-chioro-1H-indole-3-yl)éthyl)amino)-7,8-dihydro-6H-pyrimido[5,4-b][1,4]oxazine-2-yi)pyridine-2(1H)-one ; le 3-(4-((2-(2-chioro-1H-indole-3-yl)éthyl)-amino)-7,8-dihydro-6H-pyrimido[5,4-b][1,4]oxazine-2-yl)pyridine-2-ol ; la 3-[4-[2-(7-fluoro-2-méthyl-1H-indole-3-yl)éthylamino]-7,8-dihydro-6H-pyrimido[5,4-b][1,4]oxazine-2-yl]-1H-pyridine-2-one ; le 3-(4-((2-(7-fluoro-2-méthyl-1H-indole-3-yl)éthyl)amino)-7,8-dihydro-6H-pyrimido[5,4-b][1,4]oxazine-2-yl)pyridine-2-ol ; la 3[4-[2-(5,7-difluoro-2-méthyl-1H-indole-3-yl)éthylamino]-7,8-dihydro-6H-pyrimido[5,4-b][1,4]oxazine-2-yl-1H-pyridine-2-one ; le 34-((2-(5,7-difluoro-2-méthyl-1H-indole-3-yl)éthyl)amino)-7,8-dihydro-6H-pyrimido[5,4-b][1,4]oxazine-2-yl)pyridine-2-ol ; la 3-(4((2-(6-(trifluorométhoxy)-1H-indole-3-yl)éthyl)amino)-7,8-dihydro-6H-pyrimido[5,4-b][1,4]oxazine-2-yl)pyridine-2(1H)-one ; le 3(4-((2-(6-(trifluorométhoxy)-1H-indole-3-yl)éthyl)-amino)-7,8-dihydro-6H-pyrimido[5,4-b][1,4]oxazine-2-yl)pyridine-2-ol ; la 3-(4-((2-(1H-indole-3-yl)éthyl)amino)-8-méthyl-7,8-dihydro-6H-pyrimido[5,4-b][1,4]oxazine-2-yl)pyridine-2(1H)-one ; le 3-(4-((2-(1H-indole-3-yl)éthyl)amino)-8-méthyl-7,8-dihydro-6H-pyrimido[5,4-b][1,4]oxazine-2-yi)-pyridine-2-ol, la (S)-3-(4-((2-(1H-indole-3-yl)éthyl)amino)-7-(méthoxyméthyl)-7,8-dihydro-6H-pyrimido[5,4-b][1,4]oxazine-2-yl)pyridine-2(1H)-one; le (S)-3-(4-((2-(1H-indole-3-yl)éthyl)-amino)-7-(méthoxyméthyl)-7,8-dihydro-6H-pyrimido[5,4-b][1,4]oxazine-2-yl)pyridine-2-ol ; la (7R)-2-(5-fluoro-3-pyridyl)-N-[2-(1H-indole-3-yl)éthyl]-7-(méthoxyméthyl)-7,8-dihydro-6H-pyrimido-[5,4-b][1,4]oxazine-4-amine ; la (R)-3-(4-((2-6-fluoro-1H-indole-3-yl)éthyl)amino)-7-(méthoxy-méthyl)-7,8-dihydro-6H-pyrimido[5,4-b][1,4]oxazine-2-yl)pyridine-2(1H)-one ; le (R)-3-(4-((2-(6-fluoro-1H-indole-3-yl)éthyl)amino)-7-(méthoxyméthyl)-7,8-dihydro-6H-pyrimido[5,4-b][1,4]-oxazine-2-yl)pyridine-2-ol ; la 3-[(7R)-4-[2-(1H-indole-3-yl)éthoxy]-7-(méthoxyméthyl)-7,8-dihydro-6H-pyrimido[5,4][1,4]oxazine-2-yl-1H-pyridine-2-one ; le (R)-3-(4-(2-(1H-lndole-3-yl)-éthoxy)-7-(méthoxyméthyl)-7,8-dihydro-6H-pyrimido[5,4-b][1,4]oxazine-2-yl)pyridine-2-ol ; la (7R)-2-(3,5-difluorophényl)-N-[2-(1H-indole-3-yl)éthyl]-7-(méthoxyméthyl)-7,8-dihydro-6H-pyrimido[5,4-b][1,4]oxazine-4-amine ; la 2-(3-fluorophenyl)-N-[2-(1H-indole-3-yl)éthyl]-7,8-dihydro-6H-pyrimido[5,4-b][1,4]oxazine-4-amine ; la 2-(2-méthylthiazole-5-yl)-N-[(3R)-2,3,4.9-tétrahydro-1H-carbazole-3-yl]-7,8-dihydro-6H-pyrimido[5,4-b][1,4]oxazine-4-amine ; la 2-(2,4-diméthylthiazole-5-yl)-N-[2-(1H-indole-3-yl)éthyl]-7,8-dihydro-6H-pyrimido[5,4-b][1,4]oxazine-4-amine ; la (7R)-7-(méthoxyméthyl)-2-(2-méthylthiazole-5-yl)-N[(3R)-2,3,4,9-tétrahydro-1H-carbazole-3-yl]-7,8-dihydro-6H-pyrimido[5,4-b][1,4]oxazine-4-amine ; la [(7R)-2-(2-méthyl-thiazole-5-yl)-4-[[(3R)-2,3,4,9-tetrahydro-1H-carbazole-3-yl]amino]-7,8-dihydro-6H-pyrimido[5,4-b][1,4]oxazine-7-yl]methanol ; la N-[2-(1H-indole-3-yl)éthyl]-2-(1-methyltriazole-4-yl)-7,8-dihydro-6H-pyrimido[5,4-b][1,4]oxazine-4-amine ; la (7R)-2-(4-fluorophényl)-N-[2-(1H-indole-3-yl)éthyl]-7-(methoxymethyl)-7,8-dihydro-6H-pyrimido[5,4][1,4]oxazine-4-amine ; la (7R)-2-(3-fluoro-phényl)-N-[2-(1H-indole-3-yl)éthyl]-7-(méthoxyméthyl)-7,8-dihydro-6H-pyrimido[5,4-b][1,4]oxazine-4-amine ; la 5-[4-[2-(1H-indole-3-yl)éthylamino]-7,8-dihydro-6H-pyrimido[5,4-b][1,4]oxazine-2-yl]-1-méthyl-pyridine-2-one ; la (7R)-N-[2-(1H-indole-3-yl)éthyl]-7-(méthoxyméthyl)-2-2-méthylthiazole-5-yl)-7,8-dihydro-6H-pyrimido[5,4-b][1,4]oxazine-4-amine ; le (7R)-4-[2-(1H-indole-3-yl)éthylamino]-2-(2-méthyithiazole-5-yl)-7,8-dihydro-6H-pyrimido[5,4-b][1,4]oxazine-7-yl]methanol ; la 2-(2-méthylthiazole-5-yl)-N-[(4R)-1,3,4,5-tétrahydrobenzo[cd]lndole-4-yl]-7.8-dihydro-6H-pyrimido[5,4-b][1,4]oxazine-4-amine ; la 2-(2-méthyithiazole-5-yl)-N-[(4S)-1,3,4,5-tétrahydrobenzo[cd]indole-4-yl]-7,8-dihydro-6H-pyrimido[5,4-b][1,4]oxazine-4-amine ; la 3-[4-[[(3R)-2,3,4,9-tétrahydro-1H-carbazole-3-yl]amino]-7,8-dihydro-6H-pyrimido[5,4-b][1,4]oxazine-2-yl]-1H-pyridine-2-one, le (R)-3-(4-((2,3,4,9-tétrahydro-1H-carbazole-3-yl)amino)-7,8-dihydro-6H-pyrimido[5,4-b][1,4]oxazine-2-yl)pyridine-2-ol ; la N-[2-(1H-indole-3-yl)éthyl]-2-(2-méthoxy-3-pyridyl)-7,8-dihydro-6H-pyrimido[5,4-b][1,4]oxazine-4-amine ; la 2-(5-fluoro-2-méthoxy-3-pyridyl)-N-[(3R)-2,3,4,9-tétrahydro-1H-carbazole-3-yl]-7,8-dihydro-6H-pyrimido[5,4-b][1,4]oxazine-4-amine ; la 3(4((2-(1H-indole-3-yl)éthyl)amino)-7,8-dihydro-6H-pyrimido[5,4-b][1,4]oxazine-2-yl)-5-fluoropyridine-2(1H)-one ; le 3-(4-((2(1H-indole-3-yl)éthyl)amino)-7,8-dihydro-6H-pyrimido[5,4-b][1,4]oxazine-2-yl)-5-fluoropyridine-2-ol ; la 2-(5-fluoro-2-méthoxy-3-pyridyl)-N-[2-(1H-indole-3-yl)ethyl]-7,8-dihydro-6H-pyrimido[5,4-b][1,4]oxazine-4-amine ; la 3-(4-((2-(1H-indole-3-yl)éthyl)amino)-7,8-dihydro-6H-pyrimido[5,4-b][1,4]oxazine-2-yl)-5-méthyipyridine-2(1H)-one ; le 3-(4-((2-(1H-indole-3-yl)éthyl)amino)-7,8-dihydro-6H-pyrimido[5,4-b][1,4]oxazine-2-yl)-5-méthylpyridine-2-ol ; la 3-(4-((2-(1H-indole-3-yl)éthyl)amino)-7,8-dihydro-6H-pyrimido[5,4-b][1,4]oxazine-2-yl)-5-(trifluorométhyl)pyridine-2(1H)-one ; le 3-(4-((2-1H-indole-3-yl)éthyl)-amino)-7,8-dihydro-6H-pyrimido[5,4-b][1,4]oxazine-2-yl)-5-(trifluorométhyl)pyridine-2-ol; la 5-fluoro-3-(4-((2-(6-fluoro-1H-indole-3-yl)éthyl)amino)-7,8-dihydro-6H-pyrimido[5,4-b][1,4]oxazine-2-yl)pyridine-2(1H)-one ; le 5-fluoro-3(4-((2-(6-fluoro-1H-indole-3-yl)éthyl)amino)-7,8-dihydro-6H-pyrimido[5,4-b][1,4]oxazine-2-yl)pyridine-2-ol; la (R)-5-fluoro-3-(4-((2,3,4,9-tétrahydro-1H-carbazole-3-yl)amino)-7,8-dihydro-6H-pyrimido[5,4-b][1,4]oxazine-2-yl)pyridine-2(1H)-one ; le (R)-5-fluoro-3(4-((2,3,4,9-tétrahydro-1H-carbazole-3-yl)amino)-7,8-dihydro-6H-pyrimido[5,4-b]-[1,4]oxazine-2-yl)pyridine-2-ol ; la 3-(4-((2-(1H-indole-3-yl)éthyl)amino)-7,8-dihydro-6H-pyrimido[5,4-b][1,4]oxazine-2-yl)-5-chloropyridine-2(1H)-one ; le 3-(4-((2-(1H-indole-3-yl)éthyl)-amino)-7,8-dihydro-6H-pyrimido[5,4-b][1,4]oxazine-2-yl)-5-chioropyridine-2-ol ; la 3-[4-[2-(1H-indole-3-yl)éthylamino]-7,8-dihydro-6H-pyrimido[5,4-b][1,4]oxazine-2-yl]-6-méthyl-1H-pyridine-2-one ; le 3-(4-((2-(1H-indole-3-yl)éthyl)amino)-7,8-dihydro-6H-pyrimido[5,4-b][1,4]oxazine-2-yl)-6-méthylpyridine-2-ol et la 2-(2-amino-3-pyridyl)-N-2-(1H-indole-3-yl)éthyl]-7,8-dihydro-6H-pyrimido[5,4-b][1,4]oxazine-4-amine, ou un de leurs sels pharmaceutiquement acceptables.

16. Composé selon l'une quelconque des revendications 1 à 15, le composé étant la 3-(4-(2-(1H-indole-3-yl)éthoxy)-7,8-dihydro-6H-pyrimido[5,4-b][1,4]oxazine-2-yl)pyridine-2(1H)-one ou un de ses sels pharmaceutiquement acceptables.

17. Composition pharmaceutique comprenant un composé selon l'une quelconque des revendications 1 à 16, et un excipient, diluent ou support pharmaceutiquement acceptables.

18. Composé selon l'une quelconque des revendications 1 à 16 ou composition selon la revendication 17, pour une utilisation en traitement.

19. Composé selon l'une quelconque des revendications 1 à 16 ou composition selon la revendication 17, pour une utilisation dans le traitement du cancer.

20. Procédé de préparation d'un composé selon l'une quelconque des revendications 1 à 16, comprenant les étapes choisies parmi l'une des voies génériques suivantes :
(a) 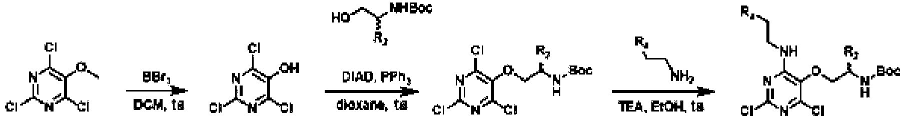 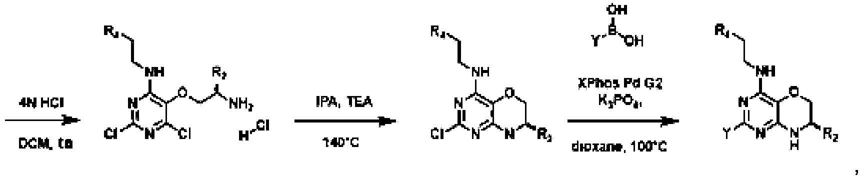 dans laquelle R2, R4 et Y sont tels que définis dans la revendication 1 et B représente le bore ;
(b) 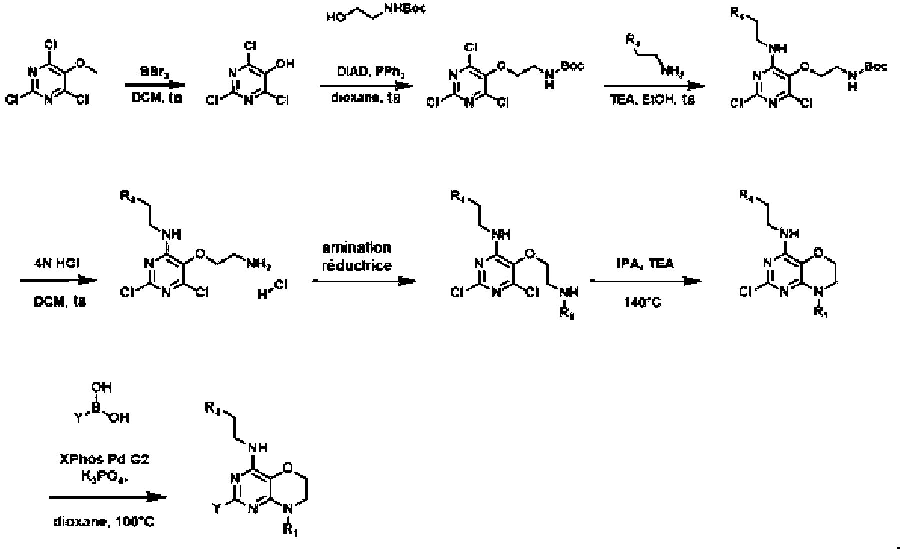 dans laquelle R₄, R₁ et Y sont tels que définis dans la revendication 1, et B représente le bore ;
(c) 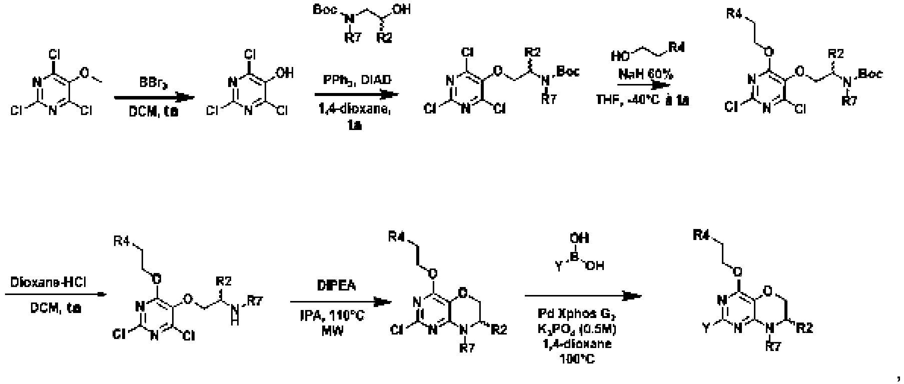 dans laquelle R2, R4, R7 et Y sont tels que définis dans la revendication 1, et B représente le bore ;
(d) 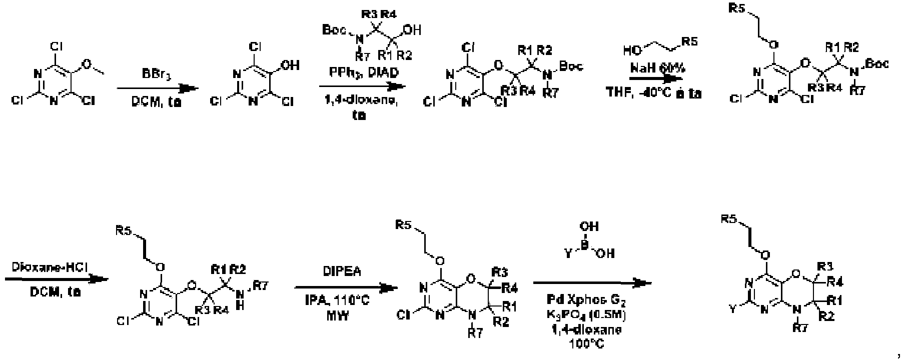 dans laquelle R1, R2, R3, R4, R5, R7 et Y sont tels que définis dans la revendication 1, et B représente le bore ;
(e) 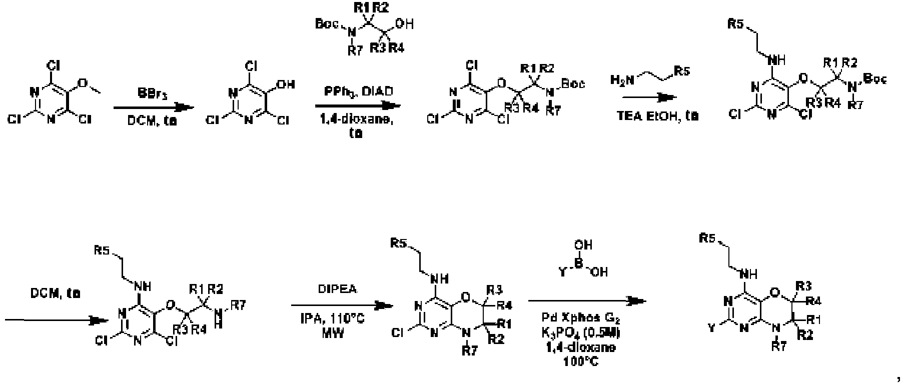 dans laquelle R1, R2, R3, R4, R5, R7 et Y sont tels que définis dans la revendication 1, et B représente le bore
(f) 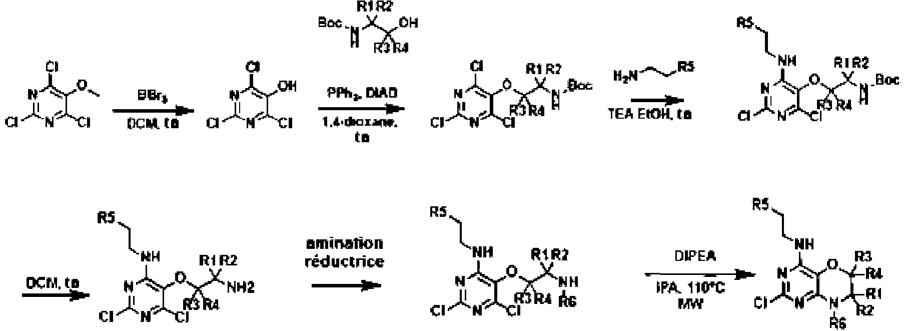 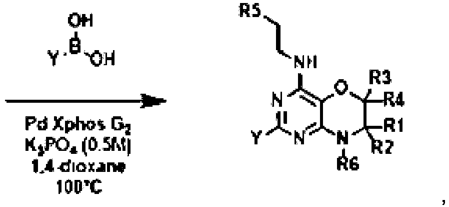 dans laquelle R1, R2, R3, R4, R5, R6 et Y sont tels que définis dans la revendication 1, et B représente le bore ;
(g) 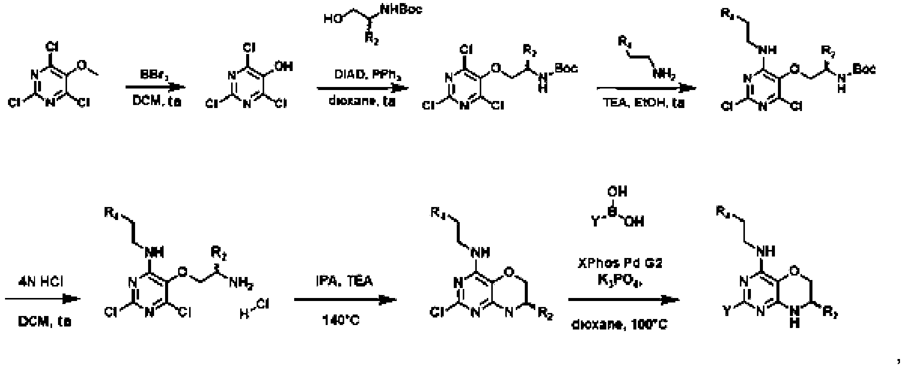 dans laquelle R₂, R₄ et Y sont tels que définis dans la revendication 1, et B représente le bore ;
(h) 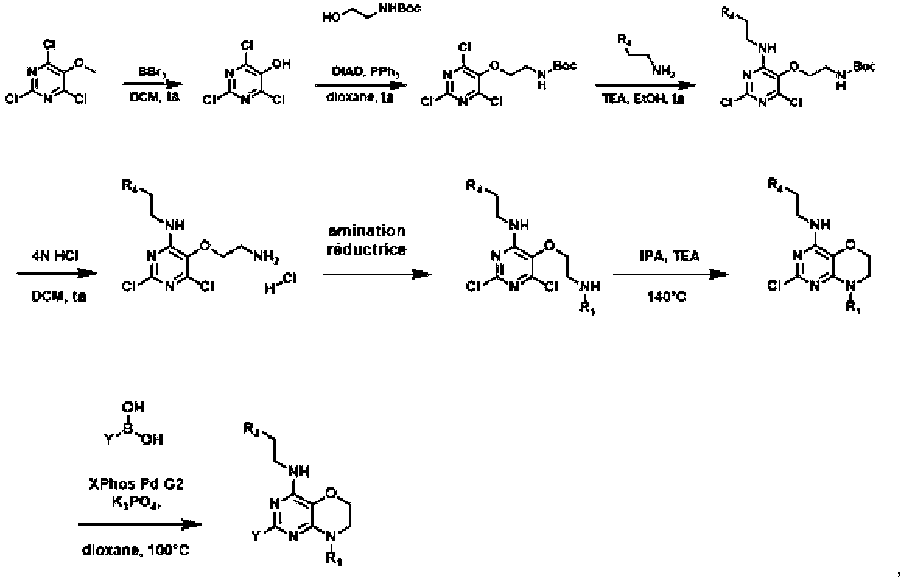 dans laquelle R₁, R₄ et Y sont tels que définis dans la revendication 1, et B représente le bore ;
(i) 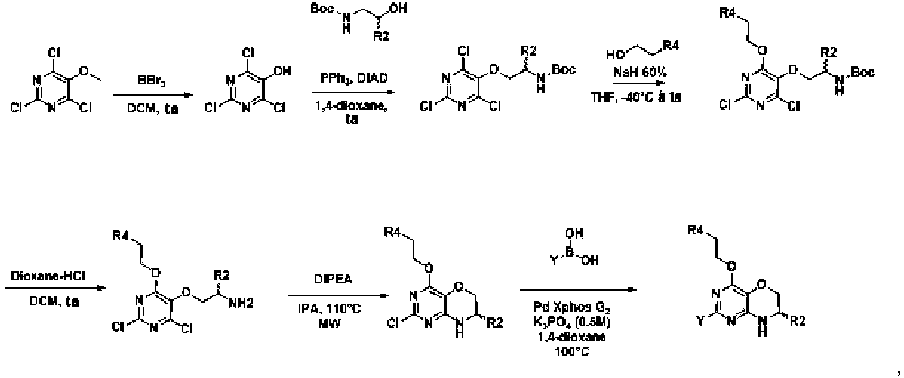 dans laquelle R₂, R₄ et Y sont tels que définis dans la revendication 1, et B représente le bore ;
(j) 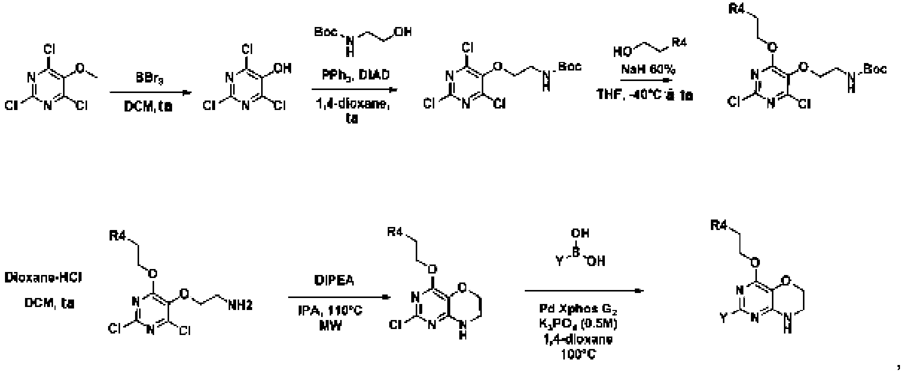 dans laquelle R4 et Y sont tels que définis dans la revendication 1, et B représente le bore ;
(k) 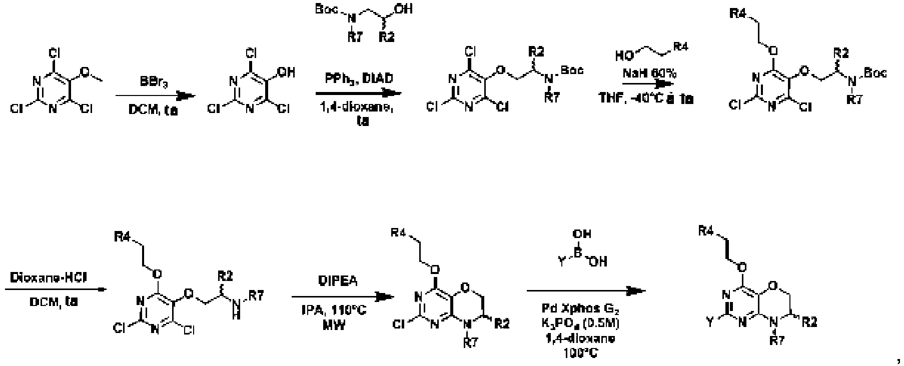 dans laquelle R2, R4, R7 et Y sont tels que définis dans la revendication 1, et B représente le bore ;
(l) 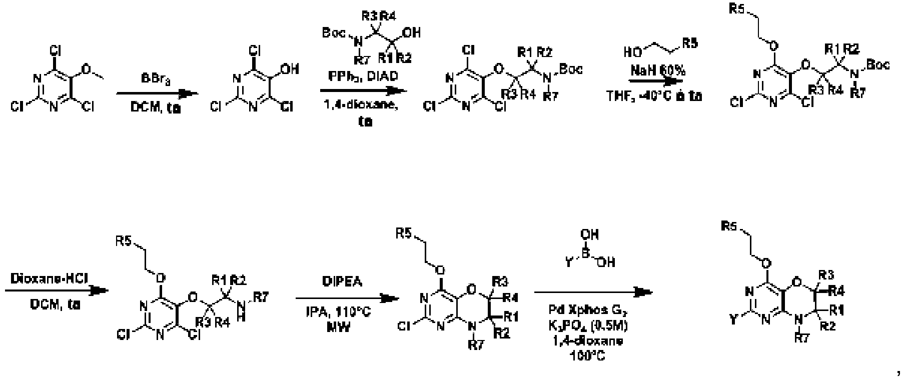 dans laquelle R1, R2, R3, R4, R5, R7 et Y sont tels que définis dans la revendication 1, et B représente le bore;
(m) 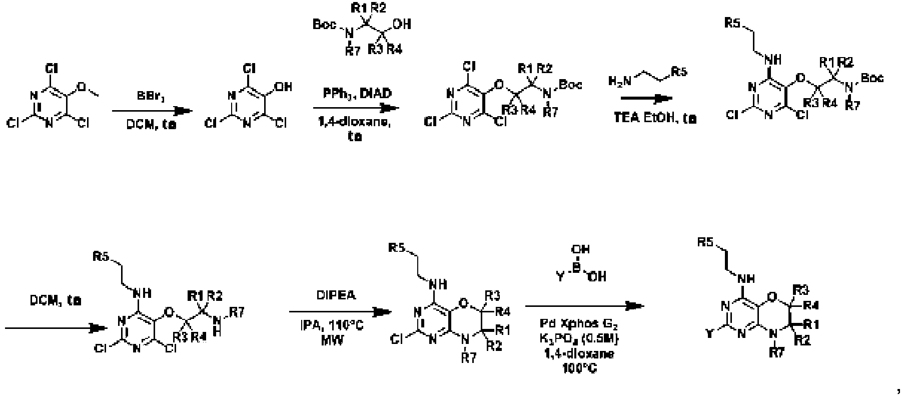 dans laquelle R1, R2, R3, R4, R5, R7 et Y sont tels que définis dans la revendication 1, et B représente le bore ; OU
(n) 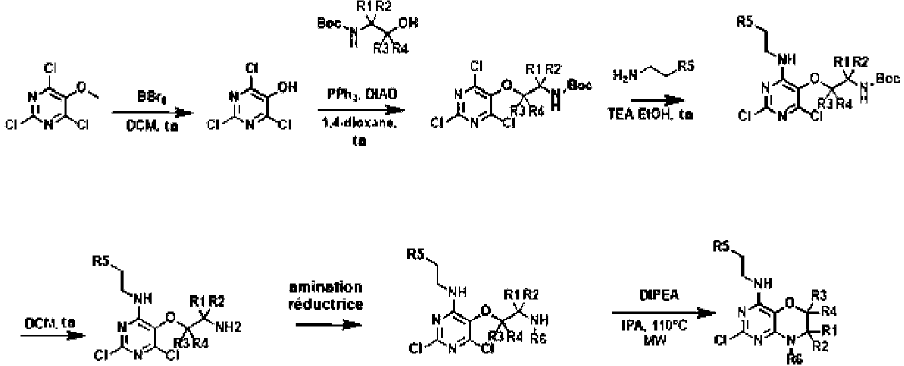 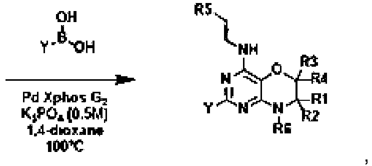 dans laquelle R1, R2, R3, R4, R5, R6 et Y sont tels que définis dans la revendication 1, et B représente le bore.
